# EUROPEAN PATENT SPECIFICATION

(11) **EP 1 828 953 B1**
(45) Date of publication and mention of the grant of the patent: **06.03.2013**
(21) Application number: 05851443.1
(22) Date of filing: 08.11.2005
(51) Int. Cl.: C12N 15/115, C12Q 1/18, C12Q 1/68, G06F 19/12

(54) **Crystalline xpt guanine riboswitch from Bacillus subtilis and structure-based compound identification employing said riboswitch**
Kristalliner Xpt Guanine Riboswitch aus Bacillus subtilis und strukturbasierte Verbindungsidentifizierung mittels besagtem Riboswitch
Riboswitch xpt guanine de Bacillus subtilis cristalline et identification de composés à base de structure utilisant ledit riboswitch

(30) Priority: 08.11.2004 US 625864 P
(43) Date of publication of application: 05.09.2007
(73) Proprietor: Yale University Corporation, New Haven Connecticut 06511 (US); The Regents of the University of Colorado, a body corporate, Denver, CO 80203 (US)
(72) Inventor: BREAKER, Ronald, R., Guilford, Connecticut 06437 (US); LIM, Jinsoo, Hamden, Connecicut 06514 (US); BATEY, Robert, Colorado 80309 (US); PUSKARZ, Izabela, West Hartford, Connecticut 06119 (US); BLOUNT, Kenneth F., New Haven, Connecticut 06512 (US)
(74) Representative: Van Someren, Petronella F. H. M.
(86) International application number: PCT/US2005/040487
(87) International publication number: WO 2006/055351

(56) References cited:
- US-A1- 2004 219 523
- MANDAL M ET AL: "Gene regulation by riboswitches" NATURE REVIEWS MOLECULAR CELL BIOLOGY, NATURE PUBLISHING, GB LNKD- DOI:10.1038/NRM1403, vol. 5, 1 June 2004 (2004-06-01), pages 451-463, XP008121394 ISSN: 1471-0072
- MANDAL M ET AL: "Adenine riboswitches and gene activation by disruption of a transcription terminator" NATURE STRUCTURAL AND MOLECULAR BIOLOGY 200401 US LNKD- DOI:10.1038/NSMB710, vol. 11, no. 1, January 2004 (2004-01), pages 29-35, XP002577594 ISSN: 1545-9993
- BATEY ROBERT T ET AL: "Structure of a natural guanine-responsive riboswitch complexed with the metabolite hypoxanthine." NATURE 18 NOV 2004 LNKD- PUBMED:15549109, vol. 432, no. 7015, 18 November 2004 (2004-11-18), pages 411-415, XP002577595 ISSN: 1476-4687
- SERGANOV A ET AL: "Structural Basis for Discriminative Regulation of Gene Expression by Adenine- and Guanine-Sensing mRNAs" CHEMISTRY AND BIOLOGY, CURRENT BIOLOGY, LONDON, GB LNKD- DOI:10.1016/J.CHEMBIOL.2004.11.018, vol. 11, no. 12, 1 December 2004 (2004-12-01), pages 1729-1741, XP004689697 ISSN: 1074-5521
- DESAI S.K. ET AL.: 'Genetic screens and selections for small molecules based on a synthetic riboswitch that activates protein translation' J. AM. CHEM. SOC. vol. 126, 24 October 2004, pages 13247 - 13254, XP002982412
- JENISON R.D. ET AL.: 'High-Resolution molecular discrimination by RNA' SCIENCE vol. 263, 11 March 1994, pages 1425 - 1429, XP000567866
- MANDAL M. ET AL.: 'Riboswitches control fundamental biochemical pathways in Bacillus subtilis and other bacteria' CELL vol. 113, 30 May 2003, pages 577 - 586, XP003009907
- MORRIS G.M. ET AL: 'Distributed automated docking of flexible lignands to proteins: Parallel applications of Autodock 2.4' JOURNAL OF COMPUTER-AIDED MOLECULAR DESIGN vol. 10, 1996, pages 293 - 304, XP008079017

## Description

### FIELD OF THE INVENTION

The disclosed invention is generally in the field of gene expression and specifically in the area of regulation of gene expression.

### BACKGROUND OF THE INVENTION

Precision genetic control is an essential feature of living systems, as cells must respond to a multitude of biochemical signals and environmental cues by varying genetic expression patterns. Most known mechanisms of genetic control involve the use of protein factors that sense chemical or physical stimuli and then modulate gene expression by selectively interacting with the relevant DNA or messenger RNA sequence. Proteins can adopt complex shapes and carry out a variety of functions that permit living systems to sense accurately their chemical and physical environments. Protein factors that respond to metabolites typically act by binding DNA to modulate transcription initiation (e.g. the lac repressor protein; Matthews, K.S., and Nichols, J.C., 1998, Prog. Nucleic Acids Res. MoL BioL 58, 127-164) or by binding RNA to control either transcription termination (e.g. the PyrR protein; Switzer, R.L., et al., 1999, Prog. Nucleic Acids Res. Mol. Biol. 62, 329-367) or translation (e.g. the TRAP protein; Babitzke, P., and Gollnick, P., 2001, J. Bacteriol. 183, 5795-5802). Protein factors respond to environmental stimuli by various mechanisms such as allosteric modulation or post-translational modification, and are adept at exploiting these mechanisms to serve as highly responsive genetic switches *(e.g.* see Ptashne, M., and Gann, A. (2002). Genes and Signals. Cold Spring Harbor Laboratory Press, Cold Spring Harbor, NY).

In addition to the widespread participation of protein factors in genetic control, it is also known that RNA can take an active role in genetic regulation. Recent studies have begun to reveal the substantial role that small non-coding RNAs play in selectively targeting mRNAs for destruction, which results in down-regulation of gene expression (*e.g.* see Hannon, G.J. 2002, Nature 418, 244-251 and references therein). This process of RNA interference takes advantage of the ability of short RNAs to recognize the intended mRNA target selectively via Watson-Crick base complementation, after which the bound mRNAs are destroyed by the action of proteins. RNAs are ideal agents for molecular recognition in this system because it is far easier to generate new target-specific RNA factors through evolutionary processes than it would be to generate protein factors with novel but highly specific RNA binding sites.

Although proteins fulfill most requirements that biology has for enzyme, receptor and structural functions, RNA also can serve in these capacities. For example, RNA has sufficient structural plasticity to form numerous ribozyme domains (Cech & Golden, Building a catalytic active site using only RNA. In: The RNA World R. F. Gesteland, T. R. Cech, J. F. Atkins, eds., pp.321-350 (1998); Breaker, In vitro selection of catalytic polynucleotides. Chem. Rev. 97, 371-390 (1997)) and receptor domains (Osborne & Ellington, Nucleic acid selection and the challenge of combinatorial chemistry. Chem. Rev. 97, 349-370 (1997); Hermann & Patel, Adaptive recognition by nucleic acid aptamers. Science 287, 820-825 (2000)) that exhibit considerable enzymatic power and precise molecular recognition. Furthermore, these activities can be combined to create allosteric ribozymes (Soukup & Breaker, Engineering precision RNA molecular switches. Proc. Natl. Acad. Sci. USA 96, 3584-3589 (1999); Seetharaman et al., Immobilized riboswitches for the analysis of complex chemical and biological mixtures. Nature Biotechnol. 19, 336-341 (2001)) that are selectively modulated by effector molecules.

These properties of RNA are consistent with speculation (Gold et al., From oligonucleotide shapes to genomic SELEX: novel biological regulatory loops. Proc. Natl. Acad. Sci. USA 94, 59-64 (1997); Gold et al., SELEX and the evolution of genomes. Curr. Opin. Gen. Dev. 7, 848-851 (1997); Nou & Kadner, Adenosylcobalamin inhibits ribosome binding to btuB RNA. Proc. Natl. Acad. Sci. USA 97, 7190-7195 (2000); Gelfand et al., A conserved RNA structure element involved in the regulation of bacterial riboflavin synthesis genes. Trends Gen. 15, 439-442 (1999); Miranda-Rios et al., A conserved RNA structure (thi box) is involved in regulation of thiamin biosynthetic gene expression in bacteria. Proc. Natl. Acad. Sci. USA 98, 9736-9741 (2001); Stormo & Ji, Do mRNAs act as direct sensors of small molecules to control their expression? Proc. Natl. Acad. Sci. USA 98, 9465-9467 (2001)) that certain mRNAs might employ allosteric mechanisms to provide genetic regulatory responses to the presence of specific metabolites. Although a thiamine pyrophosphate (TPP)-dependent sensor/regulatory protein had been proposed to participate in the control of thiamine biosynthetic genes (Webb & Downs, Characterization of thiL, encoding thiamin-monophosphate kinase, in Salmonella typhimurium. J. Biol. Chem. 272, 15702-15707 (1997)), no such protein factor has been shown to exist.

Transcription of the *lysC* gene of *B. subtilis* is repressed by high concentrations of lysine (Kochhar, S., and Paulus, H. 1996, Microbiol. 142:1635-1639; Mäder, U., et al., 2002, J. Bacteriol. 184:4288-4295; Patte, J.C. 1996. Biosynthesis of lysine and threonine. In: Escherichia coli and Salmonella: Cellular and Molecular Biology, F.C. Neidhardt, et al., eds., Vol. 1, pp. 528-541. ASM Press, Washington, DC; Patte, J.-C., et al., 1998, FEMS Microbiol. Lett. 169:165-170), but no protein factor had been identified that served as the genetic regulator (Liao, H.-H., and Hseu, T.-H. 1998, FEMS Microbiol. Lett. 168:31-36). The *lysC* gene encodes aspartokinase II, which catalyzes the first step in the metabolic pathway that converts L-aspartic acid into L-lysine (Belitsky, B.R. 2002. Biosynthesis of amino acids of the glutamate and aspartate families, alanine, and polyamines. In: Bacillus subtilis and its Closest Relatives: from Genes to Cells. A.L. Sonenshein, J.A. Hoch, and R. Losick, eds., ASM Press, Washington, D.C.).

### BRIEF SUMMARY OF THE INVENTION

It has been discovered that certain natural mRNAs serve as metabolite-sensitive genetic switches wherein the RNA directly binds a small organic molecule. This binding process changes the conformation of the mRNA, which causes a change in gene expression by a variety of different mechanisms. Modified versions of these natural "riboswitches" (created by using various nucleic acid engineering strategies) can be employed as designer genetic switches that are controlled by specific effector compounds. Such effector compounds that activate a riboswitch are referred to herein as trigger molecules. The natural switches are targets for antibiotics and other small molecule therapies. In addition, the architecture of riboswitches allows actual pieces of the natural switches to be used to construct new non-immunogenic genetic control elements, for example the aptamer (molecular recognition) domain can be swapped with other non-natural aptamers (or otherwise modified) such that the new recognition domain causes genetic modulation with user-defined effector compounds. The changed switches become part of a therapy regimen-turning on, or off, or regulating protein synthesis. Newly constructed genetic regulation networks can be applied in such areas as living biosensors, metabolic engineering of organisms, and in advanced forms of gene therapy treatments.

Disclosed are isolated and recombinant riboswitches, recombinant constructs containing such riboswitches, heterologous sequences operably linked to such riboswitches, and cells and transgenic organisms harboring such riboswitches, riboswitch recombinant constructs, and riboswitches operably linked to heterologous sequences. The heterologous sequences can be, for example, sequences encoding proteins or peptides of interest, including reporter proteins or peptides. Preferred riboswitches are, or are derived from, naturally occurring riboswitches.

Also disclosed are the crystalline atomic structures of riboswitches. These structures are useful in modeling and assessing the interaction of a riboswitch with a binding ligand. They are also useful in methods of identifying compounds that interact with the riboswitch.

Also disclosed are chimeric riboswitches containing heterologous aptamer domains and expression platform domains. That is, chimeric riboswitches are made up an aptamer domain from one source and an expression platform domain from another source. The heterologous sources can be from, for example, different specific riboswitches or different classes of riboswitches. The heterologous aptamers can also come from non-riboswitch aptamers. The heterologous expression platform domains can also come from non-riboswitch sources.

Also disclosed are compositions and methods for selecting and identifying compounds that can activate, deactivate or block a riboswitch. Activation of a riboswitch refers to the change in state of the riboswitch upon binding of a trigger molecule. A riboswitch can be activated by compounds other than the trigger molecule and in ways other than binding of a trigger molecule. The term trigger molecule is used herein to refer to molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, *in vitro* selection or *in vitro* evolution techniques). Non-natural trigger molecules can be referred to as non-natural trigger molecules.

Deactivation of a riboswitch refers to the change in state of the riboswitch when the trigger molecule is not bound. A riboswitch can be deactivated by binding of compounds other than the trigger molecule and in ways other than removal of the trigger molecule. Blocking of a riboswitch refers to a condition or state of the riboswitch where the presence of the trigger molecule does not activate the riboswitch.

Also disclosed are methods of identifying a compound that interacts with a riboswitch comprising modeling the atomic structure of the riboswitch with a test compound and determining if the test compound interacts with the riboswitch. This can be done by determining the atomic contacts of the riboswitch and test compound. Furthermore, analogs of a compound known to interact with a riboswitch can be generated by analyzing the atomic contacts, then optimizing the atomic structure of the analog to maximize interaction. These methods can be used with a high throughput screen.

Also disclosed are compounds, and compositions containing such compounds, that can activate, deactivate or block a riboswitch. Also disclosed are compositions and methods for activating, deactivating or blocking a riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch. Riboswitches can also be deactivated by, for example, removing trigger molecules from the presence of the riboswitch. A riboswitch can be blocked by, for example, binding of an analog of the trigger molecule that does not activate the riboswitch.

Also disclosed are compositions and methods for altering expression of an RNA molecule, or of a gene encoding an RNA molecule, where the RNA molecule includes a riboswitch, by bringing a compound into contact with the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch, reduce or prevent expression of the RNA molecule or promote or increase expression of the RNA molecule.

Also disclosed are compositions and methods for regulating expression of an RNA molecule, or of a gene encoding an RNA molecule, by operably linking a riboswitch to the RNA molecule. A riboswitch can be operably linked to an RNA molecule in any suitable manner, including, for example, by physically joining the riboswitch to the RNA molecule or by engineering nucleic acid encoding the RNA molecule to include and encode the riboswitch such that the RNA produced from the engineered nucleic acid has the riboswitch operably linked to the RNA molecule. Subjecting a riboswitch operably linked to an RNA molecule of interest to conditions that activate, deactivate or block the riboswitch can be used to alter expression of the RNA.

Also disclosed are compositions and methods for regulating expression of a naturally occurring gene or RNA that contains a riboswitch by activating, deactivating or blocking the riboswitch. If the gene is essential for survival of a cell or organism that harbors it, activating, deactivating or blocking the riboswitch can result in death, stasis or debilitation of the cell or organism. For example, activating a naturally occurring riboswitch in a naturally occurring gene that is essential to survival of a microorganism can result in death of the microorganism (if activation of the riboswitch turns off or represses expression). This is one basis for the use of the disclosed compounds and methods for antimicrobial and antibiotic effects.

Also disclosed are compositions and methods for regulating expression of an isolated, engineered or recombinant gene or RNA that contains a riboswitch by activating, deactivating or blocking the riboswitch. The gene or RNA can be engineered or can be recombinant in any manner. For example, the riboswitch and coding region of the RNA can be heterologous, the riboswitch can be recombinant or chimeric, or both. If the gene encodes a desired expression product, activating or deactivating the riboswitch can be used to induce expression of the gene and thus result in production of the expression product. If the gene encodes an inducer or repressor of gene expression or of another cellular process, activation, deactivation or blocking of the riboswitch can result in induction, repression, or de-repression of other, regulated genes or cellular processes. Many such secondary regulatory effects are known and can be adapted for use with riboswitches. An advantage of riboswitches as the primary control for such regulation is that riboswitch trigger molecules can be small, non-antigenic molecules.

Also disclosed are compositions and methods for altering the regulation of a riboswitch by operably linking an aptamer domain to the expression platform domain of the riboswitch (which is a chimeric riboswitch). The aptamer domain can then mediate regulation of the riboswitch through the action of, for example, a trigger molecule for the aptamer domain. Aptamer domains can be operably linked to expression platform domains of riboswitches in any suitable manner, including, for example, by replacing the normal or natural aptamer domain of the riboswitch with the new aptamer domain. Generally, any compound or condition that can activate, deactivate or block the riboswitch from which the aptamer domain is derived can be used to activate, deactivate or block the chimeric riboswitch.

Also disclosed are compositions and methods for inactivating a riboswitch by covalently altering the riboswitch (by, for example, crosslinking parts of the riboswitch or coupling a compound to the riboswitch). Inactivation of a riboswitch in this manner can result from, for example, an alteration that prevents the trigger molecule for the riboswitch from binding, that prevents the change in state of the riboswitch upon binding of the trigger molecule, or that prevents the expression platform domain of the riboswitch from affecting expression upon binding of the trigger molecule.

Also disclosed are methods of identifying compounds that activate, deactivate or block a riboswitch. For examples, compounds that activate a riboswitch can be identified by bringing into contact a test compound and a riboswitch and assessing activation of the riboswitch. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways.

Identification of compounds that block a riboswitch can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch. Also disclosed are methods of detecting compounds using biosensor riboswitches. The method can include bringing into contact a test sample and a biosensor riboswitch and assessing the activation of the biosensor riboswitch. Activation of the biosensor riboswitch indicates the presence of the trigger molecule for the biosensor riboswitch in the test sample.

Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch.

Also disclosed are methods for selecting, designing or deriving new riboswitches and/or new aptamers that recognize new trigger molecules. Such methods can involve production of a set of aptamer variants in a riboswitch, assessing the activation of the variant riboswitches in the presence of a compound of interest, selecting variant riboswitches that were activated (or, for example, the riboswitches that were the most highly or the most selectively activated), and repeating these steps until a variant riboswitch of a desired activity, specificity, combination of activity and specificity, or other combination of properties results. Also disclosed are riboswitches and aptamer domains produced by these methods.

The disclosed riboswitches, including the derivatives and recombinant forms thereof, generally can be from any source, including naturally occurring riboswitches and riboswitches designed *de novo.* Any such riboswitches can be used in or with the disclosed methods. However, different types of riboswitches can be defined and some such sub-types can be useful in or with particular methods (generally as described elsewhere herein). Types of riboswitches include, for example, naturally occurring riboswitches, derivatives and modified forms of naturally occurring riboswitches, chimeric riboswitches, and recombinant riboswitches. A naturally occurring riboswitch is a riboswitch having the sequence of a riboswitch as found in nature. Such a naturally occurring riboswitch can be an isolated or recombinant form of the naturally occurring riboswitch as it occurs in nature. That is, the riboswitch has the same primary structure but has been isolated or engineered in a new genetic or nucleic acid context. Chimeric riboswitches can be made up of, for example, part of a riboswitch of any or of a particular class or type of riboswitch and part of a different riboswitch of the same or of any different class or type of riboswitch; part of a riboswitch of any or of a particular class or type of riboswitch and any non-riboswitch sequence or component. Recombinant riboswitches are riboswitches that have been isolated or engineered in a new genetic or nucleic acid context.

Different classes of riboswitches refer to riboswitches that have the same or similar trigger molecules or riboswitches that have the same or similar overall structure (predicted, determined, or a combination). Riboswitches of the same class generally, but need not, have both the same or similar trigger molecules and the same or similar overall structure. Riboswitch classes include glycine-responsive riboswitches, guanine-responsive riboswitches, adenine-responsive riboswitches, lysine-responsive riboswitches, thiamine pyrophosphate-responsive riboswitch, adenosylcobalamin-responsive riboswitches, flavin mononucleotide-responsive riboswitches, and a S-adenosylmethionine-responsive riboswitches.

Additional advantages of the disclosed method and compositions will be set forth in part in the description which follows, and in part will be understood from the description, or can be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.

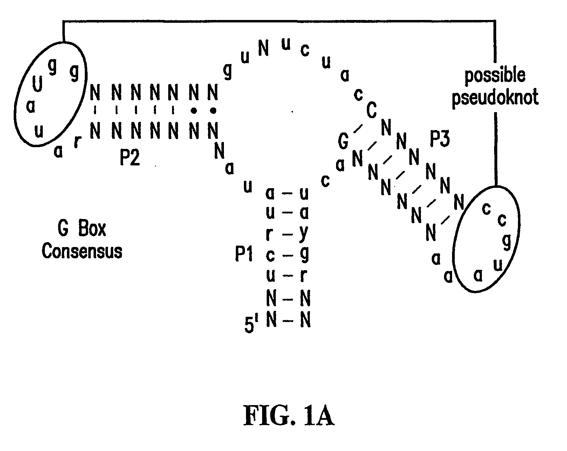

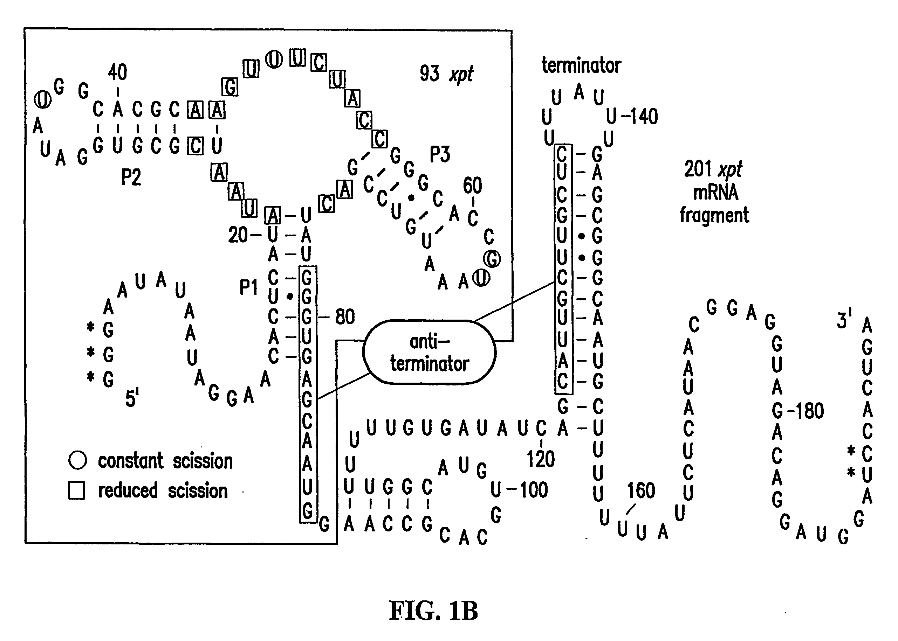

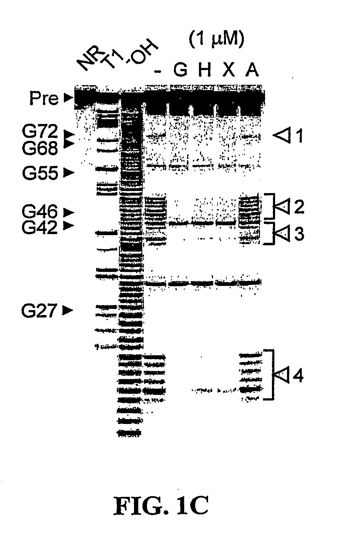

Figures 1A, 1B and 1C show the G box RNA of the *xpt-pbuX* mRNA in *B. subtilis* responds allosterically to guanine. Figure 1A shows the consensus sequence and secondary model for the G box RNA domain that resides in the 5' UTR of genes that are largely involved in purine metabolism (SEQ ID NO: 1). Phylogenetic analysis is consistent with the formation of a three-stem (P1 through P3) junction. Nucleotides depicted shown as lower case letters and capitals are present in greater than 90% and 80% of the representatives examined, respectively. Encircled nucleotides exhibit base complementation, which might indicate the formation of a pseudoknot. Figure 1B shows sequence and ligand-induced structural alterations of the 5'-UTR of the *xpt-pbuX* transcriptional unit (SEQ ID NO:2). The putative anti-terminator interaction is represented by the boxes. Nucleotides that undergo structural alteration as determined by in-line probing (from C) are identified with squares. The 93 *xpt* fragment (boxed) of the 201 *xpt* RNA retains guanine-binding function. Asterisks denote alterations to the RNA sequence that facilitate *in vitro* transcription (5' terminus) or that generate a restriction site (3' terminus). Nucleotide numbers begin at the first nucleotide of the natural transcription start site. The translation start codon begins at position 186. Figure 1C shows guanine and related purines selectively induce structural modulation of the 93 *xpt* mRNA fragment. Precursor RNAs (Pre; 5' ³²P-labeled) were subjected to in-line probing by incubation for 40 hr in the absence (-) or presence of guanine, hypoxanthine, xanthine and adenine as indicated by G, H, X and A, respectively. Lanes designated NR, T1 and ⁻OH contain RNA that was not reacted, subjected to partial digestion with RNase T1 (G-specific cleavage), or subjected to partial alkaline digestion, respectively. Selected bands corresponding to G-specific cleavage are identified. Regions 1 through 4 identify major sites of ligand-induced modulation of spontaneous RNA cleavage.

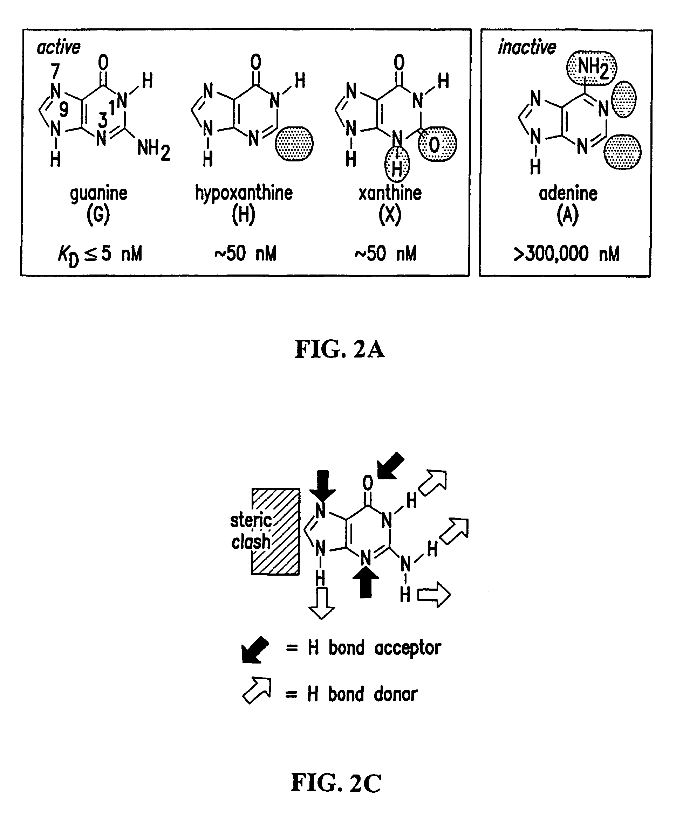

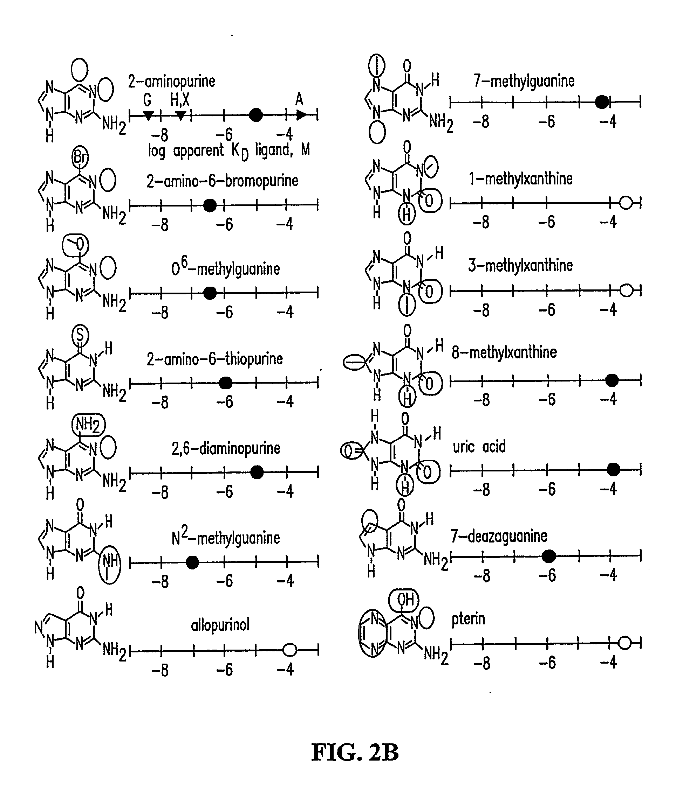

Figures 2A, 2B and 2C show a molecular discrimination by the guanine-binding aptamer of the *xpt-pbuX* mRNA. Figure 2A shows the chemical structures and apparent *K*_{D} values for guanine, hypoxanthine and xanthine (active natural regulators of *xpt-pbuX* genetic expression in *B. subtilis)* versus that of adenine (inactive). Differences in chemical structure relative to guanine are encircled. *K*_{D} values were established as shown in Figure 2 with the 201 *xpt* RNA. Numbers on guanine represent the positions of the ring nitrogen atoms. Figure 2B shows chemical structures and *K*_{D} values for various analogs of guanine reveal that all alterations of this purine cause a loss of binding affinity. Open circles identify *K*_{D} values that most likely are significantly higher than indicated, as concentrations of analog above 500 µM were not examined in this analysis. The apparent *K*_{D} values of G, H, X and A as indicated are plotted as triangles for comparison. Figure 2C shows a schematic representation of the molecular recognition features of the guanine aptamer in 201 *xpt.* Hydrogen bond formation at position 9 of guanine is expected because guanosine (*K*_{D} > 100 µM) and inosine (*K*_{D} > 100 µM), which are 9-ribosyl derivatives of guanine and hypoxanthine, respectively, do not exhibit measurable binding.

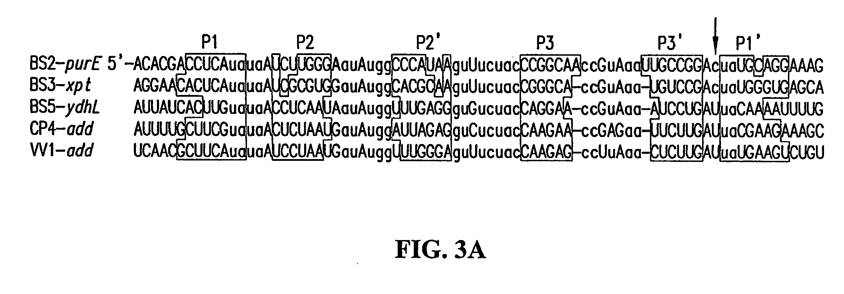

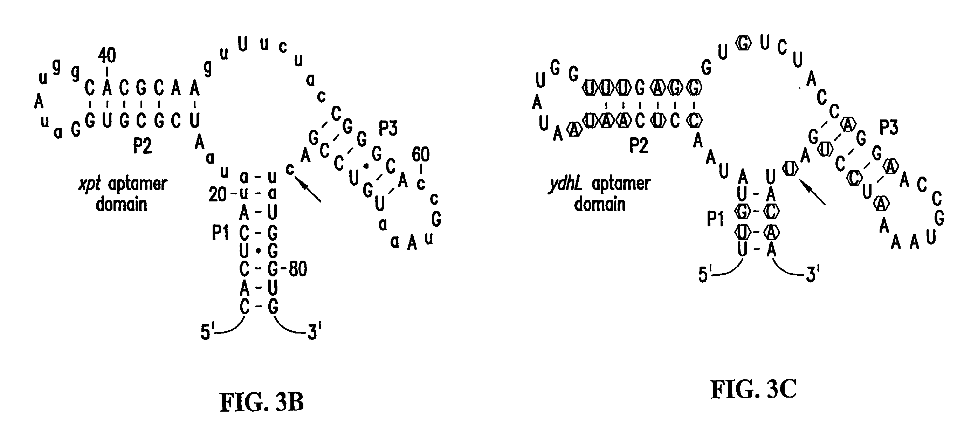

Figures 3A, 3B and 3C show guanine- and adenine-specific riboswitches. Figure 3A shows sequence and structural features of the two guanine-specific *(purE* and *xpt)* and three adenine-specific aptamer domains that are examined in this study BS2-purE, BS3-xpt, BS5-ydhL, CP4-add, VV1-add, which are represented by SEQ ID NOS:3-7, respectively. P1 through P3 identify the three base-paired stems comprising the secondary structure of the aptamer domain. Lowercase nucleotides identify positions whose base identity is conserved in greater than 90% of representatives in the phylogeny. The arrow identifies a nucleotide within the conserved core of the aptamer that is a determinant of ligand specificity. BS, CP and VV designate *B. subtilis, Clostridium perfringens* and *Vibrio vulnificus,* respectively. Figure 3B shows sequence and secondary structure of the *xpt* and *ydhL* aptamers (SEQ ID NO:8). Encircled nucleotides identify positions within the *ydhL* aptamer that differ from those in the *xpt* aptamer. The sequence disclosed in Figure 3C is SEQ ID NO:9. Nucleotides in xpt are numbered as described in Example 6 of U.S. Application Publication No. 2005-0053951. Other notations are as described in A.

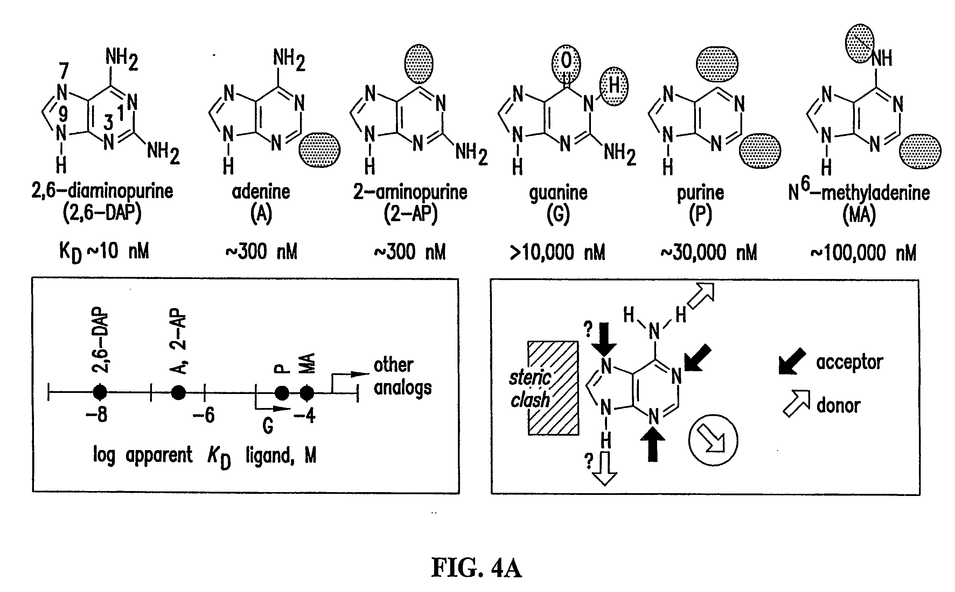

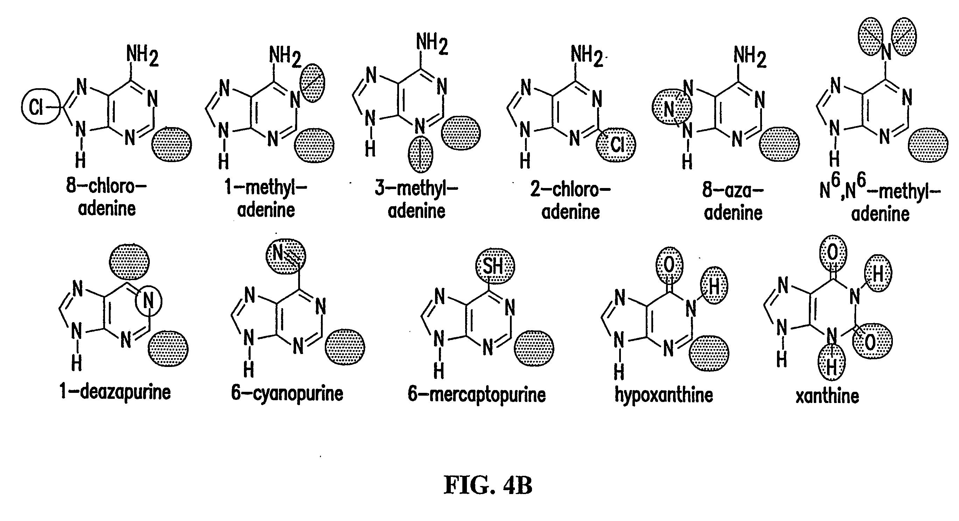

Figures 4A and 4B show the specificity of molecular recognition by the adenine aptamer from *ydhL.* Figure 4a Top: Chemical structures of adenine, guanine and other purine analogs that exhibit measurable binding to the 80 *ydhL* RNA. Chemical changes relative to 2,6-DAP, which is the tightest-binding compound, are encircled. Bottom left: Plot of the apparent *K*_{D} values for various purines. Bottom right: Model for the chemical features on adenine that serve as molecular recognition contacts for *ydhL.* Note that the importance of N7 and N9 has not been determined. Encircled arrow indicated that a contact could exist if a hydrogen bond donor is appended to C2. Figure 4b shows chemical structures of various purines that are not bound by the 80 *ydhL* RNA (*K*_{D} values poorer than 300 µM).

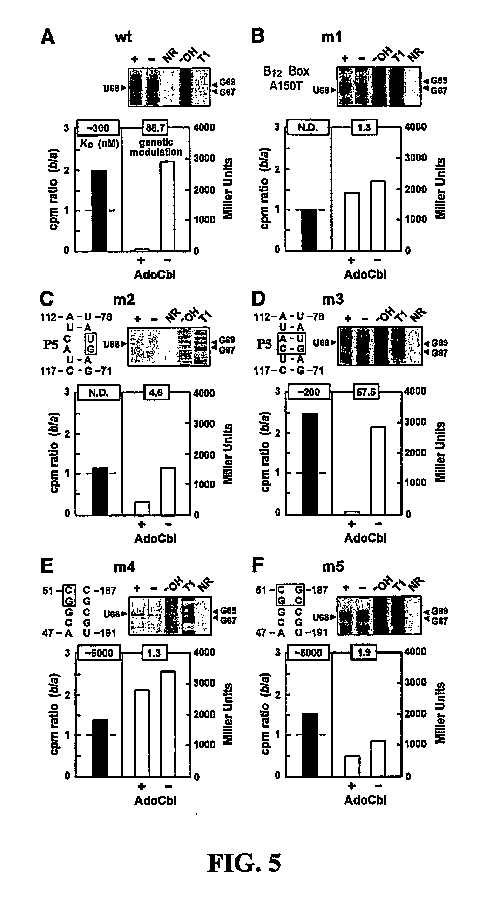

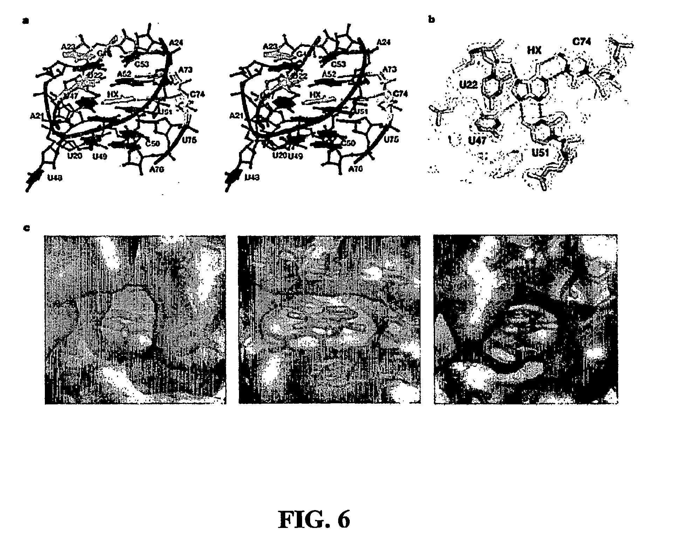

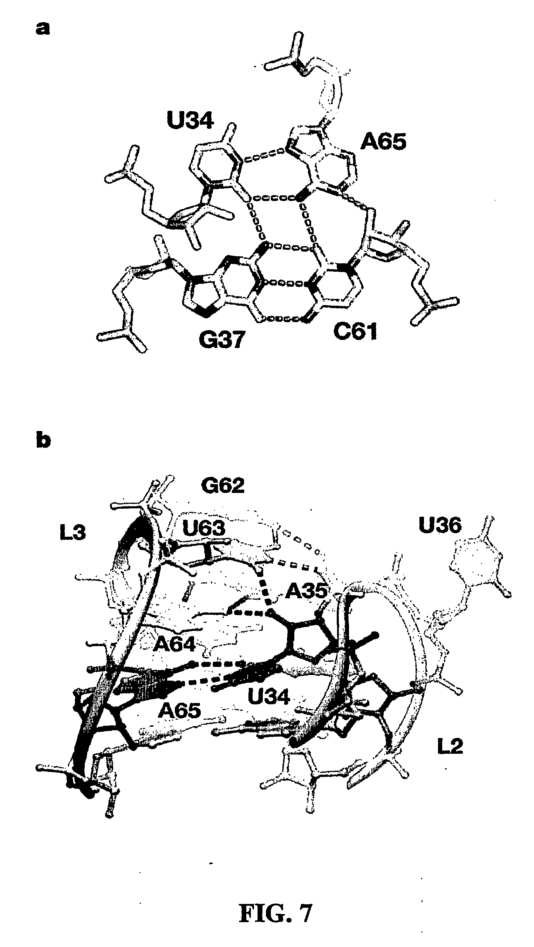

Figures 5A, 5B, 5C, and 5D show secondary and tertiary structures of the guanine riboswitch-hypoxanthine complex. Figure 5A shows left, secondary structure of the *xpt-pbuX* guanine-binding domain of the guanine riboswitch of *B. subtilis* (SEQ ID NO:213). Nucleotides conserved in more than 90% of known guanine riboswitches are shown in red; the numbering is consistent with that of the full-length mRNA. Colored boxes correspond to structural features shown in Figures 6 and 7. Right, tertiary architecture of the hypoxanthine-bound form. Key tertiary interactions between the loops are shown as thick broken lines; a water-mediated triple is indicated by the circled 'w'. Figure 5B shows gene repression by the guanine riboswitch in the 5' untranslated region of mRNA (SEQ ID NO:214). Initial transcription generates a binding domain that is primed to bind guanine (G) rapidly if it is at a sufficiently high concentration. Hypoxanthine (HX, top right) stabilizes the guanine-binding domain and particularly the P1 helix, forcing the mRNA to form a terminator element that halts transcription. In the absence of ligand (bottom right), an antiterminator forms, facilitating continued transcription. Figure 5C shows ribbon representation of the three-dimensional structure of the RNA-hypoxanthine complex. The hypoxanthine ligand is shown in red, with its surface represented by dots. Figure 5D shows the top view of the complex, emphasizing the close packing of the P2 and P3 helices.
Figures 6A, 6B, and 6C show recognition of hypoxanthine (HX) by the guanine-binding domain. Figure 6A: Stereo view of the hypoxanthine-binding pocket in the three-way junction. Figure 6B: Hydrogen-bonding interactions (grey broken lines) between hypoxanthine and the RNA. The final model (shown in stick representation) is superimposed on a simulated annealing 2Fo-Fc omit map (orange cage), in which the atoms shown were excluded from the map calculation. Figure 6C: Molecular surface representation of the binding pocket of the guanine riboswitch bound to hypoxanthine (left), compared with the theophylline-binding aptamer bound to theophylline (centre) and the *E. coli* purR repressor bound to hypoxanthine (right).
Figures 7A, 7B show stabilization of the tertiary architecture. Figure 7A: One of two base quartets that form the core of the loop-loop contact. The carbon atoms are colored as in Figure 5. Figure 7B: Side view of the loop-loop interaction, emphasizing the arrangement of base pairs and quartets. The bases of the quartet shown in A are colored blue, with the hydrogen bonding between A65 and U34 shown for orientation; the bases of the other quartet are colored green. The A35A64 pair is shown in yellow, with hydrogen bonds emphasizing its interactions with the 2'-hydroxyl group of U34. The capping G62U63 pair is shown in red.

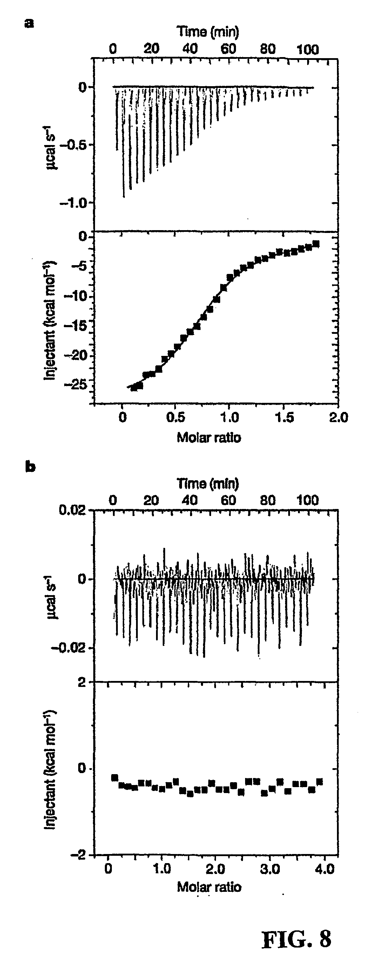

Figures 8A and 8B show an estimation of the affinity of the riboswitch for hypoxanthine. Shown are the isothermal titration calorimetry curves for the wild-type guanine-binding domain (Figure 8A) and for the guanine-binding domain lacking the tertiary interaction (Figure 8B) with hypoxanthine at 30 °C in buffer containing 10 mM K⁺-HEPES (pH 7.5), 100 mM KCl and 5 mM MgCl₂.

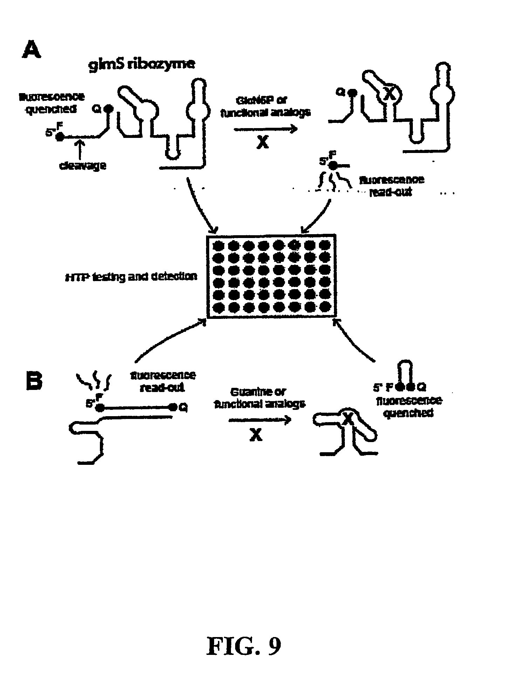

Figures 9A and 9B show schematic representations of two types of riboswitch binding assays that can be used with high throughput screens. Figure 9A: a ribozyme-based assay can be used that exploits inherent action of a self-cleaving ribozyme. X represents the compound being tested, GlcN6P is glucosamine-6-phosphate. F and Q represent fluorophore and quencher moieties, respectfully (e.g., TAMRA and CY3). Figure 9B: A molecular beacon assay can be used for non-ribozyme riboswitches such as the guanine-binding RNA. Notations are described in A.

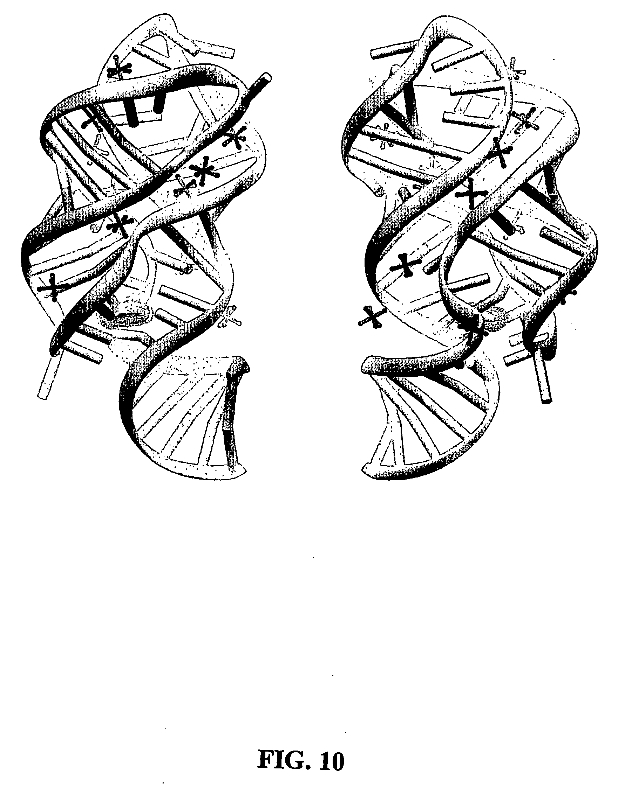

Figure 10 shows cobalt hexammine ions bound to the guanine riboswitch. The RNA (grey) on the left is shown in the same perspective as in Figure 5B, with bound hypoxanthine in red and Co(NH₃)₆³⁺ shown in green and blue. The RNA on the right is rotated 180° with respect to the left view.

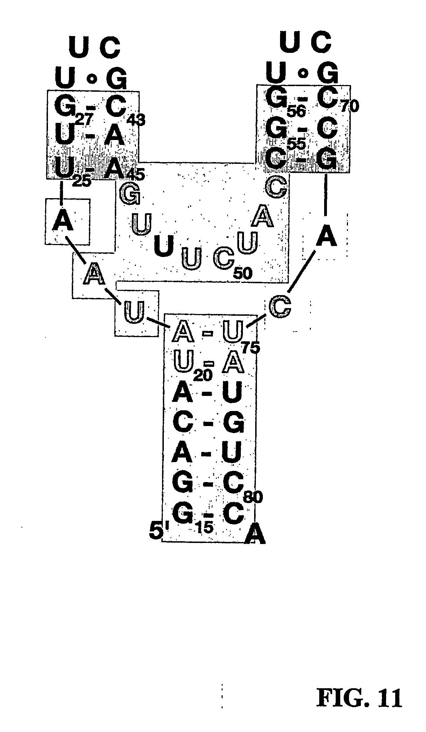

Figure 11 shows secondary structure of RNA GR-minimal (SEQ ID NO:215).
   This RNA has been designed to test the effect of the tertiary interaction formed by the loops L2 and L3 upon ligand binding. In this construct the L2 and L3 loops have been ablated along with part of P2 and P3, and replaced by extremely stable UUCG tetraloops.

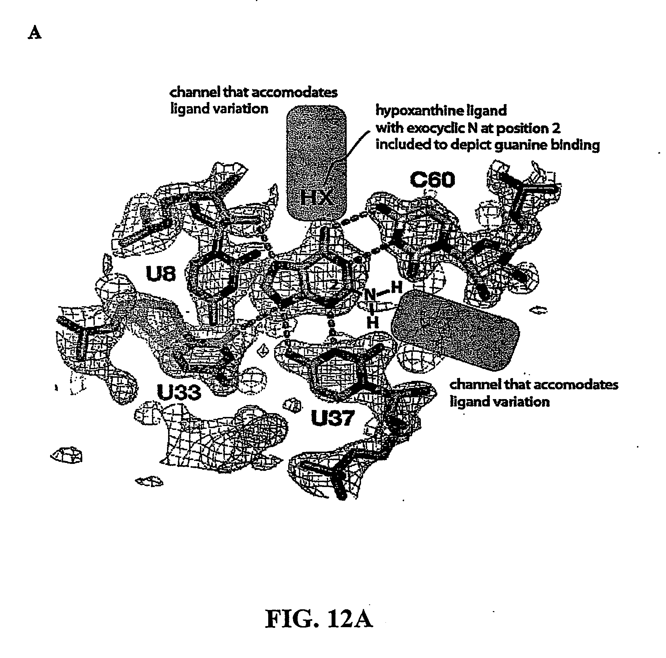

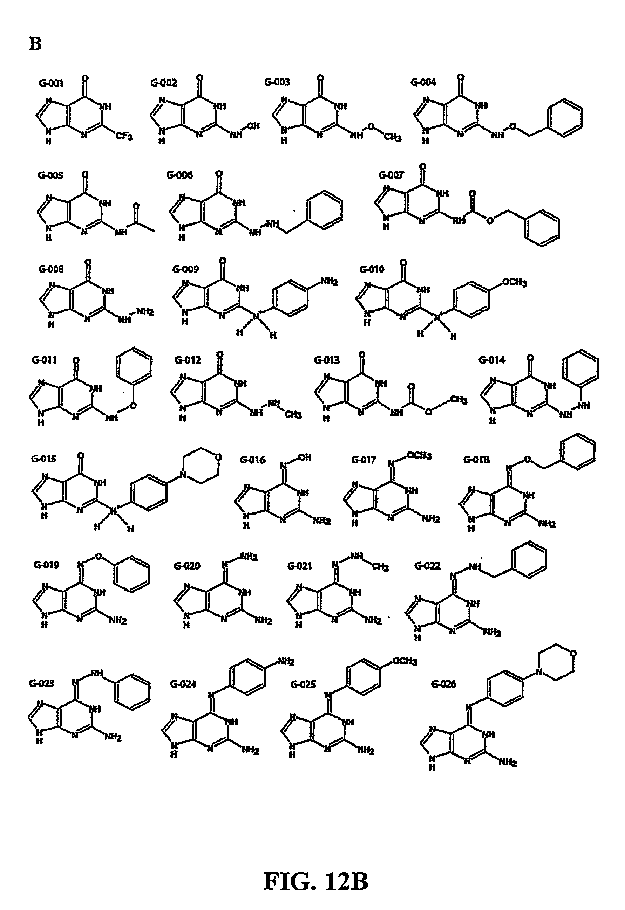

Figures 12A and 12B show structure based design of anti-riboswitch compounds.

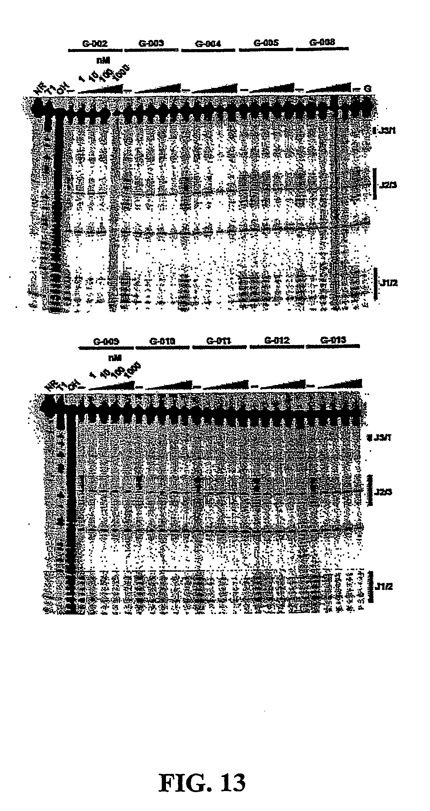

   Figure 12A: Atomic-resolution model of the guanine riboswitch bound to the guanine analog hypoxanthine (HX). Guanine binding can include two added contacts made between the exocyclic amine position 2 of the purine ring and the oxygen atoms of C60 and U37. Blue shading identifies the channels that are present, which can allow modification of the guanine ring. Along these channels, and in the vicinity of their termini, are opportunities for new contacts to be made between the aptamer and specific guanine derivatives. Figure 12B: Representatives of guanine analogs (total of 26; named G-001 though G-26) were synthesized to exploit the channels in the guanine riboswitch structure. Most compounds in this collection bind to the riboswitch with dissociation constants of lower than 1 nanomolar (see Figure 13). These compounds can be further modified to acquire new contacts between the compounds and functional groups near the channels.
Figure 13 shows representative polyacrylamde gel electrophoresis analysis of in-line probing studies that reveal allosteric modulation of the guanine aptamer by various analogs. NR, T1 and OH identify no reaction, nuclease T1 partial digestion, and alkaline partial digestion respectively. In-line probing assays were conducted for ∼1 day, and a dampening of spontaneous RNA cleavage product bands in the J1/2, J2/3 and J3/1 regions are indicative of ligand binding. This dampening occurs even when 1 nM of compound is added, indicating that the dissociation constant is equal to or better than this value.

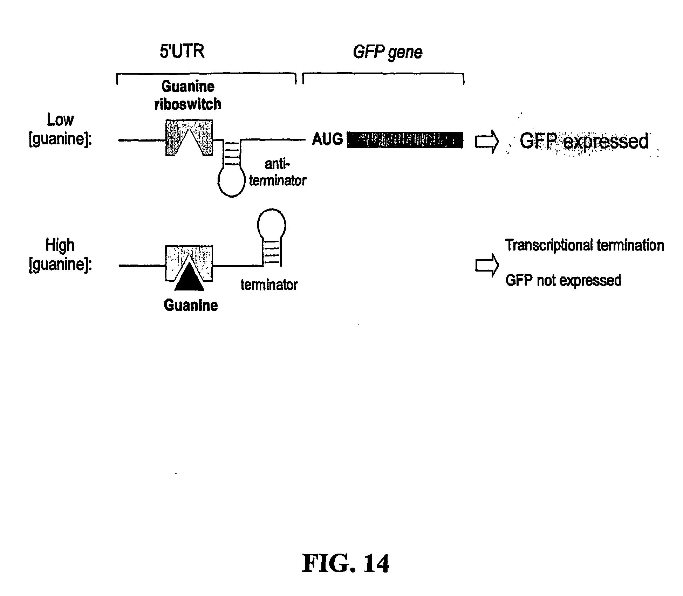

Figure 14 shows a GFP reporter assay for guanine riboswitch function *in vivo.* In the absence of guanine, a full-length mRNA is transcribed and GFP is expressed. At high concentrations of guanine, or an active guanine analog, a transcriptional terminator is formed, repressing the expression of GFP. A similar system has been created wherein the *β*-galactosidase gene is under riboswitch control.

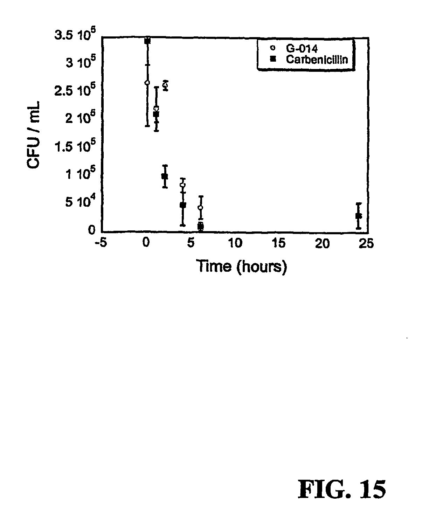

Figure 15 shows time-kill assay for G-014. G-014 reduces the number of viable *B. subtilis* cells over time at a rate equal to carbenicillin.

### DETAILED DESCRIPTION OF THE INVENTION

The disclosed methods and compositions can be understood more readily by reference to the following detailed description of particular embodiments and the Examples included therein and to the Figures and their previous and following description.

Certain natural mRNAs serve as metabolite-sensitive genetic switches wherein the RNA directly binds a small organic molecule. This binding process changes the conformation of the mRNA, which causes a change in gene expression by a variety of different mechanisms. Modified versions of these natural "riboswitches" (created by using various nucleic acid engineering strategies) can be employed as designer genetic switches that are controlled by specific effector compounds (referred to herein as trigger molecules). The natural switches are targets for antibiotics and other small molecule therapies. In addition, the architecture of riboswitches allows actual pieces of the natural switches to be used to construct new non-immunogenic genetic control elements, for example the aptamer (molecular recognition) domain can be swapped with other non-natural aptamers (or otherwise modified) such that the new recognition domain causes genetic modulation with user-defined effector compounds. The changed switches become part of a therapy regimen - turning on, or off, or regulating protein synthesis. Newly constructed genetic regulation networks can be applied in such areas as living biosensors, metabolic engineering of organisms, and in advanced forms of gene therapy treatments.

Messenger RNAs are typically thought of as passive carriers of genetic information that are acted upon by protein- or small RNA-regulatory factors and by ribosomes during the process of translation. It was discovered that certain mRNAs carry natural aptamer domains and that binding of specific metabolites directly to these RNA domains leads to modulation of gene expression. Natural riboswitches exhibit two surprising functions that are not typically associated with natural RNAs. First, the mRNA element can adopt distinct structural states wherein one structure serves as a precise binding pocket for its target metabolite. Second, the metabolite-induced allosteric interconversion between structural states causes a change in the level of gene expression by one of several distinct mechanisms. Riboswitches typically can be dissected into two separate domains: one that selectively binds the target (aptamer domain) and another that influences genetic control (expression platform). It is the dynamic interplay between these two domains that results in metabolite-dependent allosteric control of gene expression.

Distinct classes of riboswitches have been identified and are shown to selectively recognize activating compounds (referred to herein as trigger molecules). For example, coenzyme B₁₂, glycine, thiamine pyrophosphate (TPP), and flavin mononucleotide (FMN) activate riboswitches present in genes encoding key enzymes in metabolic or transport pathways of these compounds. The aptamer domain of each riboswitch class conforms to a highly conserved consensus sequence and structure. Thus, sequence homology searches can be used to identify related riboswitch domains. Riboswitch domains have been discovered in various organisms from bacteria, archaea, and eukarya.

One class of riboswitches that recognizes guanine and discriminates against most other purine analogs has been discovered. Representative RNAs that carry the consensus sequence and structural features of guanine riboswitches are located in the 5'-untranslated region (UTR) of numerous genes of prokaryotes, where they control expression of proteins involved in purine salvage and biosynthesis. Three representatives of this phylogenetic collection bind adenine with values for apparent dissociation constant (apparent *K*_{D}) that are several orders of magnitude better than for guanine. The preference for adenine is due to a single nucleotide substitution in the core of the riboswitch, wherein each representative most likely recognizes its corresponding ligand by forming a Watson/Crick base pair. In addition, the adenine-specific riboswitch associated with the *ydhL* gene of *Bacillus subtilis* functions as a genetic 'ON' switch, wherein adenine binding causes a structural rearrangement that precludes formation of an intrinsic transcription terminator stem. Guanine-sensing riboswitches are a class of RNA genetic control elements that modulate gene expression in response to changing concentrations of this compound.

The 5'-untranslated sequence of the *Escherichia coli btuB* mRNA assumes a more proactive role in metabolic monitoring and genetic control. The mRNA serves as a metabolite-sensing genetic switch by selectively binding coenzyme B₁₂ without the need for proteins. This binding event establishes a distinct RNA structure that is likely to be responsible for inhibition of ribosome binding and consequent reduction in synthesis of the cobalamin transport protein BtuB. This discovery, along with related observations described herein, supports the hypothesis that metabolic monitoring through RNA-metabolite interactions is a widespread mechanism of genetic control.

RNA structure probing data indicate that the thiamine pyrophosphate (TPP) riboswitch operates as an allosteric sensor of its target compound, wherein binding of TPP by the aptamer domain stabilizes a conformational state within the aptamer and within the neighboring expression platform that precludes translation. The diversity of expression platforms appears to be expansive. The *thiM* RNA uses a Shine-Dalgarno (SD)-blocking mechanism to control translation. In contrast, the *thic* RNA controls gene expression both at transcription and translation, and therefore might make use of a somewhat more complex expression platform that converts the TPP binding event into a transcription termination event and into inhibition of translation of completed mRNAs.

Numerous other riboswitches are known that can be used together or as part of a chimeric riboswitch along with glycine-sensing riboswitches and their components. Examples of such riboswitches and their use are described in U.S. Application Publication No. 2005-0053951.

### A. General Organization of Riboswitch RNAs

Bacterial riboswitch RNAs are genetic control elements that are located primarily within the 5'-untranslated region (5'-UTR) of the main coding region of a particular mRNA. Structural probing studies (discussed further below) reveal that riboswitch elements are generally composed of two domains: a natural aptamer (T. Hermann, D. J. Patel, Science 2000, 287, 820; L. Gold, et al., Annual Review of Biochemistry 1995, 64, 763) that serves as the ligand-binding domain, and an 'expression platform' that interfaces with RNA elements that are involved in gene expression (e.g. Shine-Dalgarno (SD) elements; transcription terminator stems). These conclusions are drawn from the observation that aptamer domains synthesized *in vitro* bind the appropriate ligand in the absence of the expression platform (see Examples 2, 3 and 6 of U.S. Application Publication No. 2005-0053951). Moreover, structural probing investigations suggest that the aptamer domain of most riboswitches adopts a particular secondary- and tertiary-structure fold when examined independently, that is essentially identical to the aptamer structure when examined in the context of the entire 5' leader RNA. This implies that, in many cases, the aptamer domain is a modular unit that folds independently of the expression platform (see Examples 2, 3 and 6 of U.S. Application Publication No. 2005-0053951).

Ultimately, the ligand-bound or unbound status of the aptamer domain is interpreted through the expression platform, which is responsible for exerting an influence upon gene expression. The view of a riboswitch as a modular element is further supported by the fact that aptamer domains are highly conserved amongst various organisms (and even between kingdoms as is observed for the TPP riboswitch), (N. Sudarsan, et al., RNA 2003, 9, 644) whereas the expression platform varies in sequence, structure, and in the mechanism by which expression of the appended open reading frame is controlled. For example, ligand binding to the TPP riboswitch of the *tenA* mRNA of *B. subtilis* causes transcription termination (A. S. Mironov, et al., Cell 2002, 111, 747). This expression platform is distinct in sequence and structure compared to the expression platform of the TPP riboswitch in the *thiM* mRNA from *E. coli,* wherein TPP binding causes inhibition of translation by a SD blocking mechanism (see Example 2 of U.S. Application Publication No. 2005-0053951). The TPP aptamer domain is easily recognizable and of near identical functional character between these two transcriptional units, but the genetic control mechanisms and the expression platforms that carry them out are very different.

Aptamer domains for riboswitch RNAs typically range from -70 to 170 nt in length (Figure 11 of U.S. Application Publication No. 2005-0053951). This observation was somewhat unexpected given that *in vitro* evolution experiments identified a wide variety of small molecule-binding aptamers, which are considerably shorter in length and structural intricacy (T. Hermann, D. J. Patel, Science 2000, 287, 820; L. Gold, et al., Annual Review of Biochemistry 1995, 64, 763; M. Famulok, Current Opinion in Structural Biology 1999, 9, 324). Although the reasons for the substantial increase in complexity and information content of the natural aptamer sequences relative to artificial aptamers remains to be proven, this complexity is believed required to form RNA receptors that function with high affinity and selectivity. Apparent *K*_{D} values for the ligand-riboswitch complexes range from low nanomolar to low micromolar. It is also worth noting that some aptamer domains, when isolated from the appended expression platform, exhibit improved affinity for the target ligand over that of the intact riboswitch. (∼10 to 100-fold) (see Example 2 of U.S. Application Publication No. 2005-0053951). Presumably, there is an energetic cost in sampling the multiple distinct RNA conformations required by a fully intact riboswitch RNA, which is reflected by a loss in ligand affinity. Since the aptamer domain must serve as a molecular switch, this might also add to the functional demands on natural aptamers that might help rationalize their more sophisticated structures.

### B. Riboswitch Regulation of Transcription Termination in Bacteria

Bacteria primarily make use of two methods for termination of transcription. Certain genes incorporate a termination signal that is dependent upon the Rho protein, (J. P. Richardson, Biochimica et Biophysica Acta 2002, 1577, 251). while others make use of Rho-independent terminators (intrinsic terminators) to destabilize the transcription elongation complex (I. Gusarov, E. Nudler, Molecular Cell 1999, 3, 495; E. Nudler, M. E. Gottesman, Genes to Cells 2002, 7, 755). The latter RNA elements are composed of a GC-rich stem-loop followed by a stretch of 6-9 uridyl residues. Intrinsic terminators are widespread throughout bacterial genomes (F. Lillo, et al., 2002, 18, 971), and are typically located at the 3'-termini of genes or operons. Interestingly, an increasing number of examples are being observed for intrinsic terminators located within 5'-UTRs.

Amongst the wide variety of genetic regulatory strategies employed by bacteria there is a growing class of examples wherein RNA polymerase responds to a termination signal within the 5'-UTR in a regulated fashion (T. M. Henkin, Current Opinion in Microbiology 2000, 3, 149). During certain conditions the RNA polymerase complex is directed by external signals either to perceive or to ignore the termination signal. Although transcription initiation might occur without regulation, control over MRNA. synthesis (and of gene expression) is ultimately dictated by regulation of the intrinsic terminator. Presumably, one of at least two mutually exclusive mRNA conformations results in the formation or disruption of the RNA structure that signals transcription termination. A trans-acting factor, which in some instances is a RNA (F. J. Grundy, et al., Proceedings of the National Academy of Sciences of the United States of America 2002, 99,11121; T. M. Henkin, C. Yanofsky, Bioessays 2002, 24, 700) and in others is a protein (J. Stulke, Archives of Microbiology 2002, 177, 433), is generally required for receiving a particular intracellular signal and subsequently stabilizing one of the RNA conformations. Riboswitches offer a direct link between RNA structure modulation and the metabolite signals that are interpreted by the genetic control machinery. A brief overview of the FMN riboswitch from a *B. subtilis* mRNA is provided below to illustrate this mechanism.

The *xpt-pbuX* operon (Christiansen, L.C., et al., 1997, J. Bacteriol. 179, 2540-2550) is controlled by a riboswitch that exhibits high affinity and high selectivity for guanine. This class of riboswitches is present in the 5'-untranslated region (5'-UTR) of five transcriptional units in *B. subtilis,* including that of the 12-gene *pur* operon. Direct binding of guanine by mRNAs serves as a critical determinant of metabolic homeostasis for purine metabolism in certain bacteria. Furthermore, the discovered classes of riboswitches, which respond to seven distinct target molecules, control at least 68 genes in *Bacillus subtilis* that are of fundamental importance to central metabolic pathways.

Also disclosed are guanine riboswitches that have been identified in *B. subtilis.* The crystal structure at 1.95 Å resolution of the purine-binding domain of the guanine riboswitch from the xpt-pbuX operon of *B. subtilis* bound to hypoxanthine, a prevalent metabolite in the bacterial purine salvage pathway, has been elucidated. This structure reveals a complex RNA fold involving several phylogenetically conserved nucleotides that create a binding pocket that almost completely envelops the ligand. Hypoxanthine functions to stabilize this structure and to promote the formation of a downstream transcriptional terminator element, thereby providing a mechanism for directly repressing gene expression in response to an increase in intracellular concentrations of metabolite.

Certain mRNAs involved in thiamine biosynthesis bind to thiamine (vitamin B₁) or its bioactive pyrophosphate derivative (TPP) without the participation of protein factors. The mRNA-effector complex adopts a distinct structure that sequesters the ribosome-binding site and leads to a reduction in gene expression. This metabolite-sensing mRNA system provides an example of a genetic "riboswitch" (referred to herein as a riboswitch) whose origin might predate the evolutionary emergence of proteins. It has been discovered that the mRNA leader sequence of the *btuB* gene of *Escherichia coli* can bind coenzyme B₁₂ selectively, and that this binding event brings about a structural change in the RNA that is important for genetic control (see Example 1 of U.S. Application Publication No. 2005-0053951). It was also discovered that mRNAs that encode thiamine biosynthetic proteins also employ a riboswitch mechanism (see Example 2 of U.S. Application Publication No. 2005-0053951).

A riboswitch class was discovered in bacteria that is selectively triggered by glycine. A representative of these glycine-sensing RNAs from *Bacillus subtilis* operates as a rare genetic on switch for the *gcυT* operon, which codes for proteins that form the glycine cleavage system. Most glycine riboswitches integrate two ligand-binding domains that function cooperatively to more closely approximate a two-state genetic switch. This advanced form of riboswitch may have evolved to ensure that excess glycine is efficiently used to provide carbon flux through the citric acid cycle and maintain adequate amounts of the amino acid for protein synthesis. Thus, riboswitches perform key regulatory roles and exhibit complex performance characteristics that previously had been observed only with protein factors.

Although the specific natural riboswitches disclosed herein are the first examples of mRNA elements that control genetic expression by metabolite binding, it is expected that this genetic control strategy is widespread in biology. It has been suggested (White III, Coenzymes as fossils of an earlier metabolic state. J. Mol. Evol. 7, 101-104 (1976); White III, In: The Pyridine Nucleotide Coenzymes. Acad. Press, NY pp. 1-17 (1982); Benner et al., Modem metabolism as a palimpsest of the RNA world. Proc. Natl. Acad. Sci. USA 86, 7054-7058 (1989)) that TPP, coenzyme B₁₂ and FMN emerged as biological cofactors during the RNA world (Joyce, The antiquity of RNA-based evolution. Nature 418, 214-221 (2002)). If these metabolites were being biosynthesized and used before the advent of proteins, then certain riboswitches might be modem examples of the most ancient form of genetic control. A search of genomic sequence databases has revealed that sequences corresponding to the TPP aptamer exist in organisms from bacteria, archaea and eukarya-largely without major alteration. Although new metabolite-binding mRNAs are likely to emerge as evolution progresses, it is possible that the known riboswitches are molecular fossils from the RNA world.

It is to be understood that the disclosed method and compositions are not limited to specific synthetic methods, specific analytical techniques, or to particular reagents unless otherwise specified, and, as such, can vary. It is also to be understood that the terminology used herein is for the purpose of describing particular embodiments only and is not intended to be limiting.

### Materials

Disclosed are materials, compositions, and components that can be used for, can be used in conjunction with, can be used in preparation for, or are products of the disclosed methods and compositions. These and other materials are disclosed herein, and it is understood that when combinations, subsets, interactions, groups, etc. of these materials are disclosed that while specific reference to each of various individual and collective combinations and permutation of these compounds can not be explicitly disclosed, each is specifically contemplated and described herein. For example, if a riboswitch or aptamer domain is disclosed and discussed and a number of modifications that can be made to a number of molecules including the riboswitch or aptamer domain are discussed, each and every combination and permutation of riboswitch or aptamer domain and the modifications that are possible are specifically contemplated unless specifically indicated to the contrary. Thus, if a class of molecules A, B, and C are disclosed as well as a class of molecules D, E, and F and an example of a combination molecule, A-D is disclosed, then even if each is not individually recited, each is individually and collectively contemplated. Thus, in this example, each of the combinations A-E, A-F, B-D, B-E, B-F, C-D, C-E, and C-F are specifically contemplated and should be considered disclosed from disclosure of A, B, and C; D, E, and F; and the example combination A-D. Likewise, any subset or combination of these is also specifically contemplated and disclosed. Thus, for example, the sub-group of A-E, B-F, and C-E are specifically contemplated and should be considered disclosed from disclosure of A, B, and C; D, E, and F; and the example combination A-D. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods, and that each such combination is specifically contemplated and should be considered disclosed.

### A. Riboswitches

Riboswitches are expression control elements that are part of an RNA molecule to be expressed and that change state when bound by a trigger molecule. Riboswitches typically can be dissected into two separate domains: one that selectively binds the target (aptamer domain) and another that influences genetic control (expression platform domain). It is the dynamic interplay between these two domains that results in metabolite-dependent allosteric control of gene expression. Disclosed are isolated and recombinant riboswitches, recombinant constructs containing such riboswitches, heterologous sequences operably linked to such riboswitches, and cells and transgenic organisms harboring such riboswitches, riboswitch recombinant constructs, and riboswitches operably linked to heterologous sequences. The heterologous sequences can be, for example, sequences encoding proteins or peptides of interest, including reporter proteins or peptides. Preferred riboswitches are, or are derived from, naturally occurring riboswitches.

The disclosed riboswitches, including the derivatives and recombinant forms thereof, generally can be from any source, including naturally occurring riboswitches and riboswitches designed de novo. Any such riboswitches can be used in or with the disclosed methods. However, different types of riboswitches can be defined and some such sub-types can be useful in or with particular methods (generally as described elsewhere herein). Types of riboswitches include, for example, naturally occurring riboswitches, derivatives and modified forms of naturally occurring riboswitches, chimeric riboswitches, and recombinant riboswitches. A naturally occurring riboswitch is a riboswitch having the sequence of a riboswitch as found in nature. Such a naturally occurring riboswitch can be an isolated or recombinant form of the naturally occurring riboswitch as it occurs in nature. That is, the riboswitch has the same primary structure but has been isolated or engineered in a new genetic or nucleic acid context. Chimeric riboswitches can be made up of, for example, part of a riboswitch of any or of a particular class or type of riboswitch and part of a different riboswitch of the same or of any different class or type of riboswitch; part of a riboswitch of any or of a particular class or type of riboswitch and any non-riboswitch sequence or component Recombinant riboswitches are riboswitches that have been isolated or engineered in a new genetic or nucleic acid context.

Riboswitches can have single or multiple aptamer domains. Aptamer domains in riboswitches having multiple aptamer domains can exhibit cooperative binding of trigger molecules or can not exhibit cooperative binding of trigger molecules (that is, the aptamers need not exhibit cooperative binding). In the latter case, the aptamer domains can be said to be independent binders. Riboswitches having multiple aptamers can have one or multiple expression platform domains. For example, a riboswitch having two aptamer domains that exhibit cooperative binding of their trigger molecules can be linked to a single expression platform domain that is regulated by both aptamer domains. Riboswitches having multiple aptamers can have one or more of the aptamers joined via a linker. Where such aptamers exhibit cooperative binding of trigger molecules, the linker can be a cooperative linker.

Aptamer domains can be said to exhibit cooperative binding if they have a Hill coefficient *n* between x and x-1, where x is the number of aptamer domains (or the number of binding sites on the aptamer domains) that are being analyzed for cooperative binding. Thus, for example, a riboswitch having two aptamer domains (such as glycine-responsive riboswitches) can be said to exhibit cooperative binding if the riboswitch has Hill coefficient between 2 and 1. It should be understood that the value of x used depends on the number of aptamer domains being analyzed for cooperative binding, not necessarily the number of aptamer domains present in the riboswitch. This makes sense because a riboswitch can have multiple aptamer domains where only some exhibit cooperative binding.

Different classes of riboswitches refer to riboswitches that have the same or similar trigger molecules or riboswitches that have the same or similar overall structure (predicted, determined, or a combination). Riboswitches of the same class generally, but need not, have both the same or similar trigger molecules and the same or similar overall structure. Riboswitch classes include glycine-responsive riboswitches, guanine-responsive riboswitches, adenine-responsive riboswitches, lysine-responsive riboswitches, thiamine pyrophosphate-responsive riboswitch, adenosylcobalamin-responsive riboswitches, flavin mononucleotide-responsive riboswitches, and a S-adenosylmethionine-responsive riboswitches.

Also disclosed are chimeric riboswitches containing heterologous aptamer domains and expression platform domains. That is, chimeric riboswitches are made up an aptamer domain from one source and an expression platform domain from another source. The heterologous sources can be from, for example, different specific riboswitches, different types of riboswitches, or different classes of riboswitches. The heterologous aptamers can also come from non-riboswitch aptamers. The heterologous expression platform domains can also come from non-riboswitch sources.

Riboswitches can be modified from other known, developed or naturally-occurring riboswitches. For example, switch domain portions can be modified by changing one or more nucleotides while preserving the known or predicted secondary, tertiary, or both secondary and tertiary structure of the riboswitch. For example, both nucleotides in a base pair can be changed to nucleotides that can also base pair. Changes that allow retention of base pairing are referred to herein as base pair conservative changes.

Modified or derivative riboswitches can also be produced using *in vitro* selection and evolution techniques. In general, *in vitro* evolution techniques as applied to riboswitches involve producing a set of variant riboswitches where part(s) of the riboswitch sequence is varied while other parts of the riboswitch are held constant. Activation, deactivation or blocking (or other functional or structural criteria) of the set of variant riboswitches can then be assessed and those variant riboswitches meeting the criteria of interest are selected for use or further rounds of evolution. Useful base riboswitches for generation of variants are the specific and consensus riboswitches disclosed herein. Consensus riboswitches can be used to inform which part(s) of a riboswitch to vary for *in vitro* selection and evolution.

Also disclosed are modified riboswitches with altered regulation. The regulation of a riboswitch can be altered by operably linking an aptamer domain to the expression platform domain of the riboswitch (which is a chimeric riboswitch). The aptamer domain can then mediate regulation of the riboswitch through the action of, for example, a trigger molecule for the aptamer domain. Aptamer domains can be operably linked to expression platform domains of riboswitches in any suitable manner, including, for example, by replacing the normal or natural aptamer domain of the riboswitch with the new aptamer domain. Generally, any compound or condition that can activate, deactivate or block the riboswitch from which the aptamer domain is derived can be used to activate, deactivate or block the chimeric riboswitch.

Also disclosed are inactivated riboswitches. Riboswitches can be inactivated by covalently altering the riboswitch (by, for example, crosslinking parts of the riboswitch or coupling a compound to the riboswitch). Inactivation of a riboswitch in this manner can result from, for example, an alteration that prevents the trigger molecule for the riboswitch from binding, that prevents the change in state of the riboswitch upon binding of the trigger molecule, or that prevents the expression platform domain of the riboswitch from affecting expression upon binding of the trigger molecule.

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo or in vitro.* For example, biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch. Biosensor riboswitches can be used in various situations and platforms. For example, biosensor riboswitches can be used with solid supports, such as plates, chips, strips and wells.

Also disclosed are modified or derivative riboswitches that recognize new trigger molecules. New riboswitches and/or new aptamers that recognize new trigger molecules can be selected for, designed or derived from known riboswitches. This can be accomplished by, for example, producing a set of aptamer variants in a riboswitch, assessing the activation of the variant riboswitches in the presence of a compound of interest, selecting variant riboswitches that were activated (or, for example, the riboswitches that were the most highly or the most selectively activated), and repeating these steps until a variant riboswitch of a desired activity, specificity, combination of activity and specificity, or other combination of properties results.

Particularly useful aptamer domains can form a stem structure referred to herein as the P1 stem structure (or simply P1). The P1 stems of a variety of riboswitches are shown in Figure 11 of U.S. Application Publication No. 2005-0053951. The hybridizing strands in the P1 stem structure are referred to as the aptamer strand (also referred to as the P1a strand) and the control strand (also referred to as the P1b strand). The control strand can form a stem structure with both the aptamer strand and a sequence in a linked expression platform that is referred to as the regulated strand (also referred to as the P1c strand). Thus, the control strand (P1b) can form alternative stem structures with the aptamer strand (P1a) and the regulated strand (P1c). Activation and deactivation of a riboswitch results in a shift from one of the stem structures to the other (from P1a/P1b to P1b/P1c or vice versa). The formation of the P1b/P1c stem structure affects expression of the RNA molecule containing the riboswitch. Riboswitches that operate via this control mechanism are referred to herein as alternative stem structure riboswitches (or as alternative stem riboswitches). Some glycine-responsive riboswitches having two aptamers utilize this mechanism using a P1 stem in the second aptamer.

In general, any aptamer domain can be adapted for use with any expression platform domain by designing or adapting a regulated strand in the expression platform domain to be complementary to the control strand of the aptamer domain. Alternatively, the sequence of the aptamer and control strands of an aptamer domain can be adapted so that the control strand is complementary to a functionally significant sequence in an expression platform. For example, the control strand can be adapted to be complementary to the Shine-Dalgarno sequence of an RNA such that, upon formation of a stem structure between the control strand and the SD sequence, the SD sequence becomes inaccessible to ribosomes, thus reducing or preventing translation initiation. Note that the aptamer strand would have corresponding changes in sequence to allow formation of a P1 stem in the aptamer domain. In the case of riboswitches having multiple aptamers exhibiting cooperative binding, one the P1 stem of the activating aptamer (the aptamer that interacts with the expression platform domain) need be designed to form a stem structure with the SD sequence.

As another example, a transcription terminator can be added to an RNA molecule (most conveniently in an untranslated region of the RNA) where part of the sequence of the transcription terminator is complementary to the control strand of an aptamer domain (the sequence will be the regulated strand). This will allow the control sequence of the aptamer domain to form alternative stem structures with the aptamer strand and the regulated strand, thus either forming or disrupting a transcription terminator stem upon activation or deactivation of the riboswitch. Any other expression element can be brought under the control of a riboswitch by similar design of alternative stem structures.

For transcription terminators controlled by riboswitches, the speed of transcription and spacing of the riboswitch and expression platform elements can be important for proper control. Transcription speed can be adjusted by, for example, including polymerase pausing elements (e.g., a series of uridine residues) to pause transcription and allow the riboswitch to form and sense trigger molecules. For example, with the FMN riboswitch, if FMN is bound to its aptamer domain, then the antiterminator sequence is sequestered and is unavailable for formation of an antiterminator structure (Figure 12 of U.S. Application Publication No. 2005-0053951). However, if FMN is absent, the antiterminator can form once its nucleotides emerge from the polymerase. RNAP then breaks free of the pause site only to reach another U-stretch and pause again. The transcriptional terminator then forms only if the *terminator* nucleotides are not tied up by the antiterminator.

Disclosed are regulatable gene expression constructs comprising a nucleic acid molecule encoding an RNA comprising a riboswitch operably linked to a coding region, wherein the riboswitch regulates expression of the RNA, wherein the riboswitch and coding region are heterologous. The riboswitch can comprise an aptamer domain and an expression platform domain, wherein the aptamer domain and the expression platform domain are heterologous. The riboswitch can comprise an aptamer domain and an expression platform domain, wherein the aptamer domain comprises a P1 stem, wherein the P1 stem comprises an aptamer strand and a control strand, wherein the expression platform domain comprises a regulated strand, wherein the regulated strand, the control strand, or both have been designed to form a stem structure. The riboswitch can comprise two or more aptamer domains and an expression platform domain, wherein at least one of the aptamer domains and the expression platform domain are heterologous. The riboswitch can comprise two or more aptamer domains and an expression platform domain, wherein at least one of the aptamer domains comprises a P1 stem, wherein the P1 stem comprises an aptamer strand and a control strand, wherein the expression platform domain comprises a regulated strand, wherein the regulated strand, the control strand, or both have been designed to form a stem structure.

Disclosed are riboswitches, wherein the riboswitch is a non-natural derivative of a naturally-occurring riboswitch. The riboswitch can comprise an aptamer domain and an expression platform domain, wherein the aptamer domain and the expression platform domain are heterologous. The riboswitch can be derived from a naturally-occurring guanine-responsive riboswitch, adenine-responsive riboswitch, lysine-responsive riboswitch, thiamine pyrophosphate-responsive riboswitch, adenosylcobalamin-responsive riboswitch, flavin mononucleotide-responsive riboswitch, glycine-responsive riboswitch, or a S-adenosylmethionine-responsive riboswitch. The riboswitch can be activated by a trigger molecule, wherein the riboswitch produces a signal when activated by the trigger molecule.

Table 5 discloses examples of various guanine riboswitches. The alignment of sequences in Table 5 is in Stockholm format as output by the cmalign program of the INFERNAL software package. The last two entries in Table 5 reflect the consensus sequence and structure for the motif as reflected by a computer algorithm described by Eddy, S. R. (2003). INFERNAL. Version 0.55. Distributed by the author. Dept of Genetics, Washington University School of Medicine. St. Louis, Missouri. Figure 41 of U.S. Application Publication No. 2005-0053951 describes consensus riboswitch sequences, natural riboswitch sequences, and riboswitch sequence alignment. Thermodynamic parameters of various nucleobases bound to adenine riboswitch RNA are shown in Table 7. Thermodynamic parameters of various nucleobases bound to guanine riboswitch RNA are shown in Table 8.

Numerous riboswitches and riboswitch constructs are described and referred to herein. The present invention relates to crystalline natural guanine-responsive riboswitches having the atomic coordinates listed in Table 6.

### 1. Aptamer Domains

Aptamers are nucleic acid segments and structures that can bind selectively to particular compounds and classes of compounds. Riboswitches have aptamer domains that, upon binding of a trigger molecule result in a change in the state or structure of the riboswitch. In functional riboswitches, the state or structure of the expression platform domain linked to the aptamer domain changes when the trigger molecule binds to the aptamer domain. Aptamer domains of riboswitches can be derived from any source, including, for example, natural aptamer domains of riboswitches, artificial aptamers, engineered, selected, evolved or derived aptamers or aptamer domains. Aptamers in riboswitches generally have at least one portion that can interact, such as by forming a stem structure, with a portion of the linked expression platform domain. This stem structure will either form or be disrupted upon binding of the trigger molecule.

Consensus aptamer domains of a variety of natural riboswitches are shown in Figure 11 of U.S. Application Publication No. 2005-0053951 and elsewhere herein. These aptamer domains (including all of the direct variants embodied therein) can be used in riboswitches. The consensus sequences and structures indicate variations in sequence and structure. Aptamer domains that are within the indicated variations are preferred to herein as direct variants. These aptamer domains can be modified to produce modified or variant aptamer domains. Conservative modifications include any change in base paired nucleotides such that the nucleotides in the pair remain complementary. Moderate modifications include changes in the length of stems or of loops (for which a length or length range is indicated) of less than or equal to 20% of the length range indicated. Loop and stem lengths are considered to be "indicated" where the consensus structure shows a stem or loop of a particular length or where a range of lengths is listed or depicted. Moderate modifications include changes in the length of stems or of loops (for which a length or length range is not indicated) of less than or equal to 40% of the length range indicated. Moderate modifications also include and functional variants of unspecified portions of the aptamer domain. Unspecified portions of the aptamer domains are indicated by solid lines in Figure 11 of U.S. Application Publication No. 2005-0053951.

The P1 stem and its constituent strands can be modified in adapting aptamer domains for use with expression platforms and RNA molecules. Such modifications, which can be extensive, are referred to herein as P1 modifications. P1 modifications include changes to the sequence and/or length of the P1 stem of an aptamer domain.

The aptamer domains shown in Figure 11 of U.S. Application Publication No. 2005-0053951 (including any direct variants) are particularly useful as initial sequences for producing derived aptamer domains via *in vitro* selection or *in vitro* evolution techniques.

Aptamer domains of the disclosed riboswitches can also be used for any other purpose, and in any other context, as aptamers. For example, aptamers can be used to control ribozymes, other molecular switches, and any RNA molecule where a change in structure can affect function of the RNA.

### 2. Expression Platform Domains

Expression platform domains are a part of riboswitches that affect expression of the RNA molecule that contains the riboswitch. Expression platform domains generally have at least one portion that can interact, such as by forming a stem structure, with a portion of the linked aptamer domain. This stem structure will either form or be disrupted upon binding of the trigger molecule. The stem structure generally either is, or prevents formation of, an expression regulatory structure. An expression regulatory structure is a structure that allows, prevents, enhances or inhibits expression of an RNA molecule containing the structure. Examples include Shine-Dalgarno sequences, initiation codons, transcription terminators, and stability and processing signals.

### B. Trigger Molecules

Trigger molecules are molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, *in vitro* selection or in *vitro* evolution techniques).

### C. Compounds

Also disclosed are compounds, and compositions containing such compounds, that can activate, deactivate or block a riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch. Riboswitches can also be deactivated by, for example, removing trigger molecules from the presence of the riboswitch. A riboswitch can be blocked by, for example, binding of an analog of the trigger molecule that does not activate the riboswitch.

Also disclosed are compounds for altering expression of an RNA molecule, or of a gene encoding an RNA molecule, where the RNA molecule includes a riboswitch. This can be accomplished by bringing a compound into contact with the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch, reduce or prevent expression of the RNA molecule or promote or increase expression of the RNA molecule.

Also disclosed are compounds for regulating expression of an RNA molecule, or of a gene encoding an RNA molecule. Also disclosed are compounds for regulating expression of a naturally occurring gene or RNA that contains a riboswitch by activating, deactivating or blocking the riboswitch. If the gene is essential for survival of a cell or organism that harbors it, activating, deactivating or blocking the riboswitch can in death, stasis or debilitation of the cell or organism.

Also disclosed are compounds for regulating expression of an isolated, engineered or recombinant gene or RNA that contains a riboswitch by activating, deactivating or blocking the riboswitch. If the gene encodes a desired expression product, activating or deactivating the riboswitch can be used to induce expression of the gene and thus result in production of the expression product. If the gene encodes an inducer or repressor of gene expression or of another cellular process, activation, deactivation or blocking of the riboswitch can result in induction, repression, or de-repression of other, regulated genes or cellular processes. Many such secondary regulatory effects are known and can be adapted for use with riboswitches. An advantage of riboswitches as the primary control for such regulation is that riboswitch trigger molecules can be small, non-antigenic molecules.

Also disclosed are methods of identifying compounds that activate, deactivate or block a riboswitch. For example, compounds that activate a riboswitch can be identified by bringing into contact a test compound and a riboswitch and assessing activation of the riboswitch. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways.

Identification of compounds that block a riboswitch can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Compounds can also be identified using the atomic crystalline structure of a riboswitch. An example of such a crystalline atomic structure of a natural guanine-responsive riboswitch can be found in Figure 5. The atomic coordinates of the atomic structure are listed in Table 6. In Figure 5, the riboswitch is shown bound to hypoxanthine. In this example, the crystal structure at 1.95 Å resolution of the purine-binding domain of the guanine riboswitch from the xpt-pbuX operon ofB. subtilis bound to hypoxanthine, a prevalent metabolite in the bacterial purine salvage pathway is shown. This structure reveals a complex RNA fold involving several phylogenetically conserved nucleotides that create a binding pocket that almost completely envelops the ligand. Hypoxanthine functions to stabilize this structure and to promote the formation of a downstream transcriptional terminator element, thereby providing a mechanism for directly repressing gene expression in response to an increase in intracellular concentrations of metabolite.

Compounds can be identified using the crystalline structure of a riboswitch by, for example, modeling the atomic structure of the riboswitch with a test compound; and determining if the test compound interacts with the riboswitch. This can be done by using a predicted minimum interaction energy, a predicted bind constant, a predicted dissociation constant, or a combination, for the test compound in the model of the riboswitch. Compounds can also be identified by, for example, asessing the fit between the riboswtich and a compound known to bind the riboswitch (such as the trigger molecule), identify sites where the compound can be changed with little or no obvious adverse effects on binding of the compound, and incorporating one or more such alterations to produce a new compound. The method of identifying compounds that interact with a riboswitch can also involve production of the compounds so identified.

Typically the method first utilizes a 3-dimensional structure of the riboswitch with a compound, also referred to as a "known compound" or "known target". Any of the trigger molecules and compounds disclosed herein can be used as such a known compound. The structure of the riboswitch can be determined using any known means, such as crystallography or solution NMR spectroscopy. That structure can also be obtained through computer molecular modeling simulation programs, such as AutoDock. The methods can involve determining the amount of binding, such as determining the binding energy, between a riboswitch, and a potential compound for that riboswitch. An active compound is a compound that has some activity against a riboswitch, such as inhibiting the riboswitch's activity or enhancing the riboswitch's activity. In addition, the potential compound can be an analog, which has some structural relationship to a known compound for the molecule. Any of the trigger molecules, known compounds, and compounds disclosed herein can be used as the basis of or to derive a potential compound.

The identity or relationship of the structure, properties, interaction or binding parameters, and the like of the known compound and potential compound can be viewed in number of ways. For example, any of the measures or interaction parameters that can be measured or assessed using the structural model, and such measures and parameters obtained for a known compound and a potential compound can be compared. One can look at the identity between the entire known compound and the potential compound. One can also look at the identity between the potential compound, such as an analog, and the know compound only in the domain where the potential compound interacts with the riboswitch. One can also look at the identity between the potential compound and the known compound at the level of a sub-domain, such as only those moieties or atoms in the potential compound which are within 7Å, 6Å, 5Å, 4Å, 3Å, or 2Å of a moiety or atom which is in contact with the riboswitch in the known compound. Generally, the more specific the sub-domain the higher the identity will be between the moieties of the potential compound and the known compound. For example, there can be 30% or greater, 35% or greater, 40% or greater, 45% or greater, 50% or greater, 55% or greater, 60% or greater, 65% or greater, 70% or greater, 75% or greater, 80% or greater, 85% or greater, 90% or greater, 95% or greater identity between the known compound and potential compound as a whole, 50% or greater, 55% or greater, 60% or greater, 65% or greater, 70% or greater, 75% or greater, 80% or greater, 85% or greater, 90% or greater, 95% or greater identity between the binding domain of the known compound and the potential compound, and 70% or greater, 75% or greater, 80% or greater, 85% or greater, 90% or greater, 95% or greater identity between the moieties or atoms of the potential compound that correspond to the moieties or atoms of the known compound which are within 5A of a moiety or atom which interacts with the riboswitch. Another sub-domain is a sub-domain of moieties or atoms which actually contact the riboswitch. In this case the identity can be, for example, greater than 50%, 55%, 60%, 65%, 70%, 75%, 80%, 85%, 90%, 95% or higher.

Typically, the potential compounds exist in a family of potential compounds, i.e. a set of analogs, all of which have some structural relationship to the known compound for the riboswitch. A family consisting of any number of members can be screened. The maximum number of members in the family is only limited by the amount of computer power available to screen each member in a desired amount of time. The methods can involve at least one template structure of the riboswitch and a target, often this would be with a known target. It is not required that this structure be existent, as it can be generated, in some cases during the disclosed methods, using standard structure determination techniques. It is preferred that a real structure exist at the time the methods are employed.

The methods can also involve modeling the structure of the potential compound, using information from the structure of the known compound. This modeling can be performed in any way, and as described herein.

The conformation and position of the potential compound can be held fixed during the calculations; that is, it can be assumed that the riboswitch binds in exactly the same orientation to the potential compound as it does to a known compound.

Then, a binding energy (or other property or parameter) can be determined between the riboswitch and the potential compound, and if the binding energy (or other property or parameter) meets certain criteria, then the potential compound can be designated as an actual compound, i.e. one that is likely to interact with the riboswitch. Although the following refers to the use of binding energy, it should be understood that any property or parameter involving the interaction or modeling of a compound and a riboswitch can be used. The criterion can be that the computed binding energy of the riboswitch with the potential compound is similar to, or more favorable than, the computed binding energy of the same riboswitch with a known compound. For example, an actual compound can be a compound where the computed binding energy as discussed herein is, for example, at least 60%, 65%, 70%, 75%, 80%, 85%, 86%, 87%, 88%, 89%, 90%, 91%, 92%, 93%, 94%, 95%, 96%, 97%, 98%, 99%, 100%, 101%, 102%, 103%, 104%, 105%, 106%, 107%, 108%, 109%, 110%, 120%, 130%, 140%, 150%, 200%, 250%, 300%, 350%, 400%, 450%, 500%, 600%, 700%, 800%, 900%, 1000%, or greater than that of the known compound binding energy. An actual compound can also be a compound which after ordering all potential compounds in terms of the strength of their binding energies, are the compounds which are in the top 20%, 19%, 18%, 17%, 16%, 15%, 14%, 13%, 12%, 11%, 10%, 9%, 8%, 7%, 6%, 5%, 4%, 3%, 2%, 1% of computed binding strengths, of for example, a set of potential compounds where the set is at least 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 125, 150, 175, 200, 225, 250, 275, 300, 350, 400, 500, 700, or a 1000 potential compounds.

It is also understood that once a potential compound is identified, as disclosed herein, traditional testing and analysis can be performed, such as performing a biological assay using the riboswitch and the actual compound to further define the ability of the actual compound to interact with and/or modulate the riboswitch. The disclosed methods can include the step of assaying the activity of the riboswitch and compound, as well as performing, for example, combinatorial chemistry studies using libraries based on the riboswitch, for example.

Energy calculations can be based on, for example, molecular or quantum mechanics. Molecular mechanics approximates the energy of a system by summing a series of empirical functions representing components of the total energy like bond stretching, van der Waals forces, or electrostatic interactions. Quantum mechanics methods use various degrees of approximation to solve the Schrödinger equation. These methods deal with electronic structure, allowing for the characterization of chemical reactions.

Potential compounds of the riboswitch can be identified. This can be accomplished by selecting potential compounds with a given similarity to the known compound. For example, compounds in the same family as the known compound can be selected.

To prepare each riboswitch for calculation, atoms can be built in that were unresolved or absent from the crystal structures of the potential compound. This can be done, for example, using the PRODRG webserver http://www.davapc1.bioch.dundee.ac.uk./programs/prodrg, or standard molecular modeling programs such as InsightII, Quanta (both at www.accelrys.com), CNS (Brunger et al., Crystallography & NMR system: a new software suite for macromolecular structure determination. Acta Crystallogr. D 54, 905-921 (1998)), or any other molecular modeling system capable of preparing the riboswitch structure.

The binding energy (or other property or parameter) of the potential compound and riboswitch can then be calculated. There are numerous means for carrying this out. For example, the sampling of sidechain positions and the computation of the binding thermodynamics can be accomplished using an empirical function that models the energy of the potential compound-molecule as a sum of electrostatic and van der Waals interactions between all pairs of atoms within the model. Any other computational method for scoring the binding energy of the potential compound with the riboswitch can be used (H. Gohlke, & G. Klebe. Approaches to the description and prediction of the binding affinity of small-molecule ligands to macromolecular receptors. Angew. Chem. Int. Ed. 41, 2644-4676 (2002)). Examples of such scoring methods include, but are not limited to, those implemented in programs such as AutoDock (G. M. Morris et al. Automated docking using a Lamarckian genetic algorithm and an empirical binding free energy function. J. Comput. Chem. 19, 1639-1662 (1998)), Gold (G. Jones et al. Molecular recognition of receptor sites using a genetic algorithm with a description of desolvation. J. Mol. Biol. 245, 43-53 (1995)), Chem-Score (M. D. Eldridge et al. J. Comput.-Aided Mol. Des. 11, 425-445 (1997) and Drug-Score (H. Gohlke et al. Knowledge-based scoring function to predict protein-ligand interactions. J. Mol. Biol. 295, 337-356 (2000)).

Rotamer libraries are known to those of skill in the art and can be obtained from a variety of sources, including the internet. Rotamers are low energy side-chain conformations. The use of a library of rotamers allows for the modeling of a structure to try the most likely side-chain conformations, saving time and producing a structure that is more likely to be correct. The use of a library of rotamers can be restricted to those residues that are within a given region of the potential compound, for example, at the binding site, or within a specified distance of the compound. The latter distance can be set at any desired length, for example, the potential compound can be 2, 3, 4, 5, 6, 7, 8, or 9 A from any atom of the molecule.

Electrostatic interactions between every pair of atoms can be calculated, for example, using a Coulombic model with the formula:
E_{elec}=332.08 q₁ q₂ /εΓ. where q₁ and q₂ are partial atomic charges, r is the distance between them, and ε is the dielectric constant.

Partial atomic charges can be taken from existing parameter sets that have been developed to describe charge distributions in molecules. Example parameter sets include, but are not limited to, PARSE (D. A. Sitkoff et al. Accurate calculation of hydration free-energies using macroscopic solvent models. J. Phys. Chem. 98, 1978-1988 (1994)), CHARMM (MacKerell et al. All-atom empirical potential for molecular modeling and dynamics studies of proteins. J. Phys. Chem. B 102, 3586-3616, 1998) and AMBER (W. D. Cornell et al. A 2nd generation force-field for the simulation of proteins, nucleic-acids, and organic-molecules. J. Am. Chem. Soc. 117. 5179-5195 (1995)). Partial charges for atoms can be assigned either by analogy with those of similar functional groups, or by empirical assignment methods such as that implemented in the PRODRG server (D. M. F. van Aalten et al. PRODRG, a program for generating molecular topologies and unique molecular descriptors from coordinates of small molecules. J. Comput.-Aided Mol. Design 10, 255-262 (1996)), or by the use of standard quantum mechanical calculation methods (for example, C. I. Bayly et al. A well-behaved electrostatic potential based method using charge restraints for deriving atomic charges - the RESP model. J. Phys. Chem. 97, 10269-10280, (1993)).

The electrostatic interaction can also be calculated by more elaborate methodologies that incorporate electrostatic desolvation effects. These can include explicit solvent and implicit solvent models: in the former, water molecules are directly included in the calculations, whereas in the latter, the effects of water are described by a dielectric continuum approach. Specific examples of implicit solvent methods for calculating electrostatic interactions include but are not limited to: Poisson-Boltzmann based methods and Generalized Born methods (M. Feig & C. L. Brooks. Recent advances in the development and application of implicit solvent models in biomolecule simulations. Curr. Opin. Struct. Biol. 14, 217-224 (2004)).

van der Waals and hydrophobic interactions between pairs of atoms (where both atoms are either sulfur or carbon) can be calculated using a simple Lennard-Jones formalism with the following equation:
E_{vdw}= ∈{σₐₜₜ¹²/r¹² - σₐₜₜ⁶/r⁶}. where ∈ is an energy, r is the distance between the two atoms and σₐₜₜ is the distance at which the energy of interaction is zero.

van der Waals interactions between pairs of atoms (where one or both atoms are neither sulfur nor carbon) can be calculated using a simple repulsive energy term:
E_{vdw}= ∈{σᵣₑₚ¹²/r¹²}. where ∈ is an energy, r is the distance between the two atoms and σᵣₑₚ determines the distance at which the repulsive interaction is equal to ∈.

Hydrophobic interactions between atoms can also be calculated using a variety of other methods known to those skilled in the art. For example, the energetic contribution can be calculated as being proportional to the amount of solvent accessible surface area of the ligand and receptor that is buried when the complex is formed. Such contributions can be expressed in terms of interactions between pairs of atoms, such as in the method proposed by Street & Mayo (A. G. Street & S. L. Mayo. Pairwise calculation of protein solvent-accessible surface areas. Folding & Design 3, 253-258 (1998)). Any other implementation of a formalism for describing hydrophobic or van der Waals or other energetic contributions can be included in the calculations.

Binding energies can be calculated for each potential compound-riboswitch interaction. For example, Monte Carlo sampling can be conducted in the presence and absence of the riboswitch, and the average energy in each simulation calculated. A binding energy for the riboswitch with the potential compound can then be calculated as the difference between the two calculated average energies.

The computed binding energy of a potential compound with the riboswitch can be compared with the computed binding energy of a known compound with the riboswitch to determine if the potential compound is likely to be an actual compound. These results can then be confirmed using experimental data, wherein the actual interaction between the riboswitch and compound can be measured. Examples of methods that can be used to determine an actual interaction between the riboswitch and the compound include but are not limited to: equilibrium dialysis measurements (wherein binding of a radioactive form of the compound to the riboswitch is detected), enzyme inhibition assays (wherein the activity of the riboswitch can be monitored in the presence and absence of the compound), and chemical shift perturbation measurements (wherein binding of the riboswitch to the potential compound is monitored by observing changes in NMR chemical shifts of atoms).

In the methods disclosed above, the riboswitch can be a guanine riboswitch, for example. This riboswitch can be selected, for example, from riboswitches in Table 5.

After the atomic crystalline structure of the riboswitch has been modeled with a potential compound, further testing can be carried out to determine the actual interaction between the riboswitch and the compound. For example, multiple different approaches can be used to detect binding RNAs, including allosteric ribozyme assays using gel-based and chip-based detection methods, and in-line probing assays. High throughput testing can also be accomplished by using, for example, fluorescent detection methods. For example, the natural catalytic activity of a glucosamine-6-phosphate sensing riboswitch that controls gene expression by activating RNA-cleaving ribozyme can be used. This ribozyme can be reconfigured to cleave separate substrate molecules with multiple turnover kinetics. Therefore, a fluorescent group held in proximity to a quenching group can be uncoupled (and therefore become more fluorescent) if a compound triggers ribozyme function. Second, molecular beacon technology can be employed. This creates a system that suppresses fluorescence if a compound prevents the beacon from docking to the riboswitch RNA. Either approach can be applied to any of the riboswitch classes by using RNA engineering strategies described herein.

Also disclosed herein are analogs that interact with the guanine riboswitch disclosed herein. Examples of such analogs can be found in Figure 12B. Many of the 26 compounds synthesized and tested bind the guanine riboswitch with constants that are equal to or better than that of guanine (-5 nM). The fact that appendages with highly variable chemical composition exhibit function shows that numerous variations of these chemical scaffolds can be generated and tested for function *in vitro* and inside cells. Specifically, further modified versions of these compounds can have improved binding to the guanine riboswitch by making new contacts to other functional groups in the RNA structure. Furthermore, modulation of bioavailability, toxicity, and synthetic ease (among other characteristics) can be tunable by making modifications in these two regions of the scaffold, as the structural model for the riboswitch shows many modifications are possible at these sites.

High-throughput screening can also be used to reveal entirely new chemical scaffolds that also bind to riboswitch RNAs either with standard or non- standard modes of molecular recognition. Since riboswitches are the first major form of natural metabolite-binding RNAs to be discovered, there has been little effort made previously to create binding assays that can be adapted for high-throughput screening. Multiple different approaches can be used to detect metabolite binding RNAs, including allosteric ribozyme, assays using gel-based and chip-based detection methods, and in-line probing assays. Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch.

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For the purposes of this disclosure, the heteroatoms, such as nitrogen, can have hydrogen substituents and/or any permissible substituents of organic compounds described herein which satisfy the valences of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, e.g., a compound that does not spontaneously undergo transformation such as by rearrangement, cyclization, elimination, etc.

"A¹," "A²," "A³," and "A⁴" are used herein as generic symbols to represent various specific substituents. These symbols can be any substituent, not limited to those disclosed herein, and when they are defined to be certain substituents in one instance, they can, in another instance, be defined as some other substituents.

The term "alkyl" as used herein is a branched or unbranched saturated hydrocarbon group of 1 to 24 carbon atoms, such as methyl, ethyl, n-propyl, isopropyl, n-butyl, isobutyl, t-butyl, pentyl, hexyl, heptyl, octyl, nonyl, decyl, dodecyl, tetradecyl, hexadecyl, eicosyl, tetracosyl, and the like. The alkyl group can also be substituted or unsubstituted. The alkyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol, as described below. The term "lower alkyl" is an alkyl group with 6 or fewer carbon atoms, e.g., methyl, ethyl, propyl, isopropyl, butyl, sec-butyl, iso-butyl, tert-butyl, pentyl, hexyl, and the like.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" specifically refers to an alkyl group that is substituted with one or more halide, e.g., fluorine, chlorine, bromine, or iodine. The term "alkoxyalkyl" specifically refers to an alkyl group that is substituted with one or more alkoxy groups, as described below. The term "alkylamino" specifically refers to an alkyl group that is substituted with one or more amino groups, as described below, and the like. When "alkyl" is used in one instance and a specific term such as "halogenated alkyl" is used in another, it is not meant to imply that the term "alkyl" does not also refer to specific terms such as "halogenated alkyl" and the like.

This practice is also used for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, e.g., an "alkylcycloalkyl." Similarly, a substituted alkoxy can be specifically referred to as, e.g., a "halogenated alkoxy," a particular substituted alkenyl can be, e.g., an "alkenylalcohol," and the like. Again, the practice of using a general term, such as "cycloalkyl," and a specific term, such as "allcylcycloalkyl," is not meant to imply that the general term does not also include the specific term.

The term "alkoxy" as used herein is an alkyl group bonded through a single, terminal ether linkage; that is, an "alkoxy" group can be defined as —OA¹ where A² is alkyl as defined above.

The term "alkenyl" as used herein is a hydrocarbon group of from 2 to 24 carbon atoms with a structural formula containing at least one carbon-carbon double bond. Asymmetric structures such as (A¹A²)C=C(A³A⁴) are intended to include both the E and Z isomers. This can be presumed in structural formulae herein wherein an asymmetric alkene is present, or it can be explicitly indicated by the bond symbol C=C. The alkenyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol, as described below.

The term "alkynyl" as used herein is a hydrocarbon group of 2 to 24 carbon atoms with a structural formula containing at least one carbon-carbon triple bond. The alkynyl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol, as described below.

The term "aryl" as used herein is a group that contains any carbon-based aromatic group including, but not limited to, benzene, naphthalene, phenyl, biphenyl, phenoxybenzene, and the like. The term "aryl" also includes "heteroaryl," which is defined as a group that contains an aromatic group that has at least one heteroatom incorporated within the ring of the aromatic group. Examples of heteroatoms include, but are not limited to, nitrogen, oxygen, sulfur, and phosphorus. Likewise, the term "non-heteroaryl," which is also included in the term "aryl," defines a group that contains an aromatic group that does not contain a heteroatom. The aryl group can be substituted or unsubstituted. The aryl group can be substituted with one or more groups including, but not limited to, alkyl, halogenated alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol as described herein. The term "biaryl" is a specific type of aryl group and is included in the definition of aryl. Biaryl refers to two aryl groups that are bound together *via* a fused ring structure, as in naphthalene, or are attached *via* one or more carbon-carbon bonds, as in biphenyl.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, etc. The term "heterocycloalkyl" is a cycloalkyl group as defined above where at least one of the carbon atoms of the ring is substituted with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol as described herein.

The term "cycloalkenyl" as used herein is a non-aromatic carbon-based ring composed of at least three carbon atoms and containing at least one double bound, *i.e.,* C=C. Examples of cycloalkenyl groups include, but are not limited to, cyclopropenyl, cyclobutenyl, cyclopentenyl, cyclopentadienyl, cyclohexenyl, cyclohexadienyl, and the like. The term "heterocycloalkenyl" is a type of cycloalkenyl group as defined above, and is included within the meaning of the term "cycloalkenyl," where at least one of the carbon atoms of the ring is substituted with a heteroatom such as, but not limited to, nitrogen, oxygen, sulfur, or phosphorus. The cycloalkenyl group and heterocycloalkenyl group can be substituted or unsubstituted. The cycloalkenyl group and heterocycloalkenyl group can be substituted with one or more groups including, but not limited to, alkyl, alkoxy, alkenyl, alkynyl, aryl, heteroaryl, aldehyde, amino, carboxylic acid, ester, ether, halide, hydroxy, ketone, sulfo-oxo, sulfonyl, sulfone, sulfoxide, or thiol as described herein.

The term "cyclic group" is used herein to refer to either aryl groups, non-aryl groups (i.e., cycloalkyl, heterocycloalkyl, cycloalkenyl, and heterocycloalkenyl groups), or both. Cyclic groups have one or more ring systems that can be substituted or unsubstituted. A cyclic group can contain one or more aryl groups, one or more non-aryl groups, or one or more aryl groups and one or more non-aryl groups.

The term "aldehyde" as used herein is represented by the formula -C(O)H. Throughout this specification "C(O)" is a short hand notation for C=O.

The terms "amine" or "amino" as used herein are represented by the formula NA¹A²A³, where A¹, A², and A³ can be, independently, hydrogen, an alkyl, halogenated alkyl, alkenyl, alkynyl, aryl, heteroaryl, cycloalkyl, cycloalkenyl, heterocycloalkyl, or heterocycloalkenyl group described above.

The term "carboxylic acid" as used herein is represented by the formula -C(O)OH. A "carboxylate" as used herein is represented by the formula - C(O)O⁻.

The term "ester" as used herein is represented by the formula -OC(O)A¹ or -C(O)OA¹, where A¹ can be an alkyl, halogenated alkyl, alkenyl, alkynyl, aryl, heteroaryl, cycloalkyl, cycloalkenyl, heterocycloalkyl, or heterocycloalkenyl group described above.

The term "ether" as used herein is represented by the formula A¹OA², where A¹ and A² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "ketone" as used herein is represented by the formula A¹C(O)A², where A¹ and A² can be, independently, an alkyl, halogenated alkyl, alkenyl, alkynyl, aryl, heteroaryl, cycloalkyl, cycloalkenyl, heterocycloalkyl, or heterocycloalkenyl group described above.

The term "halide" as used herein refers to the halogens fluorine, chlorine, bromine, and iodine.

The term "hydroxyl" as used herein is represented by the formula —OH.

The term "sulfo-oxo" as used herein is represented by the formulas -S(O)A¹ (*i.e*., "sulfonyl"), A¹S(O)A² (*i.e*., "sulfoxide"), --S(O)₂A¹**,** A¹SO₂A² (*i.e*., "sulfone"), -OS(O)₂A¹, or -OS(O)₂OA¹, where A₁ and A² can be hydrogen, an alkyl, halogenated alkyl, alkenyl, alkynyl, aryl, heteroaryl, cycloalkyl, cycloalkenyl, heterocycloalkyl, or heterocycloalkenyl group described above. Throughout this specification "S(O)" is a short hand notation for S=O.

The term "sulfonylamino" or "sulfonamide" as used herein is represented by the formula -S(O)₂NH-.

The term "thiol" as used herein is represented by the formula —SH.

As used herein, "Rⁿ" where n is some integer can independently possess one or more of the groups listed above. For example, if R¹⁰ contains an aryl group, one of the hydrogen atoms of the aryl group can optionally be substituted with a hydroxyl group, an alkoxy group, an amine group, an alkyl group, a halide, and the like. Depending upon the groups that are selected, a first group can be incorporated within second group or, alternatively, the first group can be pendant (*i.e*., attached) to the second group. For example, with the phrase "an alkyl group comprising an amino group," the amino group can be incorporated within the backbone of the alkyl group. Alternatively, the amino group can be attached to the backbone of the alkyl group. The nature of the group(s) that is (are) selected will determine if the first group is embedded or attached to the second group.

Unless stated to the contrary, a formula with chemical bonds shown only as solid lines and not as wedges or dashed lines contemplates each possible isomer, e.g., each enantiomer and diastereomer, and a mixture of isomers, such as a racemic or scalemic mixture.

Certain materials, compounds, compositions, and components disclosed herein can be obtained commercially or readily synthesized using techniques generally known to those of skill in the art. For example, the starting materials and reagents used in preparing the disclosed compounds and compositions are either available from commercial suppliers such as Aldrich Chemical Co., (Milwaukee, Wis.), Acros Organics (Morris Plains, N.J.), Fisher Scientific (Pittsburgh, Pa.), or Sigma (St. Louis, Mo.) or are prepared by methods known to those skilled in the art following procedures set forth in references such as Fieser and Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); March's Advanced Organic Chemistry, (John Wiley and Sons, 4th Edition); and Larock's Comprehensive Organic Transformations (VCH Publishers Inc., 1989).

Compounds useful with guanine-responsive riboswitches (and riboswitches derived from guanine-responsive riboswitches) include compounds represented by Formula I 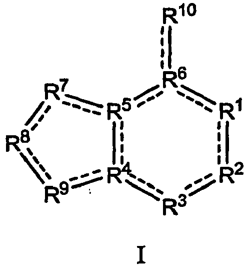 where the compound can bind a guanine-responsive riboswitch or derivative thereof, where, when the compound is bound to a guanine-responsive riboswitch or derivative, R¹ and R² serve as a hydrogen bond donor, R⁷ serves as a hydrogen bond acceptor, R⁹ serves as a hydrogen bond donor, R¹⁰ serves as a hydrogen bond acceptor, and where--- each independently represent a single or double bond. In some other examples, R³ can be a hydrogen bond acceptor.

It is to be understood that while a particular moiety or group can be referred to herein as a hydrogen bond donor or acceptor, this terminology is used to merely categorize the various substituents for ease of reference. Such language should not be interpreted to mean that a particular moiety actually participates in hydrogen bonding with the riboswitch or some other compound. It is possible that, for example, a moiety referred to herein as a hydrogen bond acceptor (or donor) could solely or additionally be involved in hydrophobic, ionic, van de Waals, or other type of interaction with the riboswitch or other compound.

It is also understood that certain groups disclosed herein can be referred to herein as both a hydrogen bond acceptor and a hydrogen bond donor. For example, -OH can be a hydrogen bond donor by donating the hydrogen atom; -OH can also be a hydrogen bond acceptor through one or more of the nonbonded electron pairs on the oxygen atom. Thus, throughout the specification various moieties can be a hydrogen bond donor and acceptor and can be referred to as such.

Suitable hydrogen bond donors that can be present in the disclosed compounds, for example as one or more of R¹, R², and R⁹, are moieties that contain a polar hydrogen bond, such as when a hydrogen atom is bonded to a more electronegative atom like C, N, O, or S. Examples of suitable hydrogen bond donors for R¹, R², and R⁹ include, but are not limited to, the following moieties: -NR¹¹-, -CHR¹¹-, =CR¹¹-, and -C(=NR¹¹)-, where R¹¹ is -H, -NH₂, -OH, -SH, -CO₂H substituted or unsubstituted alkyl, alkoxy, aryl, aryloxy, or benzyloxy, -NHalkyl, -NHalkoxy, -NHC(O)alkyl, -NHCO₂alkyl, - NHC(O)NH₂, -NH-NH₂, -NH-NHalkyl, -NH-NHalkoxy, -NH-SO₂alkyl, -NH-SO₂-R¹²,-NHCO₂CH₂-R¹², -NH-OR¹², -N⁺H₂-R¹², -NH-NH-R¹², and -NH-NH-CH₂-R¹², where R¹² can be: 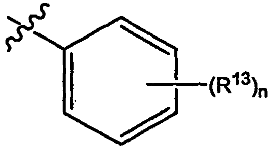 where n is from 1 to 5, and R¹³ can be one or more of -H, -NH₂, -OH, alkoxy, *-N-*morpholino, or halide.

Suitable hydrogen bond acceptors that can be present in the disclosed compounds, for example as one or more of R³, R⁷, and R¹⁰, are moieties that contain a nonbonded electron pair. Nonbonded electron pairs typically exist on N, O, S, and halogen atoms. Examples of suitable hydrogen bond acceptors for R³, R⁷ include, but are not limited to, N, O, S, and SO₂. For example, the groups R⁸ and R⁷ taken together can be represented as -CH=N-, -CH₂-O-, -CH₂-S-, or -CH₂SO₂-. R³ can be N, O, or S, which when referring to Formula I results in the moieties -N=R², -O-R², and -S-R², respectively, where R² is as defined above.

For the moiety R¹⁰, a suitable hydrogen bond acceptor can be O or S, as in O=R⁶ or S=R⁶, where R⁶ is C. Still further examples of hydrogen bond acceptors for R¹⁰ include, but are not limited to, -OH, -SH, -NH₂, -CO₂H, -alkoxy, -aryloxy, -benzyloxy, - halide, -NHalkyl, -NHalkoxy, -NHC(O)alkyl, -NHCO₂alkyl, -NHCO₂CH₂-R¹²,-NHC(O)NH₂, -NH-NH₂, -NH-NHalkyl, -NH-NHalkoxy, -SO₂alkyl, -SO₂aryl, -NH-SO₂alkyl, -NH-SO₂-R¹², -NH-OR¹², -NH-R¹², -NH-NH-R¹², -NH-NH-CH₂-R¹², or -NH-CH₂-R¹², where R¹² is as defined above.

Yet another example of a suitable hydrogen bond acceptor for R¹⁰ is NR¹⁴, as in R¹⁴N=R⁶, where R⁶ is C. In these examples, R¹⁴ can be -H, NH₂, -OH, -SH, -CO₂H, - CO₂alkyl, -CO₂aryl, -C(O)NH₂, substituted or unsubstituted alkyl, alkoxy, alkoxy, aryloxy, or benzyloxy, -NHalkyl, -NHalkoxy, -NHC(O)alkyl, -NHCO₂alkyl, - NHC(O)NH₂, -SO₂alkyl, -SO₂aryl, -NH-SO₂alkyl, -NH-SO₂-R¹², -NH-OR¹², -NH-R¹², or NH-CH2-R¹², where R¹² is as defined above.

Some specific compounds disclosed herein can be represented by Formula II: 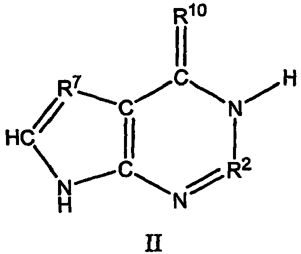 where R⁷ is N or CH;
R¹⁰ is =O, =S, =NH, =NOH, =Nalkyl, Nalkoxyl, =N-aryl, =Naryloxy, =N-benzyl, =Nbenzyloxy, =N-NH₂, =N-NHOH, =N-NHalkyl, =N-NHalkoxy, =N-NHaryl, =N-NHaryloxy, =N-NHbenzyl, =N-NHbenzyloxy, =N-NH-(p-amino-phenyl), =N-NH-(p-methoxyphenyl), =N-NH-(*p-N*-morpholino-phenyl); and
R² is =CR¹⁵-, where R¹⁵ is -NH₂, -NHNH₂, -NHOH, -NHalkyl, -NHalkoxy, - NHaryl, -NHaryloxy, -NHbenzyl, -NHbenzyloxy, -N⁺H₂aryl, -N⁺H2-(*p-N*-morpholinophenyl), -N⁺H₂-(*p*-aminophenyl), -N⁺H₂-(*p*-methoxyphenyl), -NHCO₂alkyl, - NHCO₂benzyl, -NHNHalkyl, -NHNHaryl, -NHNHbenzyl, or NHC(O)alkyl. Additional compounds can be represented by Formula III: 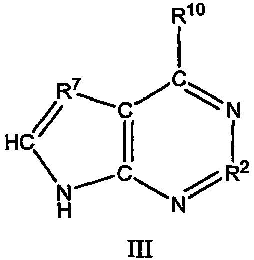 where R¹⁰ is -H, -OH, -SH, -alkoxy, halide, -NH₂, -NHOH, -NHalkyl, -NHalkoxy, - NHaryl, -NHaryloxy, -NHbenzyl, -NHbenzyoxy, -NHC(O)alkyl, -NHCO₂alkyl, NHCO₂benzyl, -NHNH₂, -NHNHalkyl, -NHNHaryl, or -NHNHbenzyl; and R² and R⁷ are as defined above.

Further specific examples of compounds are illustrated in Figure 12B.

Additional compounds useful with guanine-responsive riboswitches (and riboswitches derived from guanine-responsive riboswitches) include compounds having the formula 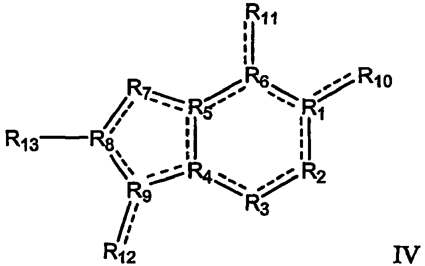 where the compound can bind a guanine-responsive riboswitch or derivative thereof, where, when the compound is bound to a guanine-responsive riboswitch or derivative, R₇ serves as a hydrogen bond acceptor, R₁₀ serves as a hydrogen bond donor, R₁₁ serves as a hydrogen bond acceptor, R₁₂ serves as a hydrogen bond donor, where R₁₃ is H, H₂ or is not present, where R₁, R₂, R₃, R₄, R₅, R₆, R₈, and R₉ are each independently C, N, O, or S, and where 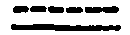 each independently represent a single or double bond.

Every compound within the above definition is intended to be and should be considered to be specifically disclosed herein. Further, every subgroup that can be identified within the above definition is intended to be and should be considered to be specifically disclosed herein. As a result, it is specifically contemplated that any compound, or subgroup of compounds can be either specifically included for or excluded from use or included in or excluded from a list of compounds. For example, as one option, a group of compounds is contemplated where each compound is as defined above but is not guanine, hypoxanthine, xanthine, or N²-methylguanine, As another example, a group of compounds is contemplated where each compound is as defined above and is able to activate a guanine-responsive riboswitch.

It should be understood that particular contacts and interactions (such as hydrogen bond donation or acceptance) described herein for compounds interacting with riboswitches are preferred but are not essential for interaction of a compound with a riboswitch. For example, compounds can interact with riboswitches with less affinity and/or specificity than compounds having the disclosed contacts and interactions. Further, different or additional functional groups on the compounds can introduce new, different and/or compensating contacts with the riboswitches. For example, for guanine riboswitches, large functional groups can be used at, for example, R¹⁰ and R². Such functional groups can have, and can be designed to have, contacts and interactions with other part of the riboswitch. Such contacts and interactions can compensate for contacts and interactions of the trigger molecules and core structure.

Compounds useful with adenine-responsive riboswitches (and riboswitches derived from adenine-responsive riboswitches) include compounds having the formula 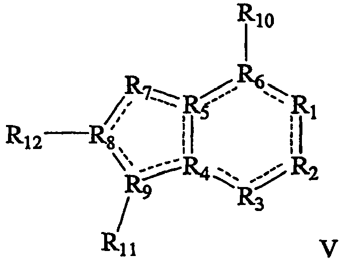 where the compound can bind an adenine-responsive riboswitch or derivative thereof, where, when the compound is bound to an adenine-responsive riboswitch or derivative, R₁, R₃ and R₇ serve as hydrogen bond acceptors, and R₁₀ and R₁₁ serve as hydrogen bond donors, where R₁₂ is H, H₂ or is not present, where R₁, R₂, R₃, R₄, R₅, R₆, R₈, and R₉ are each independently C, N, O, or S, and where 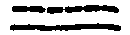 each independently represent a single or double bond.

Every compound within the above definition is intended to be and should be considered to be specifically disclosed herein. Further, every subgroup that can be identified within the above definition is intended to be and should be considered to be specifically disclosed herein. As a result, it is specifically contemplated that any compound, or subgroup of compounds can be either specifically included for or excluded from use or included in or excluded from a list of compounds. For example, as one option, a group of compounds is contemplated where each compound is as defined above but is not adenine, 2,6-diaminopurine, or 2-amino purine. As another example, a group of compounds is contemplated where each compound is as defined above and is able to activate an adenine-responsive riboswitch.

Compounds useful with lysine-responsive riboswitches (and riboswitches derived from lysine-responsive riboswitches) include compounds having the formula 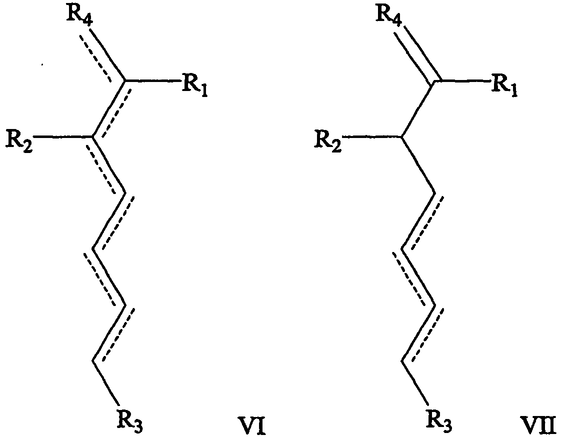 where the compound can bind a lysine-responsive riboswitch or derivative thereof, where R₂ and R₃ are each positively charged, where R₁ is negatively charged, where R₄ is C, N, O, or S, and where 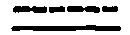 each independently represent a single or double bond.
Also contemplated are compounds as defined above where R₂ and R₃ are each NH₃⁺ and where R₁ is O⁻.

Every compound within the above definition is intended to be and should be considered to be specifically disclosed herein. Further, every subgroup that can be identified within the above definition is intended to be and should be considered to be specifically disclosed herein. As a result, it is specifically contemplated that any compound, or subgroup of compounds can be either specifically included for or excluded from use or included in or excluded from a list of compounds. For example, as one option, a group of compounds is contemplated where each compound is as defined above but is not lysine. As another example, a group of compounds is contemplated where each compound is as defined above and is able to activate a lysine-responsive riboswitch.

Compounds useful with TPP-responsive riboswitches (and riboswitches derived from lysine-responsive riboswitches) include compounds having the formula 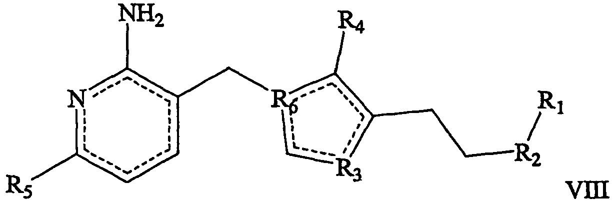 where the compound can bind a TPP-responsive riboswitch or derivative thereof, where R₁ is positively charged, where R₂ and R₃ are each independently C, O, or S, where R₄ is CH₃, NH₂, OH, SH, H or not present, where R₅ is CH₃, NH₂, OH, SH, or H, where R₆ is C or N, and where 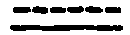 each independently represent a single or double bond. Also contemplated are compounds as defined above where R₁ is phosphate, diphosphate or triphosphate.

Every compound within the above definition is intended to be and should be considered to be specifically disclosed herein. Further, every subgroup that can be identified within the above definition is intended to be and should be considered to be specifically disclosed herein. As a result, it is specifically contemplated that any compound, or subgroup of compounds can be either specifically included for or excluded from use or included in or excluded from a list of compounds. For example, as one option, a group of compounds is contemplated where each compound is as defined above but is not TPP, TP or thiamine. As another example, a group of compounds is contemplated where each compound is as defined above and is able to activate a TPP-responsive riboswitch.

### D. Constructs, Vectors and Expression Systems

The disclosed riboswitches can be used in with any suitable expression system. Recombinant expression is usefully accomplished using a vector, such as a plasmid. The vector can include a promoter operably linked to riboswitch-encoding sequence and RNA to be expression (e.g., RNA encoding a protein). The vector can also include other elements required for transcription and translation. As used herein, vector refers to any carrier containing exogenous DNA. Thus, vectors are agents that transport the exogenous nucleic acid into a cell without degradation and include a promoter yielding expression of the nucleic acid in the cells into which it is delivered. Vectors include but are not limited to plasmids, viral nucleic acids, viruses, phage nucleic acids, phages, cosmids, and artificial chromosomes. A variety of prokaryotic and eukaryotic expression vectors suitable for carrying riboswitch-regulated constructs can be produced. Such expression vectors include, for example, pET, pET3d, pCR2.1, pBAD, pUC, and yeast vectors. The vectors can be used, for example, in a variety of *in vivo* and in *vitro* situation.

Viral vectors include adenovirus, adeno-associated virus, herpes virus, vaccinia virus, polio virus, AIDS virus, neuronal trophic virus, Sindbis and other RNA viruses, including these viruses with the HIV backbone. Also useful are any viral families which share the properties of these viruses which make them suitable for use as vectors. Retroviral vectors, which are described in Verma (1985), include Murine Maloney Leukemia virus, MMLV, and retroviruses that express the desirable properties of MMLV as a vector. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA.

A "promoter" is generally a sequence or sequences of DNA that function when in a relatively fixed location in regard to the transcription start site. A "promoter" contains core elements required for basic interaction of RNA polymerase and transcription factors and can contain upstream elements and response elements.

"Enhancer" generally refers to a sequence of DNA that functions at no fixed distance from the transcription start site and can be either 5' (Laimins, 1981) or 3' (Lusky et al., 1983) to the transcription unit. Furthermore, enhancers can be within an intron (Banerji et al., 1983) as well as within the coding sequence itself (Osborne et al., 1984). They are usually between 10 and 300 bp in length, and they function in cis. Enhancers function to increase transcription from nearby promoters. Enhancers, like promoters, also often contain response elements that mediate the regulation of transcription. Enhancers often determine the regulation of expression.

Expression vectors used in eukaryotic host cells (yeast, fungi, insect, plant, animal, human or nucleated cells) can also contain sequences necessary for the termination of transcription which can affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs.

The vector can include nucleic acid sequence encoding a marker product. This marker product is used to determine if the gene has been delivered to the cell and once delivered is being expressed. Preferred marker genes are the *E. Coli* lacZ gene which encodes *β*-galactosidase and green fluorescent protein.

The marker can be a selectable marker. When such selectable markers are successfully transferred into a host cell, the transformed host cell can survive if placed under selective pressure. There are two widely used distinct categories of selective regimes. The first category is based on a cell's metabolism and the use of a mutant cell line which lacks the ability to grow independent of a supplemented media. The second category is dominant selection which refers to a selection scheme used in any cell type and does not require the use of a mutant cell line. These schemes typically use a drug to arrest growth of a host cell. Those cells which have a novel gene would express a protein conveying drug resistance and would survive the selection. Examples of such dominant selection use the drugs neomycin, (Southern and Berg, 1982), mycophenolic acid, (Mulligan and Berg, 1980) or hygromycin (Sugden et al., 1985).

Gene transfer can be obtained using direct transfer of genetic material, in but not limited to, plasmids, viral vectors, viral nucleic acids, phage nucleic acids, phages, cosmids, and artificial chromosomes, or via transfer of genetic material in cells or carriers such as cationic liposomes. Such methods are well known in the art and readily adaptable for use in the method described herein. Transfer vectors can be any nucleotide construction used to deliver genes into cells (e.g., a plasmid), or as part of a general strategy to deliver genes, e.g., as part of recombinant retrovirus or adenovirus (Ram et al. Cancer Res. 53:83-88, (1993)). Appropriate means for transfection, including viral vectors, chemical transfectants, or physico-mechanical methods such as electroporation and direct diffusion of DNA, are described by, for example, Wolff, J. A., et al., Science, 247, 1465-1468, (1990); and Wolff, J. A. Nature, 352, 815-818, (1991).

### 1. Viral Vectors

Preferred viral vectors are Adenovirus, Adeno-associated virus, Herpes virus, Vaccinia virus, Polio virus, AIDS virus, neuronal trophic virus, Sindbis and other RNA viruses, including these viruses with the HIV backbone. Also preferred are any viral families which share the properties of these viruses which make them suitable for use as vectors. Preferred retroviruses include Murine Maloney Leukemia virus, MMLV, and retroviruses that express the desirable properties of MMLV as a vector. Retroviral vectors are able to carry a larger genetic payload, i.e., a transgene or marker gene, than other viral vectors, and for this reason are a commonly used vector. However, they are not useful in non-proliferating cells. Adenovirus vectors are relatively stable and easy to work with, have high titers, and can be delivered in aerosol formulation, and can transfect non-dividing cells. Pox viral vectors are large and have several sites for inserting genes, they are thermostable and can be stored at room temperature. Disclosed is a viral vector which has been engineered so as to suppress the immune response of the host organism, elicited by the viral antigens. Preferred vectors of this type will carry coding regions for Interleukin 8 or 10.

Viral vectors have higher transaction (ability to introduce genes) abilities than do most chemical or physical methods to introduce genes into cells. Typically, viral vectors contain, nonstructural early genes, structural late genes, an RNA polymerase III transcript, inverted terminal repeats necessary for replication and encapsidation, and promoters to control the transcription and replication of the viral genome. When engineered as vectors, viruses typically have one or more of the early genes removed and a gene or gene/promoter cassette is inserted into the viral genome in place of the removed viral DNA. Constructs of this type can carry up to about 8 kb of foreign genetic material. The necessary functions of the removed early genes are typically supplied by cell lines which have been engineered to express the gene products of the early genes in trans.

### i. Retroviral Vectors

A retrovirus is an animal virus belonging to the virus family of Retroviridae, including any types, subfamilies, genus, or tropisms. Retroviral vectors, in general, are described by Verma, I.M., Retroviral vectors for gene transfer. In Microbiology-1985, American Society for Microbiology, pp. 229-232, Washington, (1985) Examples of methods for using retroviral vectors for gene therapy are described in U.S. Patent Nos. 4,868,116 and 4,980,286; PCT applications WO 90/02806 and WO 89/07136; and Mulligan, (Science 260:926-932 (1993)).

A retrovirus is essentially a package which has packed into it nucleic acid cargo. The nucleic acid cargo carries with it a packaging signal, which ensures that the replicated daughter molecules will be efficiently packaged within the package coat. In addition to the package signal, there are a number of molecules which are needed in cis, for the replication, and packaging of the replicated virus. Typically a retroviral genome, contains the gag, pol, and env genes which are involved in the making of the protein coat. It is the gag, pol, and env genes which are typically replaced by the foreign DNA that it is to be transferred to the target cell. Retrovirus vectors typically contain a packaging signal for incorporation into the package coat, a sequence which signals the start of the gag transcription unit, elements necessary for reverse transcription, including a primer binding site to bind the tRNA primer of reverse transcription, terminal repeat sequences that guide the switch of RNA strands during DNA synthesis, a purine rich sequence 5' to the 3' LTR that serve as the priming site for the synthesis of the second strand of DNA synthesis, and specific sequences near the ends of the LTRs that enable the insertion of the DNA state of the retrovirus to insert into the host genome. The removal of the gag, pol, and env genes allows for about 8 kb of foreign sequence to be inserted into the viral genome, become reverse transcribed , and upon replication be packaged into a new retroviral particle. This amount of nucleic acid is sufficient for the delivery of a one to many genes depending on the size of each transcript. It is preferable to include either positive or negative selectable markers along with other genes in the insert.

Since the replication machinery and packaging proteins in most retroviral vectors have been removed (gag, pol, and env), the vectors are typically generated by placing them into a packaging cell line. A packaging cell line is a cell line which has been transfected or transformed with a retrovirus that contains the replication and packaging machinery, but lacks any packaging signal. When the vector carrying the DNA of choice is transfected into these cell lines, the vector containing the gene of interest is replicated and packaged into new retroviral particles, by the machinery provided in cis by the helper cell. The genomes for the machinery are not packaged because they lack the necessary signals.

### ii. Adenoviral Vectors

The construction of replication-defective adenoviruses has been described (Berkner et al., J. Virology 61:1213-1220 (1987); Massie et al., Mol. Cell. Biol. 6:2872-2883 (1986); Haj-Ahmad et al., J. Virology 57:267-274 (1986); Davidson et al., J. Virology 61:1226-1239 (1987); Zhang "Generation and identification of recombinant adenovirus by liposome-mediated transfection and PCR analysis" BioTechniques 15:868-872 (1993)). The benefit of the use of these viruses as vectors is that they are limited in the extent to which they can spread to other cell types, since they can replicate within an initial infected cell, but are unable to form new infectious viral particles. Recombinant adenoviruses have been shown to achieve high efficiency gene transfer after direct, *in vivo* delivery to airway epithelium, hepatocytes, vascular endothelium, CNS parenchyma and a number of other tissue sites (Morsy, J. Clin. Invest. 92:1580-1586 (1993); Kirshenbaum, J. Clin. Invest. 92:381-387 (1993); Roessler, J. Clin. Invest. 92:1085-1092 (1993); Moullier, Nature Genetics 4:154-159 (1993); La Salle, Science 259:988-990 (1993); Gomez-Foix, J. Biol. Chem. 267:25129-25134 (1992); Rich, Human Gene Therapy 4:461-476 (1993); Zabner, Nature Genetics 6:75-83 (1994); Guzman, Circulation Research 73:1201-1207 (1993); Bout, Human Gene Therapy 5:3-10 (1994); Zabner, Cell 75:207-216 (1993); Caillaud, Eur. J. Neuroscience 5:1287-1291 (1993); and Ragot, J. Gen. Virology 74:501-507 (1993)). Recombinant adenoviruses achieve gene transduction by binding to specific cell surface receptors, after which the virus is internalized by receptor-mediated endocytosis, in the same manner as wild type or replication-defective adenovirus (Chardonnet and Dales, Virology 40:462-477 (1970); Brown and Burlingham, J. Virology 12:386-396 (1973); Svensson and Persson, J. Virology 55:442-449 (1985); Seth, et al., J. Virol. 51:650-655 (1984); Seth, et al., Mol. Cell. Biol. 4:1528-1533 (1984); Varga et al., J. Virology 65:6061-6070 (1991); Wickham et al., Cell 73:309-319 (1993)).

A preferred viral vector is one based on an adenovirus which has had the E1 gene removed and these virons are generated in a cell line such as the human 293 cell line. In another aspect both the E1 and E3 genes are removed from the adenovirus genome.

Another type of viral vector is based on an adeno-associated virus (AAV). This defective parvovirus is a preferred vector because it can infect many cell types and is nonpathogenic to humans. AAV type vectors can transport about 4 to 5 kb and wild type AAV is known to stably insert into chromosome 19. Vectors which contain this site specific integration property are preferred. An example of this type of vector is the P4.1 C vector produced by Avigen, San Francisco, CA, which can contain the herpes simplex virus thymidine kinase gene, HSV-tk, and/or a marker gene, such as the gene encoding the green fluorescent protein, GFP.

The inserted genes in viral and retroviral usually contain promoters, and/or enhancers to help control the expression of the desired gene product. A promoter is generally a sequence or sequences of DNA that function when in a relatively fixed location in regard to the transcription start site. A promoter contains core elements required for basic interaction of RNA polymerase and transcription factors, and can contain upstream elements and response elements.

### 2. Viral Promoters and Enhancers

Preferred promoters controlling transcription from vectors in mammalian host cells can be obtained from various sources, for example, the genomes of viruses such as: polyoma, Simian Virus 40 (SV40), adenovirus, retroviruses, hepatitis-B virus and most preferably cytomegalovirus, or from heterologous mammalian promoters, e.g. beta actin promoter. The early and late promoters of the SV40 virus are conveniently obtained as an SV40 restriction fragment which also contains the SV40 viral origin of replication (Fiers et al., Nature, 273: 113 (1978)). The immediate early promoter of the human cytomegalovirus is conveniently obtained as a HindIII E restriction fragment (Greenway, P.J. et al., Gene 18: 355-360 (1982)). Of course, promoters from the host cell or related species also are useful herein.

Enhancer generally refers to a sequence of DNA that functions at no fixed distance from the transcription start site and can be either 5' (Laimins, L. et al., Proc. Natl. Acad. Sci. 78: 993 (1981)) or 3' (Lusky, M.L., et al., Mol. Cell Bio. 3: 1108 (1983)) to the transcription unit. Furthermore, enhancers can be within an intron (Banerji, J.L. et al., Cell 33: 729 (1983)) as well as within the coding sequence itself (Osborne, T.F., et al., Mol. Cell Bio. 4: 1293 (1984)). They are usually between 10 and 300 bp in length, and they function in cis. Enhancers function to increase transcription from nearby promoters. Enhancers also often contain response elements that mediate the regulation of transcription. Promoters can also contain response elements that mediate the regulation of transcription. Enhancers often determine the regulation of expression of a gene. While many enhancer sequences are now known from mammalian genes (globin, elastase, albumin, α-fetoprotein and insulin), typically one will use an enhancer from a eukaryotic cell virus. Preferred examples are the SV40 enhancer on the late side of the replication origin (bp 100-270), the cytomegalovirus early promoter enhancer, the polyoma enhancer on the late side of the replication origin, and adenovirus enhancers.

The promoter and/or enhancer can be specifically activated either by light or specific chemical events which trigger their function. Systems can be regulated by reagents such as tetracycline and dexamethasone. There are also ways to enhance viral vector gene expression by exposure to irradiation, such as gamma irradiation, or alkylating chemotherapy drugs.

It is preferred that the promoter and/or enhancer region be active in all eukaryotic cell types. A preferred promoter of this type is the CMV promoter (650 bases). Other preferred promoters are SV40 promoters, cytomegalovirus (full length promoter), and retroviral vector LTF.

It has been shown that all specific regulatory elements can be cloned and used to construct expression vectors that are selectively expressed in specific cell types such as melanoma cells. The glial fibrillary acetic protein (GFAP) promoter has been used to selectively express genes in cells of glial origin.

Expression vectors used in eukaryotic host cells (yeast, fungi, insect, plant, animal, human or nucleated cells) can also contain sequences necessary for the termination of transcription which can affect mRNA expression. These regions are transcribed as polyadenylated segments in the untranslated portion of the mRNA encoding tissue factor protein. The 3' untranslated regions also include transcription termination sites. It is preferred that the transcription unit also contain a polyadenylation region. One benefit of this region is that it increases the likelihood that the transcribed unit will be processed and transported like mRNA. The identification and use of polyadenylation signals in expression constructs is well established. It is preferred that homologous polyadenylation signals be used in the transgene constructs. In an example of the transcription unit, the polyadenylation region is derived from the SV40 early polyadenylation signal and consists of about 400 bases. It is also preferred that the transcribed units contain other standard sequences alone or in combination with the above sequences improve expression from, or stability of, the construct.

### 3. Markers

The vectors can include nucleic acid sequence encoding a marker product. This marker product is used to determine if the gene has been delivered to the cell and once delivered is being expressed. Preferred marker genes are the E. Coli lacZ gene which encodes β-galactosidase and green fluorescent protein.

The marker can be a selectable marker. Examples of suitable selectable markers for mammalian cells are dihydrofolate reductase (DHFR), thymidine kinase, neomycin, neomycin analog G418, hydromycin, and puromycin. When such selectable markers are successfully transferred into a mammalian host cell, the transformed mammalian host cell can survive if placed under selective pressure. There are two widely used distinct categories of selective regimes. The first category is based on a cell's metabolism and the use of a mutant cell line which lacks the ability to grow independent of a supplemented media. Two examples are: CHO DHFR⁻ cells and mouse LTK⁻ cells. These cells lack the ability to grow without the addition of such nutrients as thymidine or hypoxanthine. Because these cells lack certain genes necessary for a complete nucleotide synthesis pathway, they cannot survive unless the missing nucleotides are provided in a supplemented media. An alternative to supplementing the media is to introduce an intact DHFR or TK gene into cells lacking the respective genes, thus altering their growth requirements. Individual cells which were not transformed with the DHFR or TK gene will not be capable of survival in non-supplemented media.

The second category is dominant selection which refers to a selection scheme used in any cell type and does not require the use of a mutant cell line. These schemes typically use a drug to arrest growth of a host cell. Those cells which would express a protein conveying drug resistance and would survive the selection. Examples of such dominant selection use the drugs neomycin, (Southern P. and Berg, P., J. Molec. Appl. Genet. 1: 327 (1982)), mycophenolic acid, (Mulligan, R.C. and Berg, P. Science 209: 1422 (1980)) or hygromycin, (Sugden, B. et al., Mol. Cell. Biol. 5: 410-413 (1985)). The three examples employ bacterial genes under eukaryotic control to convey resistance to the appropriate drug G418 or neomycin (geneticin), xgpt (mycophenolic acid) or hygromycin, respectively. Others include the neomycin analog G418 and puramycin.

### E. Biosensor Riboswitches

Also disclosed are biosensor riboswitches. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or *in vitro.* For example, biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch.

### F. Reporter Proteins and Peptides

For assessing activation of a riboswitch, or for biosensor riboswitches, a reporter protein or peptide can be used. The reporter protein or peptide can be encoded by the RNA the expression of which is regulated by the riboswitch. The examples describe the use of some specific reporter proteins. The use of reporter proteins and peptides is well known and can be adapted easily for use with riboswitches. The reporter proteins can be any protein or peptide that can be detected or that produces a detectable signal. Preferably, the presence of the protein or peptide can be detected using standard techniques (e.g., radioimmunoassay, radio-labeling, immunoassay, assay for enzymatic activity, absorbance, fluorescence, luminescence, and Western blot). More preferably, the level of the reporter protein is easily quantifiable using standard techniques even at low levels. Useful reporter proteins include luciferases, green fluorescent proteins and their derivatives, such as firefly luciferase (FL) from Photinus pyralis, and Renilla luciferase (RL) from Renilla reniformis.

### G. Conformation Dependent Labels

Conformation dependent labels refer to all labels that produce a change in fluorescence intensity or wavelength based on a change in the form or conformation of the molecule or compound (such as a riboswitch) with which the label is associated. Examples of conformation dependent labels used in the context of probes and primers include molecular beacons, Amplifluors, FRET probes, cleavable FRET probes, TaqMan probes, scorpion primers, fluorescent triplex oligos including but not limited to triplex molecular beacons or triplex FRET probes, fluorescent water-soluble conjugated polymers, PNA probes and QPNA probes. Such labels, and, in particular, the principles of their function, can be adapted for use with riboswitches. Several types of conformation dependent labels are reviewed in Schweitzer and Kingsmore, Curr. Opin. Biotech. 12:21-27 (2001).

Stem quenched labels, a form of conformation dependent labels, are fluorescent labels positioned on a nucleic acid such that when a stem structure forms a quenching moiety is brought into proximity such that fluorescence from the label is quenched. When the stem is disrupted (such as when a riboswitch containing the label is activated), the quenching moiety is no longer in proximity to the fluorescent label and fluorescence increases. Examples of this effect can be found in molecular beacons, fluorescent triplex oligos, triplex molecular beacons, triplex FRET probes, and QPNA probes, the operational principles of which can be adapted for use with ritboswitches.

Stem activated labels, a form of conformation dependent labels, are labels or pairs of labels where fluorescence is increased or altered by formation of a stem structures. Stem activated labels can include an acceptor fluorescent label and a donor moiety such that, when the acceptor and donor are in proximity (when the nucleic acid strands containing the labels form a stem structure), fluorescence resonance energy transfer from the donor to the acceptor causes the acceptor to fluoresce. Stem activated labels are typically pairs of labels positioned on nucleic acid molecules (such as riboswitches) such that the acceptor and donor are brought into proximity when a stem structure is formed in the nucleic acid molecule. If the donor moiety of a stem activated label is itself a fluorescent label, it can release energy as fluorescence (typically at a different wavelength than the fluorescence of the acceptor) when not in proximity to an acceptor (that is, when a stem structure is not formed). When the stem structure forms, the overall effect would then be a reduction of donor fluorescence and an increase in acceptor fluorescence. FRET probes are an example of the use of stem activated labels, the operational principles of which can be adapted for use with riboswitches.

### H. Detection Labels

To aid in detection and quantitation of riboswitch activation, deactivation or blocking, or expression of nucleic acids or protein produced upon activation, deactivation or blocking of riboswitches, detection labels can be incorporated into detection probes or detection molecules or directly incorporated into expressed nucleic acids or proteins. As used herein, a detection label is any molecule that can be associated with nucleic acid or protein, directly or indirectly, and which results in a measurable, detectable signal, either directly or indirectly. Many such labels are known to those of skill in the art. Examples of detection labels suitable for use in the disclosed method are radioactive isotopes, fluorescent molecules, phosphorescent molecules, enzymes, antibodies, and ligands.

Examples of suitable fluorescent labels include fluorescein isothiocyanate (FITC), 5,6-carboxymethyl fluorescein, Texas red, nitrobenz-2-oxa-1,3-diazol-4-yl (NBD), coumarin, dansyl chloride, rhodamine, amino-methyl coumarin (AMCA), Eosin, Erythrosin, BODIPY^{®}, Cascade Blue^{®}, Oregon Green^{®}, pyrene, lissamine, xanthenes, acridines, oxazines, phycoerythrin, macrocyclic chelates of lanthanide ions such as quantum dye™, fluorescent energy transfer dyes, such as thiazole orange-ethidium heterodimer, and the cyanine dyes Cy3, Cy3.5, Cy5, Cy5.5 and Cy7. Examples of other specific fluorescent labels include 3-Hydroxypyrene 5,8,10-Tri Sulfonic acid, 5-Hydroxy Tryptamine (5-HT), Acid Fuchsin, Alizarin Complexon, Alizarin Red, Allophycocyanin, Aminocoumarin, Anthroyl Stearate, Astrazon Brilliant Red 4G, Astrazon Orange R, Astrazon Red 6B, Astrazon Yellow 7 GLL, Atabrine, Auramine, Aurophosphine, Aurophosphine G, BAO 9 (Bisaminophenyloxadiazole), BCECF, Berberine Sulphate, Bisbenzamide, Blancophor FFG Solution, Blancophor SV, Bodipy F1, Brilliant Sulphoflavin FF, Calcien Blue, Calcium Green, Calcofluor RW Solution, Calcofluor White, Calcophor White ABT Solution, Calcophor White Standard Solution, Carbostyryl, Cascade Yellow, Catecholamine, Chinacrine, Coriphosphine O, Coumarin-Phalloidin, CY3.18, CY5.1 8, CY7, Dans (1-Dimethyl Amino Naphaline 5 Sulphonic Acid), Dansa (Diamino Naphtyl Sulphonic Acid), Dansyl NH-CH3, Diamino Phenyl Oxydiazole (DAO), Dimethylamino-5-Sulphonic acid, Dipyrrometheneboron Difluoride, Diphenyl Brilliant Flavine 7GFF, Dopamine, Erythrosin ITC, Euchrysin, FIF (Formaldehyde Induced Fluorescence), Flazo Orange, Fluo 3, Fluorescamine, Fura-2, Genacryl Brilliant Red B, Genacryl Brilliant Yellow 10GF, Genacryl Pink 3G, Genacryl Yellow 5GF, Gloxalic Acid, Granular Blue, Haematoporphyrin, Indo-1, Intrawhite Cf Liquid, Leucophor PAF, Leucophor SF, Leucophor WS, Lissamine Rhodamine B200 (RD200), Lucifer Yellow CH, Lucifer Yellow VS, Magdala Red, Marina Blue, Maxilon Brilliant Flavin 10 GFF, Maxilon Brilliant Flavin 8 GFF, MPS (Methyl Green Pyronine Stilbene), Mithramycin, NBD Amine, Nitrobenzoxadidole, Noradrenaline, Nuclear Fast Red, Nuclear Yellow, Nylosan Brilliant Flavin E8G, Oxadiazole, Pacific Blue, Pararosaniline (Feulgen), Phorwite AR Solution, Phorwite BKL, Phorwite Rev, Phorwite RPA, Phosphine 3R, Phthalocyanine, Phycoerythrin R, Polyazaindacene Pontochrome Blue Black, Porphyrin, Primuline, Procion Yellow, Pyronine, Pyronine B, Pyrozal Brilliant Flavin 7GF, Quinacrine Mustard, Rhodamine 123, Rhodamine 5 GLD, Rhodamine 6G, Rhodamine B, Rhodamine B 200, Rhodamine B Extra, Rhodamine BB, Rhodamine BG, Rhodamine WT, Serotonin, Sevron Brilliant Red 2B, Sevron Brilliant Red 4G, Sevron Brilliant Red B, Sevron Orange, Sevron Yellow L, SITS (Primuline), SITS (Stilbene Isothiosulphonic acid), Stilbene, Snarf 1, sulpho Rhodamine B Can C, Sulpho Rhodamine G Extra, Tetracycline, Thiazine Red R, Thioflavin S, Thioflavin TCN, Thioflavin 5, Thiolyte, Thiozol Orange, Tinopol CBS, True Blue, Ultralite, Uranine B, Uvitex SFC, Xylene Orange, and XRITC.

Useful fluorescent labels are fluorescein (5-carboxyfluorescein-N-hydroxysuccinimide ester), rhodamine (5,6-tetramethyl rhodamine), and the cyanine dyes Cy3, Cry3.5, Cry5, Cry5.5 and Cy7. The absorption and emission maxima, respectively, for these fluors are: FITC (490 nm; 520 nm), Cy3 (554 nm; 568 nm), Cry3.5 (581 nm; 588 nm), Cy5 (652 nm: 672 nm), Cry5.5 (682 nm; 703 nm) and Cy7 (755 nm; 778 nm), thus allowing their simultaneous detection. Other examples of fluorescein dyes include 6-carboxyfluorescein (6-FAM), 2',4',1,4,-tetrachlorofluorescein (TET), 2',4',5',7',1,4-hexachlorofluorescein (HEX), 2',7'-dimethoxy-4',5'-dichloro-6-carboxyrhodamine (JOE), 2'-chloro-5'-fluoro-7',8'-fused phenyl-1,4-dichloro-6-carboxyfluorescein (NED), and 2'-chloro-7'-phenyl-1,4-dichloro-6-carboxyffuorescein (VIC). Fluorescent labels can be obtained from a variety of commercial sources, including Amersham Pharmacia Biotech, Piscataway, NJ; Molecular Probes, Eugene, OR; and Research Organics, Cleveland, Ohio.

Additional labels of interest include those that provide for signal only when the probe with which they are associated is specifically bound to a target molecule, where such labels include: "molecular beacons" as described in Tyagi & Kramer, Nature Biotechnology (1996) 14:303 and EP 0 070 685 B1. Other labels of interest include those described in U.S. Pat. No. 5,563,037; WO 97/17471 and WO 97/17076.

Labeled nucleotides are a useful form of detection label for direct incorporation into expressed nucleic acids during synthesis. Examples of detection labels that can be incorporated into nucleic acids include nucleotide analogs such as BrdUrd (5-bromodeoxyuridine, Hoy and Schimke, Mutation Research 290:217-230 (1993)), aminoallyldeoxyuridine (Henegariu et al:, Nature Biotechnology 18:345-348 (2000)), 5-methylcytosine (Sano et al., Biochim. Biophys. Acta 951:157-165 (1988)), bromouridine (Wansick et al., J. Cell Biology 122:283-293 (1993)) and nucleotides modified with biotin (Langer et al., Proc. Natl. Acad. Sci. USA 78:6633 (1981)) or with suitable haptens such as digoxygenin (Kerkhof, Anal. Biochem. 205:359-364 (1992)). Suitable fluorescence-labeled nucleotides are Fluorescein-isothiocyanate-dUTP, Cyanine-3-dUTP and Cyanine-5-dUTP (Yu et al., Nucleic Acids Res., 22:3226-3232 (1994)). A preferred nucleotide analog detection label for DNA is BrdUrd (bromodeoxyuridine, BrdUrd, BrdU, BUdR, Sigma-Aldrich Co). Other useful nucleotide analogs for incorporation of detection label into DNA are AA-dUTP (aminoallyl-deoxyuridine triphosphate, Sigma-Aldrich Co.), and 5-methyl-dCTP (Roche Molecular Biochemicals). A useful nucleotide analog for incorporation of detection label into RNA is biotin-16-UTP (biotin-16-uridine-5'-triphosphate, Roche Molecular Biochemicals). Fluorescein, Cy3, and Cy5 can be linked to dUTP for direct labeling. Cry3.5 and Cry7 are available as avidin or anti-digoxygenin conjugates for secondary detection of biotin- or digoxygenin-labeled probes.

Detection labels that are incorporated into nucleic acid, such as biotin, can be subsequently detected using sensitive methods well-known in the art. For example, biotin can be detected using streptavidin-alkaline phosphatase conjugate (Tropix, Inc.), which is bound to the biotin and subsequently detected by chemiluminescence of suitable substrates (for example, chemiluminescent substrate CSPD: disodium, 3-(4-methoxyspiro-[1,2,-dioxetane-3-2'-(5'-chloro)tricyclo [3.3.1.1^{3,7}]decane]-4-yl) phenyl phosphate; Tropix, Inc.). Labels can also be enzymes, such as alkaline phosphatase, soybean peroxidase, horseradish peroxidase and polymerases, that can be detected, for example, with chemical signal amplification or by using a substrate to the enzyme which produces light (for example, a chemiluminescent 1,2-dioxetane substrate) or fluorescent signal.

Molecules that combine two or more of these detection labels are also considered detection labels. Any of the known detection labels can be used with the disclosed probes, tags, molecules and methods to label and detect activated or deactivated riboswitches or nucleic acid or protein produced in the disclosed methods. Methods for detecting and measuring signals generated by detection labels are also known to those of skill in the art. For example, radioactive isotopes can be detected by scintillation counting or direct visualization; fluorescent molecules can be detected with fluorescent spectrophotometers; phosphorescent molecules can be detected with a spectrophotometer or directly visualized with a camera; enzymes can be detected by detection or visualization of the product of a reaction catalyzed by the enzyme; antibodies can be detected by detecting a secondary detection label coupled to the antibody. As used herein, detection molecules are molecules which interact with a compound or composition to be detected and to which one or more detection labels are coupled.

### I. Sequence Similarities

It is understood that as discussed herein the use of the terms homology and identity mean the same thing as similarity. Thus, for example, if the use of the word homology is used between two sequences (non-natural sequences, for example) it is understood that this is not necessarily indicating an evolutionary relationship between these two sequences, but rather is looking at the similarity or relatedness between their nucleic acid sequences. Many of the methods for determining homology between two evolutionarily related molecules are routinely applied to any two or more nucleic acids or proteins for the purpose of measuring sequence similarity regardless of whether they are evolutionarily related or not.

In general, it is understood that one way to define any known variants and derivatives or those that might arise, of the disclosed riboswitches, aptamers, expression platforms, genes and proteins herein, is through defining the variants and derivatives in terms of homology to specific known sequences. This identity of particular sequences disclosed herein is also discussed elsewhere herein. In general, variants of riboswitches, aptamers, expression platforms, genes and proteins herein disclosed typically have at least, about 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99 percent homology to a stated sequence or a native sequence. Those of skill in the art readily understand how to determine the homology of two proteins or nucleic acids, such as genes. For example, the homology can be calculated after aligning the two sequences so that the homology is at its highest level.

Another way of calculating homology can be performed by published algorithms. Optimal alignment of sequences for comparison can be conducted by the local homology algorithm of Smith and Waterman Adv. Appl. Math. 2: 482 (1981), by the homology alignment algorithm of Needleman and Wunsch, J. MoL Biol. 48: 443 (1970), by the search for similarity method of Pearson and Lipman, Proc. Natl. Acad. Sci. U.S.A. 85: 2444 (1988), by computerized implementations of these algorithms (GAP, BESTFIT, FASTA, and TFASTA in the Wisconsin Genetics Software Package, Genetics Computer Group, 575 Science Dr., Madison, WI), or by inspection.

The same types of homology can be obtained for nucleic acids by for example the algorithms disclosed in Zuker, M. Science 244:48-52, 1989, Jaeger et al. Proc. Natl. Acad. Sci. USA 86:7706-7710, 1989, Jaeger et al. Methods Enzymol. 183:281-306, 1989. It is understood that any of the methods typically can be used and that in certain instances the results of these various methods can differ, but the skilled artisan understands if identity is found with at least one of these methods, the sequences would be said to have the stated identity.

For example, as used herein, a sequence recited as having a particular percent homology to another sequence refers to sequences that have the recited homology as calculated by any one or more of the calculation methods described above. For example, a first sequence has 80 percent homology, as defined herein, to a second sequence if the first sequence is calculated to have 80 percent homology to the second sequence using the Zuker calculation method even if the first sequence does not have 80 percent homology to the second sequence as calculated by any of the other calculation methods. As another example, a first sequence has 80 percent homology, as defined herein, to a second sequence if the first sequence is calculated to have 80 percent homology to the second sequence using both the Zuker calculation method and the Pearson and Lipman calculation method even if the first sequence does not have 80 percent homology to the second sequence as calculated by the Smith and Waterman calculation method, the Needleman and Wunsch calculation method, the Jaeger calculation methods, or any of the other calculation methods. As yet another example, a first sequence has 80 percent homology, as defined herein, to a second sequence if the first sequence is calculated to have 80 percent homology to the second sequence using each of calculation methods (although, in practice, the different calculation methods will often result in different calculated homology percentages).

### J. Hybridization and Selective Hybridization

The term hybridization typically means a sequence driven interaction between at least two nucleic acid molecules, such as a primer or a probe and a riboswitch or a gene. Sequence driven interaction means an interaction that occurs between two nucleotides or nucleotide analogs or nucleotide derivatives in a nucleotide specific manner. For example, G interacting with C or A interacting with T are sequence driven interactions. Typically sequence driven interactions occur on the Watson-Crick face or Hoogsteen face of the nucleotide. The hybridization of two nucleic acids is affected by a number of conditions and parameters known to those of skill in the art. For example, the salt concentrations, pH, and temperature of the reaction all affect whether two nucleic acid molecules will hybridize.

Parameters for selective hybridization between two nucleic acid molecules are well known to those of skill in the art. For example, selective hybridization conditions can be defined as stringent hybridization conditions. For example, stringency of hybridization is controlled by both temperature and salt concentration of either or both of the hybridization and washing steps. For example, the conditions of hybridization to achieve selective hybridization can involve hybridization in high ionic strength solution (6X SSC or 6X SSPE) at a temperature that is about 12-25°C below the Tm (the melting temperature at which half of the molecules dissociate from their hybridization partners) followed by washing at a combination of temperature and salt concentration chosen so that the washing temperature is about 5°C to 20°C below the Tm. The temperature and salt conditions are readily determined empirically in preliminary experiments in which samples of reference DNA immobilized on filters are hybridized to a labeled nucleic acid of interest and then washed under conditions of different stringencies. Hybridization temperatures are typically higher for DNA-RNA and RNA-RNA hybridizations. The conditions can be used as described above to achieve stringency, or as is known in the art (Sambrook et al., Molecular Cloning: A Laboratory Manual, 2nd Ed., Cold Spring Harbor Laboratory, Cold Spring Harbor, New York, 1989; Kunkel et al. Methods Enzymol. 1987:154:367, 1987.
A preferable stringent hybridization condition for a DNA:DNA hybridization can be at about 68°C (in aqueous solution) in 6X SSC or 6X SSPE followed by washing at 68°C. Stringency of hybridization and washing, if desired, can be reduced accordingly as the degree of complementarity desired is decreased, and further, depending upon the G-C or A-T richness of any area wherein variability is searched for. Likewise, stringency of hybridization and washing, if desired, can be increased accordingly as homology desired is increased, and further, depending upon the G-C or A-T richness of any area wherein high homology is desired, all as known in the art.

Another way to define selective hybridization is by looking at the amount (percentage) of one of the nucleic acids bound to the other nucleic acid. For example, selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the limiting nucleic acid is bound to the non-limiting nucleic acid. Typically, the non-limiting nucleic acid is in for example, 10 or 100 or 1000 fold excess. This type of assay can be performed at under conditions where both the limiting and non-limiting nucleic acids are for example, 10 fold or 100 fold or 1000 fold below their k_{d}, or where only one of the nucleic acid molecules is 10 fold or 100 fold or 1000 fold or where one or both nucleic acid molecules are above their k_{d}.

Another way to define selective hybridization is by looking at the percentage of nucleic acid that gets enzymatically manipulated under conditions where hybridization is required to promote the desired enzymatic manipulation. For example, selective hybridization conditions would be when at least about, 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the nucleic acid is enzymatically manipulated under conditions which promote the enzymatic manipulation, for example if the enzymatic manipulation is DNA extension, then selective hybridization conditions would be when at least about 60, 65, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 percent of the nucleic acid molecules are extended. Preferred conditions also include those suggested by the manufacturer or indicated in the art as being appropriate for the enzyme performing the manipulation.

Just as with homology, it is understood that there are a variety of methods herein disclosed for determining the level of hybridization between two nucleic acid molecules. It is understood that these methods and conditions can provide different percentages of hybridization between two nucleic acid molecules, but unless otherwise indicated meeting the parameters of any of the methods would be sufficient. For example if 80% hybridization was required and as long as hybridization occurs within the required parameters in any one of these methods it is considered disclosed herein.

It is understood that those of skill in the art understand that if a composition or method meets any one of these criteria for determining hybridization either collectively or singly it is a composition or method that is disclosed herein.

### K. Nucleic Acids

There are a variety of molecules disclosed herein that are nucleic acid based, including, for example, riboswitches, aptamers, and nucleic acids that encode riboswitches and aptamers. The disclosed nucleic acids can be made up of for example, nucleotides, nucleotide analogs, or nucleotide substitutes. Non-limiting examples of these and other molecules are discussed herein. It is understood that for example, when a vector is expressed in a cell, that the expressed mRNA will typically be made up of A, C, G, and U. Likewise, it is understood that if a nucleic acid molecule is introduced into a cell or cell environment through for example exogenous delivery, it is advantageous that the nucleic acid molecule be made up of nucleotide analogs that reduce the degradation of the nucleic acid molecule in the cellular environment.

So long as their relevant function is maintained, riboswitches, aptamers, expression platforms and any other oligonucleotides and nucleic acids can be made up of or include modified nucleotides (nucleotide analogs). Many modified nucleotides are known and can be used in oligonucleotides and nucleic acids. A nucleotide analog is a nucleotide which contains some type of modification to either the base, sugar, or phosphate moieties. Modifications to the base moiety would include natural and synthetic modifications of A, C, G, and T/U as well as different purine or pyrimidine bases, such as uracil-5-yl, hypoxanthin-9-yl (I), and 2-aminoadenin-9-yl. A modified base includes but is not limited to 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine and 3-deazaadenine. Additional base modifications can be found for example in U.S. Pat. No. 3,687,808, Englisch et al., Angewandte Chemie, International Edition, 1991, 30, 613, and Sanghvi, Y. S., Chapter 15, Antisense Research and Applications, pages 289-302, Crooke, S. T. and Lebleu, B. ed., CRC Press, 1993. Certain nucleotide analogs, such as 5-substituted pyrimidines, 6-azapyrimidines and N-2, N-6 and O-6 substituted purines, including 2-aminopropyladenine, 5-propynyluracil and 5-propynylcytosine. 5-methylcytosine can increase the stability of duplex *formation*. Other modified bases are those that function as universal bases. Universal bases include 3-nitropyrrole and 5-nitroindole. Universal bases substitute for the normal bases but have no bias in base pairing. That is, universal bases can base pair with any other base. Base modifications often can be combined with for example a sugar modification, such as 2'-O-methoxyethyl, to achieve unique properties such as increased duplex stability. There are numerous United States patents such as 4,845,205; 5,130,302; 5,134,066; 5,175,273; 5,367,066; 5,432,272; 5,457,187; 5,459,255; 5,484,908; 5,502,177; 5,525,711; 5,552,540; 5,587,469; 5,594,121, 5,596,091; 5,614,617; and 5,681,941 which detail and describe a range of base modifications. Each of these patents describes rated base modifications, their synthesis, their use, and their incorporation into oligonucleotides and nucleic acids.

Nucleotide analogs can also include modifications of the sugar moiety. Modifications to the sugar moiety would include natural modifications of the ribose and deoxyribose as well as synthetic modifications. Sugar modifications include but are not limited to the following modifications at the 2' position: OH; F; O-, S-, or N-alkyl; O-, S-, or N-alkenyl; O-, S- or N-alkynyl; or O-alkyl-O-alkyl, wherein the alkyl, alkenyl and alkynyl can be substituted or unsubstituted C1 to C10, alkyl or C2 to C10 alkenyl and alkynyl. 2' sugar modifications also include but are not limited to -O[(CH₂)n O]m CH₃, - O(CH₂)n OCH₃, -O(CH₂)n NH₂, -O(CH₂)n CH₃, -O(CH₂)n -ONH₂, and - O(CH₂)nON[(CH₂)n CH₃)]₂, where n and m are from 1 to about 10.

Other modifications at the 2' position include but are not limited to: C1 to C10 lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂ CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties of an oligonucleotide, or a group for improving the pharmacodynamic properties of an oligonucleotide, and other substituents having similar properties. Similar modifications can also be made at other positions on the sugar, particularly the 3' position of the sugar on the 3' terminal nucleotide or in 2'-5' linked oligonucleotides and the 5' position of 5' terminal nucleotide. Modified sugars would also include those that contain modifications at the bridging ring oxygen, such as CH₂ and S. Nucleotide sugar analogs can also have sugar mimetics such as cyclobutyl moieties in place of the pentofuranosyl sugar. There are numerous United States patents that teach the preparation of such modified sugar structures such as 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920, each of which describes sugar structures, their synthesis, their use, and their incorporation into nucleotides, oligonucleotides and nucleic acids.

Nucleotide analogs can also be modified at the phosphate moiety. Modified phosphate moieties include but are not limited to those that can be modified so that the linkage between two nucleotides contains a phosphorothioate, chiral phosphorothioate, phosphorodithioate, phosphotriester, aminoalkylphosphotriester, methyl and other alkyl phosphonates including 3'-alkylene phosphonate and chiral phosphonates, phosphinates, phosphoramidates including 3'-amino phosphoramidate and aminoalkylphosphoramidates, thionophosphoramidates, thionoalkylphosphonates, thionoalkylphosphotriesters, and boranophosphates. It is understood that these phosphate or modified phosphate linkages between two nucleotides can be through a 3'-5' linkage or a 2'-5' linkage, and the linkage can contain inverted polarity such as 3'-5' to 5'-3' or 2'-5' to 5'-2'. Various salts, mixed salts and free acid forms are also included. Numerous United States patents teach how to make and use nucleotides containing modified phosphates and include but are not limited to, 3,687,808; 4,469,863; 4,476,301; 5,023,243; 5,177,196; 5,188,897; 5,264,423; 5,276,019; 5,278,302; 5,286,717; 5,321,131; 5,399,676; 5,405,939; 5,453,496; 5,455,233; 5,466,677; 5,476,925; 5,519,126; 5,536,821; 5,541,306; 5,550,111; 5,563,253; 5,571,799; 5,587,361; and 5,625,050, each of which describes phosphates, their synthesis, their use, and their incorporation into nucleotides, oligonucleotides and nucleic acids.

It is understood that nucleotide analogs need only contain a single modification, but can also contain multiple modifications within one of the moieties or between different moieties.

Nucleotide substitutes are molecules having similar functional properties to nucleotides, but which do not contain a phosphate moiety, such as peptide nucleic acid (PNA). Nucleotide substitutes are molecules that will recognize and hybridize to (base pair to) complementary nucleic acids in a Watson-Crick or Hoogsteen manner, but which are linked together through a moiety other than a phosphate moiety. Nucleotide substitutes are able to conform to a double helix type structure when interacting with the appropriate target nucleic acid.

Nucleotide substitutes are nucleotides or nucleotide analogs that have had the phosphate moiety and/or sugar moieties replaced. Nucleotide substitutes do not contain a standard phosphorus atom. Substitutes for the phosphate can be for example, short chain alkyl or cycloalkyl internucleoside linkages, mixed heteroatom and alkyl or cycloalkyl, internucleoside linkages, or one or more short chain heteroatomic or heterocyclic internucleoside linkages. These include those having morpholino linkages (formed in part from the sugar portion of a nucleoside); siloxane backbones; sulfide, sulfoxide and sulfone backbones; formacetyl and thioformacetyl backbones; methylene formacetyl and thioformacetyl backbones; alkene containing backbones; sulfamate backbones; methyleneimino and methylenehydrazino backbones; sulfonate and sulfonamide backbones; amide backbones; and others having mixed N, O, S and CH2 component parts. Numerous United States patents disclose how to make and use these types of phosphate replacements and include but are not limited to 5,034,506; 5,166,315; 5,185,444; 5,214,134; 5,216,141; 5,235,033; 5,264,562; 5,264,564; 5,405,938; 5,434,257; 5,466,677; 5,470,967; 5,489,677; 5,541,307; 5,561,225; 5,596,086; 5,602,240; 5,610,289; 5,602,240; 5,608,046; 5,610,289; 5,618,704; 5,623,070; 5,663,312; 5,633,360; 5,677,437; and 5,677,439 each of which phosphate replacements, their synthesis, their use, and their incorporation into nucleotides, oligonucleotides and nucleic acids.

It is also understood in a nucleotide substitute that both the sugar and the phosphate moieties of the nucleotide can be replaced, by for example an amide type linkage (aminoethylglycine) (PNA). United States patents 5,539,082; 5,714,331; and 5,719,262 teach how to make and use PNA molecules. (See also Nielsen et al., Science 254:1497-1500 (1991)).

Oligonucleotides and nucleic acids can be comprised of nucleotides and can be made up of different types of nucleotides or the same type of nucleotides. For example, one or more of the nucleotides in an oligonucleotide can be ribonucleotides, 2'-O-methyl ribonucleotides, or a mixture of ribonucleotides and 2'-O-methyl ribonucleotides; about 10% to about 50% of the nucleotides can be ribonucleotides, 2'-O-methyl ribonucleotides, or a mixture of ribonucleotides and 2'-O-methyl ribonucleotides; about 50% or more of the nucleotides can be ribonucleotides, 2'-O-methyl ribonucleotides, or a mixture of ribonucleotides and 2'-O-methyl ribonucleotides; or all of the nucleotides are ribonucleotides, 2'-O-methyl ribonucleotides, or a mixture of ribonucleotides and 2'-O-methyl ribonucleotides. Such oligonucleotides and nucleic acids can be referred to as chimeric oligonucleotides and chimeric nucleic acids.

### L. Solid Supports

Solid supports are solid-state substrates or supports with which molecules (such as trigger molecules) and riboswitches (or other components used in, or produced by, the disclosed methods) can be associated. Riboswitches and other molecules can be associated with solid supports directly or indirectly. For example, analytes (e.g., trigger molecules, test compounds) can be bound to the surface of a solid support or associated with capture agents (e.g., compounds or molecules that bind an analyte) immobilized on solid supports. As another example, riboswitches can be bound to the surface of a solid support or associated with probes immobilized on solid supports. An array is a solid support to which multiple riboswitches, probes or other molecules have been associated in an array, grid, or other organized pattern.

Solid-state substrates for use in solid supports can include any solid material with which components can be associated, directly or indirectly. This includes materials such as acrylamide, agarose, cellulose, nitrocellulose, glass, gold, polystyrene, polyethylene vinyl acetate, polypropylene, polymethacrylate, polyethylene, polyethylene oxide, polysilicates, polycarbonates, teflon, fluorocarbons, nylon, silicon rubber, polyanhydrides, polyglycolic acid, polylactic acid, polyorthoesters, functionalized silane, polypropylfumerate, collagen, glycosaminoglycans, and polyamino acids. Solid-state substrates can have any useful form including thin film, membrane, bottles, dishes, fibers, woven fibers, shaped polymers, particles, beads, microparticles, or a combination. Solid-state substrates and solid supports can be porous or non-porous. A chip is a rectangular or square small piece of material. Preferred forms for solid-state substrates are thin films, beads, or chips. A useful form for a solid-state substrate is a microtiter dish. For example a multiwell glass slide can be employed.

An array can include a plurality of riboswitches, trigger molecules, other molecules, compounds or probes immobilized at identified or predefined locations on the solid support. Each predefined location on the solid support generally has one type of component (that is, all the components at that location are the same). Alternatively, multiple types of components can be immobilized in the same predefined location on a solid support. Each location will have multiple copies of the given components. The spatial separation of different components on the solid support allows separate detection and identification.

Although useful, it is not required that the solid support be a single unit or structure. A set of riboswitches, trigger molecules, other molecules, compounds and/or probes can be distributed over any number of solid supports. For example, at one extreme, each component can be immobilized in a separate reaction tube or container, or on separate beads or microparticles.

Methods for immobilization of oligonucleotides to solid-state substrates are well established. Oligonucleotides, including address probes and detection probes, can be coupled to substrates using established coupling methods. For example, suitable attachment methods are described by Pease et al., Proc. Natl. Acad. Sci. USA 91(11):5022-5026 (1994), *and* Khrapko et al., Mol Biol (Mosk) (USSR) 25:718-730 (1991). A method for immobilization of 3'-amine oligonucleotides on casein-coated slides is described by Stimpson et al., Proc. Natl. Acad. Sci. USA 92:6379-6383 (1995). A useful method of attaching oligonucleotides to solid-state substrates is described by Guo et al., Nucleic Acids Res. 22:5456-5465 (1994).

Each of the components (for example, riboswitches, trigger molecules, or other molecules) immobilized on the solid support can be located in a different predefined region of the solid support. The different locations can be different reaction chambers. Each of the different predefined regions can be physically separated from each other of the different regions. The distance between the different predefined regions of the solid support can be either fixed or variable. For example, in an array, each of the components can be arranged at fixed distances from each other, while components associated with beads will not be in a fixed spatial relationship. In particular, the use of multiple solid support units (for example, multiple beads) will result in variable distances.

Components can be associated or immobilized on a solid support at any density. Components can be immobilized to the solid support at a density exceeding 400 different components per cubic centimeter. Arrays of components can have any number of components. For example, an array can have at least 1,000 different components immobilized on the solid support, at least 10,000 different components immobilized on the solid support, at least 100,000 different components immobilized on the solid support, or at least 1,000,000 different components immobilized on the solid support.

### M. Kits

The materials described above as well as other materials can be packaged together in any suitable combination as a kit useful for performing, or aiding in the performance of, the disclosed method. It is useful if the kit components in a given kit are designed and adapted for use together in the disclosed method. For example disclosed are kits for detecting compounds, the kit comprising one or more biosensor riboswitches. The kits also can contain reagents and labels for detecting activation of the riboswitches.

### N. Mixtures

Disclosed are mixtures formed by performing or preparing to perform the disclosed method. For example, disclosed are mixtures comprising riboswitches and trigger molecules.

Whenever the method involves mixing or bringing into contact compositions or components or reagents, performing the method creates a number of different mixtures. For example, if the method includes 3 mixing steps, after each one of these steps a unique mixture is formed if the steps are performed separately. In addition, a mixture is formed at the completion of all of the steps regardless of how the steps were performed. The present disclosure contemplates these mixtures, obtained by the performance of the disclosed methods as well as mixtures containing any disclosed reagent, composition, or component, for example, disclosed herein.

### O. Systems

Disclosed are systems useful for performing, or aiding in the performance of, the disclosed method. Systems generally comprise combinations of articles of manufacture such as structures, machines, devices, and the like, and compositions, compounds, materials, and the like. Such combinations that are disclosed or that are apparent from the disclosure are contemplated. For example, disclosed and contemplated are systems comprising biosensor riboswitches, a solid support and a signal-reading device.

### P. Data Structures and Computer Control

Disclosed are data structures used in, generated by, or generated from, the disclosed method. Data structures generally are any form of data, information, and/or objects collected, organized, stored, and/or embodied in a composition or medium. Riboswitch structures and activation measurements stored in electronic form, such as in RAM or on a storage disk, is a type of data structure.

The disclosed method, or any part thereof or preparation therefor, can be controlled, managed, or otherwise assisted by computer control. Such computer control can be accomplished by a computer controlled process or method, can use and/or generate data structures, and can use a computer program. Such computer control, computer controlled processes, data structures, and computer programs are contemplated and should be understood to be disclosed herein.

### Methods

Disclosed are methods for activating, deactivating or blocking a riboswitch. Such methods can involve, for example, bringing into contact a riboswitch and a compound or trigger molecule that can activate, deactivate or block the riboswitch. Riboswitches function to control gene expression through the binding or removal of a trigger molecule. Compounds can be used to activate, deactivate or block a riboswitch. The trigger molecule for a riboswitch (as well as other activating compounds) can be used to activate a riboswitch. Compounds other than the trigger molecule generally can be used to deactivate or block a riboswitch. Riboswitches can also be deactivated by, for example, removing trigger molecules from the presence of the riboswitch. Thus, the disclosed method of deactivating a riboswitch can involve, for example, removing a trigger molecule (or other activating compound) from the presence or contact with the riboswitch. A riboswitch can be blocked by, for example, binding of an analog of the trigger molecule that does not activate the riboswitch.

Also disclosed are methods for altering expression of an RNA molecule, or of a gene encoding an RNA molecule, where the RNA molecule includes a riboswitch, by bringing a compound into contact with the RNA molecule. Riboswitches function to control gene expression through the binding or removal of a trigger molecule. Thus, subjecting an RNA molecule of interest that includes a riboswitch to conditions that activate, deactivate or block the riboswitch can be used to alter expression of the RNA. Expression can be altered as a result of, for example, termination of transcription or blocking of ribosome binding to the RNA. Binding of a trigger molecule can, depending on the nature of the riboswitch, reduce or prevent expression of the RNA molecule or promote or increase expression of the RNA molecule.

Also disclosed are methods for regulating expression of an RNA molecule, or of a gene encoding an RNA molecule, by operably linking a riboswitch to the RNA molecule. A riboswitch can be operably linked to an RNA molecule in any suitable manner, including, for example, by physically joining the riboswitch to the RNA molecule or by engineering nucleic acid encoding the RNA molecule to include and encode the riboswitch such that the RNA produced from the engineered nucleic acid has the riboswitch operably linked to the RNA molecule. Subjecting a riboswitch operably linked to an RNA molecule of interest to conditions that activate, deactivate or block the riboswitch can be used to alter expression of the RNA.

Also disclosed are methods for regulating expression of a naturally occurring gene or RNA that contains a riboswitch by activating, deactivating or blocking the riboswitch. If the gene is essential for survival of a cell or organism that harbors it, activating, deactivating or blocking the riboswitch can result in death, stasis or debilitation of the cell or organism. For example, activating a naturally occurring riboswitch in a naturally occurring gene that is essential to survival of a microorganism can result in death of the microorganism (if activation of the riboswitch turns off or represses expression). This is one basis for the use of the disclosed compounds and methods for antimicrobial and antibiotic effects.

Also disclosed are methods for regulating expression of an isolated, engineered or recombinant gene or RNA that contains a riboswitch by activating, deactivating or blocking the riboswitch. The gene or RNA can be engineered or can be recombinant in any manner. For example, the riboswitch and coding region of the RNA can be heterologous, the riboswitch can be recombinant or chimeric, or both. If the gene encodes a desired expression product, activating or deactivating the riboswitch can be used to induce expression of the gene and thus result in production of the expression product. If the gene encodes an inducer or repressor of gene expression or of another cellular process, activation, deactivation or blocking of the riboswitch can result in induction, repression, or de-repression of other, regulated genes or cellular processes. Many such secondary regulatory effects are known and can be adapted for use with riboswitches. An advantage of riboswitches as the primary control for such regulation is that riboswitch trigger molecules can be small, non-antigenic molecules.

Also disclosed are methods for altering the regulation of a riboswitch by operably linking an aptamer domain to the expression platform domain of the riboswitch (which is a chimeric riboswitch). The aptamer domain can then mediate regulation of the riboswitch through the action of, for example, a trigger molecule for the aptamer domain. Aptamer domains can be operably linked to expression platform domains of riboswitches in any suitable manner, including, for example, by replacing the normal or natural aptamer domain of the riboswitch with the new aptamer domain. Generally, any compound or condition that can activate, deactivate or block the riboswitch from which the aptamer domain is derived can be used to activate, deactivate or block the chimeric riboswitch.

Also disclosed are methods for inactivating a riboswitch by covalently altering the riboswitch (by, for example, crosslinking parts of the riboswitch or coupling a compound to the riboswitch). Inactivation of a riboswitch in this manner can result from, for example, an alteration that prevents the trigger molecule for the riboswitch from binding, that prevents the change in state of the riboswitch upon binding of the trigger molecule, or that prevents the expression platform domain of the riboswitch from affecting expression upon binding of the trigger molecule.

Also disclosed are methods for selecting, designing or deriving new riboswitches and/or new aptamers that recognize new trigger molecules. Such methods can involve production of a set of aptamer variants in a riboswitch, assessing the activation of the variant riboswitches in the presence of a compound of interest, selecting variant riboswitches that were activated (or, for example, the riboswitches that were the most highly or the most selectively activated), and repeating these steps until a variant riboswitch of a desired activity, specificity, combination of activity and specificity, or other combination of properties results. Also disclosed are riboswitches and aptamer domains produced by these methods.

Techniques for in *vitro* selection and *in vitro* evolution of functional nucleic acid molecules are known and can be adapted for use with riboswitches and their components. Useful techniques are described by, for example, A. Roth and R. R. Breaker (2003) Selection in vitro of allosteric ribozymes. In: Methods in Molecular Biology Series - Catalytic Nucleic Acid Protocols (Sioud, M., ed.), Humana, Totowa, NJ; R. R. Breaker (2002) Engineered Allosteric Ribozymes as Biosensor Components. Curr. Opin. Biotechnol. 13:31-39; G. M. Emilsson and R. R. Breaker (2002) Deoxyribozymes: New Activities and New Applications. Cell. Mol. Life Sci. 59:596-607; Y. Li, R. R. Breaker (2001) In vitro Selection of Kinase and Ligase Deoxyribozymes. Methods 23:179-190; G. A. Soukup, R. R. Breaker (2000) Allosteric Ribozymes. In: Ribozymes: Biology and Biotechnology. R. K. Gaur and G. Krupp eds. Eaton Publishing; G. A. Soukup, R. R. Breaker (2000) Allosteric Nucleic Acid Catalysts. Curr. Opin. Struct. Biol. 10:318-325; G. A. Soukup, R. R. Breaker (1999) Nucleic Acid Molecular Switches. Trends Biotechnol. 17:469-476; R. R. Breaker (1999) In vitro Selection of Self-cleaving Ribozymes and Deoxyribozymes. In: Intracellular Ribozyme Applications: Principles and Protocols. L. Couture, J. Rossi eds. Horizon Scientific Press, Norfolk, England; R. R. Breaker (1997) In vitro Selection of Catalytic Polynucleotides. Chem. Rev. 97:371-390; and references cited therein; each of these publications describe *in vitro* selections and evolution techniques.

Also disclosed are methods for selecting and identifying compounds that can activate, deactivate or block a riboswitch. Activation of a riboswitch refers to the change in state of the riboswitch upon binding of a trigger molecule. A riboswitch can be activated by compounds other than the trigger molecule and in ways other than binding of a trigger molecule. The term trigger molecule is used herein to refer to molecules and compounds that can activate a riboswitch. This includes the natural or normal trigger molecule for the riboswitch and other compounds that can activate the riboswitch. Natural or normal trigger molecules are the trigger molecule for a given riboswitch in nature or, in the case of some non-natural riboswitches, the trigger molecule for which the riboswitch was designed or with which the riboswitch was selected (as in, for example, *in vitro* selection or *in vitro* evolution techniques). Non-natural trigger molecules can be referred to as non-natural trigger molecules.

Also disclosed herein is a method of identifying a compound that interacts with a riboswitch comprising: modeling the atomic structure the riboswitch with a test compound; and determining if the test compound interacts with the riboswitch. Determining if the test compound interacts with the riboswitch can be accomplished by, for example, determining a predicted minimum interaction energy, a predicted bind constant, a predicted dissociation constant, or a combination, for the test compound in the model of the riboswitch, as described elsewhere herein. Determining if the test compound interacts with the riboswitch can be accomplished by, for example, determining one or more predicted bonds, one or more predicted interactions, or a combination, of the test compound with the model of the riboswitch. The predicted interactions can be selected from the group consisting of, for example, van der Waals interactions, hydrogen bonds, electrostatic interactions, hydrophobic interactions, or a combination, as described above. In one example, the riboswitch is a guanine riboswitch.

Atomic contacts can be determined when interaction with the riboswitch is determined, thereby determining the interaction of the test compound with the riboswitch. Analogs of the test compound can be identified, and it can be determined if the analogs of the test compound interact with the riboswitch.

Also disclosed are methods of killing or inhibiting bacteria, comprising contacting the bacteria with a compound disclosed herein or identified by the methods disclosed herein.

Also disclosed is a method of identifying a compound that interacts with a riboswitch comprising: identifying the crystal structure of the riboswitch, modeling the riboswitch with a test compound, and determining if the test compound interacts with the riboswitch. Deactivation of a riboswitch refers to the change in state of the riboswitch when the trigger molecule is not bound. A riboswitch can be deactivated by binding of compounds other than the trigger molecule and in ways other than removal of the trigger molecule. Blocking of a riboswitch refers to a condition or state of the riboswitch where the presence of the trigger molecule does not activate the riboswitch.

Also disclosed are methods of identifying compounds that activate, deactivate or block a riboswitch. For examples, compounds that activate a riboswitch can be identified by bringing into contact a test compound and a riboswitch and assessing activation of the riboswitch. If the riboswitch is activated, the test compound is identified as a compound that activates the riboswitch. Activation of a riboswitch can be assessed in any suitable manner. For example, the riboswitch can be linked to a reporter RNA and expression, expression level, or change in expression level of the reporter RNA can be measured in the presence and absence of the test compound. As another example, the riboswitch can include a conformation dependent label, the signal from which changes depending on the activation state of the riboswitch. Such a riboswitch preferably uses an aptamer domain from or derived from a naturally occurring riboswitch. As can be seen, assessment of activation of a riboswitch can be performed with the use of a control assay or measurement or without the use of a control assay or measurement. Methods for identifying compounds that deactivate a riboswitch can be performed in analogous ways.

In addition to the methods disclosed elsewhere herein, identification of compounds that block a riboswitch can be accomplished in any suitable manner. For example, an assay can be performed for assessing activation or deactivation of a riboswitch in the presence of a compound known to activate or deactivate the riboswitch and in the presence of a test compound. If activation or deactivation is not observed as would be observed in the absence of the test compound, then the test compound is identified as a compound that blocks activation or deactivation of the riboswitch.

Also disclosed are methods of detecting compounds using biosensor riboswitches. The method can include bringing into contact a test sample and a biosensor riboswitch and assessing the activation of the biosensor riboswitch. Activation of the biosensor riboswitch indicates the presence of the trigger molecule for the biosensor riboswitch in the test sample. Biosensor riboswitches are engineered riboswitches that produce a detectable signal in the presence of their cognate trigger molecule. Useful biosensor riboswitches can be triggered at or above threshold levels of the trigger molecules. Biosensor riboswitches can be designed for use *in vivo* or in *vitro.* For example, biosensor riboswitches operably linked to a reporter RNA that encodes a protein that serves as or is involved in producing a signal can be used *in vivo* by engineering a cell or organism to harbor a nucleic acid construct encoding the riboswitch/reporter RNA. An example of a biosensor riboswitch for use *in vitro* is a riboswitch that includes a conformation dependent label, the signal from which changes depending on the activation state of the riboswitch. Such a biosensor riboswitch preferably uses an aptamer domain from or derived from a naturally occurring riboswitch.

Biosensor riboswitches can be used to monitor changing conditions because riboswitch activation is reversible when the concentration of the trigger molecule falls and so the signal can vary as concentration of the trigger molecule varies. The range of concentration of trigger molecules that can be detected can be varied by engineering riboswitches having different dissociation constants for the trigger molecule. This can easily be accomplished by, for example, "degrading" the sensitivity of a riboswitch having high affinity for the trigger molecule. A range of concentrations can be monitored by using multiple biosensor riboswitches of different sensitivities in the same sensor or assay.

Also disclosed are compounds made by identifying a compound that activates, deactivates or blocks a riboswitch and manufacturing the identified compound. This can be accomplished by, for example, combining compound identification methods as disclosed elsewhere herein with methods for manufacturing the identified compounds. For example, compounds can be made by bringing into contact a test compound and a riboswitch, assessing activation of the riboswitch, and, if the riboswitch is activated by the test compound, manufacturing the test compound that activates the riboswitch as the compound.

Also disclosed are compounds made by checking activation, deactivation or blocking of a riboswitch by a compound and manufacturing the checked compound. This can be accomplished by, for example, combining compound activation, deactivation or blocking assessment methods as disclosed elsewhere herein with methods for manufacturing the checked compounds. For example, compounds can be made by bringing into contact a test compound and a riboswitch, assessing activation of the riboswitch, and, if the riboswitch is activated by the test compound, manufacturing the test compound that activates the riboswitch as the compound. Checking compounds for their ability to activate, deactivate or block a riboswitch refers to both identification of compounds previously unknown to activate, deactivate or block a riboswitch and to assessing the ability of a compound to activate, deactivate or block a riboswitch where the compound was already known to activate, deactivate or block the riboswitch.

Disclosed is a method of detecting a compound of interest, the method comprising bringing into contact a sample and a riboswitch, wherein the riboswitch is activated by the compound of interest, wherein the riboswitch produces a signal when activated by the compound of interest, wherein the riboswitch produces a signal when the sample contains the compound of interest. The riboswitch can change conformation when activated by the compound of interest, wherein the change in conformation produces a signal via a conformation dependent label. The riboswitch can change conformation when activated by the compound of interest, wherein the change in conformation causes a change in expression of an RNA linked to the riboswitch, wherein the change in expression produces a signal. The signal can be produced by a reporter protein expressed from the RNA linked to the riboswitch.

Disclosed is a method comprising (a) testing a compound for inhibition of gene expression of a gene encoding an RNA comprising a riboswitch, wherein the inhibition is via the riboswitch, and (b) inhibiting gene expression by bringing into contact a cell and a compound that inhibited gene expression in step (a), wherein the cell comprises a gene encoding an RNA comprising a riboswitch, wherein the compound inhibits expression of the gene by binding to the riboswitch.

Also disclosed is a method of identifying riboswitches, the method comprising assessing in-line spontaneous cleavage of an RNA molecule in the presence and absence of a compound, wherein the RNA molecule is encoded by a gene regulated by the compound, wherein a change in the pattern of in-line spontaneous cleavage of the RNA molecule indicates a riboswitch.

### A. Identification of Antimicrobial Compounds

Riboswitches are a new class of structured RNAs that have evolved for the purpose of binding small organic molecules. The natural binding pocket of riboswitches can be targeted with metabolite analogs or by compounds that mimic the shape-space of the natural metabolite. Riboswitches are: (1) found in numerous Grana-positive and Gram-negative bacteria including Bacillus anthracis, (2) fundamental regulators of gene expression in these bacteria, (3) present in multiple copies that would be unlikely to evolve simultaneous resistance, and (4) not yet proven to exist in humans. This combination of features make riboswitches attractive targets for new antimicrobial compounds. Further, the small molecule ligands of riboswitches provide useful sites for derivitization to produce drug candidates. Distribution of some riboswitches is shown in Table 1 of U.S. Application Publication No. 2005-0053951.

Once a class of riboswitch has been identified and its potential as a drug target assessed (by, for example, determining how many genes in a target organism are regulated by that class of riboswitch), candidate molecules can be identified.

It has been determined that two compounds that have been known for many years to kill bacteria function by binding to riboswitches. Specifically, the compound aminoethylcysteine (an analog of lysine) binds to lysine riboswitches of bacteria and prevents expression of genes required for lysine biosynthesis. Similarly, the compound pyrithiamine (an analog of thiamine) is di-phosphorylated by bacterial cells to yield pyrithiamine pyrophosphate. It his latter compound mimics thiamine pyrophosphate (TPP) and is toxic to bacteria because it binds to TPP riboswitches. Therefore, targeting riboswitches via application of modem drug discovery strategies can lead to the discovery of new classes of antibiotic compounds. Structure-based drug design methods can be used as disclosed herein to generate metabolite analogs that bind to riboswitches and trigger their allosteric action.

The emergence of drug-resistant stains of bacteria highlights the need for the identification of new classes of antibiotics. Anti-riboswitch drugs represent a mode of anti-bacterial action that is of considerable interest for the following reasons. Riboswitches control the expression of genes that are critical for fundamental metabolic processes. Therefore manipulation of these gene control elements with drugs yields new antibiotics. Riboswitches also carry RNA structures that have evolved to selectively bind metabolites, and therefore these RNA receptors make good drug targets as do protein enzymes and receptors. Furthermore, it has been shown that two antimicrobial compounds (discussed above) kill bacteria by deactivating the antibiotics resistance to emerge through mutation of the RNA target. There are at least 11 classes of well-conserved riboswitches in many bacteria, providing numerous drug targets.

Many organisms from both Gram-positive and Gram-negative lineages have numerous classes of riboswitches (see U.S. Application Publication No. 2005-0053951). Major disease-causing organisms such as Staphylococcus and major bioterror-related organisms such as Bacillus anthracis are both rich with riboswitches. As disclosed herein, the atomic-resolution structure model for a guanine riboswitch has been elucidated, which enables the use of structure-based design methods for creating riboswitch-binding compounds. Specifically, the model for the binding site of the guanine riboswitch shows that two channels are present that would permit ligand modification (Figure 12A). 26 guanine analogs have been generated with chemical modifications at either the N2 or the 06 positions of guanine, and nearly all tested so far bind to the riboswitch with sub-nanomolar dissociation constants. Figure 12B depicts the structures of guanine analogs synthesized with modified N2 and 06 positions. Most of these compounds take advantage of the molecular recognition "blind spots" in the binding site model or the aptamer domain form a *B. subtilis* guanine riboswitch. The successful compounds can be used as a scaffold upon which further chemical variation can be introduced to create non-toxic, bioavailable, high affinity, anti-riboswitch compounds.

The following provides an illustration of this using the SAM riboswitch (see Example 7 of U.S. Application Publication No. 2005-0053951).SAM analogs that substitute the reactive methyl and sulfonium ion center with stable sulfur-based linkages (YBD-2 and YBD3) are recognized with adequate affinity (low to mid-nanomolar range) by the riboswitch to serve as a platform for synthesis of additional SAM analogs. In addition, a wider range of linkage analogs (N- and C-based linkages) can be synthesized and tested to provide the optimal platform upon which to make amino acid and nucleoside derivations.

Sulfoxide and sulfone derivatives of SAM can be used to generate analogs. Established synthetic protocols described in Ronald T. Borchardt and Yih Shiong Wu, Potential inhibitor of S-adenosylmethionine-dependent methyltransferase. 1. Modification of the amino acid portion of S-adenosylhomocysteine. J. Med. Chem. 17, 862-868, 1974, can be used, for example. These and other analogs can be synthesized and assayed for binding sequentially or in small groups. Additional SAM analogs can be designed during the progression of compound identification based on the recognition determinants that are established in each round. Simple binding assays can be conducted on *B. subtilis* and *B. anthracis* riboswitch RNAs as described elsewhere herein. More advanced assays can also be used.

The most promising SAM analog lead compounds must enter bacterial cells and bind riboswitches while remaining metabolically inert. In addition, useful SAM analogs must be bound tightly by the riboswitch, but must also fail to compete for SAM in the active sites of protein enzymes, or there is a risk of generating an undesirable toxic effect in the patient's cells. As a preliminary assessment of these issues, compounds can be tested for their ability to disrupt *B. subtilis* growth, but fail to affect *E. coli* cultures (which use SAM but lack SAM riboswitches). To screen for lead compound candidates, parallel bacterial cultures can be grown as follows:
1*. B. subtilis* can be cultured in glucose minimal media in the absence of exogenously supplied SAM analogs.
*2. B. subtilis* can be cultured in glucose minimal media in the presence of exogenously supplied SAM analogs (high doses can be selected, to be followed by repeated experiments designed to test a concentration range of the putative drug compound).
*3. E. coli* can be cultured in glucose minimal media in the presence of exogenously supplied SAM analogs (high doses will be selected, to be followed by repeated experiments designed to test a concentration range of the putative drug compound).

Fitness of the various cultures can be compared by measurement of cellular doubling times. A range of concentrations for the drug compounds can be tested using cultures grown in microtiter plates and analyzed using a microplate reader from another laboratory. Culture 1 is expected to grow well. Drugs that inhibit culture 2 may or may not inhibit growth of culture 3. Drugs that similarly inhibit both culture 2 and culture 3 upon exposure to a wide range of drug concentrations can reflect general toxicity induced by the exogenous compound *(i.e.,* inhibition of many different cellular processes, in addition or in place of riboswitch inhibition). Successful drug candidates identified in this screen will inhibit *E. coli* only at very high doses, if at all, and will inhibit *B. subtilis* at much (>10-fold) lower concentrations.

As derivization points on SAM are identified, efficient identification of lead drug compounds will require larger-scale screening of appropriate SAM analogs or generic chemical libraries. A high-throughput screen can be created by one or two different methods using nucleic acid engineering principles. Adaptation of both fluorescent sensor designs outlined below to formats that are compatible with high-throughput screening assays can be accommodated by using immobilization methods or solution-based methods.

One way to create a reporter is to add a third function to the riboswitch by adding a domain that catalyzes the release of a fluorescent tag upon SAM binding to the riboswitch domain. In the final reporter construct, this catalytic domain can be linked to the *yitJ* SAM riboswitch through a communication module that relays the ligand binding event by allowing the correct folding of the catalytic domain for generating the fluorescent signal. This can be accomplished as outlined below.

SAM RiboReporter Pool Design: A DNA template for *in vitro* transcription to RNA was constructed by PCR amplification using the appropriate DNA template and primer sequences. In this construct, stem II of the hammerhead (stem P1 of the SAM aptamer) has been randomized to present more than 250 million possible sequence combinations, wherein some inevitably will permit function of the ribozyme only when the aptamer is occupied by SAM or a related high-affmity analog. Each molecule in the population of constructs is identical in sequence except at the random domain where multiple copies of every possible combination of sequence will be represented in the population.

SAM RiboReporter Selection: The *in vitro* selection protocol can be a repetitive iteration of the following steps:
1. Transcribe RNA *in vitro* by standard methods. Include [α-³²P] UTP to incorporate radioactivity throughout the RNA.
2. Purify full length RNA on denaturing PAGE by standard methods.
3. Incubate full length RNA (∼100 pmoles) in negative selection buffer containing sufficient magnesium for catalytic activity (20 mM) but no SAM. Incubate 4 h at room temperature (∼23°C), with thermocycling or alkaline denaturation as needed to preclude the emergence of selfish molecules.
4. Purify full length RNA on denaturing PAGE and discard RNAs that react in the absence of SAM.
5. Incubate in positive selection buffer containing 20 mM Mg²⁺ and SAM (pH 7.5 at 23°C). Incubate 20 min at room temperature.
6. Purify cleaved RNA on denaturing PAGE to recover switches that bound SAM and allowed self-cleavage of the RNA.
7. Reverse transcribe RNA to DNA.
8. PCR amplify DNA with primers that reintroduced cleaved portion of RNA.

The concentration of SAM in step 4 can be 100 µM initially and can be reduced as the selection proceeds. The progress of recovering successful communication modules can be assessed by the amount of cleavage observed on the purification gel in step 6. The selection endpoint can be either when the population approaches 100% cleavage in 10 nM SAM (conditions for maximal activity of the parental ribozyme and riboswitch) or when the population approaches a plateau in activity that does not improve over multiple rounds. The end population can then be sequenced. Individual communication module clones can be assayed for generation of a fluorescent signal in the screening construct in the presence of SAM.

A fluorescent signal can also be generated by riboswitch-mediated triggering of a molecular beacon. In this design, riboswitch conformational changes cause a folded molecular beacon tagged with both a fluor and a quencher to unfold and force the fluor away from the quencher by forming a helix with the riboswitch. This mechanism is easy to adapt to existing riboswitches, as this method can take advantage of the ligand-mediated formation of terminator and anti-terminator stems that are involved in transcription control.

To use riboswitches to report ligand binding by binding a molecular beacon, the appropriate construct must be determined empirically. The optimum length and nucleotide composition of the molecular beacon and its binding site on the riboswitch can be tested systematically to result in the highest signal-to-noise ratio. The validity of the assay can be determined by comparing apparent relative binding affinities of different SAM analogs to a molecular beacon-coupled riboswitch (determined by rate of fluorescent signal generation) to the binding constants determined by standard in-line probing.

The invention relates to a crystalline natural guanine-responsive riboswitch comprising the atomic coordinates listed in Table 6. The atomic coordinates of the atomic structure can comprise The atomic coordinates of the atomic structure comprise the atomic coordinates listed in Table 6 for atoms depicted in Figure 6.

The invention relates to a method of identifying a compound that interacts with the said riboswitch comprising: (a) modeling the atomic structure of claim 1 with a test compound; and (b) determining if the test compound interacts with the riboswitch.

Determining if the test compound interacts with the riboswitch can comprise determining a predicted minimum interaction energy, a predicted bind constant a predicted dissociation constant, or a combination, for the test compound in the model of the riboswitch. Determining if the test compound interacts with the riboswitch can comprise determining one or more predicted bonds, one or more predicted interactions, or a combination, of the test compound with the model of the riboswitch.

The method can further comprise the steps of: (c) identifying analogs of the test compound; (d) determining if the analogs of the test compound interact with the riboswitch. The compound can be hypoxanthine. A gel-based assay can be used to determine if the test compound interacts with the riboswitch. A chip-based assay can be used to determine if the test compound interacts with the riboswitch.

The test compound can interact via van der Waals interactions, hydrogen bonds, electrostatic interactions, hydrophobic interactions, or a combination. The riboswitch can comprise an RNA cleaving ribozyme. A fluorescent signal can be generated when a nucleic acid comprising a quenching moiety is cleaved. Molecular beacon technology can be employed to generate tho fluorescent signal. The method can be carried out using a high throughput screen.

### Examples

### A. Example 1: Structure of a Natural Guanine-Responsive Riboswitch Complexed with the Metabolite Hypoxanthine

To understand how the nature biosensor functions, the structure of the guanine-binding domain bound to hypoxanthine has been solved by X-ray crystallography (Table 2 and Figure 12A), a biologically relevant ligand of the guanine-responsive riboswitch. In the hypoxanthine-bound state, the RNA adopts a three-dimensional fold in which the terminal loops (L2 and L3) form a series of interconnecting hydrogen bonds (see pairing scheme in Figure 5) to bring the P2 and P3 helices parallel to each other (Figure 5C). Unfavorable electrostatic interactions, a result of the juxtaposition of regions of the ribose-phosphate backbone, are neutralized through the binding of several cations between the two backbones (Figure 10). Anchoring the global helical arrangement of the RNA are numerous tertiary contacts around the three-way junction, dominated by the J2/3 loop (Figure 5C) interacting with bound hypoxanthine, the P1 helix, and the J1/2 and J3/1 loops.

The purine-binding pocket is created by conserved nucleotides in and around the three-way junction element. These nucleotides help to define the purine-binding pocket through the formation of two sets of base triples above and below (Figure 5A). The 3' side of the pocket is flanked by a water-mediated U22-A52A73 base triple and an A23G46-C53 triple; in both cases, the Watson-Crick face of the adenosine interacts with the minor groove of a Watson-Crick pair (Figure 6A). The other side is created by sequential base triples between conserved Watson-Crick pairs at the top of helix P1 (U20-A76 and A21-U75) and the Watson-Crick faces of U49 and C50, respectively, which fasten the J2/3 loop to the P1 helix. This extensive use of base triples to create a ligand-binding site is very similar to *in vitro* selected RNA aptamers that recognize planar ring systems, as exemplified by the structures of the theophylline (Zimmermann, G: R, Jenison, R. D., Wick, C. L., Simmorre, J.-P. & Pardi, A. Interlocking structural motifs mediate molecular discrimination by a theophylline-binding RNA. Nature Struct. Biol. 4, 644-649 (1997)), FMN (Fan, P., Suri, A. K., Fiala, R., Live, D. & Patel, D. J. Molecular recognition in the FMN-RNA aptamer complex. J. Mol. Biol. 258, 480-500 (1996)) and malachite green (Baugh, C., Grate, D. & Wilson, C. 2.8 A crystal structure of the malachite green aptamer. J. Mol. Biol. 301,117-128 (2000)) binders. Thus, artificially selected RNAs use some of the same principles for creating binding sites for small-molecule ligands as their naturally occurring counterpart.

Hypoxanthine is bound through an extensive series of hydrogen bonds with nucleotides U22, U47, U51 and C74 (Figure 6B), forming a base quadruple that stacks directly on the P1 helix. The structure clearly shows that the mRNA contacts all of the functional groups in the ligand, thereby explaining the specificity for hypoxanthine observed in biochemical studies (Mandal, M. & Breaker, R. R Gene regulation by riboswitches. Nature Rev. Mol. Cell. Biol. 5, 451-463 (2004)). In addition, guanine binding can be readily rationalized, because there is room in the structure to accommodate an exocyclic amine at the 2-position of the bound purine. This additional functional group can form hydrogen bonds with the carbonyl oxygens at the 2-position of C74 and U51, consistent with the tenfold higher affinity of this riboswitch for guanine over hypoxanthine.

One of the most marked features is how the ligand is almost completely enveloped by the RNA (Figure 6C): 97.8% of the surface of hypoxanthine is inaccessible to bulk solvent in the complex. The almost complete use of a ligand for recognition by an RNA is unprecedented among structurally characterized aptamers, although selection strategies that do not involve immobilization of the ligand on a solid support (Koizumi, M., Soukup, G. A., Kerr, J. N. & Breaker, R R Allosteric selection of ribozymes that respond to the second messengers cGMP and cAMP. Nature Struct. Biol. 6, 1062-1071 (1999)) hold promise for the development of RNAs that are capable of a similar degree of ligand burial. This finding also implies that the local binding site must undergo a substantial conformational change upon ligand binding, because it is not possible for hypoxanthine to gain access to a preformed binding site, which is a common feature of many RNA-ligand interactions (Leulliot, N. & Varani, G. Current topics in RNA-protein recognition: control of specificity and biological function through induced fit and conformational capture. Biochemistry 40, 7947-7956 (2001); Williamson, J. R. Induced fit in RNA-protein recognition. Nature Struct. Biol. 7, 834-837 (2000)). Finally, this mode of purine recognition also easily explains its ability to change specificity from guanine to adenine through a single point mutation at nucleotide 74 from cytosine to uracil (Mandal, M. & Breaker, R R. Adenine riboswitches and gene activation by disruption of a transcription terminator. Nature Struct. Mol. Biol. 11, 29-35 (2004)). Although the Watson-Crick pairing preference changes from guanine to adenine, the other interactions between the purine and the RNA are unchanged.

The tertiary architecture is stabilized through a unique loop-loop interaction capping helices P2 and P3 that is defined by two previously unobserved types of base quadruple. Each quadruple comprises a Watson-Crick pair with a noncanonical pair docked into its minor groove (G38-C60 and G37-C61 interacting with the A33A66 and U34A65 pairs, respectively; Figure 7A). This arrangement bears a strong similarity to how adenosines pack into an A-form helix in the commonly found type I/II A-minor triple motif (Doherty, E. A., Batey, R. T., Masquida, B. & Doudna, J. A. A universal mode of helix packing in RNA. Nature Struct. Biol. 8, 339-343 (2001); Nissen, P., Ippolito, J. A., Ban, N., Moore, P. B. & Steitz, T. A. RNA tertiary interactions in the large ribosomal subunit: the A-minor motif. Proc. Natl Acad. Sci. USA 98, 4899-4903 (2001)). Mutation of any one of these eight nucleotides would disrupt the intricate hydrogen-bonding network that cements the core of the loop-loop interaction, explaining their strict phylogenetic conservation. Stacked on these quadruples are two noncanonical pairs, including a side-by-side interaction between G62 and U63 akin to the A-platform motif (Cate, J. H. et al. RNA tertiary structure mediation by adenosine platforms. Science 273, 1696-1699 (1996)) and the bulged-G motif (Correll, C. C., Beneken, J., Plantinga, M. J., Lubbers, M. & Chan, Y. L. The common and the distinctive features of the bulged-G motif based on a 1.04 Å resolution RNA structure. Nucleic Acids Res. 31, 6806-6818 (2003)) (Figure 7B). The G62U63 pair is further stabilized through hydrogen bonding to the backbone of the opposite loop.

The interaction between the two terminal loops is essential for ligand binding by the guanine riboswitch. Differences in the stability of the RNA in the absence and presence of guanine, as determined by in-line probing experiments, indicate that this element of the RNA tertiary structure forms independently of guanine or hypoxanthine. Replacement of the wild-type loops with stable UUCG tetraloops (Molinaro, M. & Tinoco, I. Jr Use of ultra stable UNCG tetraloop hairpins to fold RNA structures: thermodynamic and spectroscopic applications. Nucleic Acids Res. 23, 3056-3063 (1995); Figure 11), which eliminates the tertiary interaction, abolishes the ability of the riboswitch to recognize hypoxanthine; this shows that it is crucial for promoting a high-affinity interaction (Figure 8). Thus, although this tertiary interaction does not contact the ligand directly, it is significant in globally organizing the riboswitch for purine recognition. Similarly, natural sequences of various hammerhead ribozymes contain loop-loop interactions that significantly accelerate their rate of cleavage under physiological conditions (De la Pena, M., Gago, S. & Flores, R. Peripheral regions of natural hammerhead ribozymes greatly increase their self-cleavage activity. EMBO J. 22, 5561-5570 (2003); Khvorova, A., Lescoute, A., Westhof, E. & Jayasena, S. D. Sequence elements outside the hammerhead ribozyme catalytic core enable intracellular activity. Nature Struct. Biol. 10, 708-712 (2003)). In each, the tertiary interaction constrains the RNA in a way that allows the three-way junction containing the functional site to respond to physiological concentrations of ligand or Mg2+ ions.

In high Mg2+ ion concentrations (20 mM MgCl2), the guanine riboswitch has a very high affinity for guanine and hypoxanthine (an observed dissociation constant (*K*d) of 5 nM and 50 nM, respectively). In *Escherichia coli,* however, repression of transcription of the *xpt-pbuX* operon by the purR repressor occurs in response to 1-10 µM concentrations of purine. To determine whether the riboswitch responds to similar concentrations of purine, which probably reflect physiological levels, its affinity for hypoxanthine was determined by isothermal titration calorimetry at varying ionic conditions (Table 1). At a more physiological ionic strength (0.25-1 mM Mg2+), the RNA showed an affinity for hypoxanthine (observed Kd = 3-4 µM) similar to that of the purR repressor protein (observed Kd = 9 µM for the E. *coli* variant) (Choi, K. Y. & Zalkin, H. Structural characterization and corepressor binding of the Escherichia coli purine repressor. J. Bacteriol. 174, 6207-6214 (1992)). Thus, both RNA- and protein-based regulatory mechanisms seem to be tuned to respond to similar concentrations of intracellular metabolite.

| Table 1 Thermodynamic parameters for hypoxanthine binding at 30°C | | | |
|---|---|---|---|
| MgCl₂(mM) | *K*_{d} (µM)* | Δ*H*_{oba} (kcalmol⁻¹)* | Δ*S*_{oba} (cal mol⁻¹K⁻¹) |
| 20 | 0.732 **±** 0.034 | -33.5 ± 0.43 | -82 |
| 5.0 | 1.34 **±** 0.094 | -28.4 ± 0.48 | -67 |
| 1.0 | 2.99 ± 0.19 | -35.4 ± 0.93 | -91 |
| 0.26 | 4.00 ± 0.19 | -41.9 ± 0.39 | -113 |
| 0† | ND | ND | ND |

| | | | |
|---|---|---|---|
| ***The reported errors represent the s.e.m. of the nonlinear least squares fit to the data. ND, no detectable binding.** **†This reaction contained 2 mM Na₂-EDTA.** | | | |

The structure further indicates how RNA directly transduces intracellular metabolite concentration into changes in gene expression through a proposed Rho-independent transcriptional regulation mechanism (Mandal, M., Boese, B., Barrick, J. E., Winkler, W. C. & Breaker, R R Riboswitches control fundamental biochemical pathways in Bacillus subtilis and other bacteria. Cell 113, 577-586 (2003); Johansen, L. E., Nygaard, P., Lassen, C., Agerso, Y. & Saxild, H. H. Definition of a second Bacillus subtilis pur regulon comprising the pur and xpt-pbuX operons plus pbuG, nupG (yxjA), and pbuE (ydhL). J. Bacteriol. 185, 5200-5209 (2003)). Transcription of the initial 90 nucleotides results in the formation of the guanine-binding domain, with the L2 and L3 loops interacting to begin to form the tertiary structure of the RNA (Figure 5B). This partially organizes the three-way junction motif for efficient ligand binding, although the junction must be unstructured to some degree to allow access of the purine to the binding pocket. At sufficiently high concentrations of guanine or hypoxanthine, the nucleobase binds the pocket, stabilizing the short P1 helix through stacking interactions and base triples with J2/3 and preventing incorporation of P1 nucleotides into an antiterminator element. The mRNA can then form a classic Rho-independent terminator stem-loop, and transcription stops. By contrast, in low intracellular concentrations of guanine or hypoxanthine, the 3' side of the isolated P1 helix is readily conscripted to form a stable antiterminator element, facilitating continued transcription. Thus, hypoxanthine is the keystone for the riboswitch, enabling the riboswitch to direct mRNA folding along two different pathways through its ability to stabilize one conformation over another, resulting in an effective biosensor of intracellular guanine, hypoxanthine and xanthine concentrations.

### 1. Methods

*Crystals:* RNA was synthesized and purified by a native affinity-tag purification method (Kieft, J. S. & Batey, R T. A general method for rapid and nondenaturing purification of RNAs. RNA 10, 988-995 (2004)) and exchanged it into a buffer containing 10 mM K+-HEPES (pH 7.5) and 1 mM hypoxanthine. Crystals were grown by mixing this solution in a 1:1 ratio with mother liquor (containing 25% PEG 3,000 (w/v), 200 mM ammonium acetate and 10 mM cobalt hexamine) and incubating it for 2-3 weeks at room temperature.

*Data collection and processing:* A single wavelength anomalous diffraction experiment was carried out at the CuKα wavelength on crystals cryoprotected in mother liquor plus 25% 2-methyl-2,4-pentanediol; clear diffraction was observed to at least 1.8 Å resolution. The data was indexed, integrated, and scaled using D*TREK (Pflugrath, J. W. The finer things in X-ray diffraction data collection. Acta Crystallogr. D 55, 1718-1725 (1999)), identified heavy atom sites with SOLVE (Terwilliger, T. SOLVE and RESOLVE: automated structure solution, density modification and model building. J. Synchrotron Radiat. 11, 49-52 (2004)), and carried out refinement with CNS (Brunger, A. T. et al. Crystallography & NMR system: a new software suite for macromolecular structure determination. Acta Crystallogr. D 54, 905-921 (1998)) to obtain a model with final *R*xtal and *R*free values of 17.8% and 22.8%, respectively. The model contains all RNA atoms except A82, for which electron density was not observed, along with the hypoxanthine ligand.

*RNA synthesis and purification:* A 68 nucleotide construct containing the sequence for the guanine riboswitch of the *pbuX-xpt* operon of *B. subtilis* was constructed using overlapping DNA oligonucleotides (Integrated DNA Technologies) and standard PCR methods. The resulting DNA fragment, which contained *Eco*RI and *Ngo*MIV restriction sites at the 5' and 3' ends, respectively, was ligated into pRAV12 (Kieft, J. S. & Batey, R. T. A general method for rapid and nondenaturing purification of RNAs. RNA 10, 988-995 (2004)), a plasmid vector designed for the native purification of RNA; the resulting vector was sequence verified. DNA template for *in vitro* transcription was generated by PCR from the resulting vector using primers directed against the T7 RNA polymerase promoter at the 5' end and the 3' side of the purification affinity tag (5', GCGCGCGAATTCTAATACGACTCACTATAG (SEQ ID NO:10; 3', GGATCCTGCCCAGGGCTG; SEQ ID NO:11). RNA was transcribed in a 12.5 mL reaction containing 30 mM Tris-HCl (pH 8.0), 10 mM DTT, 0.1 % Triton X-100, 0.1 mM spermidine-HCl, 8 mM each NTP, 40 mM MgCl₂, 50 µg/mL T7 RNA polymerase, and 1 mL of ∼0.5 µM template (Doudna, J. A. Preparation of homogeneous ribozyme RNA for crystallization. Methods Mol Biol 74, 365-70 (1997)), supplemented with 1 unit/mL inorganic pyrophosphatase to suppress formation of insoluble magnesium pyrophosphate. The reaction was incubated for 1.5 hr at 37 °C. Native purification of the RNA was performed as described. Following elution of the RNA from the affinity column, it was immediately concentrated using a 10,000 MWCO centrifugal filter device (Amicon, Ultra-15). After all of the RNA had been concentrated to ∼500.µL, it was exchanged against three 15 mL aliquots of a buffer containing 10 mM K⁺-HEPES, pH 7.5 and 1 mM hypoxanthine using the centrifugal concentrator. The final RNA was concentrated to 450 µM, as judged by it absorbance at 260 nm and a calculated extinction coefficient based upon its base composition.

*RNA crystallization:* Crystals of the guanine riboswitch were obtained using the hanging drop method in which the RNA solution was mixed in a 1:1 ratio with a reservoir solution containing 10 mM cobalt hexammine, 200 mM ammonium acetate and 25 % PEG 2K. The crystallization trays were incubated at room temperature (23 °C), with crystals obtaining their maximum size (0.05 x 0.05 x 0.2 mm) in 7-14 days. Cryoprotection of the crystals was performed by adding 30 *µ*L of a solution comprising the mother liquor plus 25 % (v/v) 2-methyl-2,4 pentanediol (MPD) for five minutes and flash-frozen in liquid nitrogen. Diffraction data was collected on a home X-ray source (Rigaku MSC) using CuKα radiation; collection of anomalous data was achieved by an inverse-beam experiment. The data was indexed, integrated and scaled with CrystalClear (Rigaku MSC) and D*TREK (Pflugrath, J. W. The finer things in X-ray diffraction data collection. Acta Crystallogr D Biol Crystallogr 55, 1718-1725 (1999)). The crystals belong to the C2 spacegroup (a = 132.30 Å, b = 35.25 Å, c = 42.23 Å, 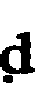 = 90.95°) and contain one molecule per asymmetric unit (refer to Table 2 for all crystallographic statistics). All data used in subsequent phasing and refinement was collected from one individual crystal.

*Phasing and structure determination.* Phases were determined using a single wavelength anomalous diffraction (SAD) experiment (Dauter, Z., Dauter, M. & Dodson, E. Jolly SAD. Acta Crystallogr D Biol Crystallogr 58, 494-506 (2002); Rice, L. M., Earnest, T. N. & Brunger, A. T. Single-wavelength anomalous diffraction phasing revisited. Acta Crystallogr D Biol Crystallogr 56 (Pt 11), 1413-20 (2000)) and diffraction data extending to 1.95 Å resolution. In this experiment, cobalt was treated as the heavy atom derivative, which has weak anomalous signal at CuKα wavelength (f' = 2.464, f' = 3.608). With SOLVE (Terwilliger, T. SOLVE and RESOLVE: automated structure solution, density modification and model building. J Synchrotron Radiat 11, 49-52 (2004)), a 10 heavy atom solution was found, with a figure of merit of 0.38 and a score of 40.9. Phases were determined using this heavy atom model and its mirror image using CNS (Brunger, A. T. et al. Crystallography & NMR system: A new software suite for macromolecular structure determination. Acta Crystallogr D Biol Crystallogr 54 (Pt 5), 905-21 (1998)) and improved with density modification. Only one of the heavy atom models yielded an electron density map in which there was clear backbone connectivity and base stacking; this map was sufficiently clear to be able to trace the majority of the RNA. Iterative rounds of model building in O (Jones, T. A., Zou, J. Y., Cowan, S. W. & Kjeldgaard. Improved methods for building protein models in electron density maps and the location of errors in these models. Acta Crystallogr A 47 (Pt 2), 110-9 (1991)) and refinement in CNS were performed while monitoring R_{free} to ensure that it improved after each round. Following building of all nucleotides in the RNA except for the 3'-terminal A82, two rounds of water picking were performed with a total of 332 water molecules placed into the model, using the restrictions that each solvent must be in hydrogen bonding distance to another atom and have a B-factor of less than 80. During this phase of model building, 12 cobalt hexammine ions were identified on the basis of having inner-sphere atoms with clear octahedral coordination geometry and their positions verified by an anomalous difference map; a single spermidine and an acetate ion were also placed within the model at this point. Sugar puckers were constrained to be C3'-endo, except for residues 22, 34, 35, 47, 49 and 62 which were restrained as C2'-endo.

*Isothermal Titration Calorimetry.* RNA for isothermal titration calorimetry (ITC) (Leavitt, S. & Freire, E. Direct measurement of protein binding energetics by isothermal titration calorimetry. Curr Opin Struct Biol 11, 560-6 (2001); Pierce, M., Raman, C. S. & Nall, B. T. Isothermal titration calorimetry of protein-protein interactions. Methods 19, 213-21 (1999)) was transcribed and purified as described above and exhaustively dialyzed against buffer containing 10 mM K⁺-HEPES, pH 7.5, 100 mM KCl and varying concentrations of MgCl₂ at 4 °C for 24-48 hours. Following dialysis, the buffer was used to prepare a solution of hypoxanthine at a concentration that was approximately 10-fold higher than the RNA (typically about 120 □M and 12 □M, respectively). All experiments were performed with a Microcal MCS ITC instrument at 30 °C. Following degassing of the RNA and hypoxanthine solutions, a titration of 29 injections of 10 µL of hypoxanthine into the RNA sample was performed, such that a final molar ratio of between 2:1 and 3:1 hypoxanthine:RNA was achieved (Recht, M. I. & Williamson, J. R. Central domain assembly: thermodynamics and kinetics of S6 and S18 binding to an S15-RNA complex. J Mol Biol 313, 35-48 (2001)). Titration data was analyzed using Origin ITC software (Microcal Software Inc.) and fit to a single-site binding model.

**Table 2: Crystallography statistics**

| Data Collection | | |
|---|---|---|
| Spacegroup: | | C2 |
| | a, b, c | 132.30, 35.25, 42.23 Å |
| | *β* | 90.95° |
| Resolution^{a}: | | 20 -1.95 Å (2.02 -1.95 A) |
| Wavelength: | | 1.5418 |
| % Completeness: | | 92.9 % (85.3 %) |
| Measured reflections: | | 76,950 |
| Unique reflections: | | 25,789 |
| Average redundancy: | | 2.9 (2.45) |
| I/σ: | | 21.5 (6.3) |
| R_{sym}^{b}: | | 3.7% (13.5 %) |

| **Phasing** | | |
|---|---|---|
| Phasing Power^{c}: | | 1.62 (0.95) |
| R_{cullis}^{d}: | | 0.67 |
| Figure of Merit | | |
| SOLVE: | | 0.38 |
| CNS, after dens. mod.: | | 0.86 |

| **Refinement** | | |
|---|---|---|
| Resolution: | | 20 -1.95 Å (2.02 -1.95 A) |
| Number of reflections: | | |
| | Working: | 23,356 (84.1%) |
| | Test set: | 2,430 (8.8 %) |
| Rₓₜₐₗ^{e}: | | 17.8 (24.8) |
| R_{free}: | | 22.8 (31.8) |
| r.m.s.d. bonds: | | 0.0095 Å |
| r.m.s.d. angles: | | 1.70° |
| cross-validated Luzzati coordinate error: | | 0.25 Å |
| cross-validated Sigma-a coordinate error: | | 0.23 Å |
| Average B-factor: | | 22.4 Å² |

| | | |
|---|---|---|
| a. Numbers in parenthesis correspond to the highest resolution shell. b. R_{merge}= ∑\|I-<I>\|/ ∑I, where I is the observed intensity and <I> is the average intensity of multiple measurements of symmetry related reflections. c. Phasing power =〈\|F_{H}\|〉/∥Fp + F_{H}\|-\|F_{PH}∥〉 reported for all reflections. d. R_{cullis} = ∑∥F_{PH} ± F_{P}\| -F_{H}(calc) \|/ ∑\|F_{PH}\| reported for all reflections. e. Rₓₜₐₗ = ∑\|Fₒ - \|F_{c}∥/ ∑\|Fₒ\|, Rₓₜₐₗ from the working set and R_{free} from the test set. | | |

### B. Example 2: Use of Riboswitch Analogs as Antimicrobials

Atomic-resolution models were generated for both a guanine riboswitch aptamer and a related aptamer for adenine (Serganov A, Yuan YR, Pikovskaya O, Polonskaia A, Malinina L, Phan AT, Hobartner C, Micura R, Breaker RR, Patel DJ. (2004) Structural basis for discriminative regulation of gene expression by adenine- and guanine-sensing mRNAs. Chem. Biol. 11:1729-1741). These models are essentially identical to that proposed by Batey et al. (Batey, R.T., Gilbert, S.D., Montange, R.K. (2004) Structure of a natural guanine-responsive riboswitch complexed with the metabolite hypoxanthine. 18:432:411-415.), and therefore shows that the structural model used to design the compounds depicted in Figure 12B is accurate. Furthermore, the similarity between the guanine and adenine riboswitch aptamers makes it clear that the same approaches used to design guanine analogs can be used to inhibit the adenine riboswitch by similarly-designed adenine analogs.

The ability of the guanine analogs depicted in Figure 12B to bind the guanine riboswitch *in vitro* has been tested, as well as their antibacterial activity toward *Bacillus subtilis.* Of these analogs, six inhibit *B. subtilis* growth in media lacking guanine (Table 3). One of these, compound G-014, has been tested for inhibitory activity toward B. *subtilis* in rich media and toward several clinically-relevant pathogens (Table 4). Importantly, G-014 inhibits most of these pathogens, including *Bacillus anthracis* and Methicillin-resistant *Staphylococcus aureus* with similar efficacy as vancomycin.

To more fully probe the antibacterial mechanism of these guanine analogs, two gene reporter systems were developed. In one system, a guanine riboswitch has been fused to Green Fluorescent Protein (GFP), such that high concentrations of guanine or a riboswitch-binding guanine analog inhibit the expression of GFP (Figure 14). Thus, a decreased level of GFP fluorescence indicates binding to and modulation of the guanine riboswitch inside bacteria. A similar system has been constructed with a guanine riboswitch upstream of *β*-galactosidase. Again, a decreased level of *β*-galactosidase activity indicates that a compound represses the guanine riboswitch *in vivo.* Table 3 summarizes the result of these assays for each of the guanine analogs. A correlation between compounds that kill cells and those that control gene expression has also been established. Similar systems can be used involving any suitable reporter gene, such as genes that encode a suitable reporter protein or a suitable reporter RNA (such as a ribozyme).

It has also been determined that G-014 is bactericidal against *Bacillus subtilis.* In a direct comparison, G-014 reduces the number of viable colony forming units ("CFU") at a rate equal to carbenicillin (Figure 15).

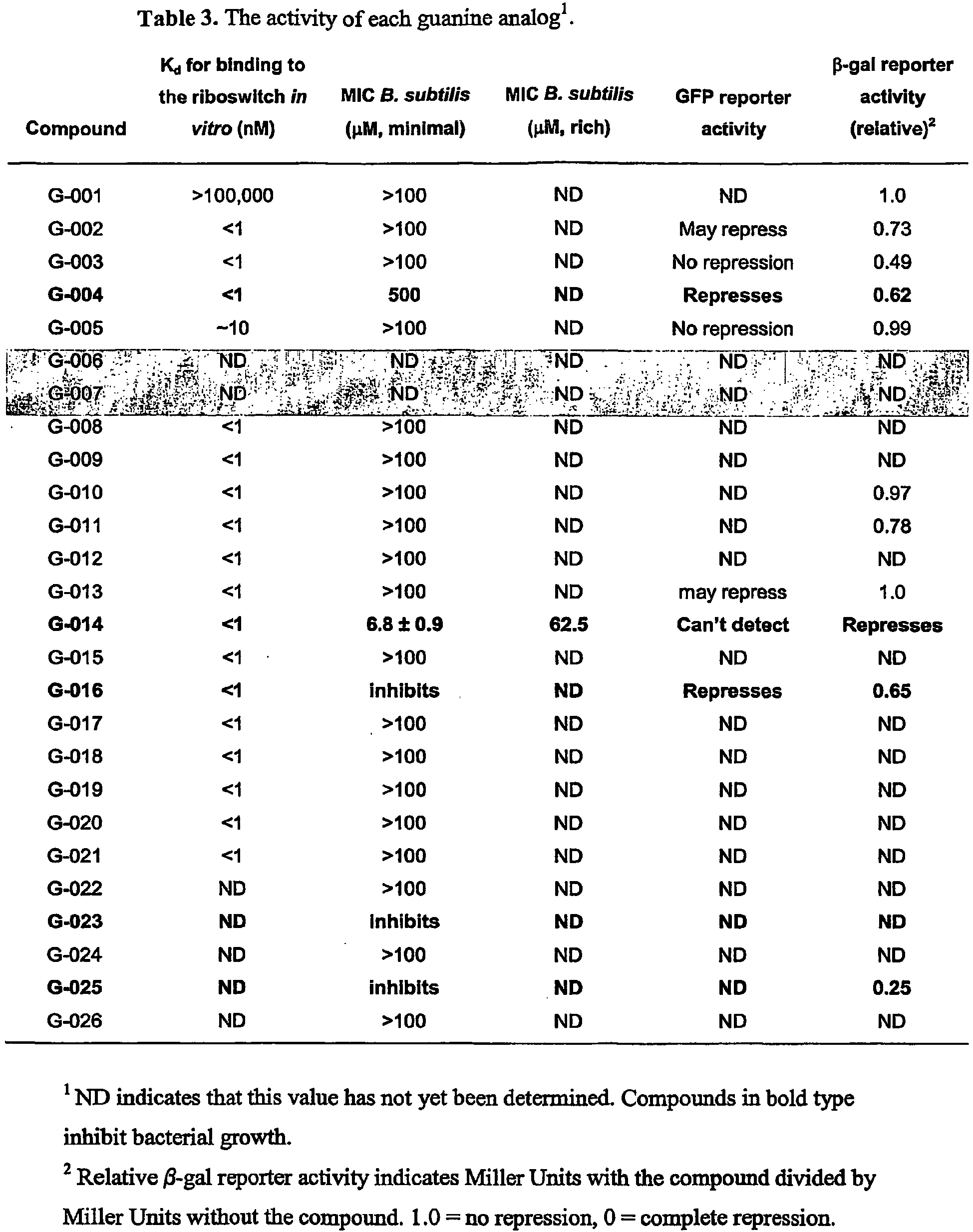

**Table 4. The antibacterial activity of G-014 toward clinically relevant pathogens.**

| | **Minimal inhibitory concentrations (µg/mL)** | | | | | | |
|---|---|---|---|---|---|---|---|
| | | | | | | | |

| **Compound** | **SA¹** | **MRSA** | **EF** | **BA** | **SP** | **HI²** | **MS²** |
|---|---|---|---|---|---|---|---|
| | | | | | | | |
| G-014 | 8 | 4 | 32 | 4 | 2 | 64 | 256 |
| Vancomycin | 1 | 1 | 2 | 1.5 | 0.25 | 64 | 24 |

| | | | | | | | |
|---|---|---|---|---|---|---|---|
| ¹SA, Staphylococcus aureus; MRSA, methicillin-resistant Staphylococcus aureus; EF, Enterococcus faecalis; SP, Streptococcus pneumoniae; HI, Haemophilus influenza; MS, Mycobacterium smegmatis. ² These pathogens do not have a guanine riboswitch. | | | | | | | |

**Table 5. Purine Riboswitches**

| # STOCKHOLM 1.0 | |
|---|---|
| #=GS NC_003869.1/586365-586467 | organism Thermoanaerobacter tengcongensis MB4 |
| #=GS NC_002570.2/1593074-1592972 | organism Bacillus halodurans C-125 |
| #=GS NC_002570.2/676475-676577 | organism Bacillus halodurans C-125 |
| #=GS NC_000964.2/4004541-4004643 | organism Bacillus subtilis subsp. subtilis str. 168 |
| #=GS NC_003366.1/513088-512986 | organism Clostridium perfringens str. 13 |
| #=GS NC_000964.2/693775-693877 | organism Bacillus subtilis subsp. subtilis str. 168 |
| #=GS NC 002570.2/650309-650411 | organism Bacillus halodurans C-125 |
| #=GS NC_006270.2/2294931-2294831 | organism Bacillus licheniformis ATCC 14580 |
| #=GS NC_006322.1/2295788-2295688 | organism Bacillus licheniformis ATCC 14580 |
| #=GS NC_004193.1/1103943-1104045 | organism Oceanobacillus iheyensis HTE831 |
| #=GS NC_003909.8/1650407-1650509 | organism Bacillus cereus ATCC 10987 |
| #=GS NC_003997.3/1497571-1497673 | organism Bacillus anthracis str. Ames |
| #=GS NC_005945.1/1497645-1497747 | organism Bacillus anthracis str. Sterne |
| #=GS NC_005957.1/1521880-1521982 | organism Bacillus thuringiensis serovar konkukian str. 97-27 |
| #=GS NC_006274.1/1532482-1532584 | organism Bacillus cereus ZK |
| #=GS NC_007530.2/1497694-1497796 | organism Bacillus anthracis str. 'Ames Ancestor' |
| #=GS NZ_AAAC02000001.1/1985987-1986089 | organism Bacillus anthracis str. A2012 |
| #=GS NZ_AAEK01000008.1/96466-96364 | organism Bacillus cereus G9241 |
| #=GS NZ_AAEN01000011.1/67143-67245 | organism Bacillus anthracis str. CNEVA-9066 |
| #=GS NZ_AAE001000025.1/66855-66957 | organism Bacillus anthracis str. A1055 |
| #=GS NZ_AAEP01000035.1/69365-69467 | organism Bacillus anthracis str. Vollum |
| #=GS NZ_AAEQ01000029.1/70848-70950 | organism Bacillus anthracis str. Kruger B |
| #=GS NZ_AAER01000023.1/58182-58284 | organism Bacillus anthracis str. Western North America USA6153 |
| #=GS NZ_AAES01000034.1/69121-69223 | organism Bacillus anthracis str. Australia 94 |
| #=GS NC_004722.1/1515239-1515341 | organism Bacillus cereus ATCC 14579 |
| #=GS NC_000964.2/697711-697813 | organism Bacillus subtilis subsp. subtilis str. 168 |
| #=GS NC_002976.3/54816-54919 | organism Staphylococcus epidermidis RP62A |
| #=GS NC_004461.1/2433047-2432944 | organism Staphylococcus epidermidis ATCC 12228 |
| #=GS NC 003030.1/2824953-2824855 | organism Clostridium acetobutylicum ATCC 824 |
| #=GS NC_003909.8/296465-296567 | organism Bacillus cereus ATCC 10987 |
| #=GS NC_003997.3/262601-262703 | organism Bacillus anthracis str. Ames |
| #=GS NC_004722.1/261558-261660 | organism Bacillus cereus ATCC 14579 |
| #=GS NC_005945.1/262614-262716 | organism Bacillus anthracis str. Sterne |
| #=GS NC_005957.1/268537-268639 | organism Bacillus thuringiensis serovar konkukian str. 97-27 |
| #=GS NC_006274.1/267835-267937 | organism Bacillus cereus ZK |
| #=GS NC_007530.2/262601-262703 | organism Bacillus anthracis str. 'Ames Ancestor' |
| #=GS NZ_AAAC02000001.1/796036-796138 | organism Bacillus anthracis str. A2012 |
| #=GS NZ_AAEK01000017.1/88397-88499 | organism Bacillus cereus G9241 |
| #=GS NZ_AAEN01000023.1/16980-17082 | organism Bacillus anthracis str. CNEVA-9066 |
| #=GS NZ_AAE001000030.1/17090-17192 | organism Bacillus anthracis str. A1055 |
| #=GS NZ_AAEP01000043.1/12962-13064 | organism Bacillus anthracis str. Vollum |
| #=GS NZ_AAEQ01000040.1/4962-4860 | organism Bacillus anthracis str. Kruger B |
| #=GS NZ_AAER01000030.1/4374-4272 | organism Bacillus anthracis str. Western North America USA6153 |
| #=GS NZ_AAES01000040.1/14760-14862 | organism Bacillus anthracis str. Australia 94 |
| #=GS NC_006270.2/693198-693300 | organism Bacillus licheniformis ATCC 14580 |
| #=GS NC_006322.1/692981-693083 | organism Bacillus licheniformis ATCC 14580 |
| #=GS NC_004722.1/259601-259703 | organism Bacillus cereus ATCC 14579 |
| #=GS NC_000964.2/2319410-2319310 | organism Bacillus subtilis subsp. subtilis str. 168 |
| #=GS NC 003030.1/2905050-2904948 | organism Clostridium acetobutylicum ATCC 824 |
| #=GS NC_003210.1/611119-611017 | organism Listeria monocytogenes EGD-e |
| #=GS NZ_AADQ01000001.1/2431-2533 | organism Listeria monocytogenes str. 1/2a F6854 |
| #=GS NC_003212.1/610156-610054 | organism Listeria innocua Clip11262 |
| #=GS NC 003366.1/422820-422922 | organism Clostridium perfringens str. 13 |
| #=GS NC_002973.5/617846-617744 | organism Listeria monocytogenes str. 4b F2365 |
| #=GS NZ_AADR01000111.1/1598-1496 | organism Listeria monocytogenes str. 4b H7858 |
| #=GS NC_003909.8/294509-294611 | organism Bacillus cereus ATCC 10987 |
| #=GS NC_005957.1/266577-266679 | organism Bacillus thuringiensis serovar konkukian str. 97-27 |
| #=GS NC_006274.1/265875-265977 | organism Bacillus cereus ZK |
| #=GS NC 003366.1/2618421-2618323 | organism Clostridium perfringens str. 13 |
| #=GS NC_002973.5/1940027-1939926 | organism Listeria monocytogenes str. 4b F2365 |
| #=GS NC 003212.1/2013345-2013244 | organism Listeria innocua Clip11262 |
| #=GS NZ_AADQ01000095.1/1690-1791 | organism Listeria monocytogenes str. 1/2a F6854 |
| #=GS NZ_AADR01000082.1/3415-3516 | organism Listeria monocytogenes str. 4b H7858 |
| #=GS NC_003366.1/2871201-2871101 | organism Clostridium perfringens str. 13 |
| #=GS NC 002745.2/430797-430900 | organism Staphylococcus aureus subsp. aureus N315 |
| #=GS NC_002758.2/430754-430857 | organism Staphylococcus aureus subsp. aureus Mu50 |
| #=GS NC_002951.2/460059-460162 | organism Staphylococcus aureus subsp. aureus COL |
| #=GS NC_002952.2/441050-441153 | organism Staphylococcus aureus subsp. aureus MRSA252 |
| #=GS NC_002953.3/409191-409294 | organism Staphylococcus aureus subsp. aureus MSSA476 |
| #=GS NC 003923.1/410546-410649 | organism Staphylococcus aureus subsp. aureus MW2 |
| #=GS NC_006582.1/1115665-1115767 | organism Bacillus clausii KSM-K16 |
| #=GS NC_006510.1/274257-274359 | organism Geobacillus kaustophilus HTA426 |
| #=GS NC_003210.1/1958922-1958821 | organism Listeria monocytogenes EGD-e |
| #=GS NC_006270.2/697054-697156 | organism Bacillus licheniformis ATCC 14580 |
| #=GS NC_006322.1/696838-696940 | organism Bacillus licheniformis ATCC 14580 |
| #=GS NC_006510.1/282580-282682 | organism Geobacillus kaustophilus HTA426 |
| #=GS NC_003997.3/260641-260743 | organism Bacillus anthracis str. Ames |
| #=GS NC_005945.1/260654-260756 | organism Bacillus anthracis str. Sterne |
| #=GS NC-007530.2/260641-260743 | organism Bacillus anthracis str. 'Ames Ancestor' |
| #=GS NZ-AAEK01000017.1/86437-86539 | organism Bacillus cereus G9241 |
| #=GS NZ_AAEN01000023.1/15020-15122 | organism Bacillus anthracis str. CNEVA-9066 |
| #=GS NZ_AAE001000030.1/15130-15232 | organism Bacillus anthracis str. A1055 |
| #=GS NZ_AAEP01000043.1/11002-11104 | organism Bacillus anthracis str. Vollum |
| #=GS NZ_AAEQ01000040.1/6922-6820 | organism Bacillus anthracis str. Kruger B |
| #=GS NZ_AAER01000030.1/6334-6232 | organism Bacillus anthracis str. Western North America USA6153 |
| #=GS NZ_AAES01000040.1/12800-12902 | organism Bacillus anthracis str. Australia 94 |
| #=GS NC_004193.1/760473-760575 | organism Oceanobacillus iheyensis HTE831 |
| #=GS NC_006270.2/4024210-4024312 | organism Bacillus licheniformis ATCC 14580 |
| #=GS NC_006322.1/4024324-4024426 | organism Bacillus licheniformis ATCC 14580 |
| #=GS NC_004193.1/786767-786868 | organism Oceanobacillus iheyensis HTE831 |
| #=GS NC_002662.1/1159509-1159607 | organism Lactococcus lactis subsp. lactis I11403 |
| #=GS NC_000964.2/625993-625893 | organism Bacillus subtilis subsp. subtilis str. 168 |
| #=GS NC_003909.8/382630-382528 | organism Bacillus cereus ATCC 10987 |
| #=GS NC_003997.3/342356-342254 | organism Bacillus anthracis str. Ames |
| #=GS NC_005945.1/342369-342267 | organism Bacillus anthracis str. Sterne |
| #=GS NC_005957.1/356354-356252 | organism Bacillus thuringiensis serovar konkukian str. 97-27 |
| #=GS NC_006274.1/357462-357360 | organism Bacillus cereus ZK |
| #=GS NC_007530.2/342356-342254 | organism Bacillus anthracis str. 'Ames Ancestor' |
| #=GS NZ_AAAC02000001.1/859268-859166 | organism Bacillus anthracis str. A2012 |
| #=GS NZ_AAEK01000051.1/8150-8252 | organism Bacillus cereus G9241 |
| #=GS NZ_AAEN01000023.1/83441-83339 | organism Bacillus anthracis str. CNEVA-9066 |
| #=GS NZ_AAE001000030.1/96886-96784 | organism Bacillus anthracis str. A1055 |
| #=GS NZ_AAEP01000046.1/48810-48708 | organism Bacillus anthracis str. Vollum |
| #=GS NZ_AAEQ01000034.1/49483-49381 | organism Bacillus anthracis str. Kruger B |
| #=GS NZ_AAER01000042.1/191339-191441 | organism Bacillus anthracis str. Western North America USA6153 |
| #=GS NZ_AAES01000043.1/48479-48377 | organism Bacillus anthracis str. Australia 94 |
| #=GS NZ_AAAC02000001.1/794076-794178 | organism Bacillus anthracis str. A2012 |
| #=GS NC_003030.1/1002176-1002275 | organism Clostridium acetobutylicum ATCC 824 |
| #=GS NC_006582.1/1554717-1554819 | organism Bacillus clausii KSM-K16 |
| #=GS NC_003909.8/336194-336296 | organism Bacillus cereus ATCC 10987 |
| #=GS NC_003997.3/295331-295433 | organism Bacillus anthracis str. Ames |
| #=GS NC_005945.1/295344-295446 | organism Bacillus anthracis str. Sterne |
| #=GS NC_005957.1/309524-309626 | organism Bacillus thuringiensis serovar konkukian str. 97-27 |
| #=GS NC_006274.1/309094-309196 | organism Bacillus cereus ZK |
| #=GS NC_007530.2/295331-295433 | organism Bacillus anthracis str. 'Ames Ancestor' |
| #=GS NZ AAAC02000001.1/812243-812345 | organism Bacillus anthracis str. A2012 |
| #=GS NZ_AAEK01000064.1/23153-23051 | organism Bacillus cereus G9241 |
| #=GS NZ AAEN01000023.1/36414-36516 | organism Bacillus anthracis str. CNEVA-9066 |
| #=GS NZ_AAE001000030.1/49824-49926 | organism Bacillus anthracis str. A1055 |
| #=GS NZ_AAEP01000046.1/1785-1887 | organism Bacillus anthracis str. Vollum |
| #=GS NZ_AA.EQ01000034.1/2457-2559 | organism Bacillus anthracis str. Kruger B |
| #=GS NZ_AAER01000042.1/238364-238262 | organism Bacillus anthracis str. Western North America USA6153 |
| #=GS NZ_AAES01000043.1/1489-1591 | organism Bacillus anthracis str. Australia 94 |
| #=GS NC_004193.1/769686-769787 | organism Oceanobacillus iheyensis HTE831 |
| #=GS NC_004722.1/343847-343745 | organism Bacillus cereus ATCC 14579 |
| #=GS NZ_AAAW03000042.1/15038-14936 | organism Desulfitobacterium hafniense DCB-2 |
| #=GS NZ_AADT03000005.1/34629-34731 | organism Moorella thermoacetica ATCC 39073 |
| #=GS NC_002570.2/648442-648544 | organism Bacillus halodurans C-125 |
| #=GS NC_004722.1/298774-298876 | organism Bacillus cereus ATCC 14579 |
| #=GS NC_006510.1/272473-272575 | organism Geobacillus kaustophilus HTA426 |
| #=GS NZ_AADW02000004.1/225764-225662 | organism Exiguobacterium sp. 255-15 |
| #=GS NC 006274.1/3685852-3685750 | organism Bacillus cereus ZK |
| #=GS NZ_AAGO01000011.1/2345-2247 | organism Lactococcus lactis subsp. cremoris SK11 |
| #=GS NZ_AAAK03000113.1/5724-5822 | organism Enterococcus faecium DO |
| #=GS NZ_AADW02000005.1/186378-186480 | organism Exiguobacterium sp. 255-15 |
| #=GS NC_003909.8/3578068-3577966 | organism Bacillus cereus ATCC 10987 |
| #=GS NC_003997.3/3605298-3605196 | organism Bacillus anthracis str. Ames |
| #=GS NC_004722.1/3766254-3766152 | organism Bacillus cereus ATCC 14579 |
| #=GS NC_005945.1/3605993-3605891 | organism Bacillus anthracis str. Sterne |
| #=GS NC_005957.1/3626969-3626867 | organism Bacillus thuringiensis serovar konkukian str. 97-27 |
| #=GS NC_007530.2/3605425-3605323 | organism Bacillus anthracis str. 'Ames Ancestor' |
| #=GS NZ_AAAC02000001.1/4059572-4059470 | organism Bacillus anthracis str. A2012 |
| #=GS NZ_AAEN01000012.1/139994-140096 | organism Bacillus anthracis str. CNEVA-9066 |
| #=GS NZ_AAE001000020.1/51249-51351 | organism Bacillus anthracis str. A1055 |
| #=GS NZ_AAEP01000042.1/12489-12387 | organism Bacillus anthracis str. Vollum |
| #=GS NZ_AAEQ01000019.1/51159-51261 | organism Bacillus anthracis str. Kruger B |
| #=GS NZ_AAER01000035.1/118314-118212 | organism Bacillus anthracis str. Western North America USA6153 |
| #=GS NZ_AAES01000032.1/51320-51422 | organism Bacillus anthracis str. Australia 94 |
| #=GS NC_006371.1/1538896-1538796 | organism Photobacterium profundum SS9 |
| #=GS NC_004460.1/504371-504471 | organism Vibrio vulnificus CMCP6 |
| #=GS NC_005140.1/1130553-1130653 | organism Vibrio vulnificus YJ016 |
| #=GS NC_004116.1/1094305-1094209 | organism Streptococcus agalactiae 2603V/R |
| #=GS NC_004368.1/1163490-1163394 | organism Streptococcus agalactiae NEM316 |
| #=GS NZ_AADT03000005.1/42245-42347 | organism Moorella thermoacetica ATCC 39073 |
| #=GS NC_004557.1/2551392-2551293 | organism Clostridium tetani E88 |
| #=GS NC_003028.1/1754786-1754882 | organism Streptococcus pneumoniae TIGR4 |
| #=GS NC_003098.1/1634825-1634921 | organism Streptococcus pneumoniae R6 |
| #=GS NZ_AAGY01000085.1/5640-5736 | organism Streptococcus pneumoniae TIGR4 |
| #=GS NZ_AAEK01000001.1/183902-184006 | organism Bacillus cereus G9241 |
| #=GS NC_003454.1/1645820-1645721 | organism Fusobacterium nucleatum subsp. nucleatum ATCC 25586 |
| #=GS NZ_AADW02000027.1/2980-2880 | organism Exiguobacterium sp. 255-15 |
| #=GS NZ_AADW02000005.1/179959-180061 | organism Exiguobacterium sp. 255-15 |
| #=GS NC_006582.1/1039390-1039492 | organism Bacillus clausii KSM-K16 |
| #=GS NC_002737.1/930749-930845 | organism Streptococcus pyogenes M1 GAS |
| #=GS NC_003485.1/910599-910695 | organism Streptococcus pyogenes MGAS8232 |
| #=GS NC_004070.1/846557-846653 | organism Streptococcus pyogenes MGAS315 |
| #=GS NC_004606.1/977077-977173 | organism Streptococcus pyogenes SSI-1 |
| #=GS NC_006086.1/857664-857760 | organism Streptococcus pyogenes MGAS10394 |
| #=GS NZ_AAFV01000199.1/507-411 | organism Streptococcus pyogenes M49 591 |
| #=GS NC_004605.1/1369721-1369821 | organism Vibrio parahaemolyticus RIMD 2210633 |
| #=GS NZ_AAAW03000004.1/66862-66959 | organism Desulfitobacterium hafniense DCB-2 |
| #=GS NC_004567.1/2968830-2968731 | organism Lactobacillus plantarum WCFS1 |
| #=GS NZ_AAEV01000003.1/13765-13667 | organism Pediococcus pentosaceus ATCC 25745 |
| #=GS NC_002570.2/806873-806971 | organism Bacillus halodurans C-125 |
| #=GS NZ_AAEK01000052.1/27552-27452 | organism Bacillus cereus G9241 |
| #=GS NC_006814.1/237722-237626 | organism Lactobacillus acidophilus NCFM |
| #=GS NC_005363.1/3414604-3414703 | organism Bdellovibrio bacteriovorus HD100 |
| #=GS NZ_AADT03000021.1/9410-9513 | organism Moorella thermoacetica ATCC 39073 |
| #=GS NC_004668.1/2288426-2288328 | organism Enterococcus faecalis V583 |
| #=GS NC_006449.1/1182949-1183044 | organism Streptococcus thermophilus CNRZ1066 |
| #=GS NZ_RAGS01000026.1/9921-10016 | organism Streptococcus thermophilus LMD-9 |
| #=GS NZ_AAGQ01000089.1/170-269 | organism Lactobacillus delbrueckii subsp. bulgaricus ATCC BAA-365 |
| #=GS NZ_AABF02000293.1/1-98 | organism Fusobacterium nucleatum subsp. vincentii ATCC 49256 |
| #=GS NC_006448.1/1185082-1185177 | organism Streptococcus thermophilus LMG 18311 |
| #=GS NC_004567.1/2410478-2410577 | organism Lactobacillus plantarum WCFS1 |
| #=GS NZ_AABJ03000010.1/47704-47802 | organism Oenococcus oeni PSU-1 |
| #=GS NZ_AADT03000002.1/89184-89083 | organism Moorella thermoacetica ATCC 39073 |
| #=GS NC_006814.1/1971978-1972076 | organism Lactobacillus acidophilus NCFM |
| #=GS NZ_AAA002000016.1/1579-1480 | organism Lactobacillus gasseri |
| #=GS NC_005362.1/1949387-1949485 | organism Lactobacillus johnsonii NCC 533 |
| #=GS NZ_AAA002000035.1/3299-3201 | organism Lactobacillus gasseri |
| #=GS NC_005362.1/263146-263049 | organism Lactobacillus johnsonii NCC 533 |
| #=GS NC_005363.1/2004933-2004835 | organism Bdellovibrio bacteriovorus HD100 |
| #=GS NZ_AADT03000002.1/98293-98192 | organism Moorella thermoacetica ATCC 39073 |
| #=GS NZ_AABH02000036.1/17403-17500 | organism Leuconostoc mesenteroides subsp. mesenteroides ATCC 8293 |
| #=GS NC_006055.1/484139-484044 | organism Mesoplasma florum L1 |
| #=GS NZ_AABH02000272.1/211-308 | organism Leuconostoc mesenteroides subsp. mesenteroides ATCC 8293 |
| #=GS NZ_AABJ03000003.1/47905-47810 | organism Oenococcus oeni PSU-1 |
| #=GS NZ_AABH02000038.1/17559-17463 | organism Leuconostoc mesenteroides subsp. mesenteroides ATCC 8293 |
| #=GS NC_006055.1/397027-397123 | organism Mesoplasma florum L1 |
| #=GS NC_003869.1/586365-586467 | taxonomy Bacteria; Firmicutes; Clostridia; Thermoanaerobacteriales; Thermoanaerobacteriaceae; Thermoanaerobacter; tengcongensis |
| #=GS NC_002570.2/1593074-1592972 | taxonomy Bacteria; Firmicutes; Bacillales; Bacillaceae; Bacillus; |
| #=GS NC_002570.2/676475-676577 | taxonomy Bacteria; Firmicutes; Bacillales; Bacillaceae; Bacillus; halodurans |
| #=GS NC_000964.2/4004541-4004643 | taxonomy Bacteria; Firmicutes; Bacillales; Bacillaceae; Bacillus; subtilis |
| #=GS NC_003366.1/513088-512986 | taxonomy Bacteria; Firmicutes; Clostridia; Clostridiales; Clostridium; perfringens |
| #=GS NC_000964.2/693775-693877 | taxonomy Bacteria; Firmicutes; Bacillales; Bacillaceae; Bacillus; subtilis |
| #=GS NC_002570.2/650309-650411 | taxonomy Bacteria; Firmicutes; Bacillales; Bacillaceae; Bacillus; |
| #=GS NC_006270.2/2294931-2294831 | taxonomy Bacteria; Firmicutes; Bacillales; Bacillaceae; Bacillus; |
| #=GS NC_006322.1/2295788-2295688 | taxonomy Bacteria; Firmicutes; Bacillales; Bacillaceae; Bacillus; licheniformis |
| #=GS NC_004193.1/1103943-1104045 | taxonomy Bacteria; Firmicutes; Bacillales; Bacillaceae; Oceanobacillus; |
| #=GS NC_003909.8/1650407-1650509 | taxonomy Bacteria; Firmicutes; Bacillales; Bacillaceae; Bacillus; Bacillus; cereus group; Bacillus; cereus |
| #=GS NC_003997.3/1497571-1497673 | taxonomy Bacteria; Firmicutes; Bacillales; Bacillaceae; Bacillus; |
| | Bacillus; cereus group; Bacillus; anthracis |
| #=GS NC_005945.1/1497645-1497747 | taxonomy Bacteria; Firmicutes; Bacillales; Bacillaceae; Bacillus; cereus group; Bacillus; anthracis |
| #=GS NC_005957.1/1521880-1521982 | taxonomy Bacteria; Firmicutes; Bacillales; Bacillaceae; Bacillus; cereus group; Bacillus; thuringiensis |
| #=GS NC_006274.1/1532482-1532584 | taxonomy Bacteria; Firmicutes; Bacillales; Bacillaceae; Bacillus; Bacillus; cereus group; Bacillus; cereus |
| #=GS NC_007530.2/1497694-1497796 | taxonomy Bacteria; Firmicutes; Bacillales; Bacillaceae; Bacillus; cereus group; Bacillus; anthracis |
| #=GS NZ_AAAC02000001.1/1985987-1986089 | taxonomy Bacteria; Firmicutes; Bacillales; Bacillaceae; Bacillus; cereus group; Bacillus; anthracis |
| #=GS NZ_AAEK01000008.1/96466-96364 | taxonomy Bacteria; Firmicutes; Bacillales; Bacillaceae; Bacillus; Bacillus; cereus group; Bacillus; cereus |
| #=GS NZ_AAEN01000011.1/67143-67245 | taxonomy Bacteria; Firmicutes; Bacillales; Bacillaceae; Bacillus; cereus group; Bacillus; anthracis |
| #=GS NZ_AAE001000025.1/66855-66957 | taxonomy Bacteria; Firmicutes; Bacillales; Bacillaceae; Bacillus; Bacillus; cereus group; Bacillus; anthracis |
| #=GS NZ_AAEP01000035.1/69365-69467 | taxonomy Bacteria; Firmicutes; Bacillales; Bacillaceae; Bacillus; cereus group; Bacillus; anthracis |
| #=GS NZ_AAEQ01000029.1/70848-70950 | taxonomy Bacteria; Firmicutes; Bacillales; Bacillaceae; Bacillus; Bacillus; cereus group; Bacillus; anthracis |
| #=GS NZ_AAER01000023.1/58182-58284 | taxonomy Bacteria; Firmicutes; Bacillales; Bacillaceae; Bacillus; cereus group; Bacillus; anthracis |
| #=GS NZ_AAES01000034.1/69121-69223 | taxonomy Bacteria; Firmicutes; Bacillales; Bacillaceae; Bacillus; cereus group; Bacillus; anthracis |
| #=GS NC_004722.1/1515239-1515341 | taxonomy Bacteria; Firmicutes; Bacillales; Bacillaceae; Bacillus; cereus group; Bacillus; cereus |
| #=GS NC_000964.2/697711-697813 | taxonomy Bacteria; Firmicutes; Bacillales; Bacillaceae; Bacillus; subtilis |
| #=GS NC_002976.3/54816-54919 | taxonomy Bacteria; Firmicutes; Bacillales; Staphylococcus; epidermidis |
| #=GS NC_004461.1/2433047-2432944 | taxonomy Bacteria; Firmicutes; Bacillales; Staphylococcus; epidermidis |
| #=GS NC_003030.1/2824953-2824855 | taxonomy Bacteria; Firmicutes; Clostridia; Clostridiales; Clostridium; acetobutylicum |
| #=GS NC_003909.8/296465-296567 | taxonomy Bacteria; Firmicutes; Bacillales; Bacillaceae; Bacillus; Bacillus; cereus group; Bacillus; cereus |
| #=GS NC_003997.3/262601-262703 | taxonomy Bacteria; Firmicutes; Bacillales; Bacillaceae; Bacillus; cereus group; Bacillus; anthracis |
| #=GS NC_004722.1/261558-261660 | taxonomy Bacteria; Firmicutes; Bacillales; Bacillaceae; Bacillus; |
| #=GS NC_005945.1/262614-262716 | Bacillus; cereus group; Bacillus; cereus taxonomy Bacteria; Firmicutes; Bacillales; Bacillaceae; Bacillus; cereus group; Bacillus; anthracis |
| #=GS NC_005957.1/268537-268639 | taxonomy Bacteria; Firmicutes; Bacillales; Bacillaceae; Bacillus; Bacillus; cereus group; Bacillus; thuringiensis |
| #=GS NC_006274.1/267835-267937 | taxonomy Bacteria; Firmicutes; Bacillales; Bacillaceae; Bacillus; Bacillus; cereus group; Bacillus; cereus |
| #=GS NC_007530.2/262601-262703 | taxonomy Bacteria; Firmicutes; Bacillales; Bacillaceae; Bacillus; Bacillus; cereus group; Bacillus; anthracis |
| #=GS NZ_AAAC02000001.1/796036-796138 | taxonomy Bacteria; Firmicutes; Bacillales; Bacillaceae; Bacillus; Bacillus; cereus group; Bacillus; anthracis |
| #=GS NZ_AAEK01000017.1/88397-88499 | taxonomy Bacteria; Firmicutes; Bacillales; Bacillaceae; Bacillus; Bacillus; cereus group; Bacillus; cereus |
| #=GS NZ_AAEN01000023.1/16980-17082 | taxonomy Bacteria; Firmicutes; Bacillales; Bacillaceae; Bacillus; cereus group; Bacillus; anthracis |
| #=GS NZ_AAE001000030.1/17090-17192 | taxonomy Bacteria; Firmicutes; Bacillales; Bacillaceae; Bacillus; cereus group; Bacillus; anthracis |
| #=GS NZ_AAEP01000043.1/12962-13064 | taxonomy Bacteria; Firmicutes; Bacillales; Bacillaceae; Bacillus; Bacillus; cereus group; Bacillus; anthracis |
| #=GS NZ_AAEQ01000040.1/4962-4860 | taxonomy Bacteria; Firmicutes; Bacillales; Bacillaceae; Bacillus; cereus group; Bacillus; anthracis |
| #=GS NZ_AAER01000030.1/4374-4272 | taxonomy Bacteria; Firmicutes; Bacillales; Bacillaceae; Bacillus; Bacillus; cereus group; Bacillus; anthracis |
| #=GS NZ_AAES01000040.1/14760-14862 | taxonomy Bacteria; Firmicutes; Bacillales; Bacillaceae; Bacillus; Bacillus; cereus group; Bacillus; anthracis |
| #=GS NC_006270.2/693198-693300 | taxonomy Bacteria; Firmicutes; Bacillales; Bacillaceae; Bacillus; |
| #=GS NC_006322.1/692981-693083 | taxonomy Bacteria; Firmicutes; Bacillales; Bacillaceae; Bacillus; |
| #=GS NC_004722.1/259601-259703 | taxonomy Bacteria; Firmicutes; Bacillales; Bacillaceae; Bacillus; cereus group; Bacillus; cereus |
| #=GS NC_000964.2/2319410-2319310 | taxonomy Bacteria; Firmicutes; Bacillales; Bacillaceae; Bacillus; subtilis |
| #=GS NC_003030.1/2905050-2904948 | taxonomy Bacteria; Firmicutes; Clostridia; Clostridiales; Clostridium; acetobutylicum |
| #=GS NC_003210.1/611119-611017 | taxonomy Bacteria; Firmicutes; Bacillales; Listeriaceae; Listeria; monocytogenes |
| #=GS NZ_AADQ01000001.1/2431-2533 | taxonomy Bacteria; Firmicutes; Bacillales; Listeriaceae; Listeria; |
| #=GS NC_003212.1/610156-610054 | monocytogenes taxonomy Bacteria; Firmicutes; Bacillales; Listeriaceae; Listeria; |
| #=GS NC_003366.1/422820-422922 | taxonomy Bacteria; Firmicutes; Clostridia; Clostridiales; Clostridiaceae; Clostridium; perfringens |
| #=GS NC_002973.5/617846-617744 | taxonomy Bacteria; Firmicutes; Bacillales; Listeriaceae; Listeria; monocytogenes |
| #=GS NZ_AADR01000111.1/1598-1496 | taxonomy Bacteria; Firmicutes; Bacillales; Listeriaceae; Listeria; monocytogenes |
| #=GS NC_003909.8/294509-294611 | taxonomy Bacteria; Firmicutes; Bacillales; Bacillaceae; Bacillus; Bacillus; cereus group; Bacillus; cereus |
| #=GS NC_005957.1/266577-266679 | taxonomy Bacteria; Firmicutes; Bacillales; Bacillaceae; Bacillus; Bacillus; cereus group; Bacillus; thuringiensis |
| #=GS NC_006274.1/265875-265977 | taxonomy Bacteria; Firmicutes; Bacillales; Bacillaceae; Bacillus; Bacillus; cereus group; Bacillus; cereus |
| #=GS NC_003366.1/2618421-2618323 | taxonomy Bacteria; Firmicutes; Clostridia; Clostridiales; Clostridium; perfringens |
| #=GS NC_002373.5/1940027-1939926 | taxonomy Bacteria; Firmicutes; Bacillales; Listeriaceae; Listeria; monocytogenes |
| #=GS NC_003212.1/2013345-2013244 | taxonomy Bacteria; Firmicutes; Bacillales; Listeriaceae; Listeria; innocua |
| #=GS NZ_AADQ01000095.1/1690-1791 | taxonomy Bacteria; Firmicutes; Bacillales; Listeriaceae; Listeria; monocytogenes |
| #=GS NZ_AADR01000082.1/3415-3516 | taxonomy Bacteria; Firmicutes; Bacillales; Listeriaceae; Listeria; monocytogenes |
| #=GS NC_003366.1/2871201-2871101 | taxonomy Bacteria; Firmicutes; Clostridia; Clostridiales; Clostridiaceae; Clostridium; perfringens |
| #=GS NC_002745.2/430797-430900 | taxonomy Bacteria; Firmicutes; Bacillales; Staphylococcus; aureus |
| #=GS NC_002758.2/430754-430857 | taxonomy Bacteria; Firmicutes; Bacillales; Staphylococcus; aureus |
| #=GS NC_002951.2/460059-460162 | taxonomy Bacteria; Firmicutes; Bacillales; Staphylococcus; aureus |
| #=GS NC_002952.2/441050-441153 | taxonomy Bacteria; Firmicutes; Bacillales; Staphylococcus; aureus |
| #=GS NC_002953.3/409191-409294 | taxonomy Bacteria; Firmicutes; Bacillales; Staphylococcus; aureus |
| #=GS NC_003923.1/410546-410649 | taxonomy Bacteria; Firmicutes; Bacillales; Staphylococcus; aureus |
| #=GS NC_006582.1/1115665-1115767 | taxonomy Bacteria; Firmicutes; Bacillales; Bacillaceae; Bacillus; clausii |
| #=GS NC_006510.1/274257-274359 | taxonomy Bacteria; Firmicutes; Bacillales; Bacillaceae; Geobacillus; kaustophilus |
| #=GS NC_003210.1/1958922-1958821 | taxonomy Bacteria; Firmicutes; Bacillales; Listeriaceae; Listeria; |
| | monocytogenes |
| #=GS NC_006270.2/697054-697156 | taxonomy Bacteria; Firmicutes; Bacillales; Bacillaceae; Bacillus; licheniformis |
| #=GS NC_006322.1/696838-696940 | taxonomy Bacteria; Firmicutes; Bacillales; Bacillaceae; Bacillus; |
| #=GS NC_006510.1/282580-282682 | taxonomy Bacteria; Firmicutes; Bacillales; Bacillaceae; Geobacillus; kaustophilus |
| #=GS NC_003997.3/260641-260743 Bacillus; | taxonomy Bacteria; Firmicutes; Bacillales; Bacillaceae; Bacillus; cereus group; Bacillus; anthracis |
| #=GS NC_005945.1/260654-260756 Bacillus; | taxonomy Bacteria; Firmicutes; Bacillales; Bacillaceae; Bacillus; cereus group; Bacillus; anthracis |
| #=GS NC_007530.2/260641-260743 | taxonomy Bacteria; Firmicutes; Bacillales; Bacillaceae; Bacillus; cereus group; Bacillus; anthracis |
| #=GS NZ_AAEK01000017.1/86437-86539 | taxonomy Bacteria; Firmicutes; Bacillales; Bacillaceae; Bacillus; Bacillus; cereus group; Bacillus; cereus |
| #=GS NZ_AAEN01000023.1/15020-15122 | taxonomy Bacteria; Firmicutes; Bacillales; Bacillaceae; Bacillus; Bacillus; cereus group; Bacillus; anthracis |
| #=GS NZ_AAEO01000030.1/15130-15232 | taxonomy Bacteria; Firmicutes; Bacillales; Bacillaceae; Bacillus; Bacillus; cereus group; Bacillus; anthracis |
| #=GS NZ_AAEP01000043.1/11002-11104 | taxonomy Bacteria; Firmicutes; Bacillales; Bacillaceae; Bacillus; Bacillus; cereus group; Bacillus; anthracis |
| #=GS NZ_AAEQ01000040.1/6922-6820 | taxonomy Bacteria; Firmicutes; Bacillales; Bacillaceae; Bacillus; Bacillus; cereus group; Bacillus; anthracis |
| #=GS NZ_AAER01000030.1/6334-6232 | taxonomy Bacteria; Firmicutes; Bacillales; Bacillaceae; Bacillus; cereus group; Bacillus; anthracis |
| #=GS NZ_AAES01000040.1/12800-12902 | taxonomy Bacteria; Firmicutes; Bacillales; Bacillaceae; Bacillus; Bacillus; cereus group; Bacillus; anthracis |
| #=GS NC_004193.1/760473-760575 iheyensis | taxonomy Bacteria; Firmicutes; Bacillales; Bacillaceae; Oceanobacillus; |
| #=GS NC_006270.2/4024210-4024312 | taxonomy Bacteria; Firmicutes; Bacillales; Bacillaceae; Bacillus; licheniformis |
| #=GS NC_006322.1/4024324-4024426 | taxonomy Bacteria; Firmicutes; Bacillales; Bacillaceae; Bacillus; licheniformis |
| #=GS NC_004193.1/786767-786868 | taxonomy Bacteria; Firmicutes; Bacillales; Bacillaceae; Oceanobacillus; iheyensis |
| #=GS NC_002662.1/1159509-1159607 | taxonomy Bacteria; Firmicutes; Lactobacillales; Streptococcaceae; Lactococcus; lactis |
| #=GS NC_000964.2/625993-625893 | taxonomy Bacteria; Firmicutes; Bacillales; Bacillaceae; Bacillus; |
| | subtilis |
| #=GS NC_003909.8/382630-382528 | taxonomy Bacteria; Firmicutes; Bacillales; Bacillaceae; Bacillus; Bacillus; cereus group; Bacillus; cereus |
| #=GS NC_003997.3/342356-342254 | taxonomy Bacteria; Firmicutes; Bacillales; Bacillaceae; Bacillus; cereus group; Bacillus; anthracis |
| #=GS NC_005945.1/342369-342267 | taxonomy Bacteria; Firmicutes; Bacillales; Bacillaceae; Bacillus; cereus group; Bacillus; anthracis |
| #=GS NC_005957.1/356354-356252 | taxonomy Bacteria; Firmicutes; Bacillales; Bacillaceae; Bacillus; Bacillus; cereus group; Bacillus; thuringiensis |
| #=GS NC_006274.1/357462-357360 | taxonomy Bacteria; Firmicutes; Bacillales; Bacillaceae; Bacillus; Bacillus; cereus group; Bacillus; cereus |
| #=GS NC_007530.2/342356-342254 | taxonomy Bacteria; Firmicutes; Bacillales; Bacillaceae; Bacillus; cereus group; Bacillus; anthracis |
| #=GS NZ_AAAC02000001.1/859268-859166 | taxonomy Bacteria; Firmicutes; Bacillales; Bacillaceae; Bacillus; cereus group; Bacillus; anthracis |
| #=GS NZ_AAEKOI000051.1/8150-8252 | taxonomy Bacteria; Firmicutes; Bacillales; Bacillaceae; Bacillus; Bacillus; cereus group; Bacillus; cereus |
| #=GS NZ_AAENOI000023.1/83441-83339 | taxonomy Bacteria; Firmicutes; Bacillales; Bacillaceae; Bacillus; cereus group; Bacillus; anthracis |
| #=GS NZ_AAEO01000030.1/96886-96784 | taxonomy Bacteria; Firmicutes; Bacillales; Bacillaceae; Bacillus; cereus group; Bacillus; anthracis |
| #=GS NZ_AAEP01000046.1/48810-48708 | taxonomy Bacteria; Firmicutes; Bacillales; Bacillaceae; Bacillus; cereus group; Bacillus; anthracis |
| #=GS NZ_AAEQ01000034.1/49483-49381 | taxonomy Bacteria; Firmicutes; Bacillales; Bacillaceae; Bacillus; cereus group; Bacillus; anthracis |
| #=GS NZ_AAER01000042.1/191339-191441 | taxonomy Bacteria; Firmicutes; Bacillales; Bacillaceae; Bacillus; cereus group; Bacillus; anthracis |
| #=GS NZ_AAES01000043.1/48479-48377 | taxonomy Bacteria; Firmicutes; Bacillales; Bacillaceae; Bacillus; Bacillus; cereus group; Bacillus; anthracis |
| #=GS NZ_AAAC02000001.1/794076-794178 | taxonomy Bacteria; Firmicutes; Bacillales; Bacillaceae; Bacillus; cereus group; Bacillus; anthracis |
| #=GS NC_003030.1/1002176-1002275 | taxonomy Bacteria; Firmicutes; Clostridia; Clostridiales; Clostridium; acetobutylicum |
| #=GS NC_006582.1/1554717-1554819 | taxonomy Bacteria; Firmicutes; Bacillales; Bacillaceae; Bacillus; clausii |
| #=GS NC_003909.8/336194-336296 | taxonomy Bacteria; Firmicutes; Bacillales; Bacillaceae; Bacillus; Bacillus; cereus group; Bacillus; cereus |
| #=GS NC_003997.3/295331-295433 | taxonomy Bacteria; Firmicutes; Bacillales; Bacillaceae; Bacillus; |
| | Bacillus; cereus group; Bacillus; anthracis |
| #=GS NC_005945.1/295344-295446 | taxonomy Bacteria; Firmicutes; Bacillales; Bacillaceae; Bacillus; Bacillus; cereus group; Bacillus; anthracis |
| #=GS NC_005957.1/309524-309626 | taxonomy Bacteria; Firmicutes; Bacillales; Bacillaceae; Bacillus; Bacillus; cereus group; Bacillus; thuringiensis |
| #=GS NC_006274.1/309094-309196 | taxonomy Bacteria; Firmicutes; Bacillales; Bacillaceae; Bacillus; Bacillus; cereus group; Bacillus; cereus |
| #=GS NC_007530.2/295331-295433 | taxonomy Bacteria; Firmicutes; Bacillales; Bacillaceae; Bacillus; Bacillus; cereus group; Bacillus; anthracis |
| #=GS NZ_AAAC02000001.1/812243-812345 | taxonomy Bacteria; Firmicutes; Bacillales; Bacillaceae; Bacillus; Bacillus; cereus group; Bacillus; anthracis |
| #=GS NZ_AAEK01000064.1/23153-23051 | taxonomy Bacteria; Firmicutes; Bacillales; Bacillaceae; Bacillus; Bacillus; cereus group; Bacillus; cereus |
| #=GS NZ_AAEN01000023.1/36414-36516 | taxonomy Bacteria; Firmicutes; Bacillales; Bacillaceae; Bacillus; Bacillus; cereus group; Bacillus; anthracis |
| #=GS NZ_AAEO01000030.1/49824-49926 | taxonomy Bacteria; Firmicutes; Bacillales; Bacillaceae; Bacillus; Bacillus; cereus group; Bacillus; anthracis |
| #=GS NZ_AAEP01000046.1/1785-1887 | taxonomy Bacteria; Firmicutes; Bacillales; Bacillaceae; Bacillus; Bacillus; cereus group; Bacillus; anthracis |
| #=GS NZ_AAEQ01000034.1/2457-2559 | taxonomy Bacteria; Firmicutes; Bacillales; Bacillaceae; Bacillus; Bacillus; cereus group; Bacillus; anthracis |
| #=GS NZ_AAER01000042.1/238364-238262 | taxonomy Bacteria; Firmicutes; Bacillales; Bacillaceae; Bacillus; Bacillus; cereus group; Bacillus; anthracis |
| #=GS NZ_AAES01000043.1/1489-1591 | taxonomy Bacteria; Firmicutes; Bacillales; Bacillaceae; Bacillus; Bacillus; cereus group; Bacillus; anthracis |
| #=GS NC_004193.1/769686-769787 | taxonomy Bacteria; Firmicutes; Bacillales; Bacillaceae; Oceanobacillus; iheyensis |
| #=GS NC_004722.1/343847-343745 | taxonomy Bacteria; Firmicutes; Bacillales; Bacillaceae; Bacillus; Bacillus; cereus group; Bacillus; cereus |
| #=GS NZ_AAAW03000042.1/15038-14936 | taxonomy Bacteria; Firmicutes; Clostridia; Clostridiales; Peptococcaceae; Desulfitobacterium; hafniense |
| #=GS NZ_AADT03000005.1/34629-34731 | taxonomy Bacteria; Firmicutes; Clostridia; Thermoanaerobacteriales; Thermoanaerobacteriaceae; Moorella group; Moorella; thermoacetica |
| #=GS NC_002570.2/648442-648544 | taxonomy Bacteria; Firmicutes; Bacillales; Bacillaceae; Bacillus; halodurans |
| #=GS NC_004722.1/298774-298876 | taxonomy Bacteria; Firmicutes; Bacillales; Bacillaceae; Bacillus; Bacillus; cereus group; Bacillus; cereus |
| #=GS NC_006510.1/272473-272575 | taxonomy Bacteria; Firmicutes; Bacillales; Bacillaceae; Geobacillus; kaustophilus |
| #=GS NZ_AADW02000004.1/225764-225662 | taxonomy Bacteria; Firmicutes; Bacillales; Bacillaceae; Exiguobacterium; sp. |
| #=GS NC_006274.1/3685852-3685750 | taxonomy Bacteria; Firmicutes; Bacillales; Bacillaceae; Bacillus; Bacillus; cereus group; Bacillus; cereus |
| #=GS NZ_AAGO01000011.1/2345-2247 | taxonomy Bacteria; Firmicutes; Lactobacillales; Streptococcaceae; Lactococcus; lactis |
| #=GS NZ_AAAK03000113.1/5724-5822 | taxonomy Bacteria; Firmicutes; Lactobacillales; Enterococcaceae; Enterococcus; faecium |
| #=GS NZ_AADW02000005.1/186378-186480 | taxonomy Bacteria; Firmicutes; Bacillales; Bacillaceae; Exiguobacterium; sp. |
| #=GS NC_003909.8/3578068-3577966 | taxonomy Bacteria; Firmicutes; Bacillales; Bacillaceae; Bacillus; Bacillus; cereus group; Bacillus; cereus |
| #=GS NC_003997.3/3605298-3605196 | taxonomy Bacteria; Firmicutes; Bacillales; Bacillaceae; Bacillus; Bacillus; cereus group; Bacillus; anthracis |
| #=GS NC_004722.1/3766254-3766152 | taxonomy Bacteria; Firmicutes; Bacillales; Bacillaceae; Bacillus; Bacillus; cereus group; Bacillus; cereus |
| #=GS NC_005945.1/3605993-3605891 | taxonomy Bacteria; Firmicutes; Bacillales; Bacillaceae; Bacillus; Bacillus; cereus group; Bacillus; anthracis |
| #=GS NC_005957.1/3626969-3626867 | taxonomy Bacteria; Firmicutes; Bacillales; Bacillaceae; Bacillus; Bacillus; cereus group; Bacillus; thuringiensis |
| #=GS NC_007530.2/3605425-3605323 | taxonomy Bacteria; Firmicutes; Bacillales; Bacillaceae; Bacillus; Bacillus; cereus group; Bacillus; anthracis |
| #=GS NZ_AAAC02000001.1/4059572-4059470 | taxonomy Bacteria; Firmicutes; Bacillales; Bacillaceae; Bacillus; Bacillus; cereus group; Bacillus; anthracis |
| #=GS NZ_AAEN01000012.1/139994-140096 | taxonomy Bacteria; Firmicutes; Bacillales; Bacillaceae; Bacillus; Bacillus; cereus group; Bacillus; anthracis |
| #=GS NZ_AAEO01000020.1/51249-51351 | taxonomy Bacteria; Firmicutes; Bacillales; Bacillaceae; Bacillus; Bacillus; cereus group; Bacillus; anthracis |
| #=GS NZ_AAEP01000042.1/12489-12387 | taxonomy Bacteria; Firmicutes; Bacillales; Bacillaceae; Bacillus; Bacillus; cereus group; Bacillus; anthracis |
| #=GS NZ_AAEQ01000019.1/51159-51261 | taxonomy Bacteria; Firmicutes; Bacillales; Bacillaceae; Bacillus; Bacillus; cereus group; Bacillus; anthracis |
| #=GS NZ_AAER01000035.1/118314-118212 | taxonomy Bacteria; Firmicutes; Bacillales; Bacillaceae; Bacillus; Bacillus; cereus group; Bacillus; anthracis |
| #=GS NZ_AAAES01000032.1/51320-51422 | taxonomy Bacteria; Firmicutes; Bacillales; Bacillaceae; Bacillus; Bacillus; cereus group; Bacillus; anthracis |
| #=GS NC_006371.1/1538896-1538796 | taxonomy Bacteria; Proteobacteria; Gammaproteobacteria; Vibrionales; Vibrionaceae; Photobacterium; profundum |
| #=GS NC_004460.1/504371-504471 | taxonomy Bacteria; Proteobacteria; Gammaproteobacteria; Vibrionales; Vibrionaceae; Vibrio; vulnificus |
| #=GS NC_005140.1/1130553-1130653 | taxonomy Bacteria; Proteobacteria; Gammaproteobacteria; Vibrionales; Vibrio; vulnificus |
| #=GS NC_004116.1/1094305-1094209 | taxonomy Bacteria; Firmicutes; Lactobacillales; Streptococcaceae; agalactiae |
| #=GS NC_004368.1/1163490-1163394 | taxonomy Bacteria; Firmicutes; Lactobacillales; Streptococcaceae; Streptococcus; agalactiae |
| #=GS NZ_AADT03000005.1/42245-42347 | taxonomy Bacteria; Firmicutes; Clostridia; Thermoanaerobacteriales; Thermoanaerobacteriaceae; Moorella group; Moorella; thermoacetica |
| #=GS NC_004557.1/2551392-2551293 | taxonomy Bacteria; Firmicutes; Clostridia; Clostridiales; Clostridiaceae; Clostridium; tetani |
| #=GS NC_003028.1/1754786-1754882 | taxonomy Bacteria; Firmicutes; Lactobacillales; Streptococcaceae; Streptococcus; pneumoniae |
| #=GS NC_003098.1/1634825-1634921 | taxonomy Bacteria; Firmicutes; Lactobacillales; Streptococcaceae; pneumoniae |
| #=GS NZ_AAGY01000085.1/5640-5736 | taxonomy Bacteria; Firmicutes; Lactobacillales; Streptococcaceae; Streptococcus; pneumoniae |
| #=GS NZ_AAEK01000001.1/183902-184006 | taxonomy Bacteria; Firmicutes; Bacillales; Bacillaceae; Bacillus; cereus group; Bacillus; cereus |
| #=GS NC_003454.1/1645820-1645721 | taxonomy Bacteria; Fusobacteria; Fusobacterales; Fusobacteriaceae; Fusobacterium; nucleatum |
| #=GS NZ_AADW02000027.1/2980-2880 | taxonomy Bacteria; Firmicutes; Bacillales; Bacillaceae; Exiguobacterium; sp. |
| #=GS NZ_AADW02000005.1/179959-180061 | taxonomy Bacteria; Firmicutes; Bacillales; Bacillaceae; Exiguobacterium; sp. |
| #=GS NC_006582.1/1039390-1039492 | taxonomy Bacteria; Firmicutes; Bacillales; Bacillaceae; Bacillus; clausii |
| #=GS NC_002737.1/930749-930845 | taxonomy Bacteria; Firmicutes; Lactobacillales; Streptococcaceae; Streptococcus; pyogenes |
| #=GS NC_003485.1/910599-910695 | taxonomy Bacteria; Firmicutes; Lactobacillales; Streptococcaceae; pyogenes |
| #=GS NC_004070.1/846557-846653 | taxonomy Bacteria; Firmicutes; Lactobacillales; Streptococcaceae; pyogenes |
| #=GS NC_004606.1/977077-977173 | taxonomy Bacteria; Firmicutes; Lactobacillales; Streptococcaceae; pyogenes |
| #=GS NC_006086.1/857664-857760 | taxonomy Bacteria; Firmicutes; Lactobacillales; Streptococcaceae; Streptococcus; pyogenes |
| #=GS NZ_AAFV01000199.1/507-411 | taxonomy Bacteria; Firmicutes; Lactobacillales; Streptococcaceae; Streptococcus; pyogenes |
| #=GS NC_004605.1/1369721-1369821 | taxonomy Bacteria; Proteobacteria; Gammaproteobacteria; Vibrionales; Vibrionaceae; Vibrio; parahaemolyticus |
| #=GS NZ_AAAW03000004.1/66862-66959 | taxonomy Bacteria; Firmicutes; Clostridia; Clostridiales; Peptococcaceae; Desulfitobacterium; hafniense |
| #=GS NC_004567.1/2968830-2968731 | taxonomy Bacteria; Firmicutes; Lactobacillales; Lactobacillaceae; Lactobacillus; plantarum |
| #=GS NZ_AAEV01000003.1/13765-13667 | taxonomy Bacteria; Firmicutes; Lactobacillales; Lactobacillaceae; Pediococcus; pentosaceus |
| #=GS NC_002570.2/806873-806971 | taxonomy Bacteria; Firmicutes; Bacillales; Bacillaceae; Bacillus; |
| #=GS NZ_AAEK01000052.1/27552-27452 | taxonomy Bacteria; Firmicutes; Bacillales; Bacillaceae; Bacillus; Bacillus; cereus group; Bacillus; cereus |
| #=GS NC_006814.1/237722-237626 | taxonomy Bacteria; Firmicutes; Lactobacillales; Lactobacillaceae; achidophilus |
| #=GS NC_005363.1/3414604-3414703 | taxonomy Bacteria; Proteobacteria; Deltaproteobacteria; Bdellovibrionales; Bdellovibrionaceae; Bdellovibrio; bacteriovorus |
| #=GS NZ_AADT03000021.1/9410-9513 | taxonomy Bacteria; Firmicutes; Clostridia; Thermoanaerobacteriales; Thermoanaerobacteriaceae; Moorella group; Moorella; thermoacetica |
| #=GS NC_004668.1/2288426-2288328 | taxonomy Bacteria; Firmicutes; Lactobacillales; Enterococcaceae; faecalis |
| #=GS NC_006449.1/1182949-1183044 | taxonomy Bacteria; Firmicutes; Lactobacillales; Streptococcaceae; Streptococcus; thermophilus |
| #=GS NZ_AAGS01000026.1/9921-10016 | taxonomy Bacteria; Firmicutes; Lactobacillales; Streptococcaceae; Streptococcus; thermophilus |
| #=GS NZ_AAGQ01000089.1/170-269 | taxonomy Bacteria; Firmicutes; Lactobacillales; Lactobacillaceae; Lactobacillus; delbrueckii |
| #=GS NZ_AABF02000293.1/1-98 | taxonomy Bacteria; Fusobacteria; Fusobacteria (class); Fusobacterales; Fusobacteriaceae; Fusobacterium; nucleatum |
| #=GS NC_006448.1/1185082-1185177 | taxonomy Bacteria; Firmicutes; Lactobacillales; Streptococcaceae; Streptococcus; thermophilus |
| #=GS NC_004567.1/2410478-2410577 | taxonomy Bacteria; Firmicutes; Lactobacillales; Lactobacillaceae; Lactobacillus; plantarum |
| #=GS NZ_AABJ03000010.1/47704-47802 | taxonomy Bacteria; Firmicutes; Lactobacillales; Oenococcus; oeni |
| #=GS NZ_AADT03000002.1/89184-89083 | taxonomy Bacteria; Firmicutes; Clostridia; Thermoanaerobacteriales; |
| #=GS NC_006814.1/1971978-1972076 | Thermoanaerobacteriaceae; Moorella group; Moorella; thermoacetica taxonomy Bacteria; Firmicutes; Lactobacillales; Lactobacillaceae; Lactobacillus; acidophilus |
| #=GS NZ_AAA002000016.1/1579-1480 | taxonomy Bacteria; Firmicutes; Lactobacillales; Lactobacillaceae; Lactobacillus; gasseri |
| #=GS NC_005362.1/1949387-1949485 | taxonomy Bacteria; Firmicutes; Lactobacillales; Lactobacillaceae; Lactobacillus; johnsonii |
| #=GS NZ AAA002000035.1/3299-3201 | taxonomy Bacteria; Firmicutes; Lactobacillales; Lactobacillaceae; Lactobacillus; gasseri |
| #=GS NC_005362.1/263146-263049 | taxonomy Bacteria; Firmicutes; Lactobacillales; Lactobacillaceae; Lactobacillus; johnsonii |
| #=_{G}S NC_005363.1/2004933-2004835 | taxonomy Bacteria; Proteobacteria; Deltaproteobacteria; Bdellovibrionales; Bdellovibrionaceae; Bdellovibrio; bacteriovorus |
| #=GS NZ AADT03000002.1/98293-98192 | taxonomy Bacteria; Firmicutes; Clostridia; Thermoanaerobacteriales; Thermoanaerobacteriaceae; Moorella group; Moorella; thermoacetica |
| #=GS NZ_AABH02000036.1/17403-17500 | taxonomy Bacteria; Firmicutes; Lactobacillales; Leuconostoc; mesenteroides |
| #=GS NC_006055.1/484139-484044 | taxonomy Bacteria; Firmicutes; Mollicutes; Entomoplasmatales; Entomoplasmataceae; Mesoplasma; florum |
| #=GS NZ_AABH02000272.1/211-308 | taxonomy Bacteria; Firmicutes; Lactobacillales; Leuconostoc; mesenteroides |
| #=GS NZ_AABJ03000003.1/47905-47810 | taxonomy Bacteria; Firmicutes; Lactobacillales; Oenococcus; oeni |
| #=GS NZ_AABH02000038.1/17559-17463 | taxonomy Bacteria; Firmicutes; Lactobacillales; Leuconostoc; mesenteroides |
| #=GS NC_006055.1/397027-397123 | taxonomy Bacteria; Firmicutes; Mollicutes; Entomoplasmatales; Entomoplasmataceae; Mesoplasma; florum |
| NC_003869.1/586365-586467 | SEQ ID NO:12 |
| AAAAAUUUAAUAAGA.AG.CACUCAUAUAAUCCCGAGA.AU.AUGGCUCGGGA.GUCUCUACCGAACAACC..GUAAAUUGUUC.G.ACUAUGAGUGAAAGU.GUACCUAGGG | |
| NC_002570.2/1593074-1592972 | SEQ ID NO:13 |
| AUUUACAUUAAAAAA.AG.CACUCGUAUAAUCGCGGGA.AU.AGGGCCCGCAA.GUUUCUACCAGGCUGCC..GUAAACAGCCU.G.ACUACGAGUGAUACU.UUGACAUAGA | |
| NC_002570.2/676475-676577 | SEQ ID NO:14 |
| CGUUCUUUAUAUAAA.GU.ACCUCAUAUAAUCUUGGGA.AU.AUGGCCCAAAA.GUUUCUACCUGCUGACC.. GUAAAUCGGCG.G.ACUAUGGGGAAAGAU.UUUGGAUCUU | |
| NC_000964.2/4004541-4004643 | SEQ ID NO:15 |
| CAUCUUAGAAAAAGA.CA.UUCUUGUAUAUGAUCAGUA.AU.AUGGUCUGAUU.GUUUCUACCUAGUAACC.. GUAAAAAACUA.G.ACUACAAGAAAGUUU.GAAUAAAUUU | |
| NC_003366.1/513088-512986 | SEQ ID NO:16 |
| UAAGUGUAUUAAAUU.UU.AACUCGUAUAUAAUCGGUA.AU.AUGGUCCGAAA.GUUUCUACCUGCUAACC..GUAAAAUAGCA.G.ACUACGAGGAGUUGU.ACUAUAAAUU | |
| NC_000964.2/693775-693877 | SEQ ID NO:17 |
| AGAAAUCAAAUAAGA.UG.AAUUCGUAUAAUCGCGGGA.AU.AUGGCUCGCAA.GUCUCUACCAAGCUACC..GUAAAUGGCUU.G.ACUACGUAAACAUUU.CUUUCGUUUG | |
| NC_002570.2/650309-650411 | SEQ ID NO:18 |
| AAUAAAUCGAAAACA.UC.AUUUCGUAUAAUGGCAGGA.AU.AGGGCCUGCGA.GUUUCUACCAAGCUACC..GUAAAUAGCUU.G.ACUACGAAAAUAAUG.GGUUUUUUAC | |
| NC_006270.2/2294931-2294831 | SEQ ID NO:19 |
| AAUUUGAUACAUUAU.AU.CACUCAUAUAAUCGCGUGG.AU.AUGGCACGCAA.GUUUCUACCGGGCA-CC..GUAAA-UGUCC.G.ACUAUGAGUGGGCGA.UAAGAAAACG | |
| NC_006322.1/2295788-2295688 | SEQ ID NO:20 |
| AAUUUGAUACAUUAU.AU.CACUCAUAUAAUCGCGUGG.AU.AUGGCACGCAA.GUUUCUACCGGGCA-CC..GUAAA-UGUCC.G.ACUAUGAGUGGGCGA.UAAGAAAACG | |
| NC_004193.1/1103943-1104045 | SEQ ID NO:21 |
| AAACCUUAUAUAUAG.UU.UUUUCAUAUAAUCGCGGGG.AU.AUGGCCUGCAA.GUUUCUACCGGUUUACC..GUAAAUGAACC.G.ACUAUGGAAAAGCGG.AAAAUUCGAU | |
| NC_003909.8/1650407-1650509 | SEQ ID NO:22 |
| AAAUAAAUAGUUAGC.UA.CACUCAUAUAAUCGCGGGG.AU.AUGGCCUGCAA.GUUUCUACCGAAGUACC.. GUAAAUACUUU.G.ACUAUGAGUGAGGAC.GAAUAUAUUU | |
| NC_003997.3/1497571-1497673 | SEQ ID NO:23 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NC_005945.1/1497645-1497747 | SEQ ID NO:24 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NC_005957.1/1521880-1521982 | SEQ ID NO:25 |
| AAAUAAAUAGUUAGC.UA.CACUCAUAUAAUCGCGGGG.AU.AUGGCCUGCAA.GUUUCUACCGAAGUACC.. GUAAAUACUUU.G.ACUAUGAGUGAGGAC.GAAUAUAUUU | |
| NC_006274.1/1532482-1532584 | SEQ ID NO:26 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NC_007530.2/1497694-1497796 | SEQ ID NO:27 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAAC02000001.1/1985987-1986089 | SEQ ID NO:28 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAEK01000008.1/96466-96364 | SEQ ID NO:29 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAEN01000011.1/67143-67245 | SEQ ID NO:30 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAE001000025.1/66855-66957 | SEQ ID NO:31 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAEP01000035.1/69365-69467 | SEQ ID NO:32 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAEQ01000029.1/70848-70950 | SEQ ID NO:33 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAER01000023.1/58182-58284 | SEQ ID NO:34 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NZ_AAES01000034.1/69121-69223 | SEQ ID NO:35 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAUUU | |
| NC_004722.1/1515239-1515341 | SEQ ID NO:36 |
| AAAUAAAUAGUUAGC.UA.CACUCAUAUAAUCGCGGGG.AU.AUGGCCUGCAA.GUUUCUACCGAAGUACC..GUAAAUACUUU.G.ACUAUGAGUGAGGAC.GAAUAUAAUU | |
| NC_000964.2/697711-697813 | SEQ ID NO:37 |
| CAUGAAAUCAAAACA.CG.ACCUCAUAUAAUCUUGGGA.AU.AUGGCCCAUAA.GUUUCUACCCGGCAACC..GUAAAUUGCCG.G.ACUAUGCAGGAAAGU.GAUCGAUAAA | |
| NC_002976.3/54816-54919 | SEQ ID NO:38 |
| CAUAAAAUAAUUUAU.AU.GACUCAUAUAAUCUAGAGA.AU.AUGGCUUUAGAaGUUUCUACCGUGUCGCC..AUAAACGACAC.G.ACUAUGAGUAACAAU.CCAAUACAUU | |
| NC_004461.1/2433047-2432944 | SEQ ID NO:39 |
| CAUAAAAUAAUUUAU.AU.GACUCAUAUAAUCUAGAGA.AU.AUGGCUUUAGAaGUUUCUACCGUGUCGCC..AUAAACGACAC.G.ACUAUGAGUAACAAU.CCAAUACAUU | |
| NC_003030.1/2824953-2824855 | SEQ ID NO:40 |
| AAUCGUUAAUAUAGU.UU.AACUCAUAUAU-UUCCUGA.AU.AUGGCAGGAU-.GUUUCUACAAGGAA-CC..UUAAA-UUUCU.U.ACUAUGAGUGAUUUG.UUUGUAUGCA | |
| NC_003909.8/296465-296567 | SEQ ID NO:41 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_003997.3/262601-262703 | SEQ ID NO:42 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_004722.1/261558-261660 | SEQ ID NO:43 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_005945.1/262614-262716 | SEQ ID NO:44 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_005957.1/268537-268639 | SEQ ID NO:45 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_006274.1/267835-267937 | SEQ ID NO:46 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_007530.2/262601-262703 | SEQ ID NO:47 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NZ_AAAC02000001.1/796036-796138 | SEQ ID NO:48 |
| GAAAAGUGAAUAUUA.UG.CCGUCGUAUAAUAUCGGGG.AU.AUGGCCCGAAA.GUUUCUACCUAGCUACC.. GUAAAUGGCUU.G.ACUACGAGGCGUUUU.UAUAAAGGUG | |
| NZ_AAEK01000017.1/88397-88499 | SEQ ID NO:49 |
| GAAAAGUGAAUAUUA.UG.CCGUCGUAUAAUAUCGGGG.AU.AUGGCCCGAAA.GUUUCUACCUAGCUACC.. GUAAAUGGCUU.G.ACUACGAGGCGUUUU.UAUAAAGGUG | |
| NZ_AAEN01000023.1/16980-17082 | SEQ ID NO:50 |
| GAAAAGUGAAUAUUA.UG.CCGUCGUAUAAUAUCGGGG.AU.AUGGCCCGAAA.GUUUCUACCUAGCUACC.. GUAAAUGGCUU.G.ACUACGAGGCGUUUU.UAUAAAGGUG | |
| NZ_AAEO01000030.1/17090-17192 | SEQ ID NO:51 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NZ_AAEP01000043.1/12962-13064 | SEQ ID NO:52 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NZ_AAEQ01000040.1/4962-4860 | SEQ ID NO:53 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NZ_AAER01000030.1/4374-4272 | SEQ ID NO:54 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NZ_AAES01000040.1/14760-14862 | SEQ ID NO:55 |
| GAAAAGUGAAUAUUA.UG.CCGUCGUAUAAUAUCGGGG.AU.AUGGCCCGAAA.GUUUCUACCUAGCUACC..GUAAAUGGCUU.G.ACUACGAGGCGUUUU.UAUAAAGGUG | |
| NC_006270.2/693198-693300 | SEQ ID NO:56 |
| AGUAAUUUAAAAAAG.AC.UUGUCGUAUAAUCAUGGGG.AU.AUGGCCCAUAA.GUUUCUACCAAGCUACC..GUAAAUAGCUU.G.ACUACGCUUGUAUAC.AAUAUUUUAU | |
| NC_006322.1/692981-693083 | SEQ ID NO:57 |
| AGUAAUUUAAAAAAG.AC.UUGUCGUAUAAUCAUGGGG.AU.AUGGCCCAUAA.GUCUACCAAGCUACC..GUAAAUAUUGCUU.G.ACUACGCUUGUAUAC.AAUAUUUUAU | |
| NC_004722.1/259601-259703 | SEQ ID NO:58 |
| AGAUAAUAUAAAACG.AU.CCUUCAUAUAUCCUCAAUG.AU.AUGGUUUGAGA.GUCUCUACCGGGUUACC..GUAAACAACCU. G. ACUAUGAAGGCAGUG. UGUCUUAUAU | |
| NC_000964.2/2319410-2319310 | SEQ ID NO:59 |
| UUACAAUAUAAUAGG.AA.CACUCAUAUAAUCGCGUGG.AU.AUGGCACGCAA.GUUUCUACCGGGCA-CC..GUAAA-UGUCC.G.ACUAUGGGUGAGCAA.UGGAACCGCA | |
| NC_003030.1/2905050-2904948 | SEQ ID NO:60 |
| GAAAAGUAAUAACAU.AU.UACCCGUAUAUGCUUAGAA.AU.AUGGUCUAAGC.GUCUCUACCGGACUGCC..GUAAAUUGUCU.G.ACUAUGGGUGUUUAU.AAGUAUUUUA | |
| NC_003210.1/611119-611017 | SEQ ID NO:61 |
| AAUCCGCUACAAUAA.UA.UAGUCGUAUAAGUUCGGUA.AU.AUGGACCGUUC.GUUUCUACCAGGCAACC..GUAAAAUGCCA.G.GCUACGAGCUAUUGU.AAAAUUUAAU | |
| NZ_AADQ01000001.1/2431-2533 | SEQ ID NO:62 |
| AAUCCGCUACAAUAA.UA.UAGUCGUAUAAGUUCGGUA.AU.AUGGACCGUUC.GUUUCUACCAGGCAACC..GUAAAAUGCCA.G.GCUACGAGCUAUUGU.AAAAUUUAAU | |
| NC_003212.1/610156-610054 | SEQ ID NO:63 |
| AAUCGUCUACAAUAA.UA.AAGUCGUAUAAGUUCGGUA.AU.AUGGACCGUUC.GUUUCUACCAGGCAACC..GUAAAAUGCCA.G.GCUACGAGCUAUUGU.AAAAUUUAAU | |
| NC_003366.1/422820-422922 | SEQ ID NO:64 |
| UAUGUACUUAUAUAA.GU.AUAUCGUAUAUGCUCGACG.AU.AUGGGUUGAGU.GUUUCUACUAGGAGGCC..GUAAACAUCCU.A.ACUACGAAUAUAUAG.GUGAUUUCUA | |
| NC_002973.5/617846-617744 | SEQ ID NO:65 |
| AAUCCGCUACAAUAA.UA.AAGUCGUAUAAGUUCGGUA.AU.AUGGACCGUUC.GUUUCUACCAGGCAACC..GUAAAAUGCCA.G.GCUACGAGCUAUUGU.AAAAUUUAAU | |
| NZ_AADR01000111.1/1598-1496 | SEQ ID NO:66 |
| AAUCCGCUACAAUAA.UA.AAGUCGUAUAAGUUCGGUA.AU.AUGGACCGUUC.GUUUCUACCAGGCAACC..GUAAAAUGCCA.G.GCUACGAGCUAUUGU.AAAAUUUAAU | |
| NC_003909.8/294509-294611 | SEQ ID NO:67 |
| AGAUAAUAUAAAACG.AU.CCUUCAUAUAUCCUCAAAG.AU.AUGGUUUGAGA.GUCUCUACCGGGUUACC..GUAAACAACCU.G.ACUAUGAAGGCAGUG.UGUCUUAUAU | |
| NC_005957.1/266577-266679 | SEQ ID NO:68 |
| AGAUAAUAUAAAACG.AU.CCUUCAUAUAUCCUCAAAG.AU.AUGGUUUGAGA.GUCUCUACCGGGUUACC..GUAAACAACCU.G.ACUAUGAAGGCAGUG.UGUCUUAUAU | |
| NC_006274.1/265875-265977 | SEQ ID NO:69 |
| AGAUAAUAUAAAACG.AU.CCUUCAUAUAUCCUCAAAG.AU.AUGGUUUGAGA.GUCUCUACCGGGUUACC..GUAAACAACCU.G.ACUAUGAAGGCAGUG.UGUCUUAUAU | |
| NC_003366.1/2618421-2618323 | SEQ ID NO:70 |
| AAAACGGAAUAUAAA.CA.AACUCGUAUAA-GCUUUGA.AU.AAGGCAAGGC-.GUUUCUACCGGAAA-CC..UUAAA-UUUCC.G.UCUAUGAGUGAAUUU.GAUAUACUAU | |
| NC_002973.5/1940027-1939926 | SEQ ID NO:71 |
| AUAACUUAAAACCGA.AA.UACUUAUAUAAUAGUUGCG.AU.-UGGGCGACGA.GUUUCUACCUGGUUACC..GUAAAUAACCG. G. ACUAUGAGUAGUUUG. UAUAAAGAAG | |
| NC_003212.1/2013345-2013244 | SEQ ID NO:72 |
| AUAACUUAAAACCGA.AA.UACUUAUAUAAUAGUUGCG.AU.-UGGGCGACGA.GUUUCUACCUGGUUACC..GUAAAUAACCG.G.ACUAUGAGUAGUUUG.UAUAAAGAAG | |
| NZ_AADQ01000095.1/1690-1791 | SEQ ID NO:73 |
| AUAACUUAAAACCGA. AA. UACUUAUAUAAUAGUUGCG. AU. -UGGGCGACGA. GUUUCUACCUGGUUACC.. GUAAAUAACCG. G. ACUAUGAGUAGUUUG. UAUAAAGAAG | |
| NZ AADR01000082.1/3415-3516 | SEQ ID NO:74 |
| AUAACUUAAAACCGA.AA.UACUUAUAUAAUAGUUGCG.AU.UGGGCGACGA.GUUUCUACCUGGUUACC..GUAAAUAACCG.G.ACUAUGAGUAGUUUG.UAUAAAGAAG | |
| NC_003366.1/2871201-2871101 | SEQ ID NO:75 |
| AUAAAAAAAUAAAUU.UU.GCUUCGUAUAACUCUAAUG.AU.AUGGAUUAGAG.GUCUCUACCAAGAA-CC..GAGAA-UUCUU.G.AUUACGAAGAAAGCU.UAUUUGCUUU | |
| NC_002745.2/430797-430900 | SEQ ID NO:76 |
| GUUAAAUAAUUUACA.UA.AACUCAUAUAAUCUAAAGA.AU.AUGGCUUUAGAaGUUUCUACCAUGUUGCC..UUGAACGACAU.G.ACUAUGAGUAACAAC.ACAAUACUAG | |
| NC_002758.2/430754-430857 | SEQ ID NO:77 |
| GUUAAAUAAUUUACA.UA.AACUCAUAUAAUCUAAAGA.AU.AUGGCUUUAGAaGUUUCUACCAUGUUGCC..UUGAACGACAU.G.ACUAUGAGUAACAAC.ACAAUACUAG | |
| NC_002951.2/460059-460162 | SEQ ID NO:78 |
| GUUAAAUAAUUUACA.UA.AACUCAUAUAAUCUAAAGA.AU.AUGGCUUUAGAaGUUUCUACCAUGUUGCC..UUGAACGACAU.G.ACUAUGAGUAACAAC.ACAAUACUAG | |
| NC_002952.2/441050-441153 | SEQ ID NO:79 |
| GUUAAAUAAUUUACA.UA.AACUCAUAUAAUCUAAAGA.AU.AUGGCUUUAGAaGUUUCUACCAUGUUGCC..UUGAACGACAU.G.ACUAUGAGUAACAAC.ACAAUACUAG | |
| NC_002953.3/409191-409294 | SEQ ID NO:80 |
| GUUAAAUAAUUUACA.UA.AACUCAUAUAAUCUAAAGA.AU.AUGGCUUUAGAaGUUUCUACCAUGUUGCC..UUGAACGACAU.G.ACUAUGAGUAACAAC.ACAAUACUAG | |
| NC_003923.1/410546-410649 | SEQ ID NO:81 |
| GUUAAAUAAUUUACA.UA.AACUCAUAUAAUCUAAAGA.AU.AUGGCUUUAGAaGUUUCUACCAUGUUGCC..UUGAACGACAU.G.ACUAUGAGUAACAAC.ACAAUACUAG | |
| NC_006582.1/1115665-1115767 | SEQ ID NO:82 |
| AAGUUAAAAACGAAA.AC.ACCUCAUAUAUACUCGGGA.AU.AUGGCUCGAAC.GUUUCUACCCGGCAACC..GUAAAUUGCCG.G.ACUAUGAGGGGAAGU.CAUUACGCGC | |
| NC_006510.1/274257-274359 | SEQ ID NO:83 |
| AUGAAUAUUGUUGAA.UU.CCGUCGUAUAAUCCCGGGA.AU.AUGGCUCGGGA.GUUUCUACCAAGCUACC..GUAAAUAGCUU.G.ACUACGAGGGAUGCG.GGAUCGGAGA | |
| NC_003210.1/1958922-1958821 | SEQ ID NO:84 |
| AUAACUUAAAACCGA.AA.UACUUGUAUAAUAGUUGCG.AU.-UGGGCGACGA.GUUUCUACCUGGUUACC..GUAAAUAACCG.G.ACUAUGAGUAGUUUG.UAUAAAGAAG | |
| NC_006270.2/697054-697156 | SEQ ID NO:85 |
| CAUGACAUGAAAACA.CA.UCCUCAUAUAAUCUUGGGA.AU.AUGGCCCAUAA.GUCUCUACCCGAUGACC..GUAAAUCAUCG.G.ACUAUGCAGGAAAGU.GGACAAUAAA | |
| NC_006322.1/696838-696940 | SEQ ID NO:86 |
| CAUGACAUGAAAACA.CA.UCCUCAUAUAAUCUUGGGA.AU.AUGGCCCAUAA.GUCUCUACCCGAUGACC..GUAAAUCAUCG.G.ACUAUGCAGGAAAGU.GGACAAUAAA | |
| NC_006510.1/282580-282682 | SEQ ID NO:87 |
| AUAGUGUAUGAGAAG.AU.CCCUCAUAUAAUUUUGGGA.AU.AUGGCCCAAAA.GUUUCUACCCAAUCACC..GUAAAUGAUUG.G.ACUAUGAGGGAAAGG.AUCGGUUUUG | |
| NC_003997.3/260641-260743 | SEQ ID NO:88 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NC_005945.1/260654-260756 | SEQ ID NO:89 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NC_007530.2/260641-260743 | SEQ ID NO:90 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ_AAEK01000017.1/86437-86539 | SEQ ID NO:91 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ_AAEN01000023.1/15020-15122 | SEQ ID NO:92 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ_AAE001000030.1/15130-15232 | SEQ ID NO:93 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ AAEP01000043.1/11002-11104 | SEQ ID NO:94 |
| AGAUAAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ_AAEQ01000040.1/6922-6820 | SEQ ID NO:95 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ_AAER01000030.1/6334-6232 | SEQ ID NO:96 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NZ_AAES01000040.1/12800-12902 | SEQ ID NO:97 |
| AGAUAAUAUAAAACG.AU.CCUUCAUAUAUCCUCAAAG.AU.AAGGUUUGAGA.GUCUCUACCGGGUUACC..GUAAACAACCU.G.ACUAUGAAGGCAGUG.UGUCUUAUAU | |
| NC_004193.1/760473-760575 | SEQ ID NO:98 |
| CAAUUUUUAUCCAAU.GC.CUUUCGUAUAUCCUCGAUA.AU.AUGGUUCGAAA.GUAUCUACCGGGUCACC..GUAAAUGAUCU.G.ACUAUGAAGGCAGAA.GCAGGUUCGG | |
| NC_006270.2/4024210-4024312 | SEQ ID NO:99 |
| AAAUAAUAGAAGCCC.AC.UUCUUGUAUAUAAUCAGUA.AU.AGGGUCUGAUU.GUUUCUACCUGGCAACC..GUAAAUCGCCA.G.ACUACAAGGAAGUUU.GAAUAGAUUU | |
| NC_006322.1/4024324-4024426 | SEQ ID NO:100 |
| AAAUAAUAGAAGCCC.AC.UUCUUGUAUAUAAUCAGUA.AU.AGGGUCUGAUU.GUUUCUACCUGGCAACC..GUAAAUCGCCA.G.ACUACAAGGAAGUUU.GAAUAGAUUU | |
| NC_004193.1/786767-786868 | SEQ ID NO:101 |
| CCGACAAUUGAAAAU.GA.ACCUCAUAUAAAUUUGAGA.AU.AUGGCUCAGAA.GUUUCUACCCAGC-ACC..GUAAAUGGCUG.G.ACUAUGAGGGAAGAU.GGAUCAUUUC | |
| NC_002662.1/1159509-1159607 | SEQ ID NO:102 |
| UAGUCUAUAAUAGAA.CA.AUCUUAUUUAU-ACCUAGG.AU.AUGGCUGGGC-.GUUUCUACCUCGUA-CC..GUAAA-UGCGA.G.ACAAUAAGGAAAUUC.GAUUUUUUAG | |
| NC_000964.2/625993-625893 | SEQ ID NO:103 |
| AAUUAAAUAGCUAUU.AU.CACUUGUAUAACCUCAAUA.AU.AUGGUUUGAGG.GUGUCUACCAGGAA-CC..GUAAA-AUCCU.G.AUUACAAAAUUUGUU.UAUGACAUUU | |
| NC 003909.8/382630-382528 | SEQ ID NO:104 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NC 003997.3/342356-342254 | SEQ ID NO:105 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NC_005945.1/342369-342267 | SEQ ID NO:106 |
| UUAAUACGGACGAUG..ACCUCAUAUAUACUCGAUA.AU.AUGGAUCGAGA.GUUUCUACCCGGCAACC..UUAAAUUGCUUUG.G.ACUAUGGGGAAAACU.AAUGAAUAUU | |
| NC_005957.1/356354-356252 | SEQ ID NO:107 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NC_006274.1/357462-357360 | SEQ ID NO:108 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NC_007530.2/342356-342254 | SEQ ID NO:109 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAAC02000001.1/859268-859166 | SEQ ID NO:110 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAEK01000051.1/8150-8252 | SEQ ID NO:111 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAEN01000023.1/83441-83339 | SEQ ID NO:112 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAE001000030.1/96886-96784 | SEQ ID NO:113 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAEP01000046.1/48810-48708 | SEQ ID NO:114 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAEQ01000034.1/49483-49381 | SEQ ID NO:115 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAER01000042.1/191339-191441 | SEQ ID NO:116 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAES01000043.1/48479-48377 | SEQ ID NO:117 |
| UUAAUACGGACGAUG.UU.ACCUCAUAUAUACUCGAUA.AU.AUGGAUCGAGA.GUUUCUACCCGGCAACC..UUAAAUUGCUG.G.ACUAUGGGGAAAACU.AAUGAAUAUU | |
| NZ_AAAC02000001.1/794076-794178 | SEQ ID NO:118 |
| AGAUAAUAUAAAACG.AU.CCUUCAUAUAUCCUCAAAG.AU.ARGGUUUGAGA.GUCUCUACCGGGUUACC..GUAAACAACCU.G.ACUAUGAWGGCAGUG.UGUCUUAUAU | |
| NC_003030.1/1002176-1002275 | SEQ ID NO:119 |
| UAUAUAAAAAACUAA.AU.UUCUCGUAUAC-ACCGGUA.AU.AUGGUCCGGAA.GUUUCUACCUGCUG-CC..AUAAA-UAGCA.G.ACUACGGGGUGUUAU.UGAUAAUAUA | |
| NC_006582.1/1554717-1554819 | SEQ ID NO:120 |
| UAAACGAACAAAGCA.UC.AGCUCGUAUAAUAGCGGUA.AU.AUGGUCCGCGA.GUCUCUACCAGGCUGCC..GAUAACGGCCU.G.ACUACGAGUGGUCUU.UUUCAGUUGU | |
| NC_003909.8/336194-336296 | SEQ ID NO:121 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_003997.3/295331-295433 | SEQ ID NO:122 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_005945.1/295344-295446 | SEQ ID NO:123 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_005957.1/309524-309626 | SEQ ID NO:124 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_006274.1/309094-309196 | SEQ ID NO:125 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_007530.2/295331-295433 | SEQ ID NO:126 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAAC02000001.1/812243-812345 | SEQ ID NO:127 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAEK01000064.1/23153-23051 | SEQ ID NO:128 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAEN01000023.1/36414-36516 | SEQ ID NO:129 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAE001000030.1/49824-49926 | SEQ ID NO:130 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAEP01000046.1/1785-1887 | SEQ ID NO:131 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAEQ01000034.1/2457-2559 | SEQ ID NO:132 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAER01000042.1/238364-238262 | SEQ ID NO:133 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NZ_AAES01000043.1/1489-1591 | SEQ ID NO:134 |
| AAAGAAUAAUAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_004193.1/769686-769787 | SEQ ID NO:135 |
| UGAUGUAAUUGAAUA.GA.AAUGCGUAUAAUUAAGGGG.AU.AUGGCCC-ACA.GUUUCUACCAGACCACC..GUAAAUGGUUU.G.ACUACGCAGUAAUUA.UAUUUGUAUC | |
| NC_004722.1/343847-343745 | SEQ ID NO:136 |
| UUAAUACGGACGAUG.UU.ACCUCAUAUAUACUUGAUA.AU.AUGGAUCGAGA.GUUUCUACCCGGCAACC..UUAAAUUGCUG.G.ACUAUGGGGAAAACU.AAUGAAUAUU | |
| NZ₋AAAW03000042.1/15038-14936 | SEQ ID NO:137 |
| AAAAAAUUUAAAUAG.GG.CGUUCAUAUAAUCGCGGAG.AU.AGGGUCCGCAA.GUCUACCGGGCUGCC..GUAAAUGGCCU.G.ACUAUGAGCGAAACU.GUGCCCAGGG | |
| NZ_AADT03000005.1/34629-34731 | SEQ ID NO:138 |
| AAAAAUAAUUUAACC.CU.GCUUCGUAUAUUCCCGGAA.AU.GCGGUCCGGGA.GUUUCUACCAGGCAACC..GUAAAUUGCCC.G.GCUACGAAGGUUAUU.CUUCGUCGUC | |
| NC_002570.2/648442-648544 | SEQ ID NO:139 |
| ACAUGUAGAUAUCAU.CC.CUUUCGUAUAUACUUGGAG.AU.AAGGUCCAGGA.GUUUCUACCAGAUCACC..GUAAAUGAUCU.G.ACUAUGAAGGUGGAA.UGGCUCGAUA | |
| NC_004722.1/298774-298876 | SEQ ID NO:140 |
| AGAAACAAUAAUAUA.AG.ACCUCAUAUAAUCGCGGGG.AU.AUGGCCUGCAA.GUCUCUACCUAACGACC..GUUAUUCGUUA.G.ACUAUGAGGGAAAGU.CACUCGGUAU | |
| NC_006510.1/272473-272575 | SEQ ID NO:141 |
| UACGGAUAGACGAAA.GC.CCUUCAUAUAAGCGCAAGA.AU.AUGGCUUGCGC.GUCUCUACCGGGCCGCC..GUAAACGGCCC.G.ACUAUGAAGGCAGAA.GACGCUGCUA | |
| NZ_AADW02000004.1/225764-225662 | SEQ ID NO:142 |
| GGAAGAUUGAAUAUA.AC.ACCUCGUAUAAUAGCAGGG.AU.AUGGCUUGCAA.GUUUCUACCCGACGACC..CUAAAUCGUUG.G.ACUAUGGGGUAUAUG.GAUGUUCGUC | |
| NC_006274.1/3685852-3685750 | SEQ ID NO:143 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGAAAAA.GUGUGUAACA | |
| NZ_AAG001000011.1/2345-2247 | SEQ ID NO:144 |
| UUGAGCUGCUAUAAA.CA.AUCUUAUUUAU-ACCUAGA.AU.AUGGCUGGGC-.GUUUCUACCUCGUA-CC..GUAAA-UGCGA.G.ACAAUAAGGAAAUUC.GAUUUUUCAA | |
| NZ_AAAK03000113.1/5724-5822 | SEQ ID NO:145 |
| UAUUAGUUUUACAUA.CC.UACUUAUAUAU-CGUCAUA.AU.AUGGAUGACA-.GUUUCUAGCCAGUA-CC..GUAAA-UGCUG.G.ACUAUAAGUAAAAGA.UUGGCUAUUU | |
| NZ_AADW02000005.1/186378-186480 | SEQ ID NO:146 |
| CUAAAAAACAAAAAA.UA.AUGCCGUAUAAUUCUGGGG.AU.AUGGCCCGGAA.GUCUCUACAGGAACACC..UUAAAGGUUCC.U.ACUACGGCGUGCACU.GAUUUCCGGU | |
| NC003909.8/3578068-3577966 | SEQ ID NO:147 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NC_003997.3/3605298-3605196 | SEQ ID NO:148 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NC_004722.1/3766254-3766152 | SEQ ID NO:149 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NC_005945.1/3605993-3605891 | SEQ ID NO:150 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NC_005957.1/3626969-3626867 | SEQ ID NO:151 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NC_007530.2/3605425-3605323 | SEQ ID NO:152 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ_AAAC02000001.1/4059572-4059470 | SEQ ID NO:153 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ_AAEN01000012.1/139994-140096 | SEQ ID NO:154 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ_AAE001000020.1/51249-51351 | SEQ ID NO:155 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ AAEP01000042.1/12489-12387 | SEQ ID NO:156 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ_AAEQ01000019.1/51159-51261 | SEQ ID NO:157 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ_AAERO1000035.1/118314-118212 | SEQ ID NO:158 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NZ_AAES01000032.1/51320-51422 | SEQ ID NO:159 |
| CUUCGAAAAGAAUCA.CU.GCCUCAUAUAAUCUUGGAG.AU.AAGGUCCAUAA.GUUUCUACCUGGCAACC..AUGAAUUGCUA.G.ACUAUGAGGGGAAAA.GUGUGUAACA | |
| NC_006371.1/1538896-1538796 | SEQ ID NO:160 |
| UAAUAAUGGGUAAAG.UU.ACUUCGUAUAACCCCACUU.AU.AGGGUGUGGGG.GUCUCUACCAGAAU-CC..GUAAA-AUUCU.G.AUUACGAAGAGUUGA.GUGAUUGAUC | |
| NC₋004460.1/504371-504471 | SEQ ID NO:161 |
| GACUUUCGGCGAUCA.AC.GCUUCAUAUAAUCCUAAUG.AU.AUGGUUUGGGA.GUUUCUACCAAGAG-CC..UUAAA-CUCUU.G.AUUAUGAAGUCUGUC.GCUUUAUCCG | |
| NC_005140.1/1130553-1130653 | SEQ ID NO:162 |
| GACUUUCGGCGAUCA.AC.GCUUCAUAUAAUCCUAAUG.AU.AUGGUUUGGGA.GUUUCUACCAAGAG-CC..UUAAA-CUCUU.G.AUUAUGAAGUCUGUC.GCUUUAUCCG | |
| NC_004116.1/1094305-1094209 | SEQ ID NO:163 |
| CAAUUAAAUAUAUGA.UU.UACUUAUUUAU-GCUGAGG.AU.-UGGCUUAGC-.GUCUCUACAAGACA-CC..GU-AA-UGUCU.A.ACAAUAAGUAAGCUA.AUAAAUAGCU | |
| NC_004368.1/1163490-1163394 | SEQ ID NO:164 |
| CAAUUAAAUAUAUGA.UU.UACUUAUUUAU-GCUGAGG.AU.-UGGCUUAGC-.GUCUCUACAAGACA-CC..GU-AA-UGUCU.A.ACAAUAAGUAAGCUA.AUAAAUAGCU | |
| NZ_AADT03000005.1/42245-42347 | SEQ ID NO:165 |
| CAAUAAAGCCAGUCU.UA.ACUUCGUAUAUCCCCGGCA.AU.AGGGACCGGGG.GUUUCUACCAGGCAACC..GCAAAUUGCCC.G.GCUACGAAGGUAUAU.CUUCGUUGCU | |
| NC_004557.1/2551392-2551293 | SEQ ID NO:166 |
| CUCUAUAAUAAAUUA.UU.GACUCAUAUAU-CCCCUUA.AU.AAGGUAGGGA-.GUAUCUACCAGAAG-CC..UUAAA-CUUCU.G.ACUAUGAGUGAAUAAaGCAUUGUCAU | |
| NC_003028.1/1754786-1754882 | SEQ ID NO:167 |
| AAAAUUGAAUAUCGU.UU.UACUUGUUUAU-GUCGUGA.AU.-UGGCACGAC-.GUUUCUACAAGGUG-CC..GG-AA-CACCU.A.ACAAUAAGUAAGUCA.GCAGUGAGAU | |
| NC_003098.1/1634825-1634921 | SEQ ID NO:168 |
| AAAAUUGAAUAUCGU.UU.UACUUGUUUAU-GUCGUGA.AU.-UGGCACGAC-.GUUUCUACAAGGUG-CC..GG-AA-CACCU.A.ACAAUAAGUAAGUCA.GCAGUGAGAU | |
| NZ_AAGY01000085.1/5640-5736 | SEQ ID NO:169 |
| AAAAUUGAAUAUCGU.UU.UACUUGUUUAU-GUCGUGA.AU.-UGGCACGAC-.GUUUCUACAAGGUG-CC..GG-AA-CACCU.A.ACAAUAAGUAAGUCA.GCAGUGAGAU | |
| NZ_AAEK01000001.1/183902-184006 | SEQ ID NO:170 |
| AUAAUUUUACACAUU.AU.CACUCGUAUAUACUCGGUA.AU.AUGGUCCGAGC.GUUUCUACCUAGUUCCCaaUGAAAGAACUG.G.ACUACGGGUUAAAGU.AUUCGGUCGC | |
| NC_003454.1/1645820-1645721 | SEQ ID NO:171 |
| UAAAUAAUUUUAAUA.AA.AAUUCGUAUAA-GCCUAAU.AU.AUGGAAGGGU-.GUCCCUAC-GGUUAACC..AUAAAUUAACC.A.GCUACGAAAAAUGUU.UUACUGUGUU | |
| NZ_AADW02000027.1/2980-2880 | SEQ ID NO:172 |
| GAAUAAACGUAUAGC.AA.CGCUCGUAUAAUAGUGGGG.AU.-UGGCCCACGA.GUCUCUACCGGAUCGCC..GU-AACGAUCC.G.ACUACGGGUGGUGAG.UUACUGCUCU | |
| NZ_AADW02000005.1/179959-180061 | SEQ ID NO:173 |
| AAAUCAUACAUGCAU.CU.CCUUUGUAUAUACUCGCGA.AU.AUGGCGUGAGA.GUCUCUACCGGGUCACC..UUAAACGACCU.G.ACUAUGAAGGAGCAG.ACCCUUCGUA | |
| NC_006582.1/1039390-1039492 | SEQ ID NO:174 |
| AAUGUCCAAUAGGAA.AA.UACCCGUAUAAUUGCAGGA.AU.AAGGCCUGCAC.GUUUCUACCGAGCCACC..GUAAAUGGCUU.G.ACUACGGCAUGAUAA.AUGGAGCGCA | |
| NC_002737.1/930749-930845 | SEQ ID NO:175 |
| UGAAUUCAAUAAUGA.CA.UACUUAUUUAU-GCUGUGA.AU.-UGGCGCAGC-.GUCUCUACAAGACA-CC..UU-AA-UGUCU.A.ACAAUAAGUAAGCUU.UUAGGCUUGC | |
| NC_003485.1/910599-910695 | SEQ ID NO:176 |
| UGAAUUCAAUAAUGA.CA.UACUUAUUUAU-GCUGUGA.AU.-UGGCGCAGC-.GUCUCUACAAGACA-CC..UU-AA-UGUCU.A.ACAAUAAGUAAGCUU.UUAGGCUUGC | |
| NC_004070.1/846557-846653 | SEQ ID NO:177 |
| UGAAUUCAAUAAUGA.CA.UACUUAUUUAU-GCUGUGA.AU.-UGGCGCAGC-.GUCUCUACAAGACA-CC..UU-AA-UGUCU.A.ACAAUAAGUAAGCUU.UUAGGCUUGC | |
| NC_004606.1/977077-977173 | SEQ ID NO:178 |
| UGAAUUCAAUAAUGA.CA.UACUUAUUUAU-GCUGUGA.AU.-UGGCGCAGC-.GUCUCUACAAGACA-CC..UU-AA-UGUCU.A.ACAAUAAGUAAGCUU.UUAGGCUUGC | |
| NC_006086.1/857664-857760 | SEQ ID NO:179 |
| UGAAUUCAAUAAUGA.CA.UACUUAUUUAU-GCUGUGA.AU.-UGGCGCAGC-.GUCUCUACAAGACA-CC..UU-AA-UGUCU.A.ACAAUAAGUAAGCUU.UUAGGCUUGC | |
| NZ₋AAFV01000199.1/507-411 | SEQ ID NO:180 |
| UGAAUUCAAUAAUGA.CA.UACUUAUUUAU-GCUGUGA.AU.-UGGCGCAGC-.GUCUCUACAAGACA-CC..UU-AA-UGUCU.A.ACAAUAAGUAAGCUU.UUAGGCUUGC | |
| NC_004605.1/1369721-1369821 | SEQ ID NO:181 |
| UAUAAUCGCAAGCGU.UU.GCUUCGUAUAACCCCAAUG.AU.AUGGUUUGGGG.GUCUCUACCAGUUC-CC..GCAAA-GUGCU.G.AUUACGAAGAGUUGA.GAUCACUGUG | |
| NZ_AAAW03000004.1/66862-66959 | SEQ ID NO:182 |
| UGAACUGAAUAAUAA.AA.UUUUUAUAUAA-GUUCAUA.AU.-GGGUUGAAC-.GUCUCUACCAACUA-CC..GUAAA-UAGUU.G.AUUAUAAAAAUUUCG.AACGGAAUGA | |
| NC_004567.1/2968830-2968731 | SEQ ID NO:183 |
| UUAUCAAUACAACUA.AU.UGCCUAUAUAAU-GCCAUG.AU.AUGGAUGGCGA.GUUUCUACCCAGUG-CC..GUAAA-CACUG.G.ACUAUAAGCGAAUUG.AGUCGACGGG | |
| NZAAEV01000003.1/13765-13667 | SEQ ID NO:184 |
| ACAAUCAAAUAAAAC.UU.AGCCUAUAUAAUGUC-UUA.AU.CUGGU-UGACA.GUUUCUACCCAACG-CC..GUAAA-UGUUG.G.ACUAUAGGAAAACUA.ACUCUUAUGU | |
| NC_002570.2/806873-806971 | SEQ ID NO:185 |
| UUAAUCGAGCUCAAC.AC.UCUUCGUAUAUCCUC-UCA.AU.AUGGGAUGAGG.GUCUCUAC-AGGUA-CC..GUAAA-UACCU.A.GCUACGAAAAGAAUG.CAGUUAAUGU | |
| NZ_AAEKOI000052.1/27552-27452 | SEQ ID NO:186 |
| AUUAAUUACAUAUGA.GA.AUCAUGUAUAACUCCAAGA.AU.AUGGCUUGGGG.GUCUCUACCAGGAA-CC..AAUAA-CUCCU.G.ACUACAAAAUGCGUA.UUAUAGCGUU | |
| NC_006814.1/237722-237626 | SEQ ID NO:187 |
| AUUAAGCCAAUACAA.AU.AUCU-AUAUAU-CGUCGAA.AU.AAGGUCGACA-.GUUUCUACCCACUA-CC..GUAAA-UGGUG.G.ACUAU-AGGUAAACG.AAAUUAAGAA | |
| NC_005363.1/3414604-3414703 | SEQ ID NO:188 |
| AAAUAACUUCAUAGUgUU.UCCCCGUAUAU-GUUGCGA.AU.AGGGCGCAGC-.GUUUCUACCAGGCA-CC...UCAAA-UGCCU.G.ACUAUGGAGGUUCUU.UGUGAAGUUG | |
| NZ_AADT03000021.1/9410-9513 | SEQ ID NO:189 |
| AAAAUAAAUAUGGCA.AU.GGCCUGUAUAAUUGGGGGA.AU.AGGGCUCCCAA.GUUUCUACCGGGCAACC..GUAAAUUGCCCUG.GCUACAGCCUGAAAG.UGUACCUCAG | |
| NC_004668.1/2288426-2288328 | SEQ ID NO:190 |
| UCCUAGAGAAACAUA.GA.AGCUUGUAUAA-GGUCAAA.AU.ACGGUUGACC-.GUCUCUACCCAGCA-CC..GUAAA-UGCUG.G.UCUAUAAGUGAAGAA.GAGCGGAUAG | |
| NC_006449.1/1182949-1183044 | SEQ ID NO:191 |
| CAAACAAUGAGAACA.-U.UACUUAUUUAU-GUCACGA.AU.-GGGCGUGAC-.GUUUCUACAAGGUG-CC..GU-AA-CACCU.A.ACAAUAAGUAAGCUA.AUUUAGUCAU | |
| NZ_AAGS01000026.1/9921-10016 | SEQ ID NO:192 |
| CAAACAAUGAGAACA.-U.UACUUAUUUAU-GUCACGA.AU.-GGGCGUGAC-.GUUUCUACAAGGUG-CC..GU-AA-CACCU.A.ACAAUAAGUAAGCUA.AUUUAGUCAU | |
| NZ_AAGQ01000089.1/170-269 | SEQ ID NO:193 |
| UAACUUAACAAAUGU.UUcUGCUUAUAUAU-CGCUGCG.AU.ACGGGUAGCA-.GUCUCUACCCGGAG-CC..GUAAA-CUCCG.G.ACUAUAGGUAAAGAA.GGGCCGGUAU | |
| NZ_AABF02000293.1/1-98 | SEQ ID NO:194 |
| UAGAUAUUAAAUAAA.--.AAUUCGUAUAA-GCCUAAU.AU.AUGGAAAGGU-.GUCCCUAC-GGUUAACC..GUAAAUUAACC.A.GCUACGAAAAAUGUU.UUUGCUGUAU | |
| NC_006448.1/1185082-1185177 | SEQ ID NO:195 |
| CAAACAAUGAGAACA.-U.UACUUAUUUAU-GUCACGA.AU.-GGGCGUGAC-.GUUUCUACAAGGUG-CC..GU-AA-CUCCU.A.ACAAUAAGUAAGCUA.AUUUAGUCAU | |
| NC_004567.1/2410478-2410577 | SEQ ID NO:196 |
| UUCAAAUAAGUGGUA.AU.UGCCUAUAUAAU-GUCAUG.AU.AUGGUUGACGA.GUUUCUACCCAACC-CC..GUAAA-GGUUG.G.ACUAUAAGCAAACGA.GGUCAUCCCG | |
| NZ_AABJ03000010.1/47704-47802 | SEQ ID NO:197 |
| AUUCAGAAUGUUGAA.AA.AGCUUAUAUAUGGUCGU-A.AU.AAGG-AUGACC.GUUUCUACCCGGAG-CC..ACAAA-CUCAG.G.ACUAUAAGCAAUUAA.GUACUUGUGC | |
| NZ_AADT03000002.1/89184-89083 | SEQ ID NO:198 |
| GAGUCUUCUUUUAGG.UU.UCUUCGUAUAGUCCCGGAG.AU.-UGGUCCGGGG.GUUUCUACCAGGUGACC..GG-AAUCACCUuG.GCUACGAAGGGUUAU.UUCCUUUGUG | |
| NC_006814.1/1971978-1972076 | SEQ ID NO:199 |
| AUACUUAACAAUCAA.GU.UAUCUAUAUAU-CGUCGAA.AU.AAGGUCGACA-.GUAUCUACCCUGAG-CC..AUAAA-UUCAG.G.ACUAUAGGUAUCAGA.CGUCAUAAUU | |
| NZ_AAA002000016.1/1579-1480 | SEQ ID NO:200 |
| CACUCUAGAUAUCAA.AA.UAUCUAUAUAU-CGUCGUA.AU.AAGGUCGACA-.GUUUCUACCCGGAA-CCa.AUUAA-UUCUG.G.ACUAUAGGUAAUCGA.UGUCAUAAGU | |
| NC_005362.1/1949387-1949485 | SEQ ID NO:201 |
| AUACUUAACAAUCAA.GU.UAUCUAUAUAU-CGUCGAA.AU.AAGGUCGACA-.GUAUCUACCCUGAG-CC..AUAAA-UUCAG.G.ACUAUAGGUAUCAGA.CGUCAUAAAU | |
| NZ_AAA002000035.1/3299-3201 | SEQ ID NO:202 |
| AUACUUAACAAUCAA.GU.UAUCUAUAUAU-CGUCGAA.AU.AAGGUCGACA-.GUAUCUACCCUGAG-CC..AUAAA-UUCAG.G.ACUAUAGGUAUCAGA.CGUCAUAAAU | |
| NC_005362.1/263146-263049 | SEQ ID NO:203 |
| CACUCUAGAUAUCAA.AU.AUCU-AUAUAU-CGUCGUA.AU.AAGGUCGACA-.GUUUCUACCCGGAA-CCa.AUUAA-UUCUG.G.ACUAU-AGGUAAUCG.AUGUCAUAAG | |
| NC_005363.1/2004933-2004835 | SEQ ID NO:204 |
| ACUACUAACCGCGGU.UA.CACAAAUAUAA-GUCGGAG.AU.AGGGUCUGAC-.GUUUCUACCUGCCA-CC..GUAAA-GGGCA.G.UCUAUUUGGAGCGAA.UAUAUGUUGA | |
| NZ_AADT03000002.1/98293-98192 | SEQ ID NO:205 |
| GACGAAUAUUACUAU.UA.GACUCGUAUAACCCCGGCG.AU.-GGGGCCGGGG.GUCUCUACCAGGUGACC..GG-AAUCACCUcG.GCUACGAGGGUGAGC.GGCAGCUGGU | |
| NZ_AABH02000036.1/17403-17500 | SEQ ID NO:206 |
| CAACUAAAGAAGUUA.UU.UGCAGAUAUAU-CGUUGGA.AA.ACGGCCAACA-.GUUUCUACCACGCC-CC..-AAAA-GUCGU.G.ACUAUCCGCAAAUGU.UUUUGACGAU | |
| NC_006055.1/484139-484044 | SEQ ID NO:207 |
| UAUAAAAUUAAUAUG.AA.AACUUGUAUAAUCCUUC--.AU.AUCGGGAAGGA.GUCUCUACCUAACA-CC..---AA-UGUUA.G.AUUAUGAGUUUUAUG.GUUUUCGCUA | |
| NZ_AABH02000272.1/211-308 | SEQ ID NO:208 |
| CAACUAAAGAAGUUA.UU.UGCAGAUAUAU-CGUUGGA.AA.ACGGCCAACA-.GUUUCUACCACGCC-CC..-AAAA-GUCGU.G.ACUAUCCGCAAAUGU.UUCUGACGAU | |
| NZ AABJ03000003.1/47905-47810 | SEQ ID NO:209 |
| AAGAUAAAUAGCAAC.CA.AGCAGGUAUAU-CGUCGGA.UA.AUGGCUGACA-.GUUUCUACCCAACA-CC..---AA-UGUUG.G.ACUAUCUGUGGAUGU.CUUUUUGGCG | |
| NZ_AABH02000038.1/17559-17463 | SEQ ID NO:210 |
| UAUUCUAUGUGAAAU.UU.AGCUGAUAUAGUAUCGA--.AUaAUGG-UCGAUU.GUUUCUAGCCAGCA-CC..---CA-UGCUG.GaACUAUCAUAAACAUG.UUAUUUAAUU | |
| NC_006055.1/397027-397123 | SEQ ID NO:211 |
| AAUAAUUAAAUAUAA.AA.AACUUAUACAU-GACAACAuAU.-UGGGUUGUC-.GAC-CUGCCUCUGGACC..-U--AUCCUUA.G.ACUAUAAGCGUGAGG.UUUUUUUACA | |
| #=GC RF | SEQ ID NO:212 |
| aAAauuaaAaAAAaA.au.aacUCgUAUAAucucgggA.AU.AUGGcccgaga.GUUUCUACCaggcaaCC..GUAAAuugccu.G.ACUAcGAguaAauuu.uauuUaUuuu | |
| #=GC | |
| SS_cons :::::::::::::::.::.((((((((,,,<<<<<<<_.__.___>>>>>>>.,,,,,,,,<<<<<<<__..>>>>>>.>.,,))))))))::::::.::::::::::: | |
| // | |

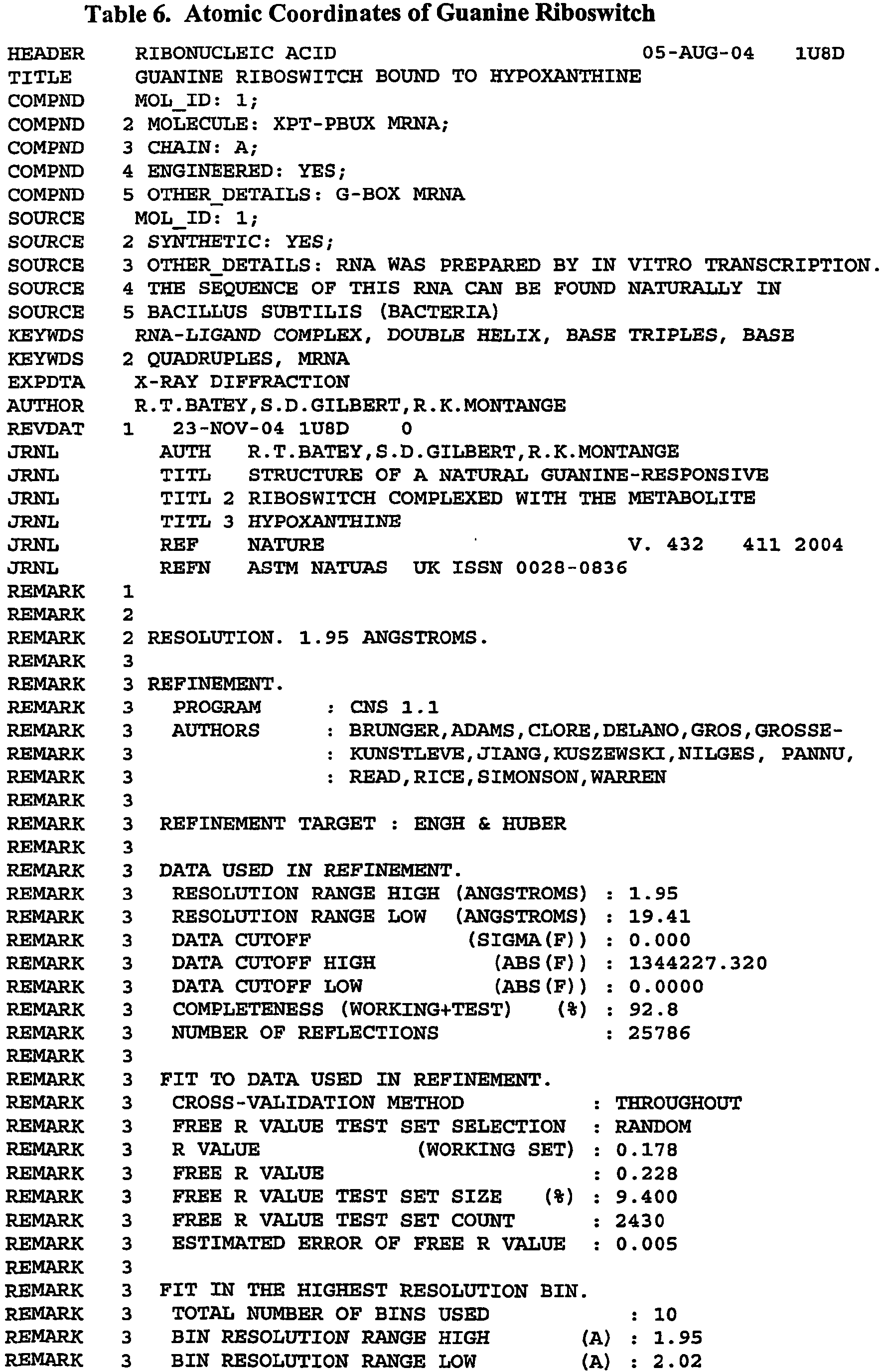

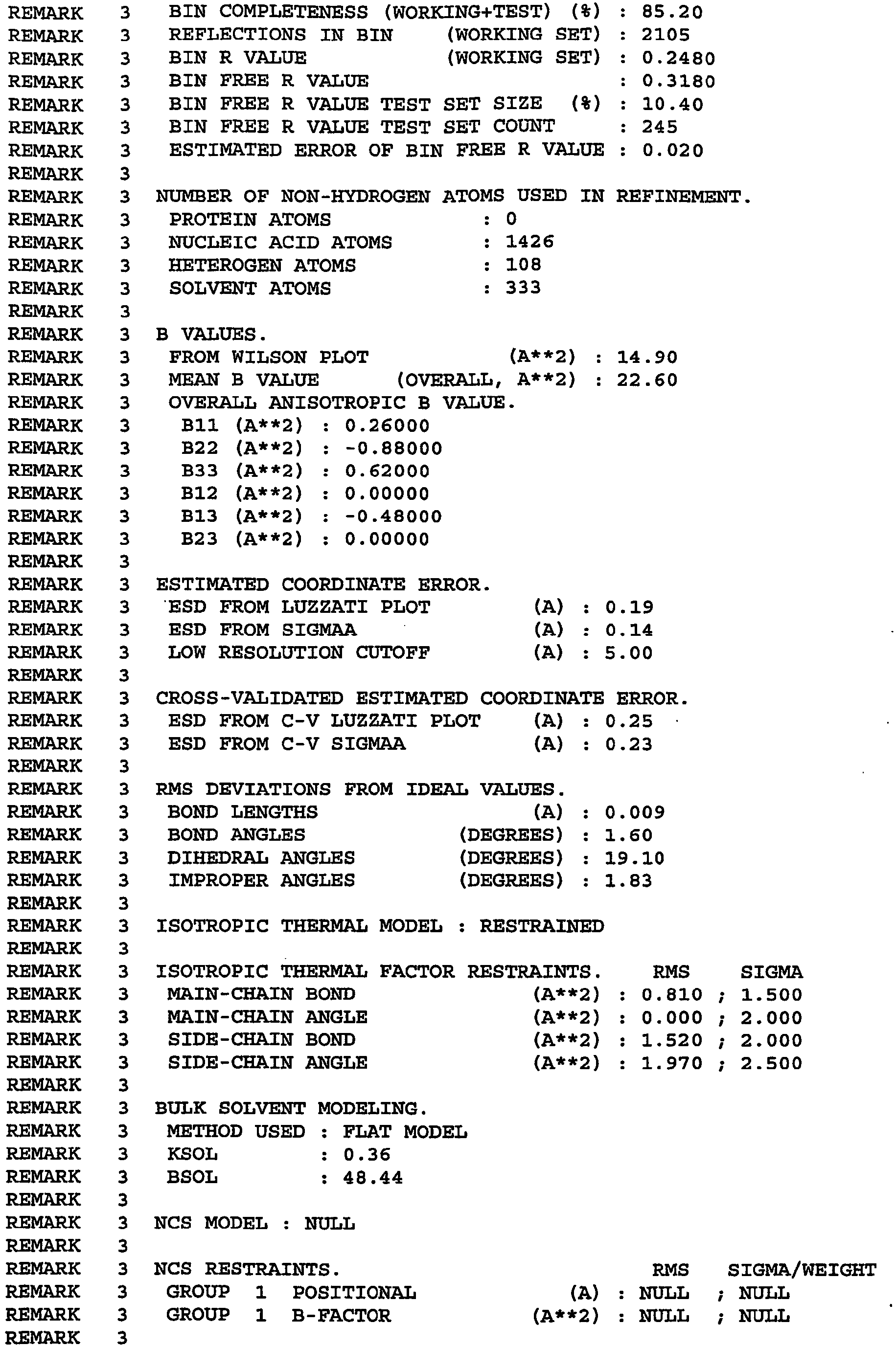

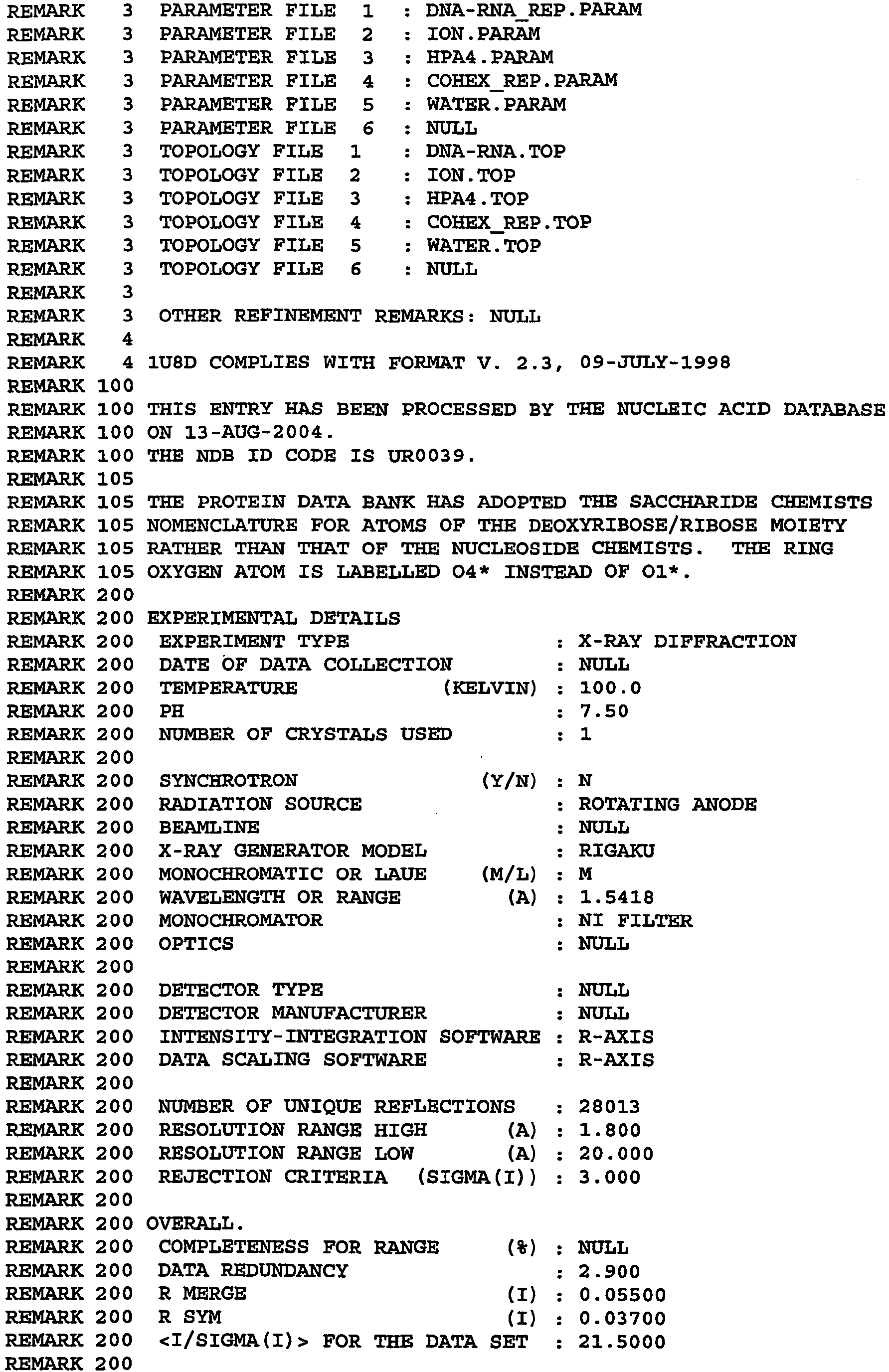

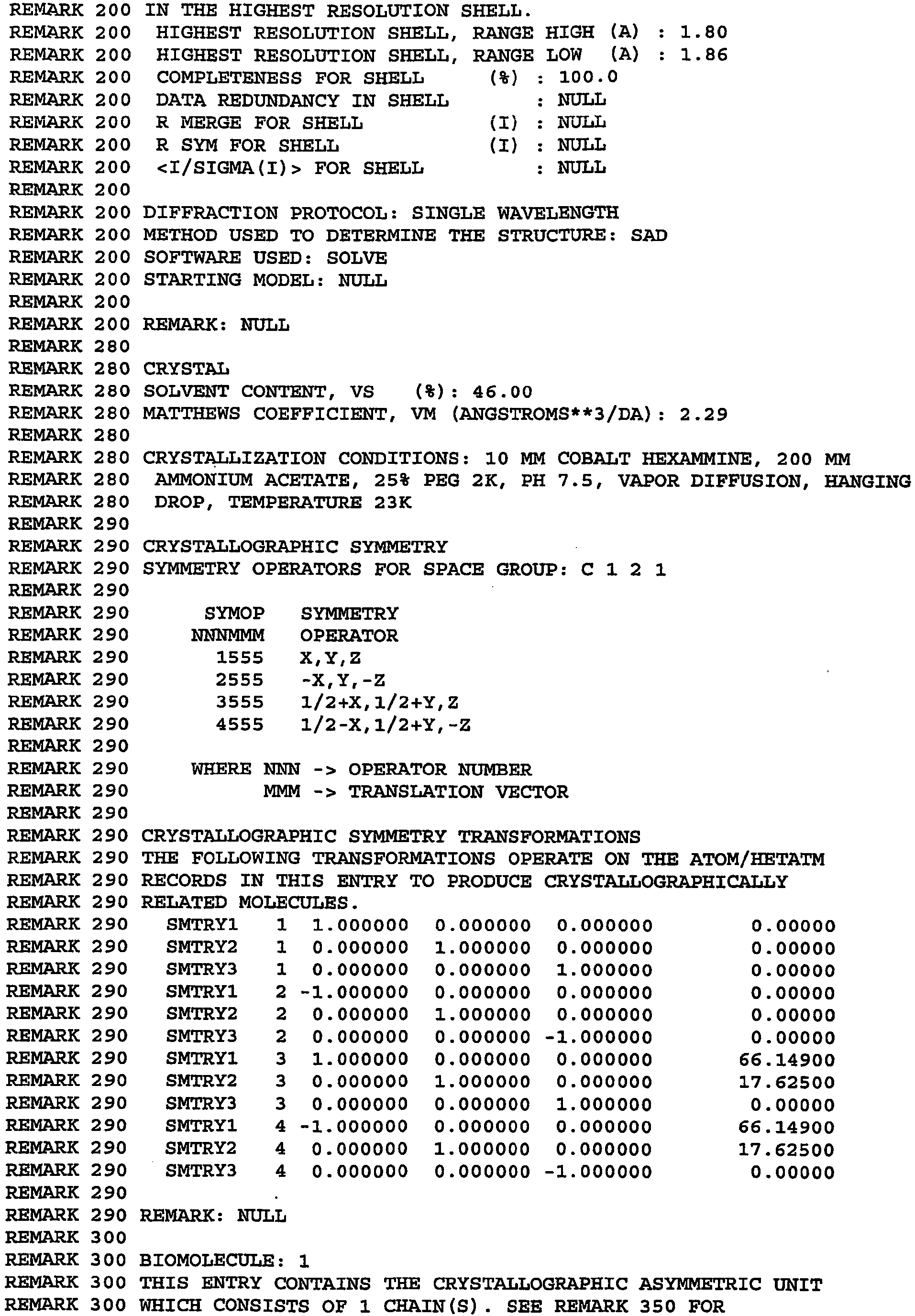

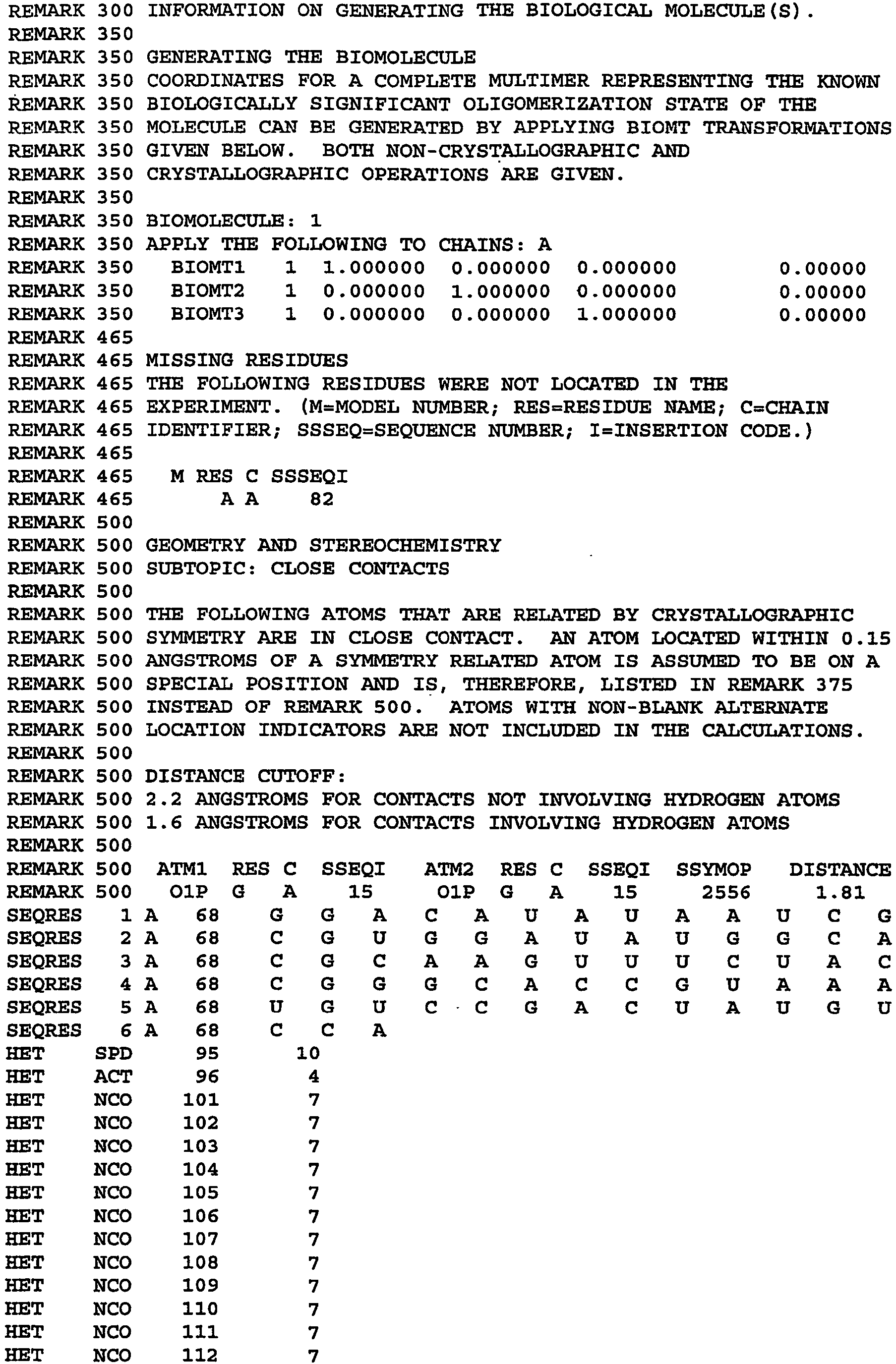

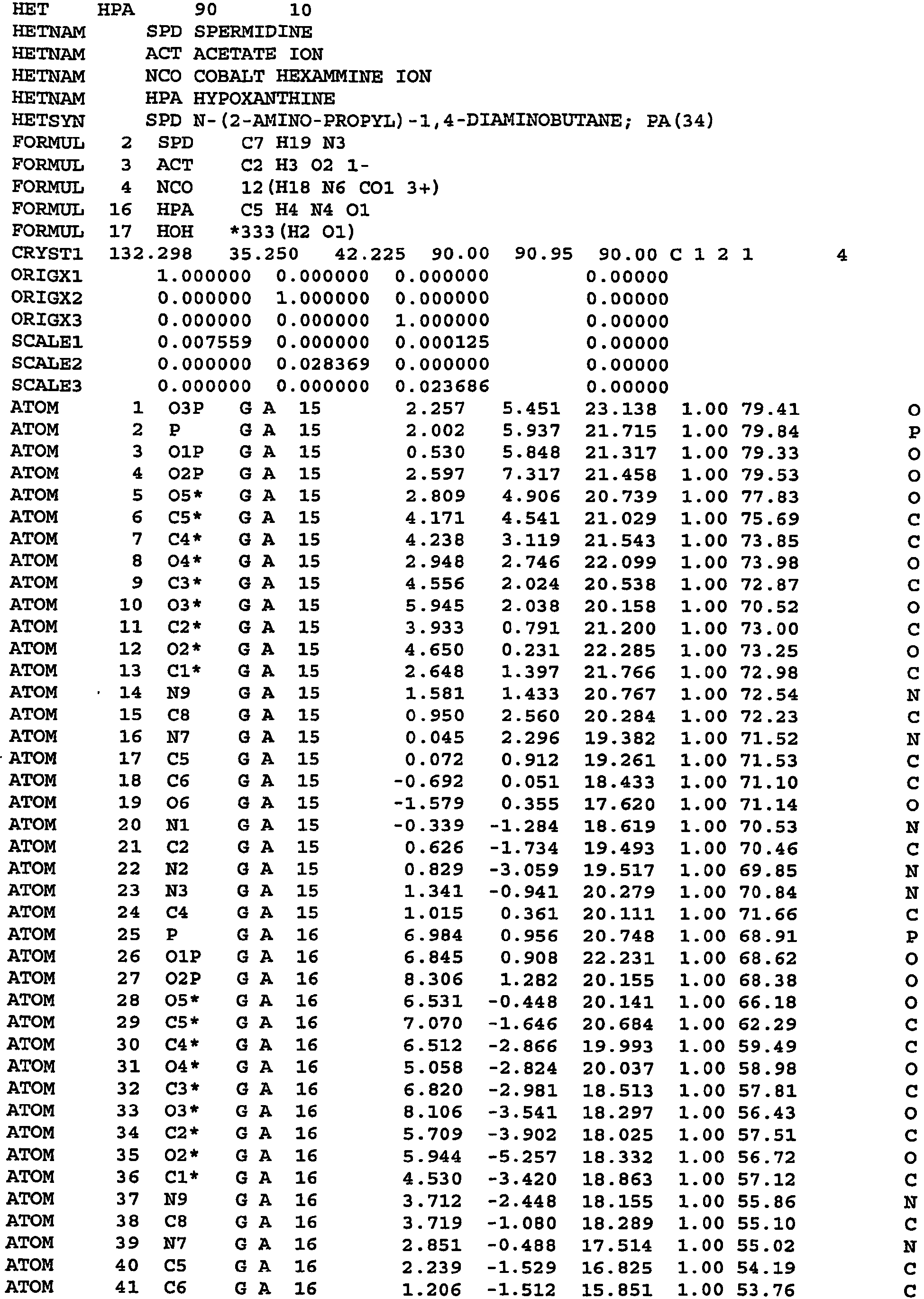

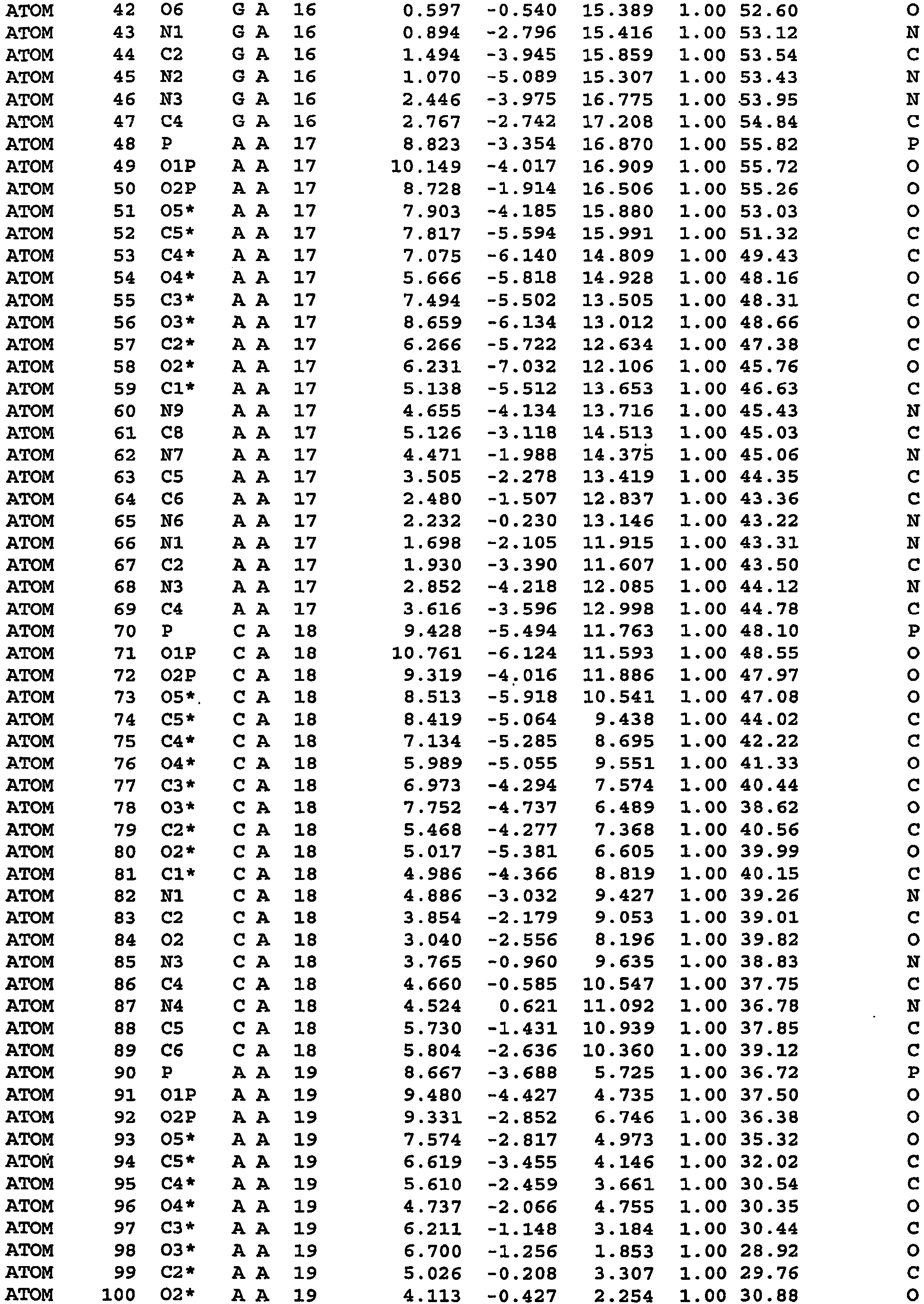

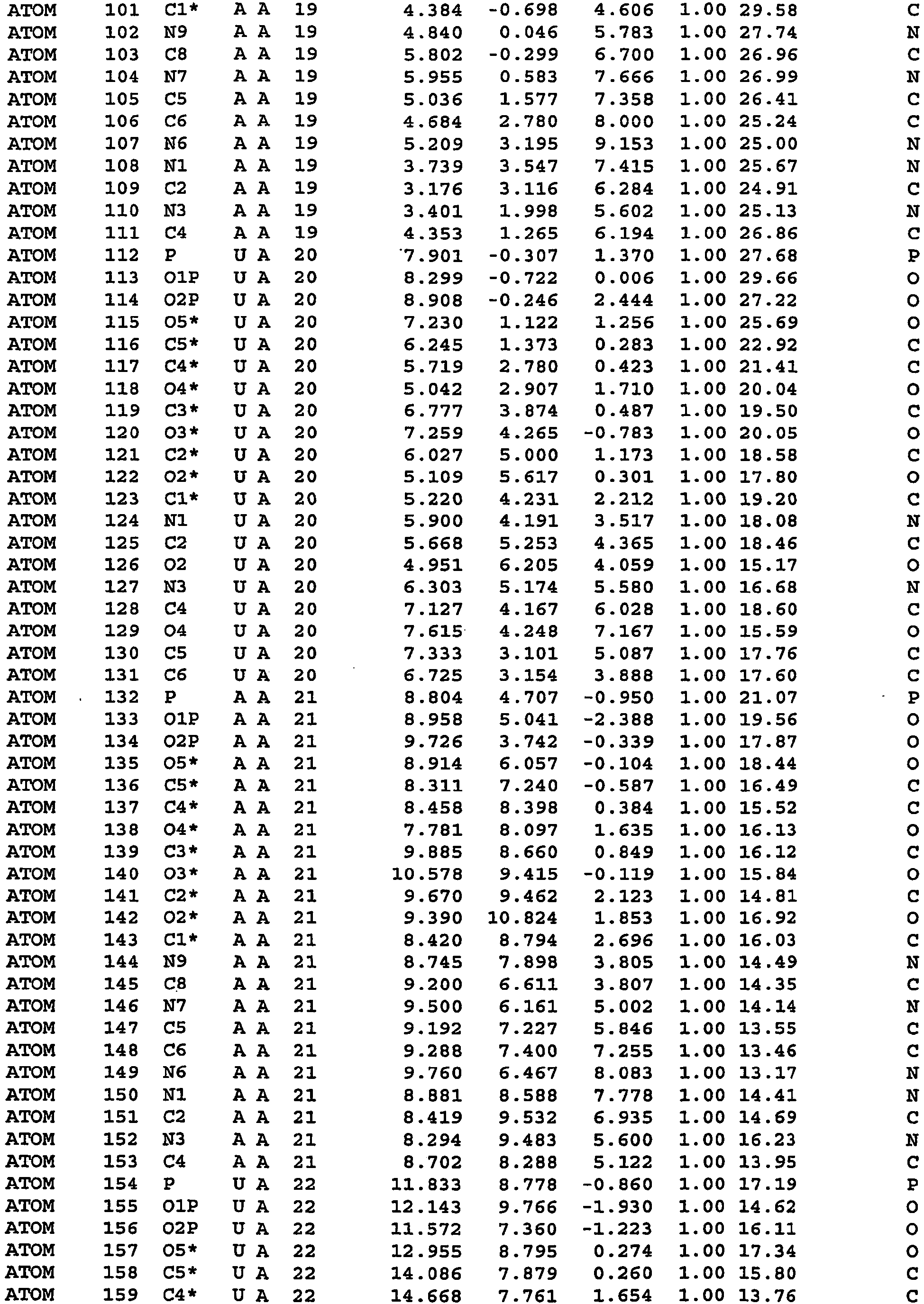

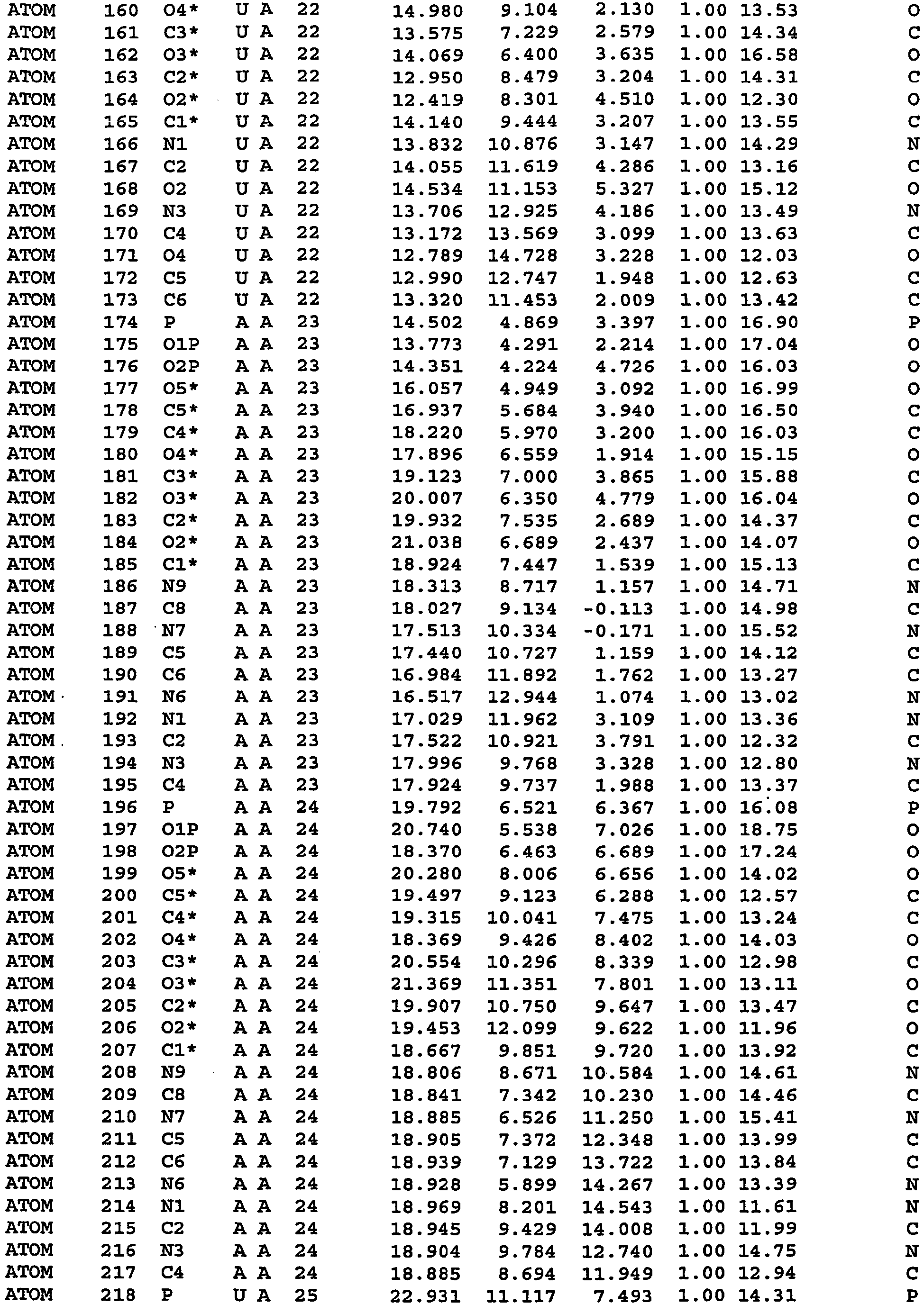

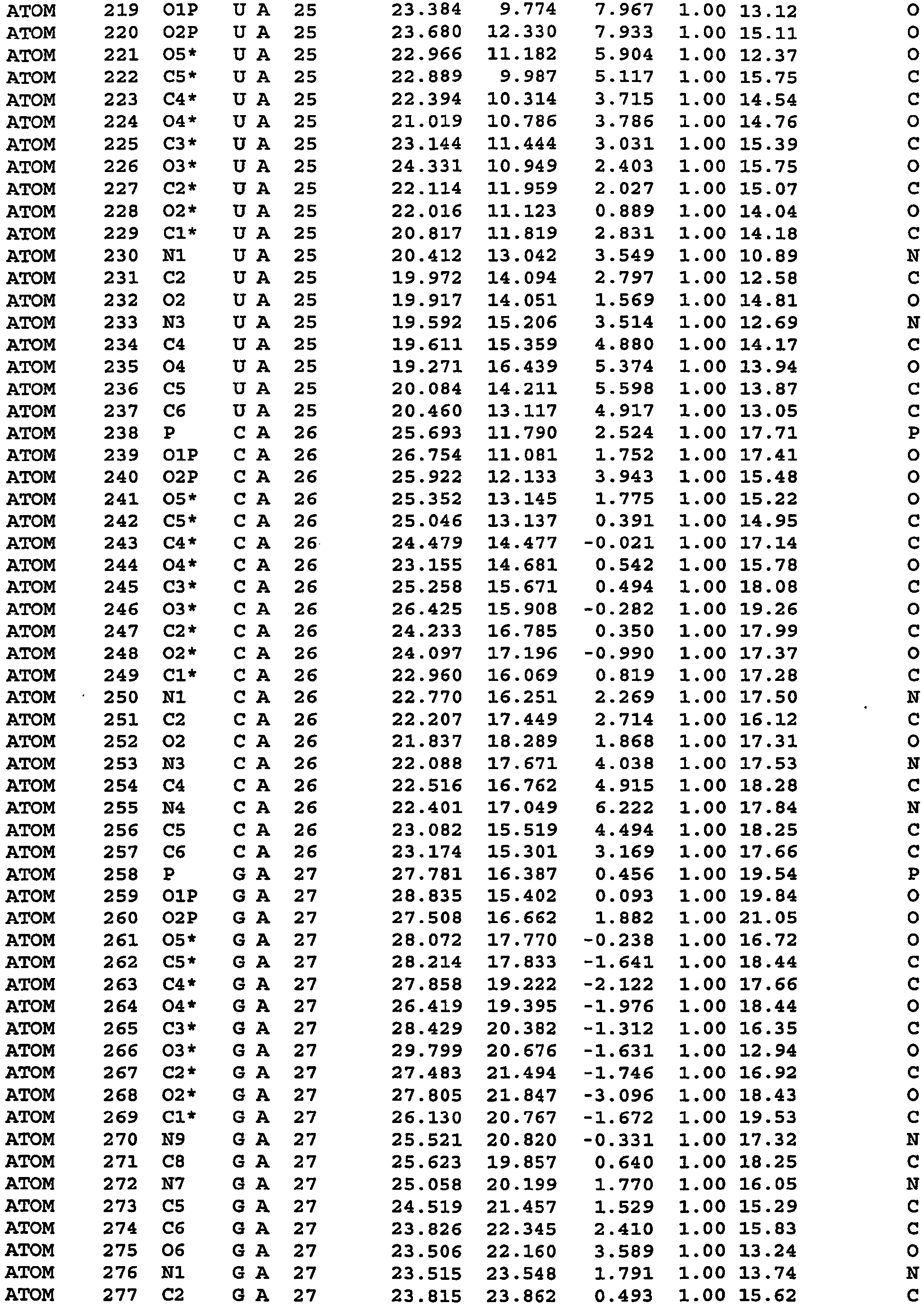

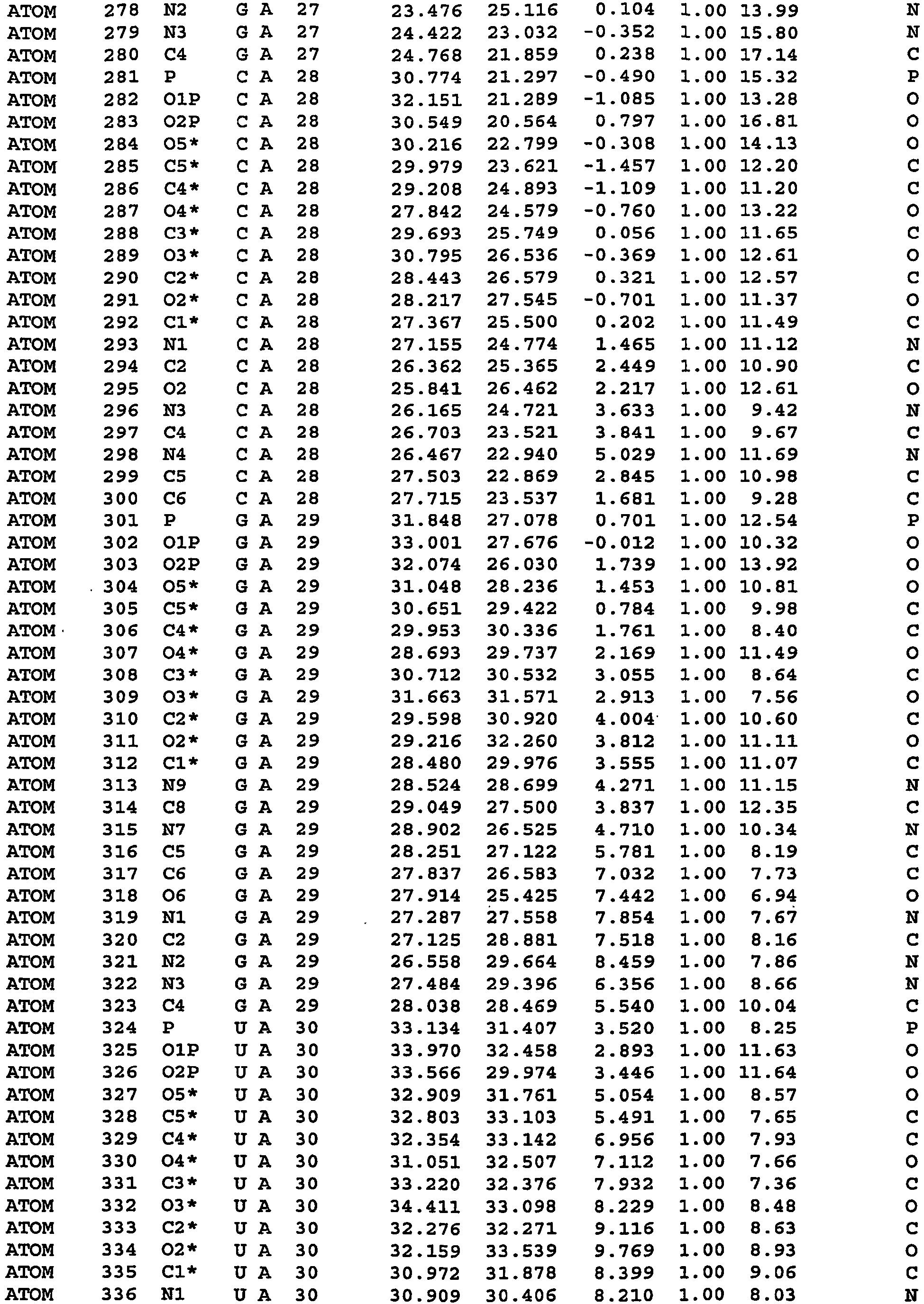

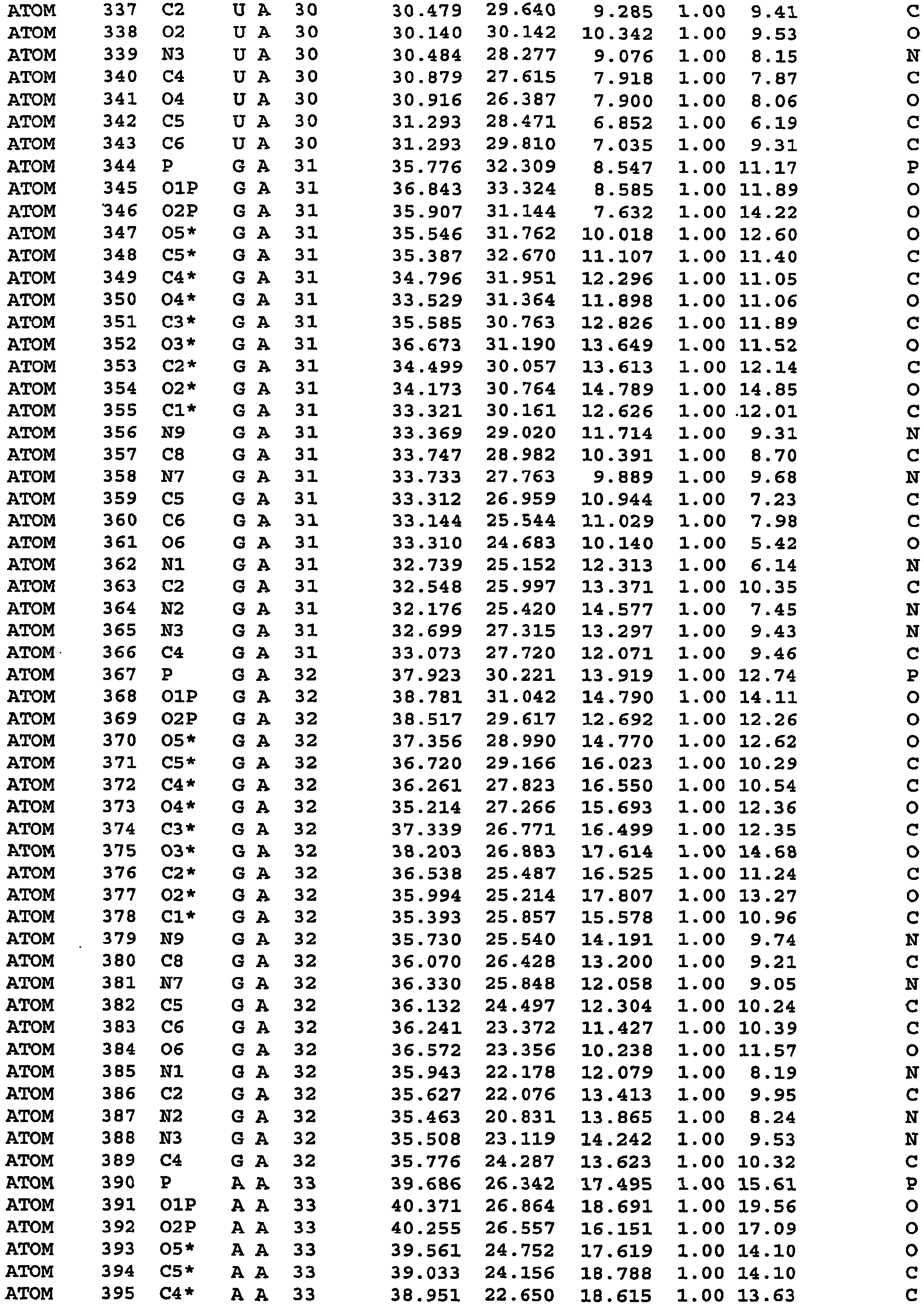

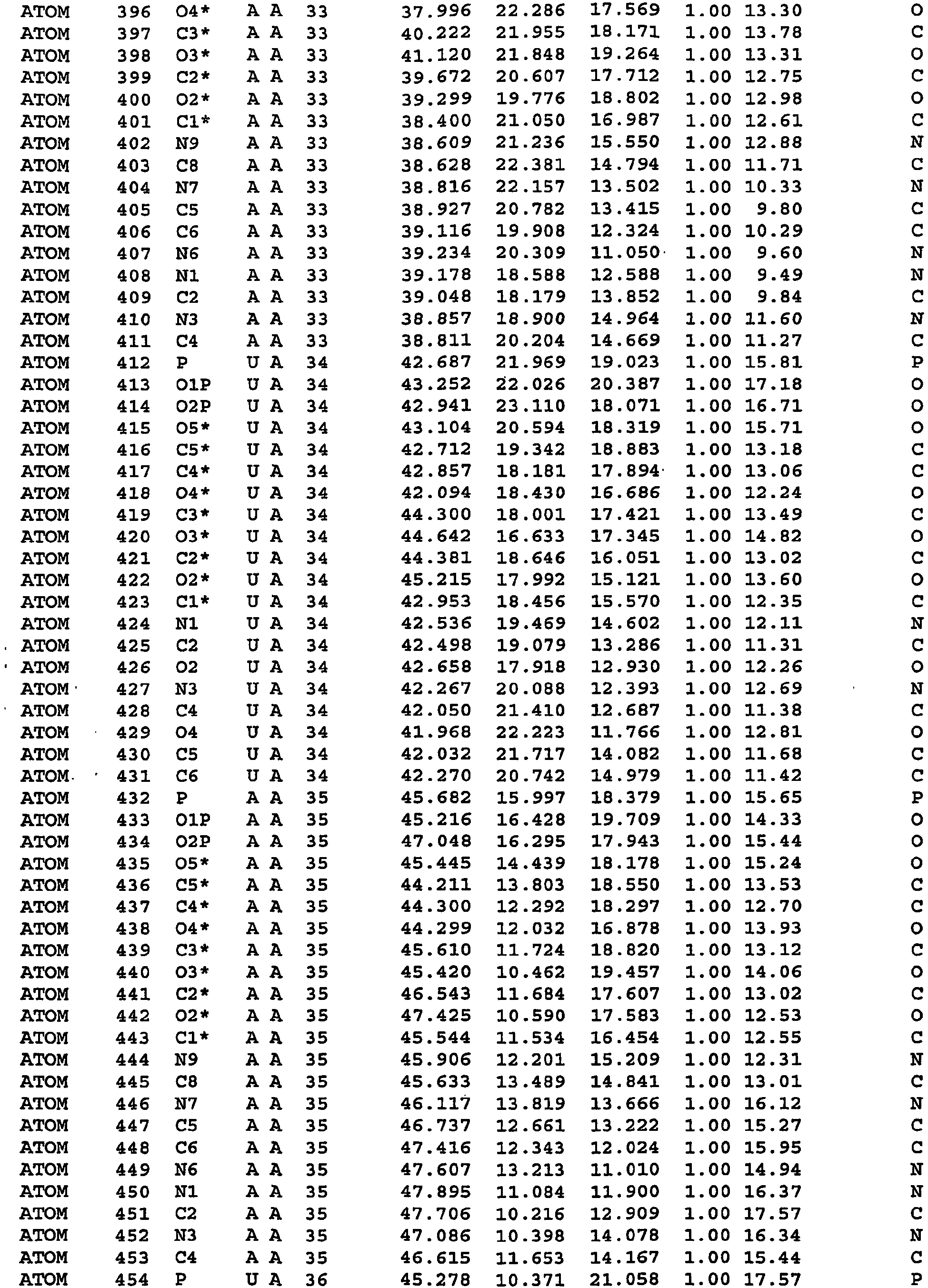

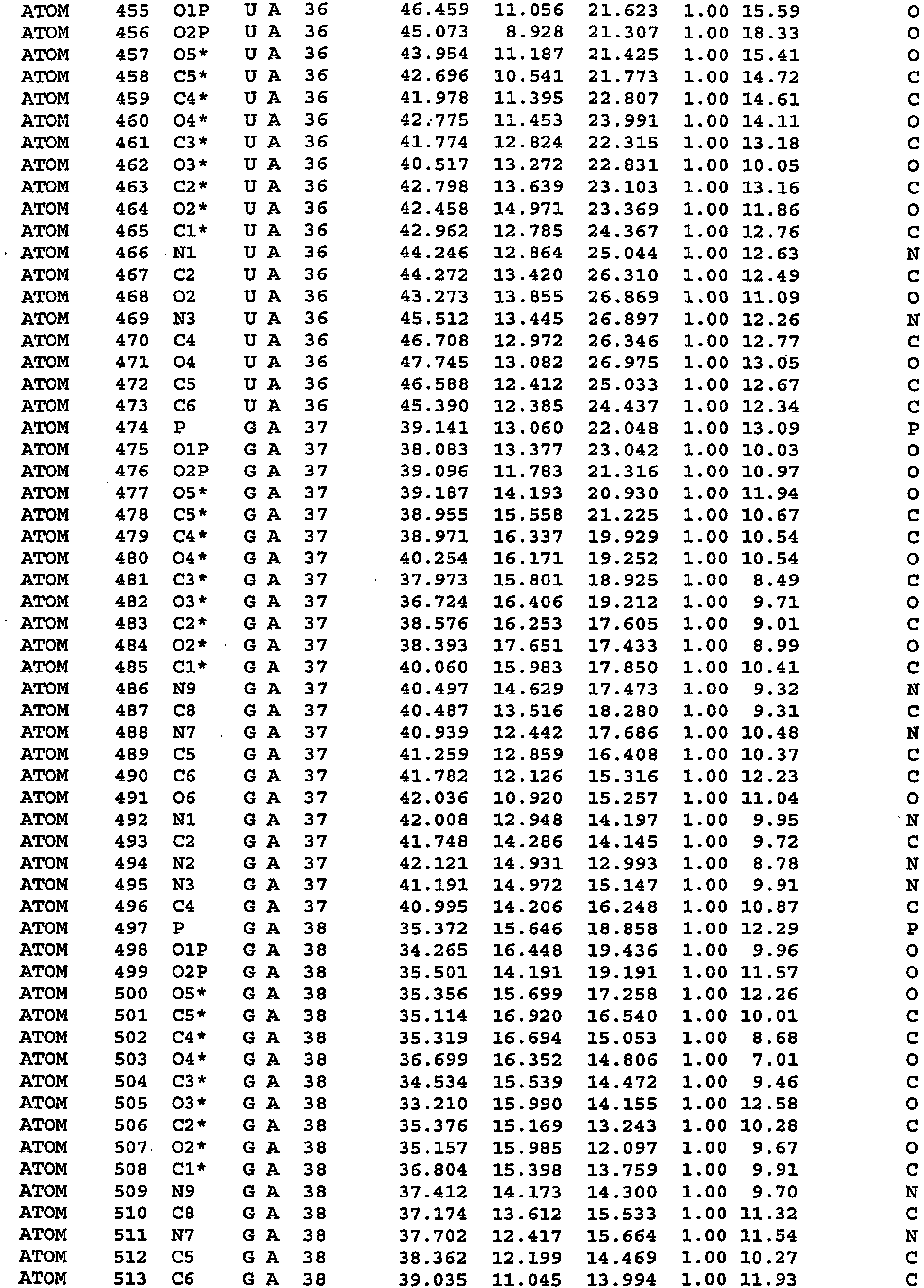

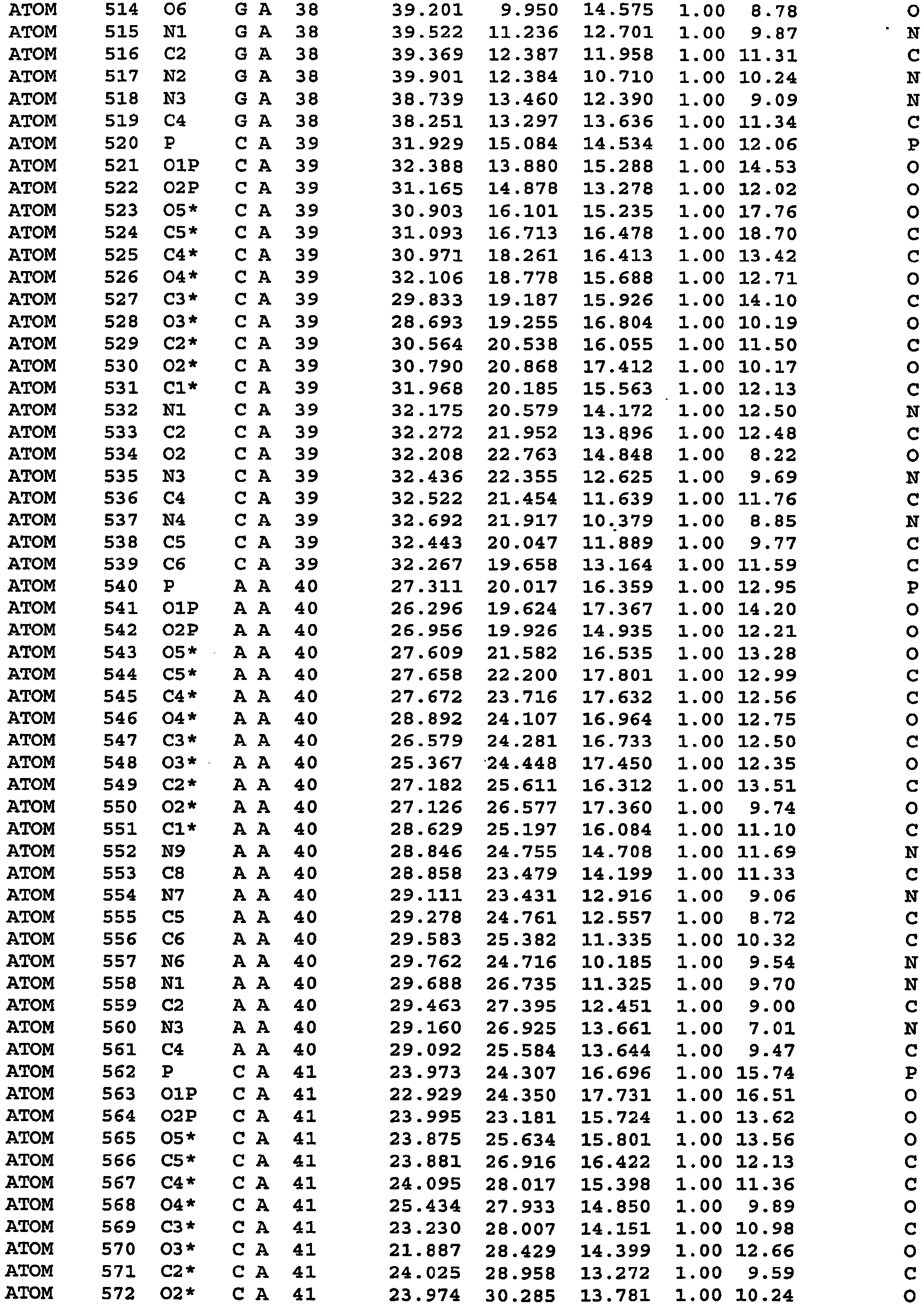

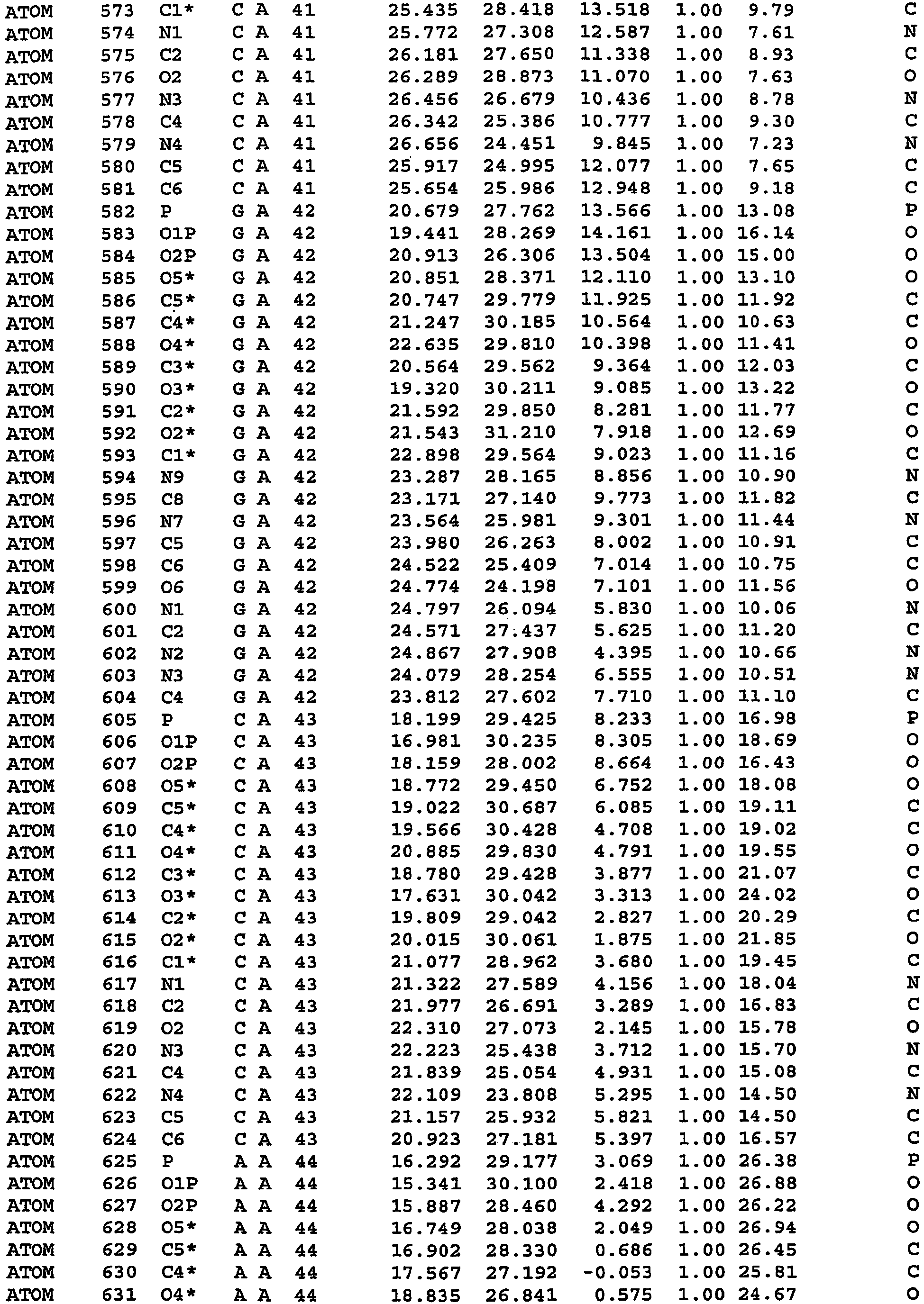

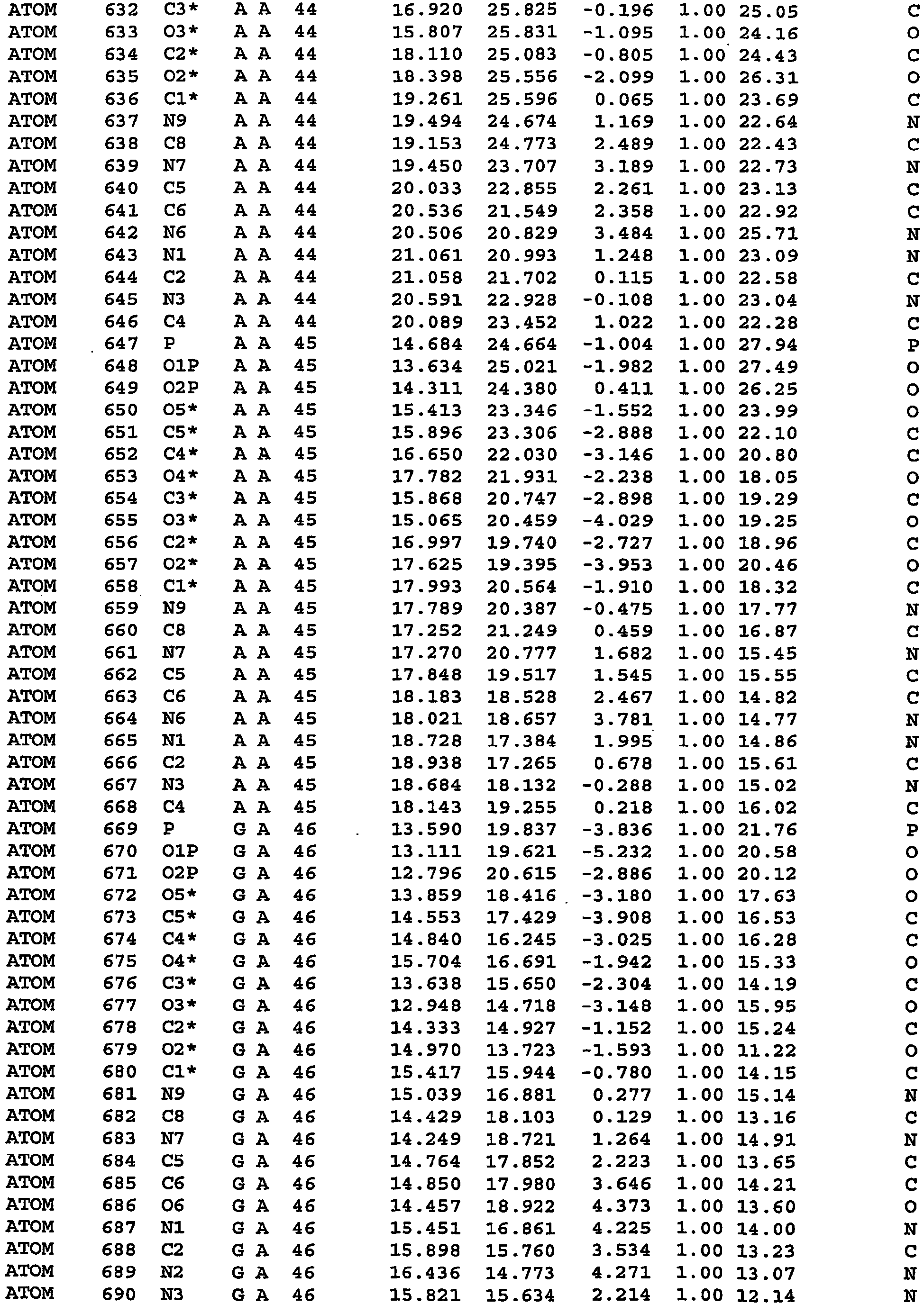

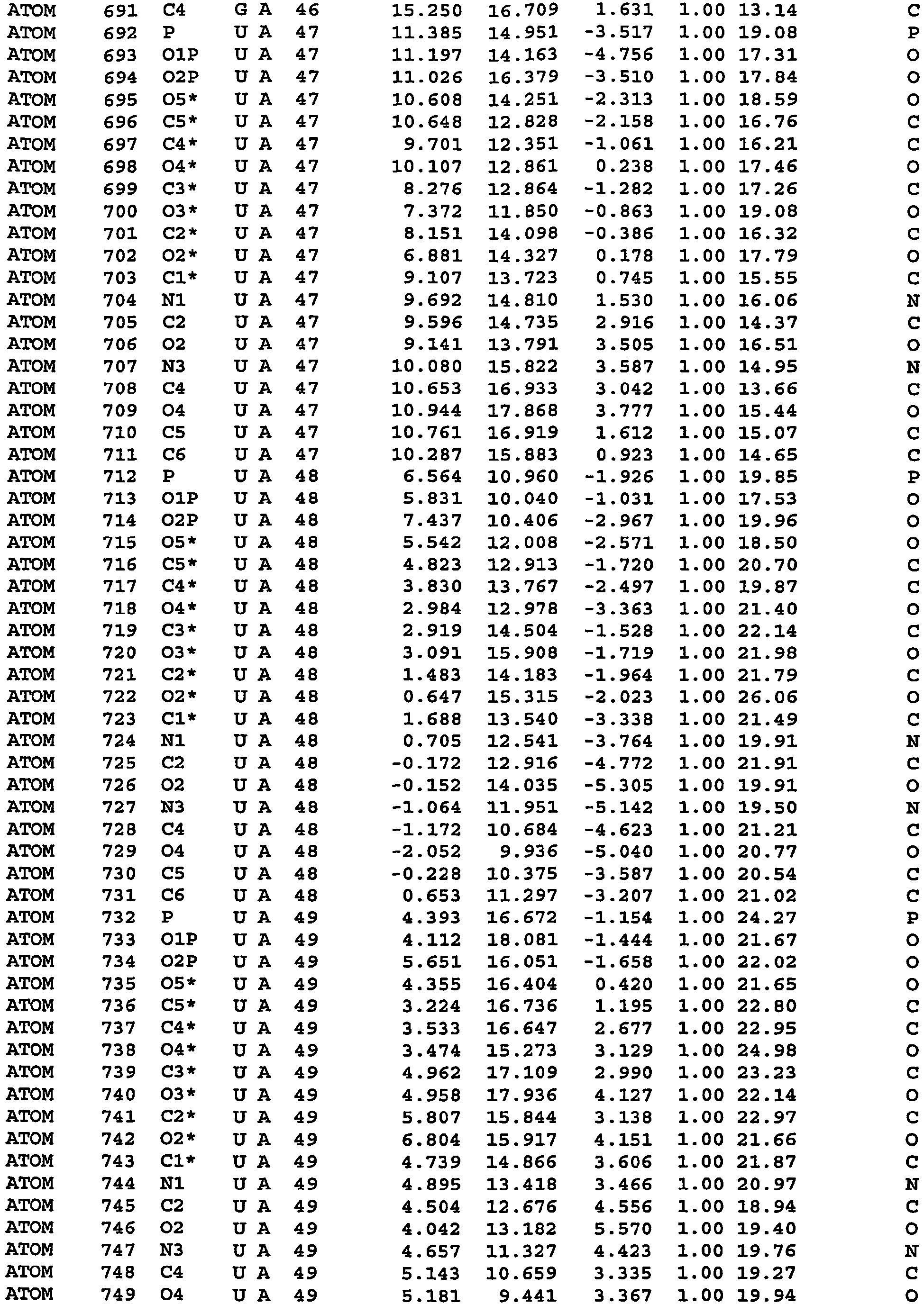

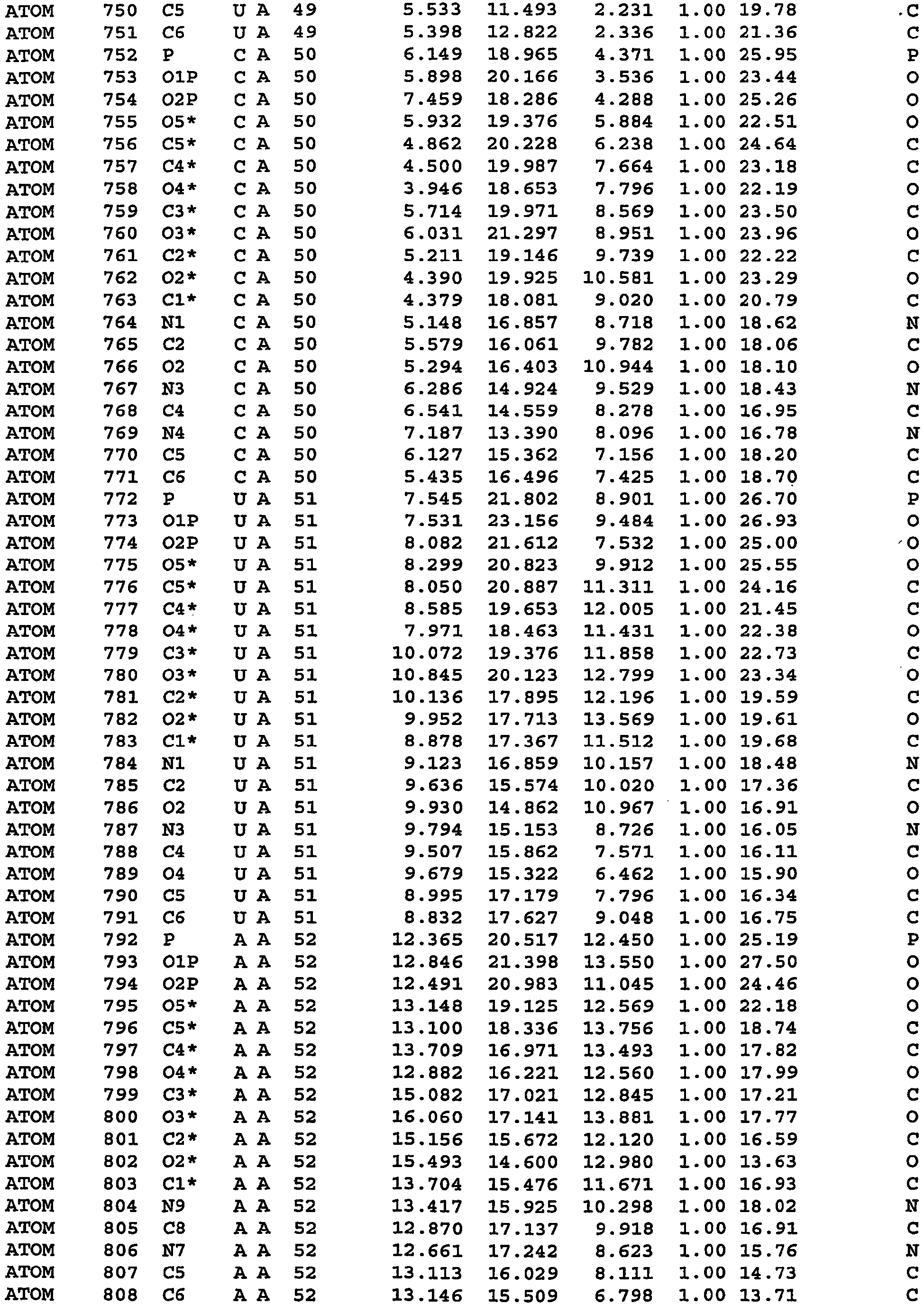

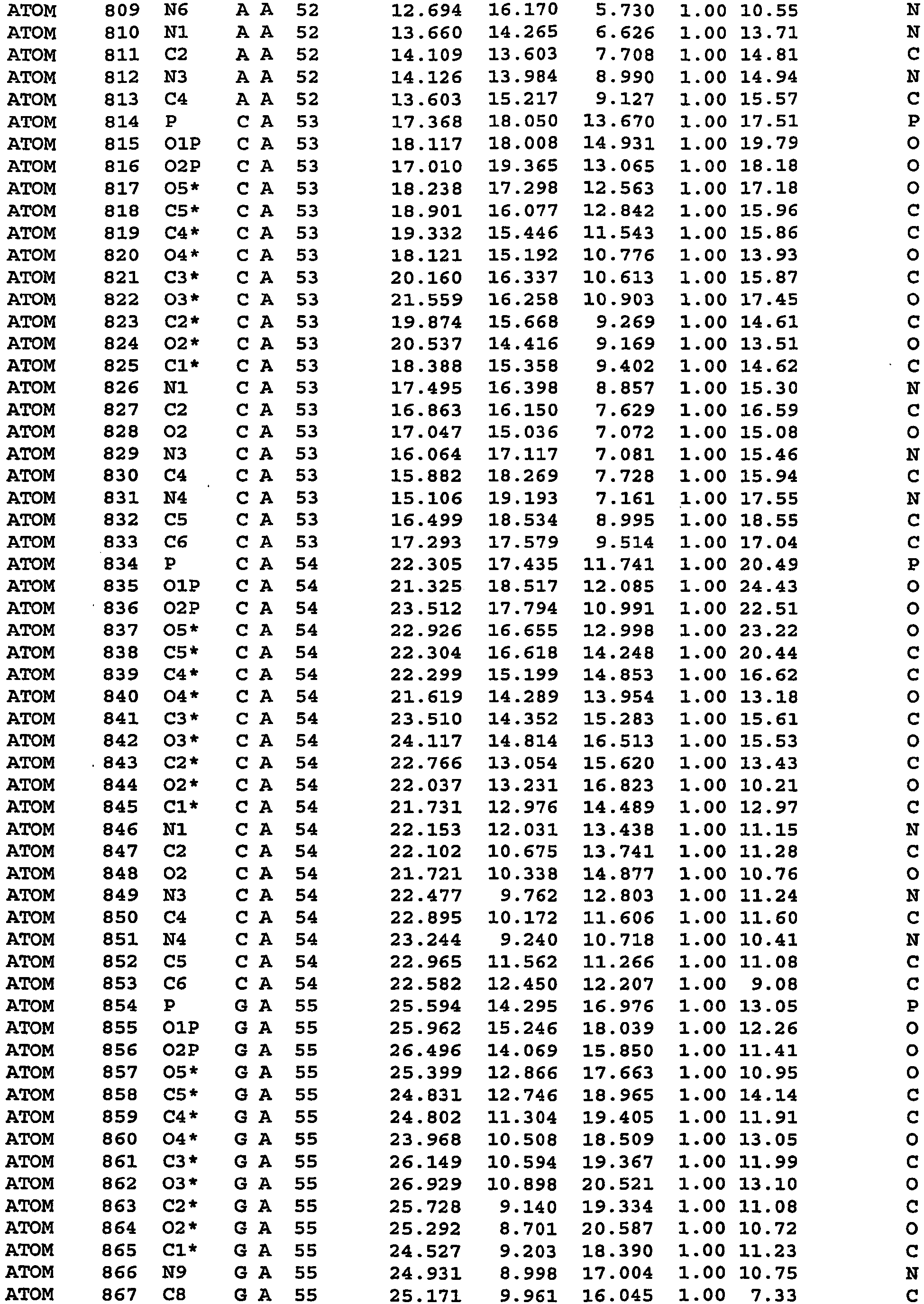

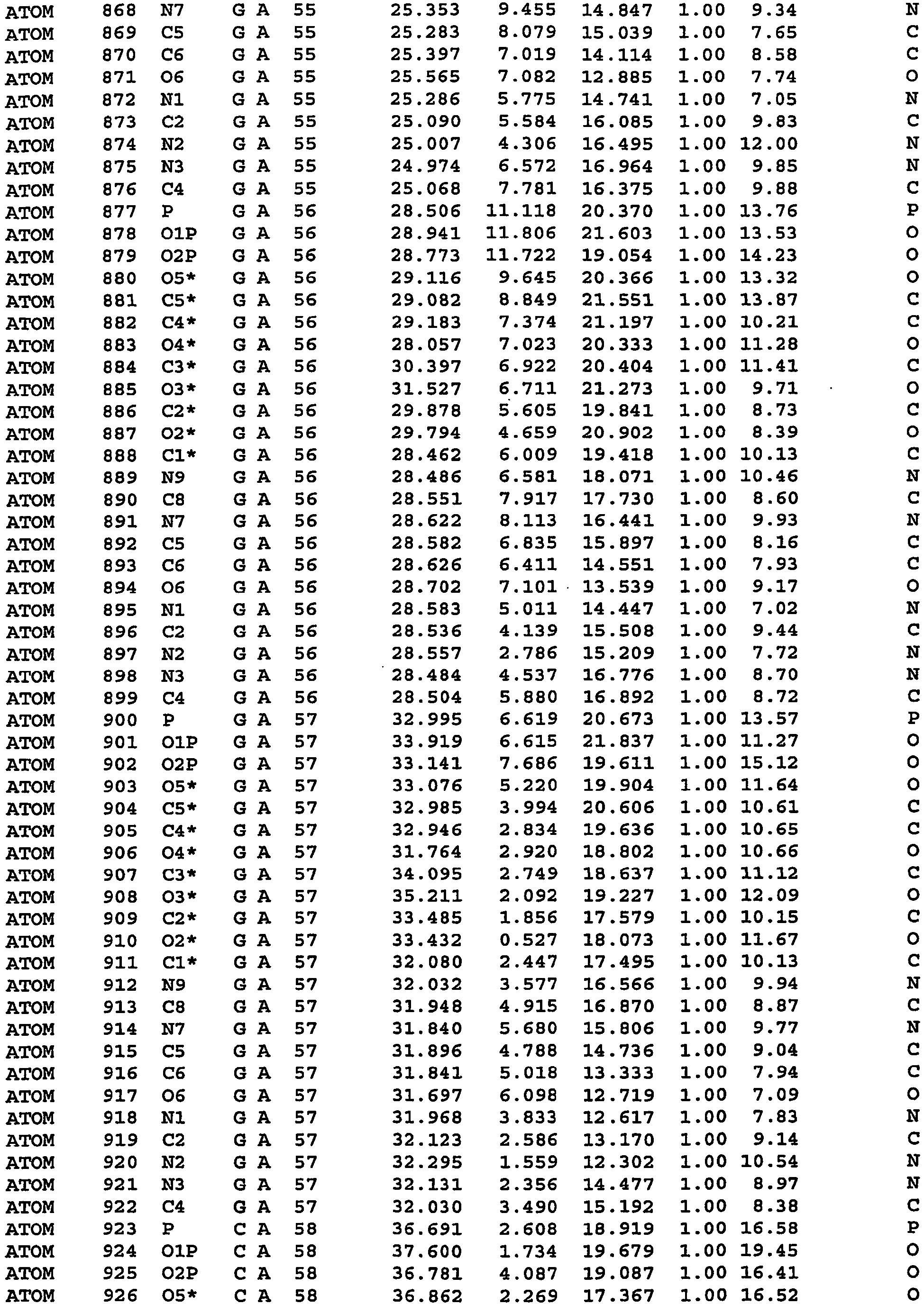

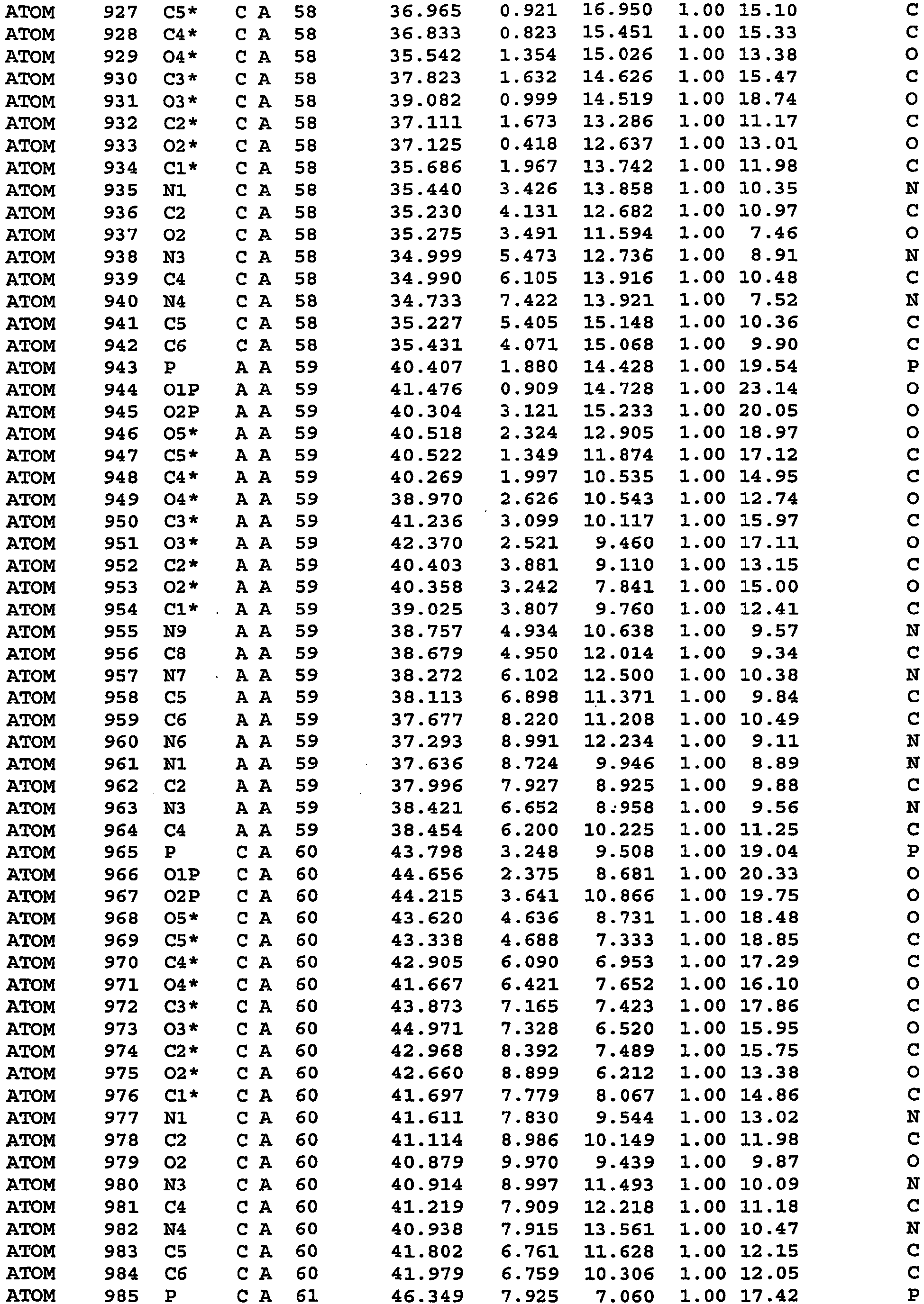

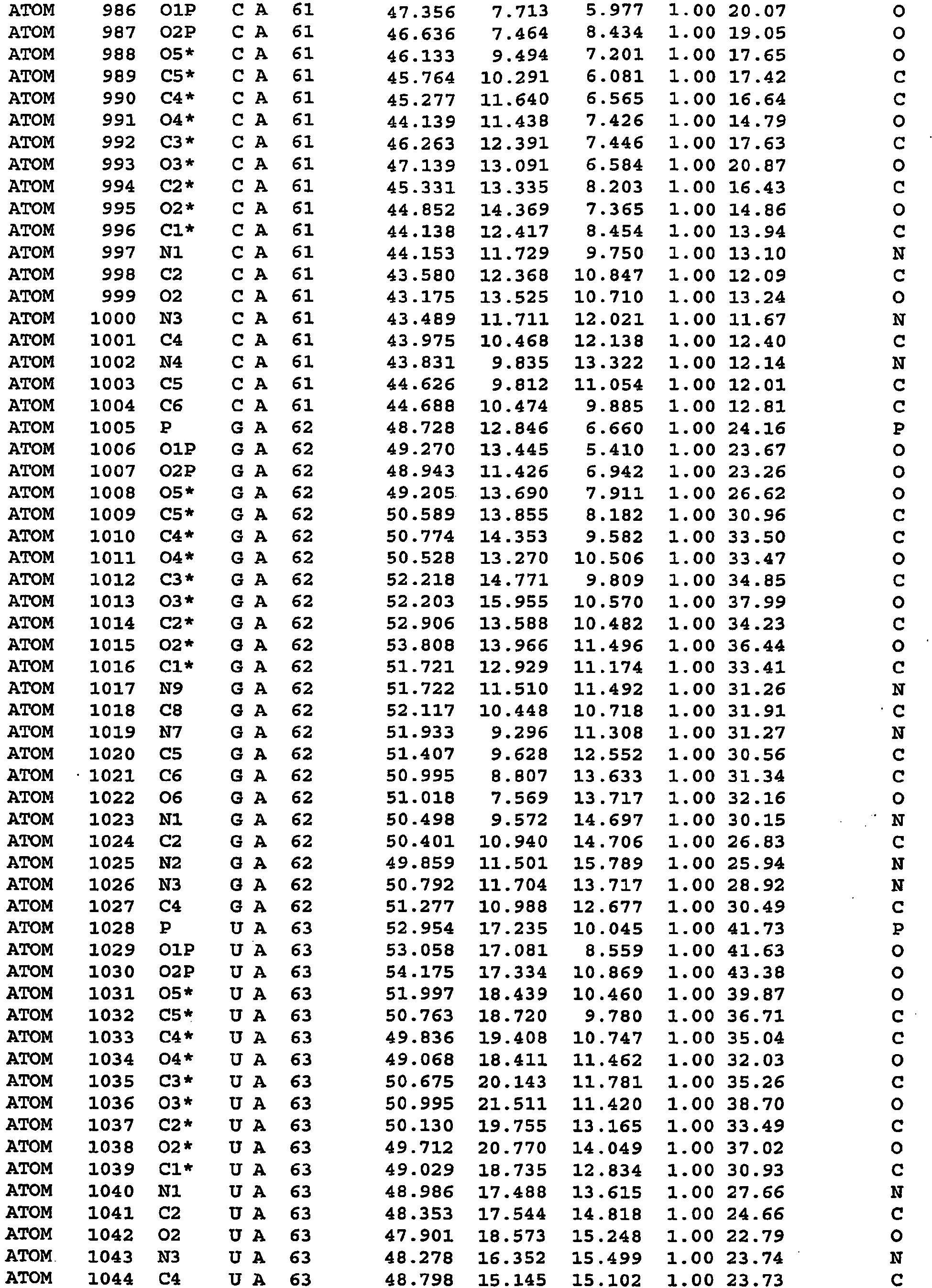

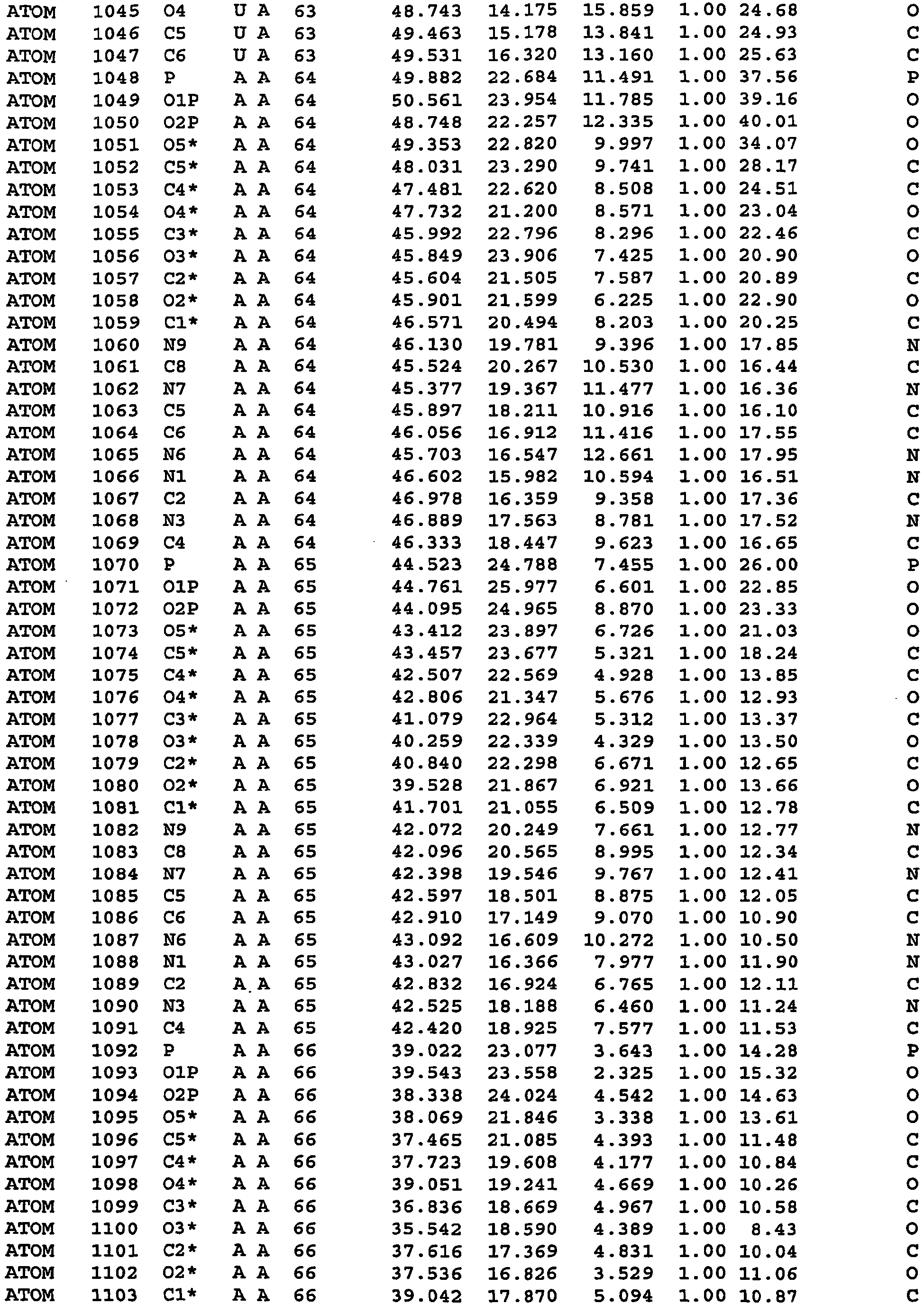

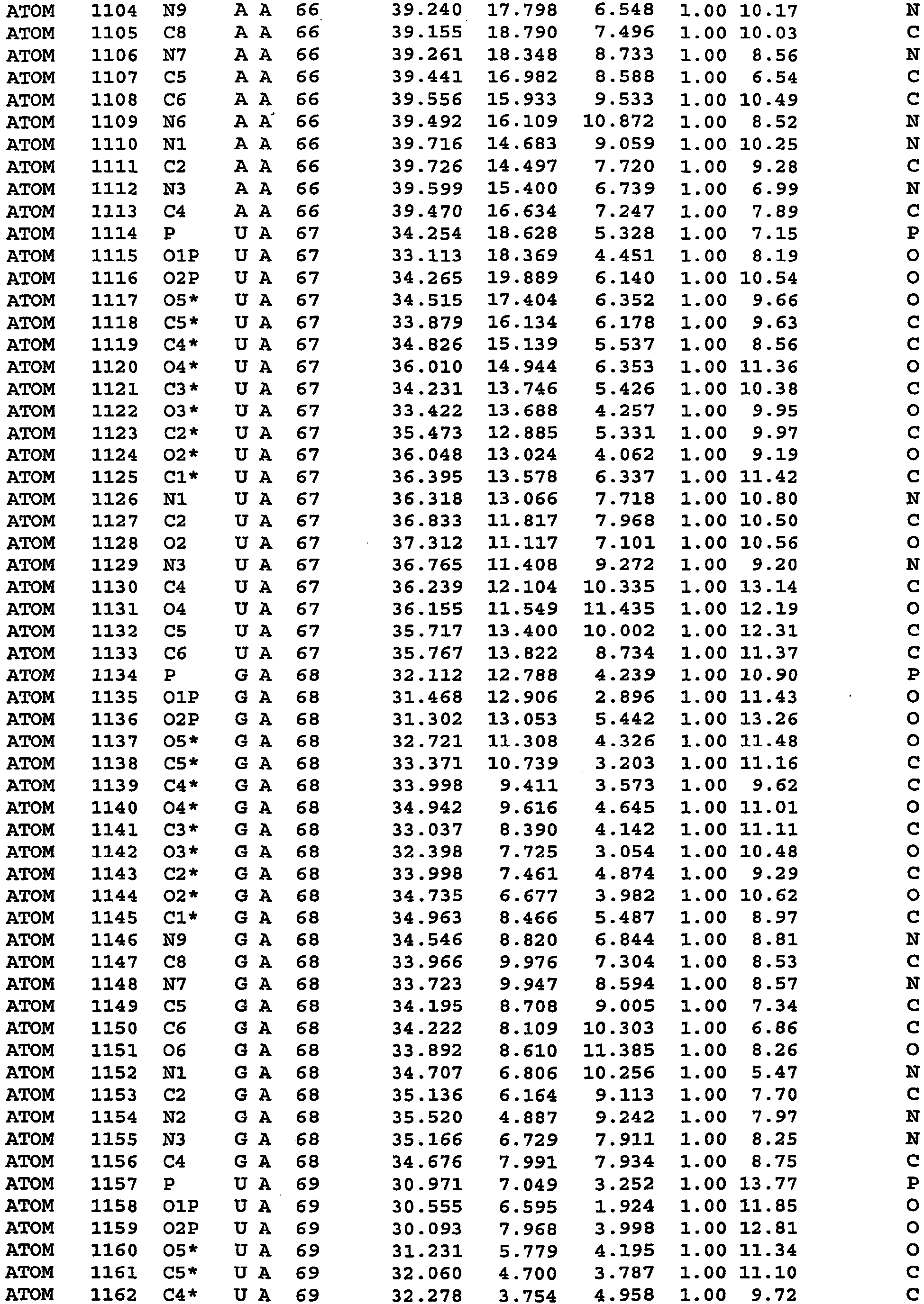

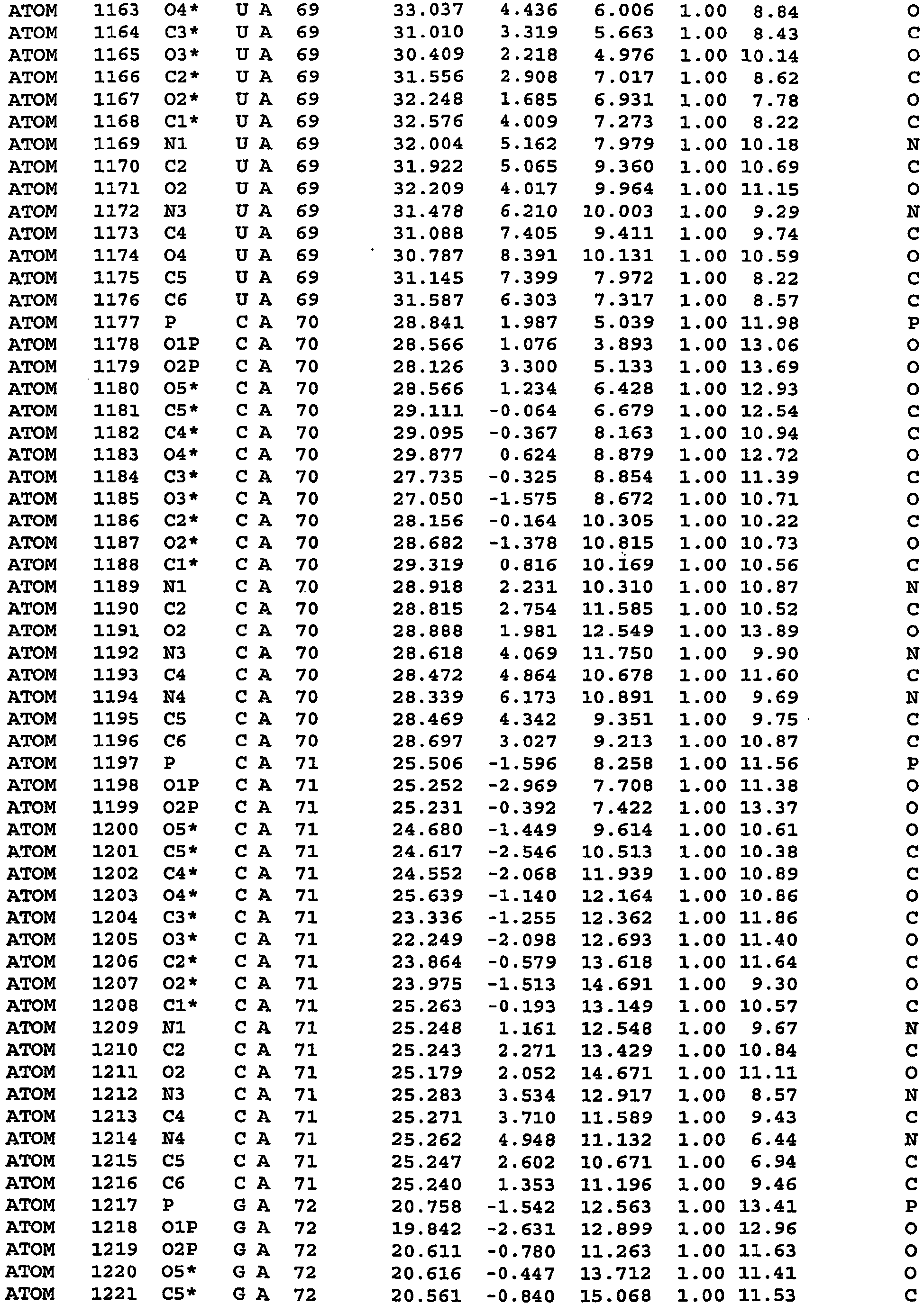

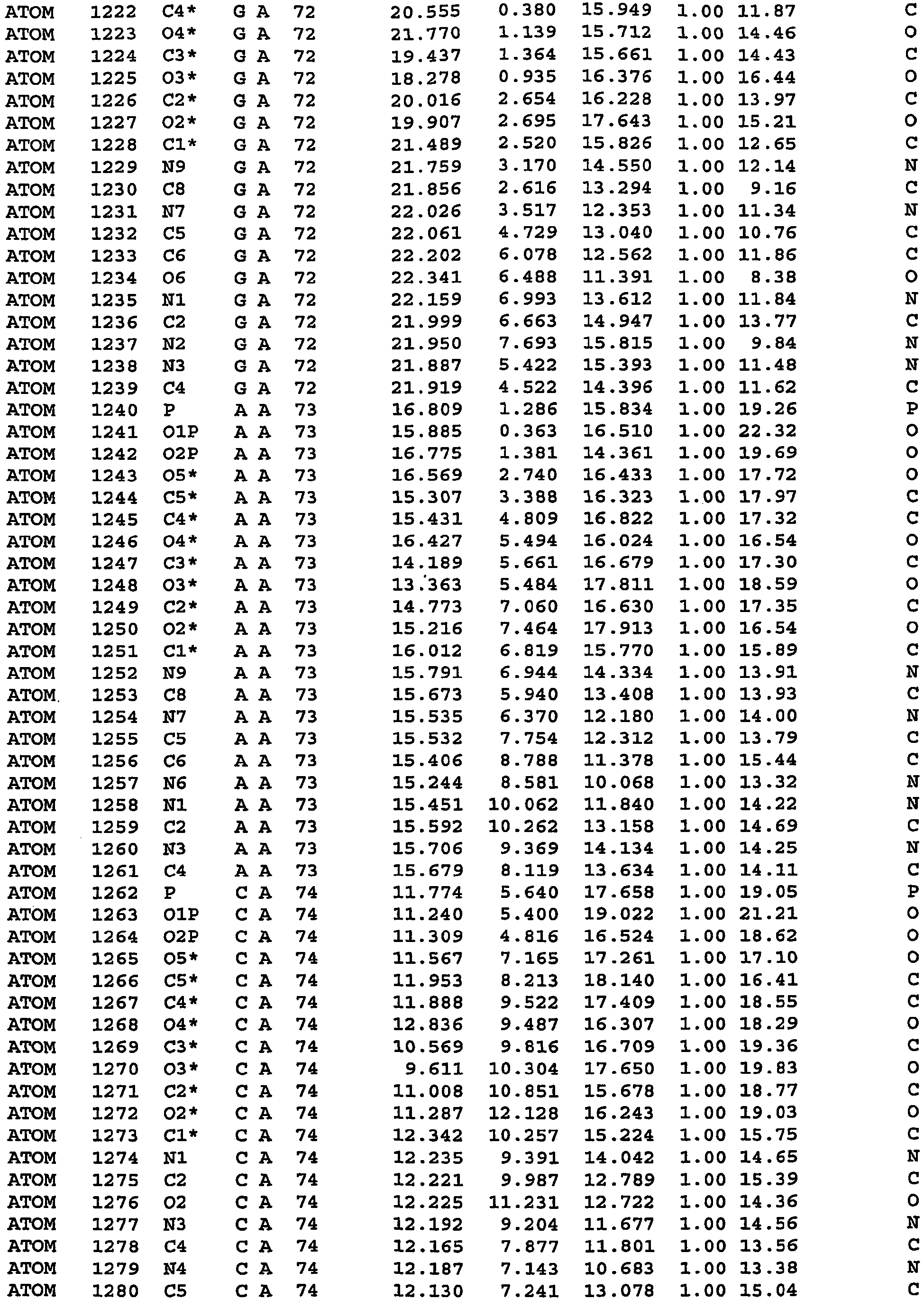

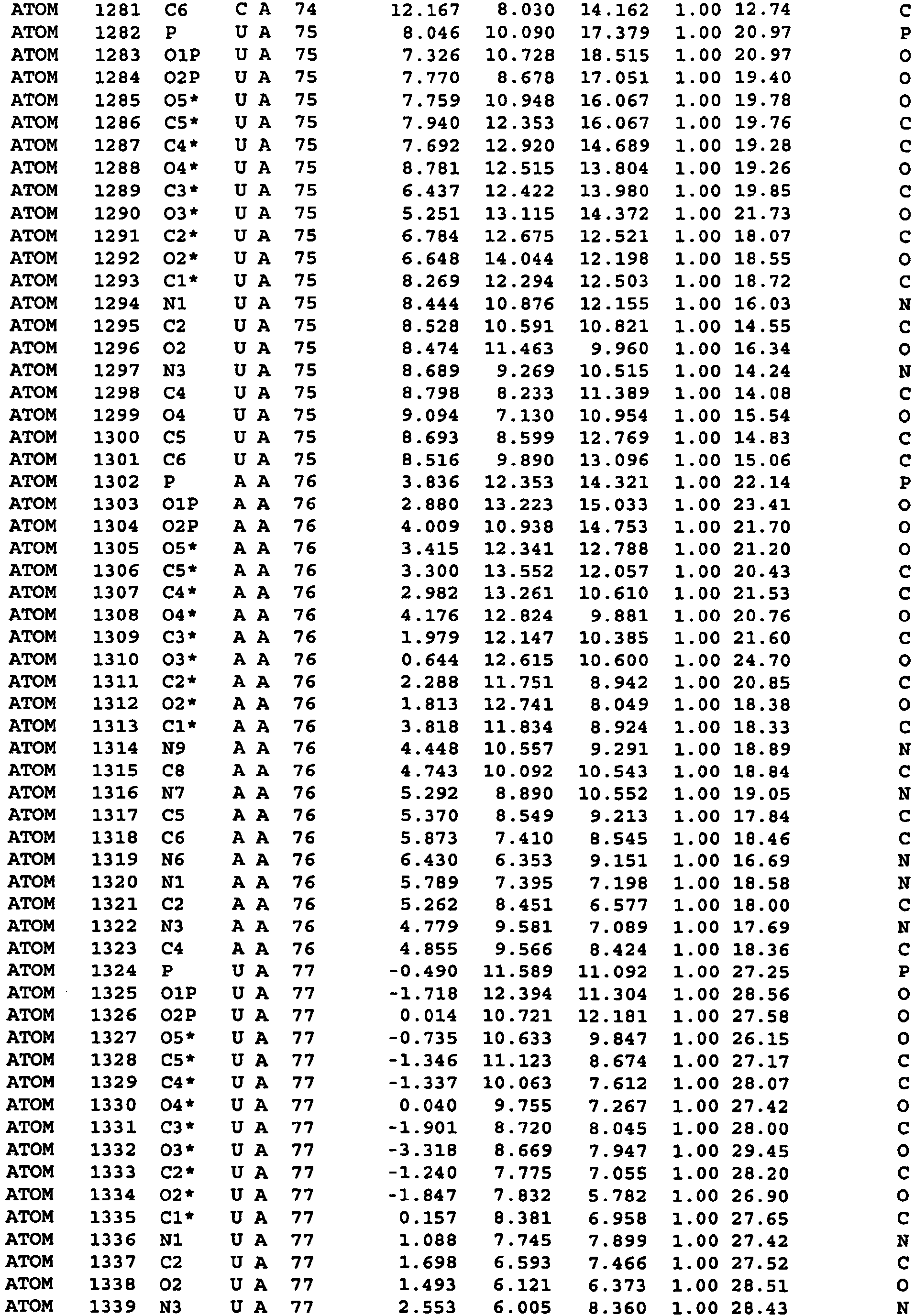

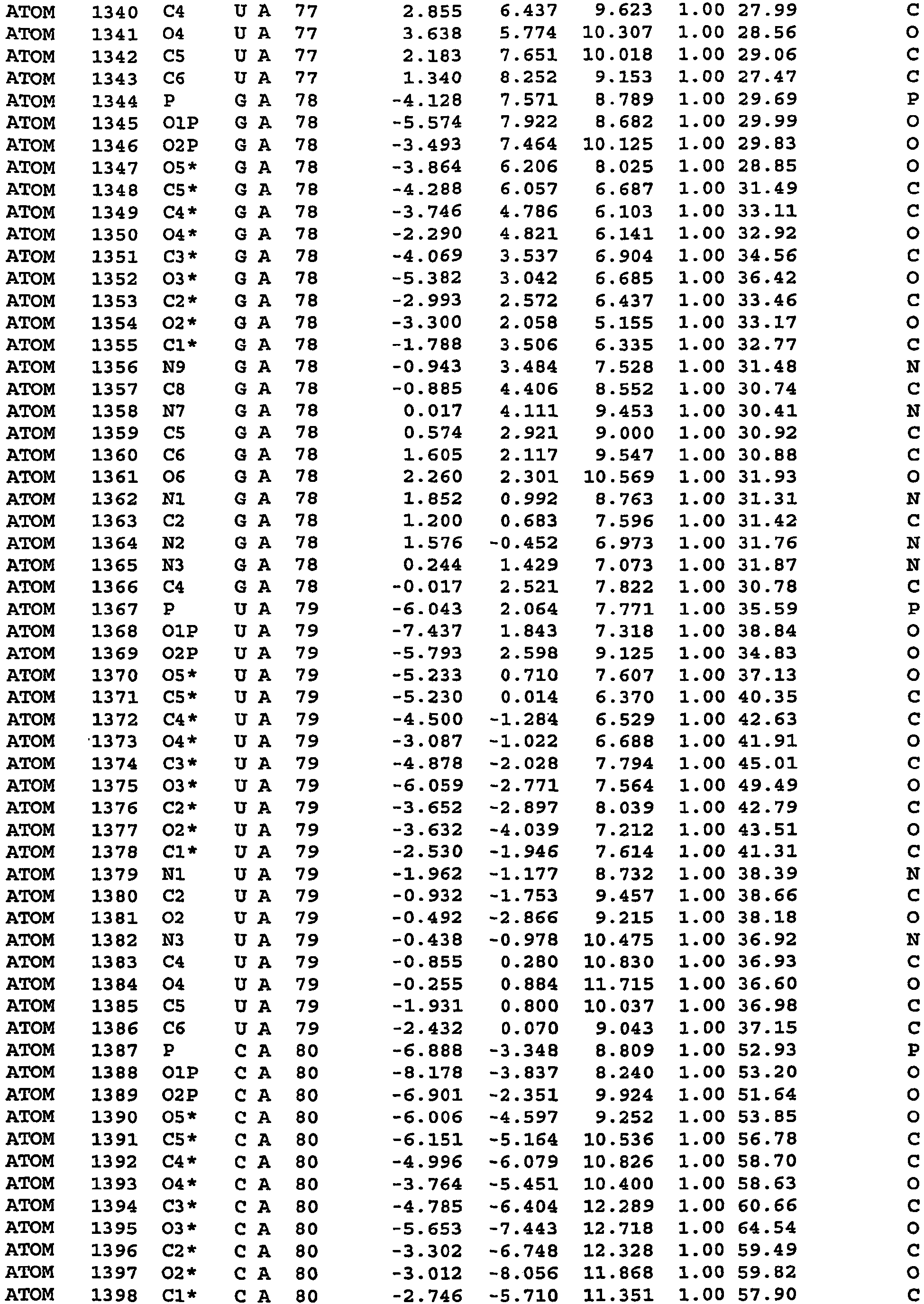

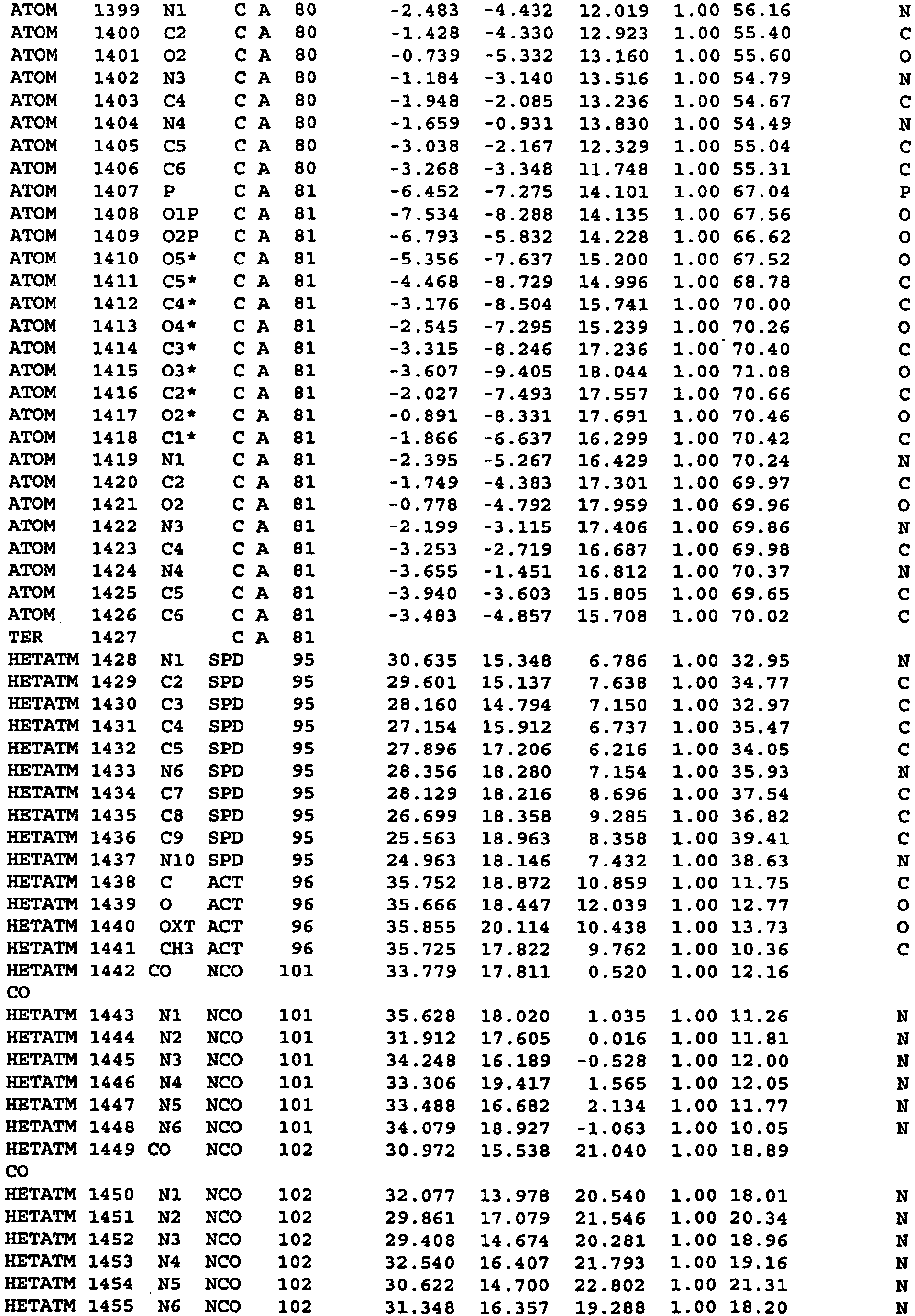

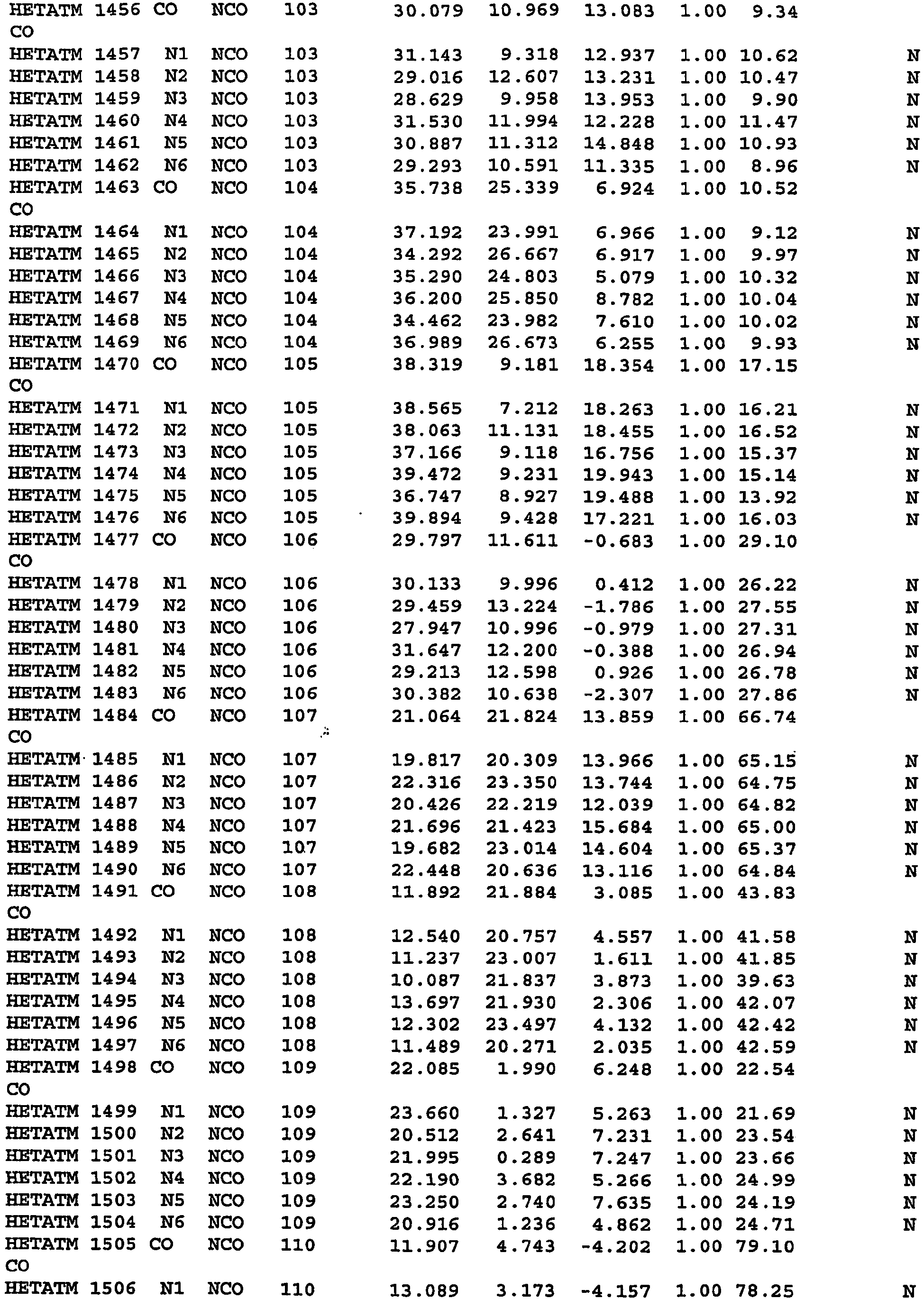

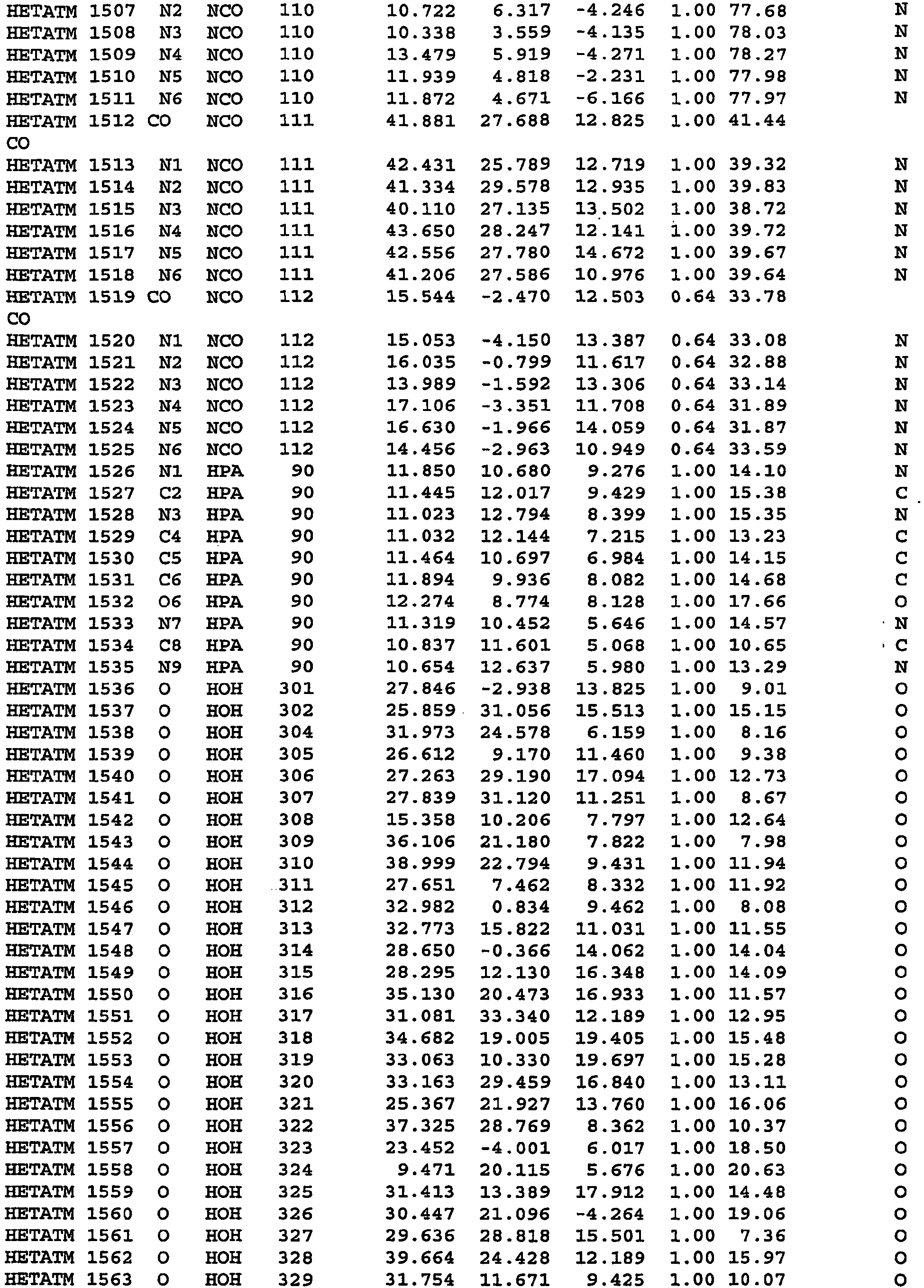

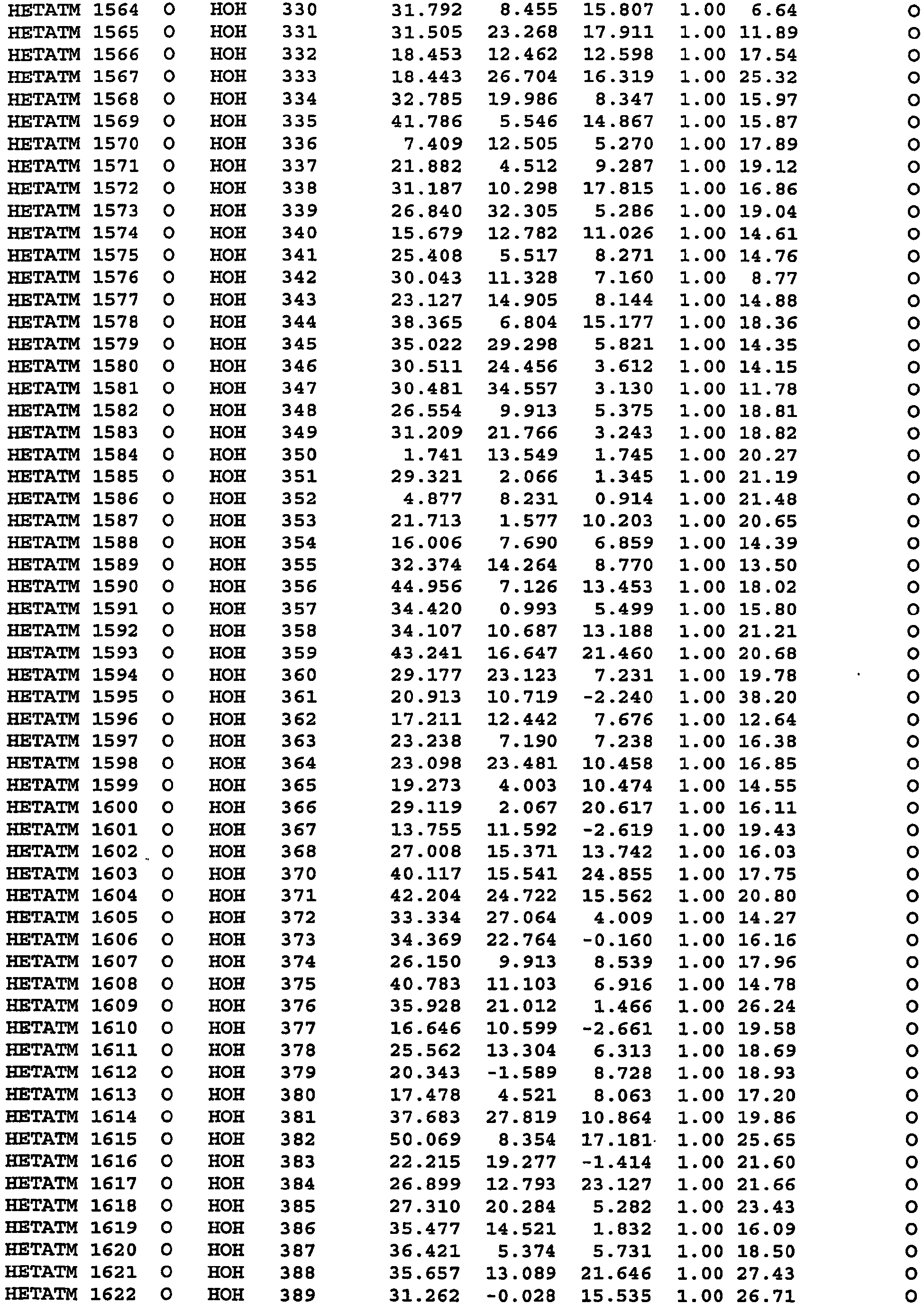

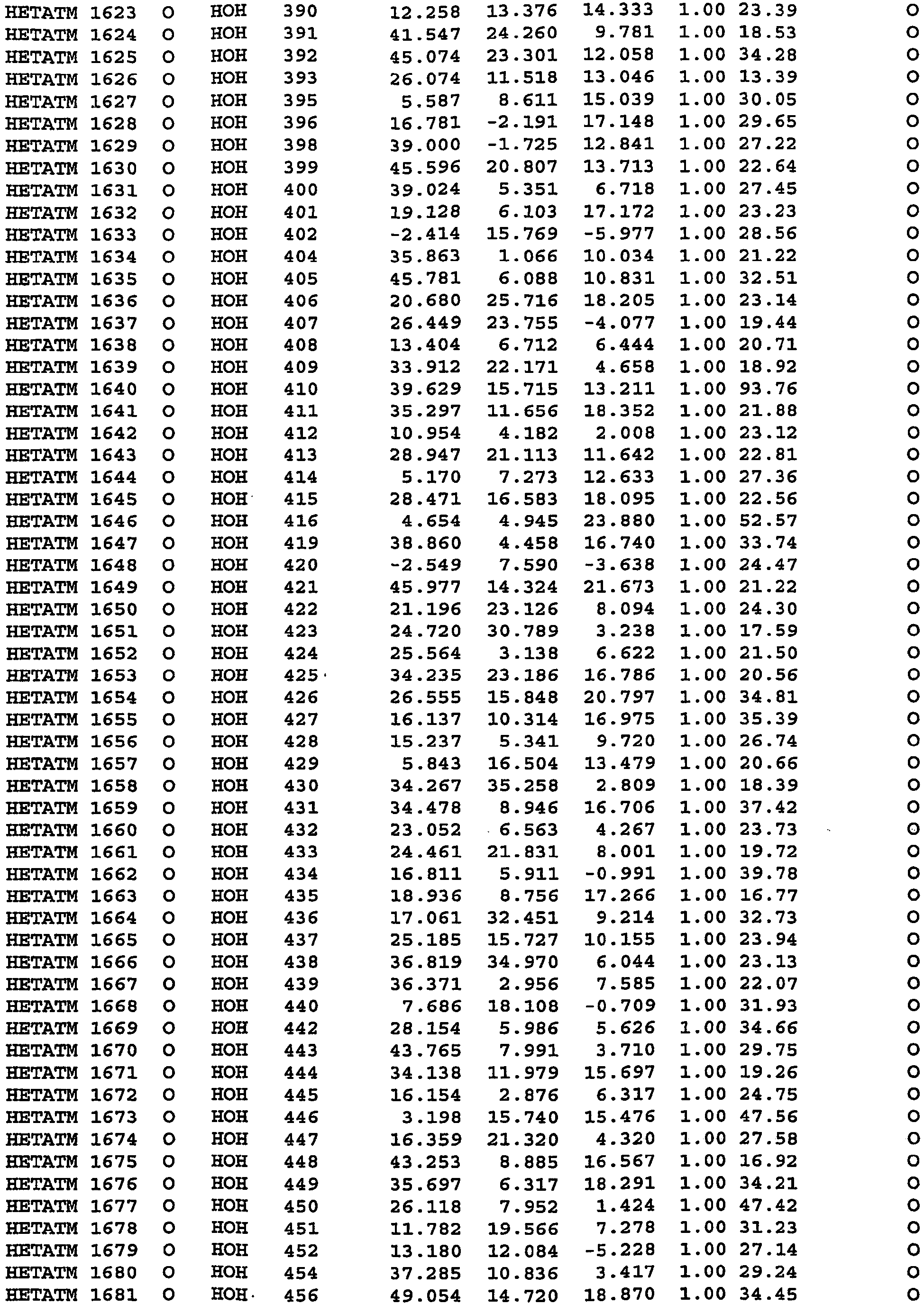

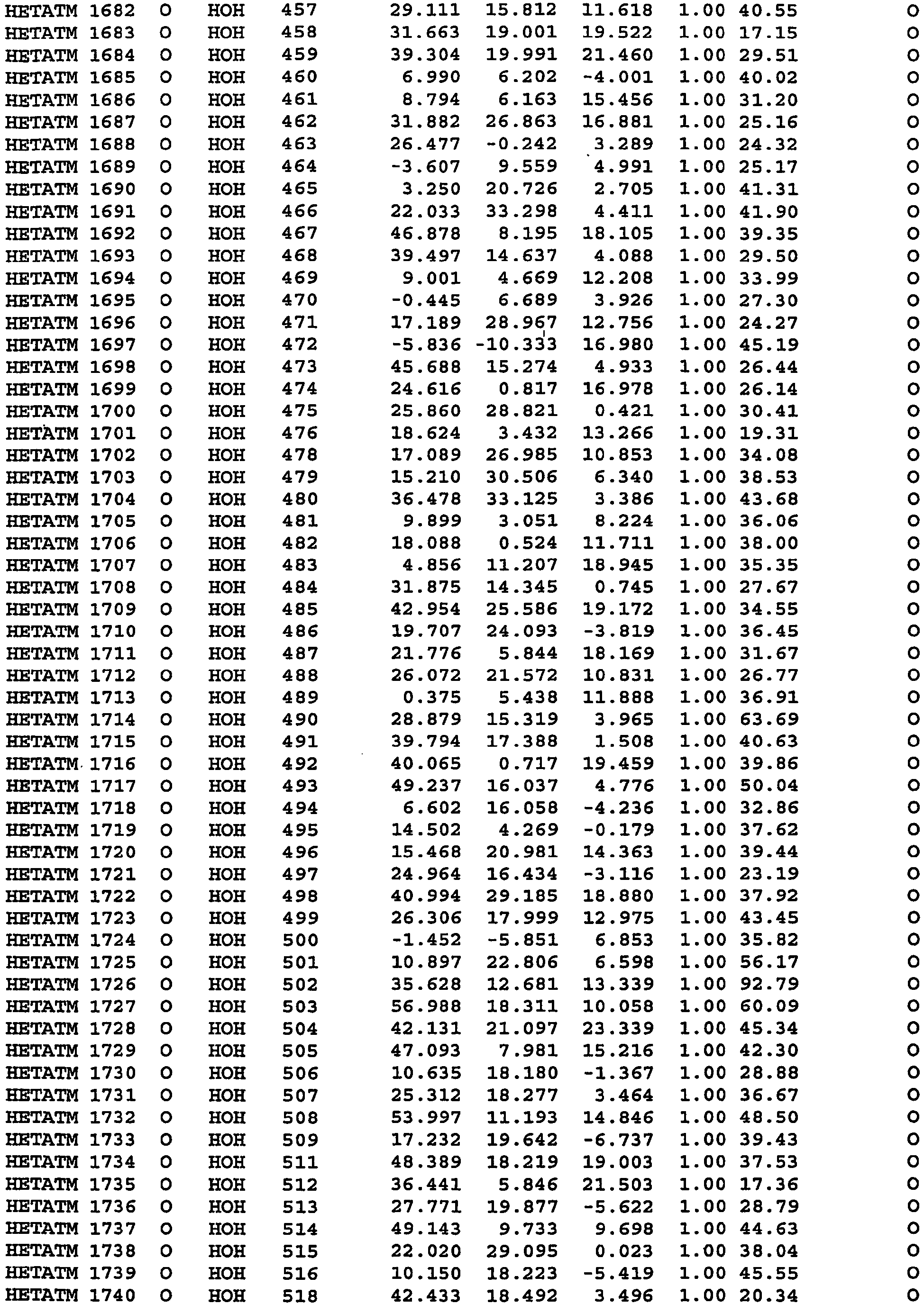

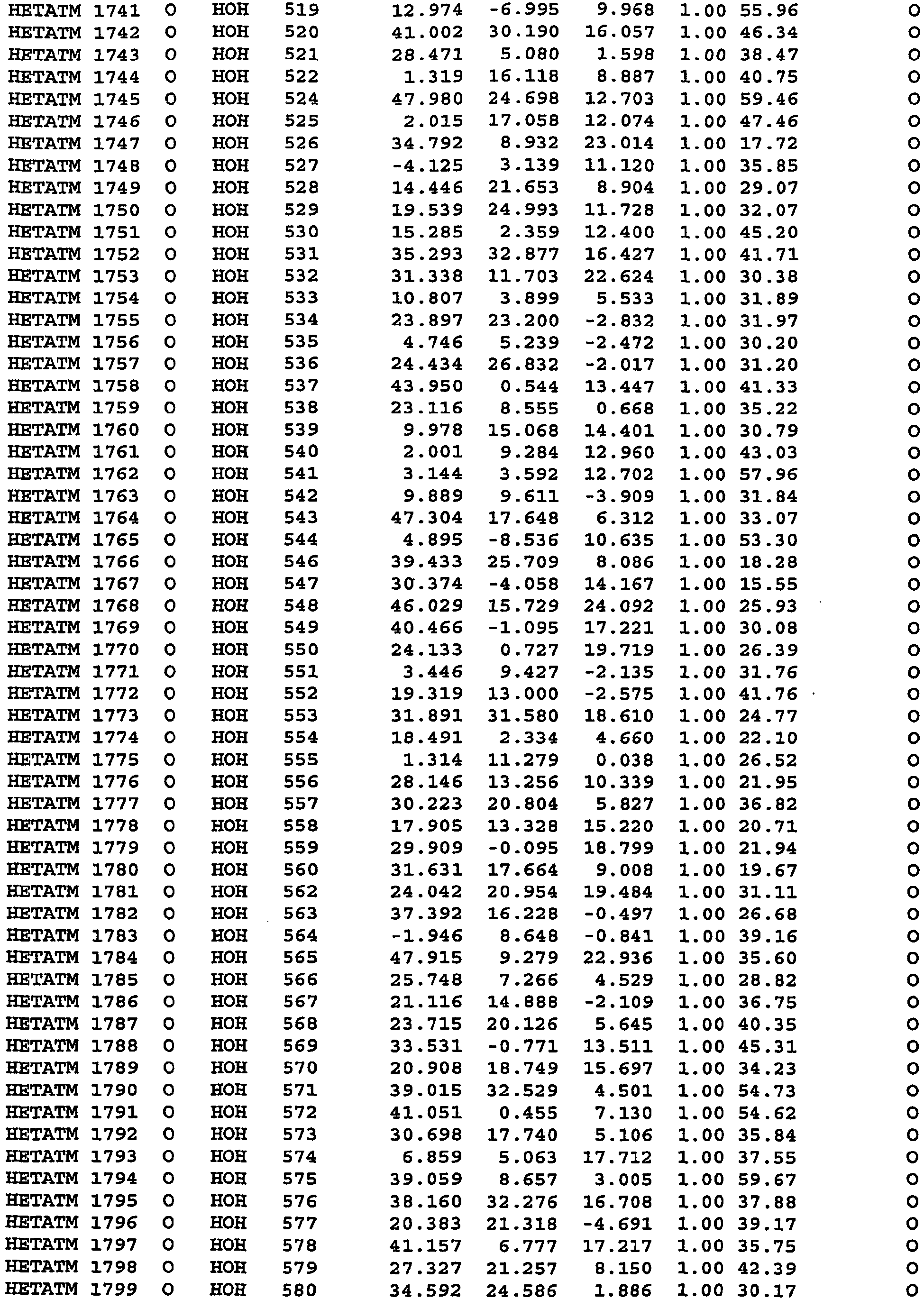

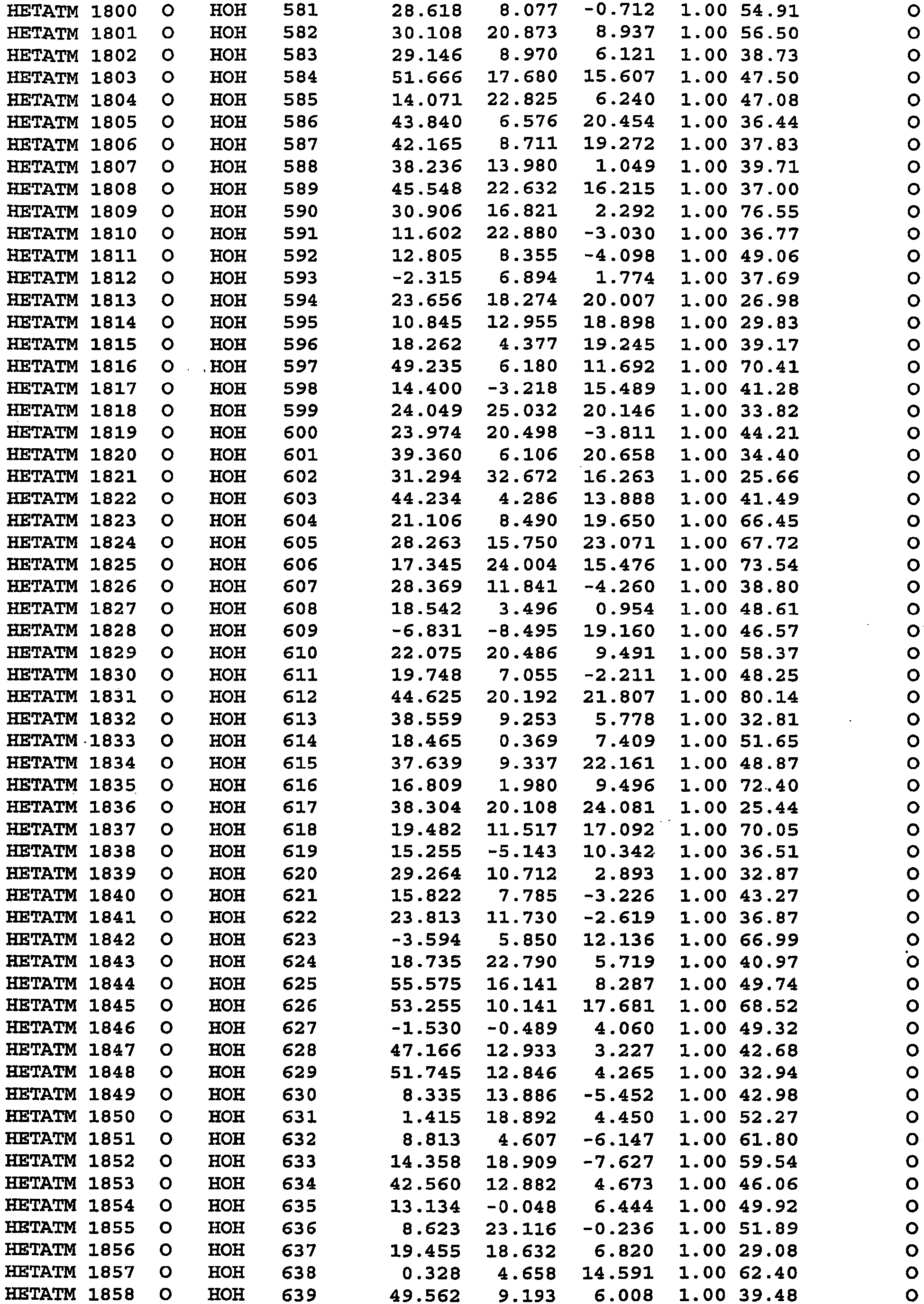

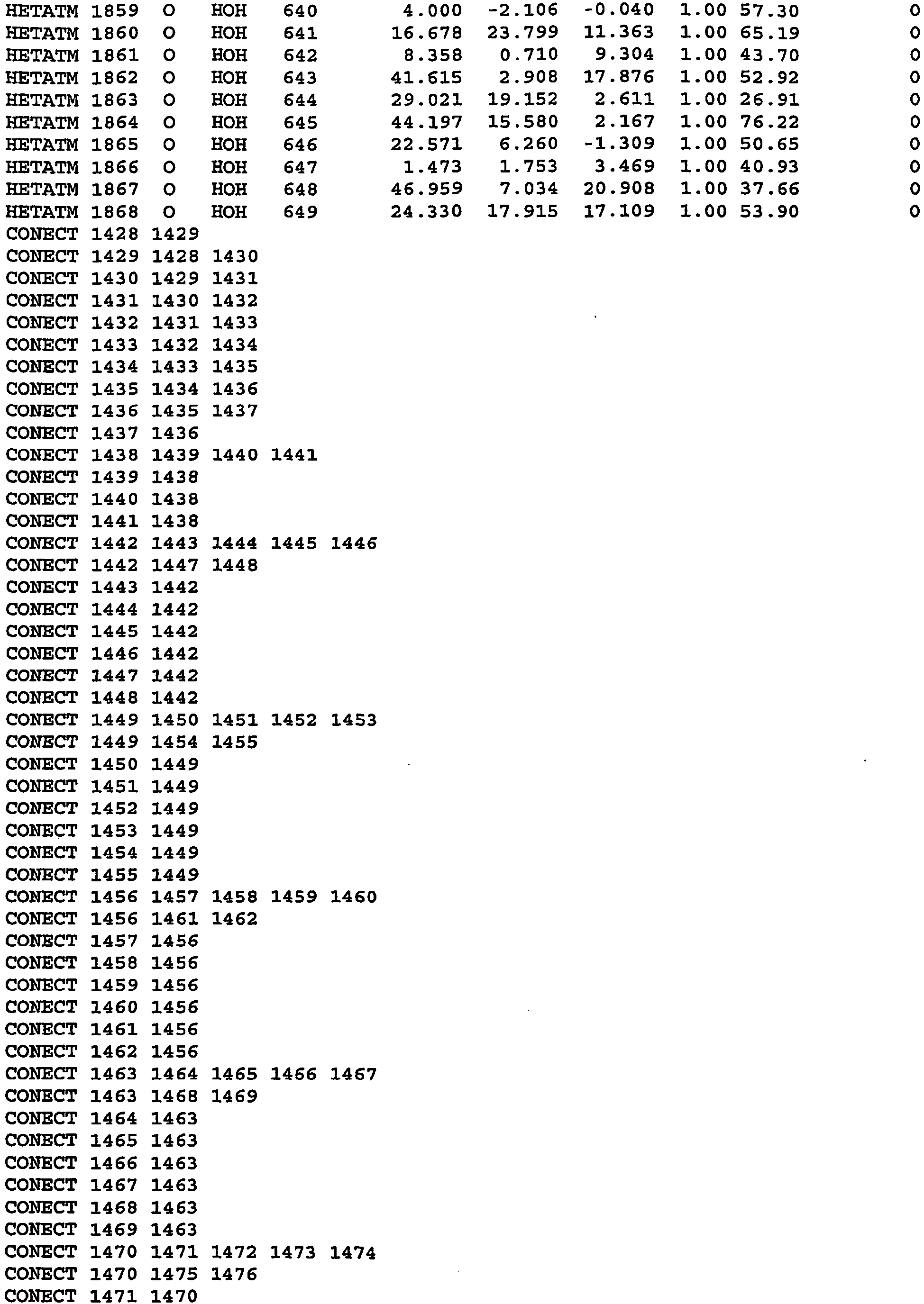

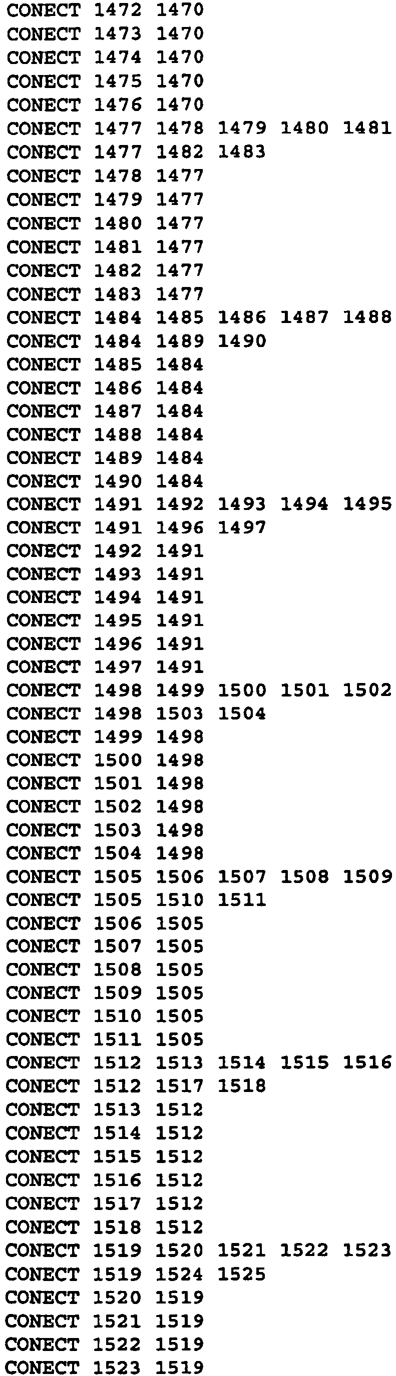

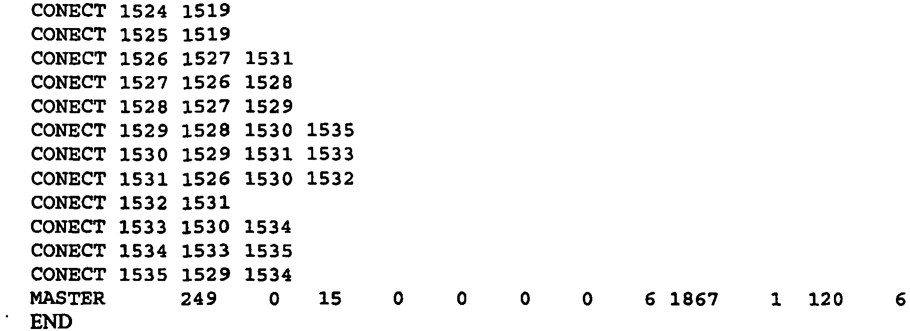

**Table 7. Thermodynamic parameters associated with various nucleobases bound to the adenine riboswitch RNA (AR)**

| # of exp. | Analog | K_{d} (µM) | ΔH (kcal/mol) | ΔS (Cal/mol●deg) |
|---|---|---|---|---|
| 5 | 2,6-Diaminopurine* | 0.017 ± 0.006 | -40.3 ± 2.91 | -97.6 ± 4.00 |
| 2 | 2-Aminopurine | 0.251 ± 0.026 | -31.8 ± 2.64 | -73.9 ± 9.26 |
| 2 | Adenine | 0.469 ± 0.041 | -34.5 ± 0.05 | -84.8 ± 0.30 |
| 2 | Purine | 110 ± 3 | -25.4 ± 0.99 | -65.4 ± 3.00 |
| 2 | 2-Fluoroadenine | 4.02 ± 1.07 | -37.4 ± 3.41 | -98.7 ± 11.8 |
| 2 | 2,4,5,6-Tetraaminopyrimidine | 24.9 ± 4.25 | -43.2 ± 3.50 | -122 ± 12.0 |
| 2 | 2,4,6-Triaminopyrimidine | 18.3 ± 1.67 | -37.7 ± 0.23 | -102 ± 0.70 |
| 1 | 2,4-Diaminopyrimidine | n.d. | | |
| 1 | Histamine | n.d. | | |
| 1 | L-Histidinol | n.d. | | |
| 2 | 6-Bromoguanine | 2.7 ± 1.16 | -50.4 ± 0.16 | -141 ± 1.90 |
| 2 | 6-Chloroguanine | 0.721 ± 0.044 | -44.3 ± 7.2 | -98.6 ± 4.39 |
| 1 | 2,6-Dichloropurine | n.d. | | |
| 4 | O-Methylguanine | n.d. | | |
| 1 | Kinetin | n.d. | | |
| 1 | 6-n-Hexylaminopurine | n.d. | | |
| 2 | 6-Benzylaminopurine | n.d. | | |
| 2 | 6-Methylpurine | 25.2 ± 1.63 | -31.5 ± 0.42 | -82.9 ± 1.25 |
| 2 | O-Benzylguanine | n.d. | | |

| | | | | |
|---|---|---|---|---|
| All experiments were done in 50mM K⁺ PES, pH 7.5, 100mM KCl, 10mM MgCl₂ at 30°C. n.d. designates reactions where no detectable binding was indicated. * The heat capacity (ΔCp) for this reaction is -1.10 kcal mol⁻¹ K⁻¹, as measured between 298 and 323 K. | | | | |

**Table 8. Thermodynamic parameters associated with various nucleobases bound to the guanine riboswitch RNA (GR)**

| # of exp | Analog | Kd (µM) | ΔH (kcal/mol) | ΔS (cal/mol●deg) |
|---|---|---|---|---|
| 2 | Guanine | 0.004 ± 0.003 | -53.5 ± 6.98 | -137 ± 24.8 |
| 2 | Hypoxanthine | 0.759 ± 0.066 | -31.3 ± 0.01 | -75.3 ± 0.145 |
| 2 | Xanthine | 50.5 ± 5.8* | -34.6 ± 10.4* | -94.4 ± 34.5* |
| 2 | 7-Deazaguanine | 0.049 ± 0.013 | -42.0 ± 3.72 | -105 + 11.7 |
| 2 | 8-Azaguanine | 0.0487 ± 0.1 | -46.9 ± 9.87 | -121 ± 32.6 |
| 1 | 4,6-Dihyroxypyrimidine | n.d | | |
| 1 | 5,6Diamino2,4Dihydroxyupyrimidine | n.d. | | |
| 2 | 6-Bromoguanine | 2.12 ± 0.56 | -39.0 ± 7.79 | -103 ± 26.2 |
| 2 | 6-Chloroguanine | 0.856 ± 0.014 | -43.6 ± 13.1 | -116 ± 43.2 |
| 1 | 2,6-Dichloropurine | n.d. | | |
| 1 | O-Methylguanine | 0.003 | -18.7 | -23.3 |
| 2 | 2-Aminopurine | 4.36 ± 1.2 | -46.4 ± 2.66 | -129 ± 8.20 |
| 1 | O-Benzylguanine | n.d. | | |
| 2 | 6-Thioguanine | 0.0737 ± 0.01 | -40.6 ± 14.5 | -101 ± 47.5 |
| 2 | 6-Methylthioguanine | 0.0418 ± 0.003 | -24.5 ± 0.20 | -46.9 ± 0.24 |

| | | | | |
|---|---|---|---|---|
| All experiments were done in 50mM K⁺HEPES, 100mM KCI, 10mM MgCl₂ at 30°C. n.d. designates reactions where no detectable binding was indicated. *Software was not able to fit data and report parameters unless n was held at 1. | | | | |

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

It must be noted that as used herein and in the appended claims, the singular forms "a", "an", and "the" include plural reference unless the context clearly dictates otherwise. Thus, for example, reference to "a riboswitch" includes a plurality of such riboswitches, reference to "the riboswitch" is a reference to one or more riboswitches and equivalents thereof known to those skilled in the art, and so forth.

"Optional" or "optionally" means that the subsequently described event, circumstance, or material may or may not occur or be present, and that the description includes instances where the event, circumstance, or material occurs or is present and instances where it does not occur or is not present.

Ranges may be expressed herein as from "about" one particular value, and/or to "about" another particular value. When such a range is expressed, also specifically contemplated and considered disclosed is the range from the one particular value and/or to the other particular value unless the context specifically indicates otherwise. Similarly, when values are expressed as approximations, by use of the antecedent "about," it will be understood that the particular value forms another, specifically contemplated embodiment that should be considered disclosed unless the context specifically indicates otherwise. It will be further understood that the endpoints of each of the ranges are significant both in relation to the other endpoint, and independently of the other endpoint unless the context specifically indicates otherwise. Finally, it should be understood that all of the individual values and sub-ranges of values contained within an explicitly disclosed range are also specifically contemplated and should be considered disclosed unless the context specifically indicates otherwise. The foregoing applies regardless of whether in particular cases some or all of these embodiments are explicitly disclosed.

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described. Nothing herein is to be construed as an admission that the present invention is not entitled to antedate such disclosure by virtue of prior invention. No admission is made that any reference constitutes prior art. The discussion of references states what their authors assert, and applicants reserve the right to challenge the accuracy and pertinency of the cited documents. It will be clearly understood that, although a number of publications are referred to herein, such reference does not constitute an admission that any of these documents forms part of the common general knowledge in the art.

Throughout the description and claims of this specification, the word "comprise" and variations of the word, such as "comprising" and "comprises," means "including but not limited to," and is not intended to exclude, for example, other additives, components, integers or steps.

Those skilled in the art will recognize, or be able to ascertain using no more than routine experimentation, many equivalents to the specific embodiments of the method and compositions described herein. Such equivalents are intended to be encompassed by the following claims.

## Claims

1. Crystalline natural guanine-responsive riboswitch having the atomic coordinates listed in Table 6.

2. A method of identifying a compound that interacts with the guanine-responsive riboswitch of claim 1 comprising:
(a) modeling the atomic structure of claim 1 with a test compound; and
(b) determining if the test compound interacts with the riboswitch.

3. The method of claim 2, wherein determining if the test compound interacts with the riboswitch comprises determining a predicted minimum interaction energy, a predicted bind constant, a predicted dissociation constant, or a combination, for the test compound in the model of the riboswitch.

4. The method of claim 2, wherein determining if the test compound interacts with the riboswitch comprises determining one or more predicted bonds, one or more predicted interactions, or a combination, of the test compound with the model of the riboswitch.

5. The method of claim 2, wherein atomic contacts are determined in step (b), thereby determining the interaction of the test compound with the riboswitch.

6. The method of claim 5, further comprising the steps of:
(c) identifying analogs of the test compound;
(d) determining if the analogs of the test compound interact with the riboswitch.

7. The method of claim 6, wherein the compound is hypoxanthine.

8. The method of claim 2, wherein a gel-based assay is used to determine if the test compound interacts with the riboswitch.

9. The method of claim 2, wherein a chip-based assay is used to determine if the test compound interacts with the riboswitch.

10. The method of claim 2, wherein the test compound interacts via van der Waals interactions, hydrogen bonds, electrostatic interactions, hydrophobic interactions, or a combination.

11. The method of claim 2, wherein the riboswitch comprises an RNA cleaving ribozyme.

12. The method of claim 2, wherein a fluorescent signal is generated when a nucleic acid comprising a quenching moiety is cleaved.

13. The method of claim 12, wherein molecular beacon technology is employed to generate the fluorescent signal.

14. The method of claim 2, wherein the method is carried out using a high throughput screen.

## Patentansprüche

1. Kristalliner natürlicher, auf Guanin ansprechender Riboswitch mit den in Tabelle 6 aufgeführten Atomkoordinaten.

2. Verfahren zur Erkennung einer Verbindung, die mit dem auf Guanin ansprechenden Riboswitch gemäß Anspruch 1 zusammenwirkt, mit den Schritten:
(a) Nachbilden der Atomstruktur gemäß Anspruch 1 mit einer Testverbindung; und
(b) Bestimmen, ob die Testverbindung mit dem Riboswitch zusammenwirkt.

3. Verfahren gemäß Anspruch 2, wobei die Bestimmung, ob die Testverbindung mit dem Riboswitch zusammenwirkt, das Bestimmen einer prognostizierten minimalen Wechselwirkungsenergie, einer prognostizierten Bindungskonstante, einer prognostizierten Spaltungskonstante oder einer Kombination für die Testverbindung in dem Modell des Riboswitch umfasst.

4. Verfahren gemäß Anspruch 2, wobei die Bestimmung, ob die Testverbindung mit dem Riboswitch zusammenwirkt, das Bestimmen einer oder mehr prognostizierten Bindungen, einer oder mehr prognostizierten Wechselwirkungen oder einer Kombination der Testverbindung mit dem Modell des Riboswitch umfasst.

5. Verfahren gemäß Anspruch 2, wobei atomare Verbindungen in Schritt (b) bestimmt werden, wodurch die Wechselwirkung der Testverbindung mit dem Riboswitch bestimmt wird.

6. Verfahren gemäß Anspruch 5, weiterhin mit folgenden Schritten:
(c) Erkennen von Analogen der Testverbindung;
(d) Bestimmen, ob die Analoge der Testverbindung mit dem Riboswitch zusammenwirken.

7. Verfahren gemäß Anspruch 6, wobei die Verbindung aus Hypoxanthin besteht.

8. Verfahren gemäß Anspruch 2, wobei eine Analyse auf Gelbasis eingesetzt wird, um zu bestimmen, ob die Testverbindung mit dem Riboswitch zusammenwirkt.

9. Verfahren gemäß Anspruch 2, wobei eine Analyse auf Chipbasis eingesetzt wird, um zu bestimmen, ob die Testverbindung mit dem Riboswitch zusammenwirkt.

10. Verfahren gemäß Anspruch 2, wobei die Testverbindung über van der Waals Wechselwirkungen, Wasserstoffbindungen, elektrostatische Wechselwirkungen, hydrophobe Wechselwirkungen oder eine Kombination zusammenwirkt.

11. Verfahren gemäß Anspruch 2, wobei der Riboswitch ein RNA spaltendes Ribozym enthält.

12. Verfahren gemäß Anspruch 2, wobei ein fluoreszierendes Signal erzeugt wird, wenn eine Nukleinsäure mit einem Quenching-Anteil abgespalten wird.

13. Verfahren gemäß Anspruch 12, wobei Molecular Beacon Technologie zur Erzeugung des fluoreszierenden Signals eingesetzt wird.

14. Verfahren gemäß Anspruch 2, wobei das Verfahren unter Verwendung eines Hochdurchsatzfilters durchgeführt wird.

## Revendications

1. Riborégulateur réagissant à la guanine naturel cristallin présentant les coordonnées atomiques énumérées dans le tableau 6.

2. Procédé d'identification d'un composé qui interagit avec le riborégulateur réagissant à la guanine selon la revendication 1 comprenant :
(a) la modélisation de la structure atomique selon la revendication 1 avec un composé à tester ; et
(b) la détermination si le composé à tester interagit avec le riborégulateur.

3. Procédé selon la revendication 2, dans lequel la détermination si le composé à tester interagit avec le riborégulateur comprend la détermination d'une énergie d'interaction minimale prédite, d'une constante de liaison prédite, d'une constante de dissociation prédite, ou d'une combinaison, pour le composé à tester dans le modèle du riborégulateur.

4. Procédé selon la revendication 2, dans lequel la détermination si le composé à tester interagit avec le riborégulateur comprend la détermination d'une ou de plusieurs liaisons prédites, d'une ou de plusieurs interactions prédites, ou d'une combinaison, du composé à tester avec le modèle du riborégulateur.

5. Procédé selon la revendication 2, dans lequel les contacts atomiques sont déterminés dans l'étape (b), déterminant de cette manière l'interaction du composé à tester avec le riborégulateur.

6. Procédé selon la revendication 5, comprenant en outre les étapes suivantes :
(c) l'identification d'analogues du composé à tester ;
(d) la détermination si les analogues du composé à tester interagissent avec le riborégulateur.

7. Procédé selon la revendication 6, dans lequel le composé est l'hypoxanthine.

8. Procédé selon la revendication 2, dans lequel un test à base de gel est utilisé pour déterminer si le composé à tester interagit avec le riborégulateur.

9. Procédé selon la revendication 2, dans lequel un test à base de puce est utilisé pour déterminer si le composé à tester interagit avec le riborégulateur.

10. Procédé selon la revendication 2, dans lequel le composé à tester interagit par l'intermédiaire d'interactions de van der Waals, de liaisons hydrogène, d'interactions électrostatiques, d'interactions hydrophobes, ou d'une combinaison.

11. Procédé selon la revendication 2, dans lequel le riborégulateur comprend un ribozyme clivant l'ARN.

12. Procédé selon la revendication 2, dans lequel un signal fluorescent est généré lorsqu'un acide nucléique comprenant un radical d'extinction est clivé.

13. Procédé selon la revendication 12, dans lequel la technologie des balises moléculaires est employée pour générer le signal fluorescent.

14. Procédé selon la revendication 2, où le procédé est réalisé en utilisant un criblage à haut débit.
